# EUROPEAN PATENT SPECIFICATION

(11) **EP 1 747 465 B1**
(45) Date of publication and mention of the grant of the patent: **23.03.2011**
(21) Application number: 05731863.6
(22) Date of filing: 21.04.2005
(51) Int. Cl.: G01N 33/574

(54) **USE OF NOVEL BIOMARKERS FOR DETECTION OF TESTICULAR CARCINOMA IN SITU AND DERIVED CANCERS IN HUMAN SAMPLES**
VERWENDUNG NEUARTIGER BIOMARKER ZUM NACHWEIS VON HODENKREBS IN SITU UND ABGELEITETEN KREBSERKRANKUNGEN IN MENSCHLICHEN PROBEN
UTILISATION DE NOUVEAUX BIOMARQUEURS DE DETECTION D'UN CARCINOME TESTICULAIRE IN SITU ET DE CANCERS DERIVES DANS DES PRELEVEMENTS HUMAINS

(30) Priority: 23.04.2004 DK 200400647; 30.12.2004 DK 200402032
(43) Date of publication of application: 31.01.2007
(60) Divisional application: 08172455.1
(73) Proprietor: Rigshospitalet, Copenhagen University Hospital, 2100 Copenhagen Ø (DK); University of Copenhagen, 2200 Copenhagen N (DK); European Molecular Biology Laboratories Heidelberg, 69117 Heidelberg (DE)
(72) Inventor: ALMSTRUP, Kristian, Vindinge DK-4000 Roskilde (DK); HØI-HANSEN, Christina, E., DK-2830 Virum (DK); RAJPERT-DE MEYTS, Ewa, DK-2670 Greve (DK); WIRKNER, Ute, D-74925 Epfenbach (DE); ANSORGE, Wilhelm, 1278 La Rippe (CH); LEFFERS, Henrik, DK-2200 Copenhagen N (DK); SKAKKEBÆK, Niels, Erik, DK-3520 Farum (DK); SCHWAGER, Christian, 67117 Limburgerhof (DE); BLAKE, Jonathon, 68723 Oftersheim (DE)
(74) Representative: Plougmann & Vingtoft A/S
(86) International application number: PCT/DK2005/000280
(87) International publication number: WO 2005/103703

(56) References cited:
- WO-A-03/051308
- WO-A-2004/031410
- WO-A-2004/085654
- LOOIJENGA LEENDERT H J ET AL: "POU5F1 (OCT3/4) identifies cells with pluripotent potential in human germ cell tumors." CANCER RESEARCH, vol. 63, no. 9, 1 May 2003 (2003-05-01), pages 2244-2250, XP002343970 ISSN: 0008-5472
- HOEI-HANSEN C E ET AL: "A subfertile patient diagnosed with testicular carcinoma in situ by immunocytological staining for AP-2gamma in semen samples: Case report" HUMAN REPRODUCTION (OXFORD), vol. 20, no. 3, March 2005 (2005-03), pages 579-582, XP002365149 ISSN: 0268-1161
- HOEI-HANSEN CHRISTINA E ET AL: "Transcription factor AP-2gamma is a developmentally regulated marker of testicular carcinoma in situ and germ cell tumors." CLINICAL CANCER RESEARCH : AN OFFICIAL JOURNAL OF THE AMERICAN ASSOCIATION FOR CANCER RESEARCH. 15 DEC 2004, vol. 10, no. 24, 15 December 2004 (2004-12-15), pages 8521-8530, XP002365150 ISSN: 1078-0432
- HOEI-HANSEN C E ET AL: "New evidence for the origin of intracranial germ cell tumours from primordial germ cells: expression of pluripotency and cell differentiation markers." THE JOURNAL OF PATHOLOGY MAY 2006, vol. 209, no. 1, May 2006 (2006-05), pages 25-33, ISSN: 0022-3417
- SCHNEIDER D T ET AL: "Multipoint imprinting analysis indicates a common precursor cell for gonadal and nongonadal pediatric germ cell tumors." CANCER RESEARCH 1 OCT 2001, vol. 61, no. 19, 1 October 2001 (2001-10-01), pages 7268-7276, ISSN: 0008-5472
- PAULS KATHARINA ET AL: "Transcription factor AP-2gamma, a novel marker of gonocytes and seminomatous germ cell tumors." INTERNATIONAL JOURNAL OF CANCER. JOURNAL INTERNATIONAL DU CANCER 20 JUN 2005, vol. 115, no. 3, 20 June 2005 (2005-06-20) , pages 470-477, ISSN: 0020-7136
- NIELSEN C T ET AL: "Onset of the release of spermatozoa (spermarche) in boys in relation to age, testicular growth, pubic hair, and height." THE JOURNAL OF CLINICAL ENDOCRINOLOGY AND METABOLISM MAR 1986, vol. 62, no. 3, March 1986 (1986-03), pages 532-535, ISSN: 0021-972X

## Description

### Field of the Invention

The present invention provides a biomarker that characterizes testicular carcinoma in situ (CIS), which is the precursor for classical testicular seminoma and non-seminomas. The same biomarker may also be used to characterize gonadoblastoma, a CIS-like pre-cancerous lesion found in dysgenetic gonads. The biomarker is AP-2γ.

### Background of the invention

Testicular carcinoma in situ (CIS) is the common precursor of nearly all testicular germ cell tumors (TGCTs) that occur in young adults. The incidence of TGCTs has increased markedly over the past several decades, and these tumors are now the most common type of cancer in young men.

CIS cells may transform into either a seminoma or a non-seminoma. The seminoma retains a germ-cell-like phenotype, whereas the tumour known as non-seminoma or teratoma retains embryonic stem cell features; pluripotency and the ability to differentiate into virtually all somatic tissues. Non-seminomas comprise embryonal carcinoma (EC), various mixtures of differentiated teratomatous tissue components and extraembryonic tissues, such as yolk sac tumour and choriocarcinoma. In addition to CIS, gonadoblastoma, a CIS-like lesion occurs in dysgenetic testes and intersex gonads, which frequently may contain some ovarian structures.

The only difference between CIS and gonadoblastoma concerns the overall architecture of the lesion in the surrounding gonad (CIS is found usually in single rows along the basement membrane and there is a single layer of Sertoli cells between CIS cells and the lumen of seminiferous tubules, while gonadoblastoma contains nests (clumps) of CIS-like cells surrounded by small somatic granulosa-like cells and sometimes spermatogonia-like cells). The morphology and gene expression patterns of CIS cells and gonadoblastoma cells are indistinguishable (Jørgensen et al. Histopathology 1997), therefore, further in this application, the present inventors use the term CIS for both precursor lesions.

The most commonly used marker for CIS in clinical practice is placental-like alkaline phosphatase (ALPP or PLAP) (Manivel, JC, et al. Am J Surg Pathol 1987, 11:21-9), which is also a known marker of primordial germ cells. Epidemiological evidence and evidence based on immunohistochemical comparative studies of proteins expressed in CIS and fetal germ cells indicate that CIS is an inborn lesion, probably arising from gonocytes in early fetal life and progress to an overt TGCT after puberty. The precise nature of the molecular events underlying the initiation of malignant transformation from the gonocyte to the CIS cell has not yet been elucidated, and the following progression into overt tumors remains largely unknown.

Looijenga et al. 2003, POU5F1 (OCT3/4) Identifies Cells with Pluripotent Potential in Human Germ Cell Tumors, Cancer Research 63, p. 2244-2255, discloses a study pertaining to the use of POU5F1 (OCT3/4) as a marker for human germ cell tumors. The study analyses the presence of POU5F1 in human germ cell tumors and other tumor types using immunohistochemistry. The study verifies that POU5F1 expression is specific for particular subtypes of human germ cell tumors of adults.

In order to identify new markers for early detection of CIS, the present inventors analyzed gene expression in CIS cells and tumors in a systematic manner, by employing both a differential display (DD) methodology and by using a cDNA microarray covering the entire human transcriptome.

### Summary of the invention

In the microarray studies the present inventors have circumvented detecting changes of gene expression not related to CIS (as CIS cells constitute only a small percentage of cells in a typical tissue sample) by using testis tissues containing different amounts of tubules with CIS and searched for genes regulated in a manner proportional to the content of CIS in the sample.

Selected candidates for CIS and tumor markers from both the DD and the microarray study have been verified by semi-quantitative reverse transcriptase PCR (RT-PCR) and the expressing cell type determined by in situ hybridization. Furthermore, the present inventors use antibodies against the some promising candidates and used immunohistochemistry to identify the expressing cell types in tissues with CIS/gonadoblastoma and in tumors derived from CIS.

The present inventors also investigated the gene level of transcripts (using the same large cDNA microarray) of testicular seminoma and embryonic carcinoma (EC, an undifferentiated component of non-seminomas) and compared that to the level of transcripts of CIS.

This facilitated identification of genes that mark the progression of CIS into either the seminoma or the non-seminoma. Results were confirmed by RT-PCR and markers were localized to tumor cells by in situ hybridization as well as for selected markers at the protein level by immunohistochemistry.

Carcinoma in situ (CIS) is the common precursor of histologically heterogeneous testicular germ cell tumors, which have markedly increased in frequency in the recent decades and now are the most common malignancy of young adult men. Using genome-wide gene expression profiling, the present inventors identified more than 200 genes highly expressed in testicular CIS, including many never reported in testicular neoplasms. Expression was further verified by semi-quantitative RT-PCR and in situ hybridization, and by immunohistochemistry for a few protein products, for which antibodies were available.

### Description of the invention

The biomarker disclosed herein can be used for the detection of CIS and gonadoblastoma but also the biomarker AP-2γ for the detection of the seminomas and non-seminomas derived from CIS and thus for the progression of CIS is included in the present invention. In one embodiment the marker is set forth in SEQ ID NO 20.

The biomarker was identified using differential display and genome-wide cDNA microarray analysis and subsequent verified by other methods, including in situ hybridization, which showed that the CIS markers were expressed in CIS cells.

For some biomarkers localization to CIS cells and some CIS-derived overt tumours was also done at the protein level by immunohistochemistry.

The present invention thus relates to the use of the identified biomarker, AP-2γ, to identify CIS and the status of CIS derived cancers in human samples such as semen samples, blood, testicular biopsies, and biopsies from any other human tissue.

In one aspect, the present invention relates to a method for determining the presence of precursors of germ cell tumours in a sample from an individual. Such a diagnostic method would comprise determining the level of transcripts of AP-2γ in a sample, and concluding from said level of transcripts whether or not the sample contains precursors of germ cell tumours.

Specifically, the method comprises the following steps:
a) determining the level of transcripts of a marker in said sample, wherein the marker is AP-2γ;
b) comparing said level of transcripts with a reference level of transcripts;
c) identifying whether the level of transcripts is different from said reference level of transcripts and
evaluating whether the sample contains precursors of germ cell tumours if the level of transcripts is different from the reference level of transcripts.

In the present context the term "precursors of germ cell tumours" relates to the commonly accepted fact that CIS is a common precursor for all germ cell tumours of the adolescents and young adults. CIS is also known as intratubular germ cell neoplasia of the unclassified type, testicular intraepithelial neoplasia and gonocytoma in situ, with the last term probably the most accurate from the biological point of view.

In the present context the term "expression profile" relates to assaying the level of transcripts, such as mRNA, either relative to a reference value, relatively to another transcript or sample and/or absolutely values. In example cDNA micro arrays measure the abundance of a transcript in two different samples relative to each other, whereas e.g. real-time PCR measures the absolute copy number of transcripts in the sample. Both relative and absolute measurements enables a person skilled in the art to make a diagnostic decision based on the data presented.

As known to the skilled addressee, a level of a transcript could also be obtained from e.g. amplified RNA, aRNA, and/or complementary DNA, cDNA, by various techniques. Thus, the present invention also relates to any methods using such manipulations. The genes listed in table 1 are in no particular order, and thus both the listed and the complementary strand of AP-2γ is an object of the present inventions.

The sequences listed in table 1, may represent only a part of the sequence of the gene listed, e.g. an EST sequence, fragment of sequence or similar. However, the full genomic sequence, the coding sequence, untranslated regions, and promoter regions of AP-2γ is an object of the present invention.

In general, assaying the level of transcripts, and thus determining the level of transcripts of a marker can be made by several technical approaches, such as, but not limited to, micro *arrays, filter arrays, DNA chips, gene chips, differential display, SAGE, northern blot, RT-PCR, quantitative PCR, PER, real-time PCR or any other known technique known to the skilled addressee

When assaying levels of expression of the sequences presented in table 1, the levels refers to the measurable expression level of a given nucleic acid. The level of expression of a nucleic acid is determined by methods well known in the art. "Differentially expressed" or "changes in the level of expression" may refer to an increase or decrease in the measurable expression level of a given nucleic acid and "Differentially expressed" when referring to microarray analysis means the ratio of the expression level of a given nucleic acid in one sample and the expression level of the given nucleic acid in another sample is not equal to 1.0. "Differentially expressed" when referring to microarray analysis according to the invention also means the ratio of the expression level of a given nucleic acid in one sample and the expression level of the given nucleic acid in another sample is greater than or less than 1.0 and includes greater than 1.2 and less than 0.7, as well as greater than 1.5 and less than 0.5. A nucleic acid also is said to be differentially expressed in two samples if one of the two samples contains no detectable expression of the nucleic acid.

Absolute quantification of the level of expression of a nucleic acid can be accomplished by including known concentration(s) of one or more control nucleic acid species, generating a standard curve based on the amount of the control nucleic acid and extrapolating the expression level of the "unknown" nucleic acid species from the hybridization intensities of the unknown with respect to the standard curve. The level of expression is measured by hybridization analysis using labeled target nucleic acids according to methods well known in the art. The label on the target nucleic acid can be a luminescent label, an enzymatic label, a radioactive label, a chemical label or a physical label. Preferably, target nucleic acids are labeled with a fluorescent molecule. Preferred fluorescent labels include, but are not limited to, fluorescein, amino coumarin acetic acid, tetramethylrhodamine isothiocyanate (TRITC), Texas Red, Cy3 and Cy5.

Concluding from said level of transcripts whether or not the sample contains precursors of germ cell tumours can be obtained by assaying the level of transcripts and determine if the markers are either present and/or up-regulated and/or down-regulated. Depending on the experimental set-up the marker genes may appear up-regulated but could also appear down-regulated, if e.g. the ratio were switched. The settings in the present application focus on the up-regulated markers.

Depending on the assay method and set-up the measurement of the marker gene may be done in reference to either another transcript, a sample or measured absolutely.

The term "sample" could be a semen sample, a blood sample, a serum sample, a urine sample, a testicular biopsy sample, or a biopsy from any other human tissue. The marker genes described here may be used to identify CIS or seminoma or non-seminoma cells in other clinical samples than those related to the testis and semen samples, i.e. in extra-gonadal brain tumors or blood samples from patients with disseminated seminoma.

In a presently preferred embodiment the sample is a semen sample.

The general scope of the present invention relates to detection of precursors of germ cell tumours in various samples as described above. It is obvious to the skilled addressee that the methods described herein enables a person skilled in the art to detect the presence of and/or the development of even very small amounts of precursors of germ cell tumours in the selected samples. In a presently preferred embodiment the methods described herein enables detection of at least one cell which is a precursors of germ cell tumours.

The level of transcripts and as described below the intensity values are indicative of precursors of germ cell tumours since these data are found significantly more often in patients with a precursors of germ cell tumours than in patients without the precursors of germ cell tumours.

As used herein, "expression pattern" or "expression profile" comprises the pattern (i.e., qualitatively and/or quantitatively) of expression of one or more expressed nucleic acid sequences where one or more members of the set are differentially expressed.

As the skilled addressee would recognise the present invention enables a person skilled in the art in both diagnosing and/or monitoring precursors of germ cell tumours in a sample from a patient by detecting the level of transcripts of AP-2γ.

The present invention also relates to use of AP-2γ sequence/gene/mRNAs for diagnostics of precursors of germ cell tumours located in the testis or in all other locations, using any method that can detect the products on the mRNA level (PCR, hybridisation, micro-array, etc.) or on the protein level (immunoassays, Western blot, protein-microarrays etc.) in any human samples including semen samples, blood, tissue samples, urine, etc. in patients or healthy individuals.

In other words, the present invention relates to a method for diagnosing precursors of germ cell tumours, the method comprising the steps of assaying an expression level of AP-2γ in a sample; and, identifying said precursor of germ cell tumours if at least one of said expression levels is greater than a reference expression level.

Alternatively, the method for determining the presence of precursors of germ cell tumours in a sample may be based on measurements of the polypeptides expressed by the nucleotide sequence SEQ ID NO:20.

Thus, another aspect of the present invention relates to a method for determining the presence of precursors of germ cell tumours in a sample from an individual comprising determining the intensity signal(s) of a marker in a patient sample, and concluding from said intensity signal(s) whether the patient sample contains precursors of germ cell tumours, wherein the marker is AP-2γ.

Such a method comprises the following steps:
a) determining the intensity signal of the protein encoded by a marker in said sample, wherein the marker is AP-2γ;
b) comparing the intensity signal with a corresponding reference intensity signal;
c) identifying whether the intensity signal is different from said corresponding reference intensity signal and
evaluating whether the sample contains precursors of germ cell tumours if the intensity signal is different from the corresponding reference intensity signal.

The term "intensity signal" relates to assaying the protein level of the proteins encoded by the transcripts from table 1. Any immuno-assay is able to perform such measurement e.g. ELISA, RIA, Western blots, or dot blots. Also other methods capable of measuring the amount of a protein quantitatively or relatively. Such techniques include, but are not limited to; mass spectrometry base measurements, protein microarrays, ProteinChip® (Ciphergen) and derived methods.

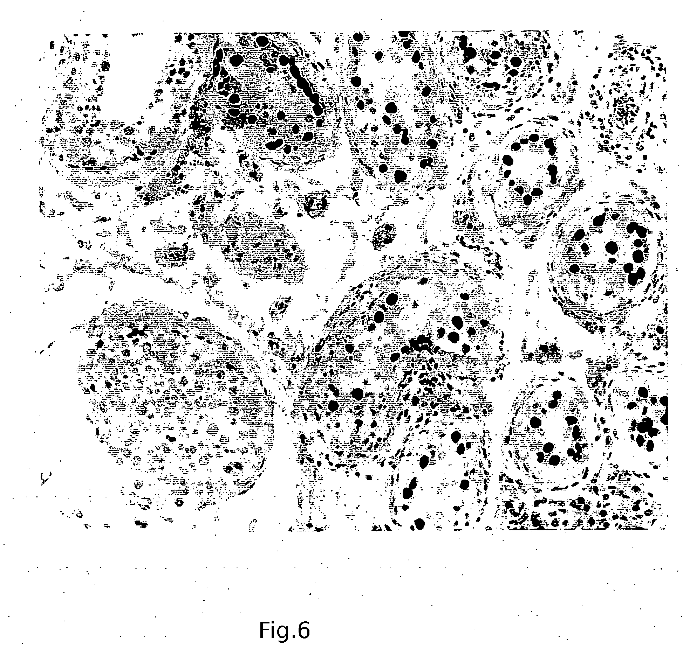

As exemplified below, the present inventors are generating antibodies against the gene products of the present inventions. Figure 6 displays an example of one such antibody used in an immuno-staining of an tissue section comprising CIS cells.

Antibodies generated against the gene products of the present invention may enable monitoring of metastasis and invasion of germ cell tumours.

As shown in table 1, several markers are up-regulated in highly abundant CIS cells, thus the present invention is described in relation to such up-regulated markers.

Such specific markers are the markers selected from the group consisting of SEQ ID NO 1-157.

A presently preferred embodiment of the present invention further relates to a method according to the invention, wherein AP-2γ is in a sample with CIS in 100% of the seminiferous tubules up-regulated more than 7 fold when compared to a reference sample, such as 7.1 fold, e.g. 7.2 fold, such as 7.5 fold, e.g. 8 fold, e.g. 9 fold, such as 10 fold, e.g. 11 fold, e.g. 12 fold, such as 13 fold, 14 fold, 15 fold, e.g. 16 fold, such as 17 fold, 18 fold, 19 fold, e.g. 20 fold, such as 25 fold, e.g. 30 fold, 35 fold, 40 fold, e.g. 45 fold, such as 50 fold, e.g. 55 fold, e.g. 60 fold, such as 65 fold, e.g. 70 fold, such as 75 fold, e.g. 80 fold, such as 85 fold, e.g. 90 fold, e.g. 95 fold, such as 100 fold.

Another presently preferred embodiment of the present invention relates to a method according to the invention, wherein the level of transcripts distinguish between classical testicular seminoma and testicular non-seminoma in gonads or in extra-gonadal localizations.

One presently preferred embodiment relates to a method which can distinguish seminoma from non-seminoma.

Another presently preferred embodiment relates to a method which can distinguish seminoma from CIS.

Yet another presently preferred embodiment relates to a method which can distinguish non-seminoma from CIS.

Therefore, one aspect of the present invention relates to a method for monitoring progression from precursors of germ cell tumours to overt cancer in an individual comprising determining the level of transcripts and/or the intensity signal(s) of AP-2γin a sample and concluding from the level of transcripts and/or the intensity signal(s), whether the sample contains precursors of germ cell tumours.

In one detailed embodiment the method comprises the following steps:
a) determining the level of transcripts of a marker in a sample, wherein the marker is AP-2γ;
b) comparing said level of transcripts with a reference level of transcripts;
c) identifying whether the level of transcripts is different from said reference level of transcripts and
evaluating the development stage of precursors of germ cell tumours to overt cancer cells if the level of transcripts is different from the reference level of transcripts.

Alternatively, the method comprises the following steps:
a) determining the intensity signal of the protein encoded by a marker in a sample, wherein the marker is AP-2γ;
b) comparing the intensity signal with a corresponding reference intensity signal;
c) identifying whether the intensity signal is different from said corresponding reference intensity signal and
evaluating the development stage of precursors of germ cell tumours to overt cancer cells if the intensity signal is different from the corresponding reference intensity signal.In yet another aspect, the present invention relates to a method for identifying precursors of germ cell tumours in a sample from a mammal, the method comprising
assaying said sample by a quantitative detection assay and determining the level of transcripts and/or the intensity signal of a marker, wherein said marker is AP-2γ;
comparing said level of transcripts and/or the intensity signal with a reference value
identifying whether the level of transcripts and/or the intensity signal of the marker from the sample is significantly different from the reference value, and
evaluating whether the sample contains precursors of germ cell tumours if the level of transcripts and/or the intensity signal is different from the reference value.

As the skilled addressee would recognise the present invention is useful as a method for determining the presence of precursors of germ cell tumours in an individual comprising detecting the presence or absence of at least one expression product, wherein said at least one expression product comprise a nucleotide sequence from AP-2γIn another aspect, the present invention also relates to a method of assessing the efficacy of a therapy for inhibiting precursors of germ cell tumours in a subject, the method comprising
the following steps:
comparing the level of transcripts of a marker in a first sample obtained from the subject prior to providing at least a portion of the therapy to the subject and the level of transcripts of the marker in a second sample obtained from the subject following provision of the portion of the therapy, wherein a significantly altered level of transcripts of the marker in the second sample, relative to the first sample, is an indication that the therapy is efficacious for inhibiting precursors of germ cell tumours in the subject
wherein the marker is AP-2γ. Alternatively, the above method aspect can also be assessed by comparing the intensity signals of the proteins encoded by the marker instead of comparing the level of transcripts.

An another alternative method of assessing the efficacy of a therapy for inhibiting precursors of germ cell tumours in a subject comprises the following steps:
comparing the intensity signal of the protein encoded by a marker in a first sample obtained from the subject prior to providing at least a portion of the therapy to the subject and the intensity signal of the proteins encoded by the marker in a second sample obtained from the subject following provision of the portion of the therapy, wherein a significantly altered intensity signal of the protein encoded by the marker in the second sample, relative to the first sample, is an indication that the therapy is efficacious for inhibiting precursors of germ cell tumours in the subject
wherein the marker is AP-2γ. The method of assessing the efficacy of a therapy of the present invention also relates to methods for identifying, evaluating, and monitoring drug candidates for the treatment of precursors of germ cell tumours and of course any of the later stages. According to the invention, a candidate drug or therapy is assayed for its ability to decrease the expression of one or more markers of precursors of germ cell tumours. In one embodiment, a specific drug or therapy may reduce the expression of markers for a specific type or subclass of precursors of germ cell tumours described herein. Alternatively, a preferred drug may have a general effect on precursors of germ cell tumours and decrease the expression of different markers characteristic of different types or classes of precursors of germ cell tumours. In one embodiment, a preferred drug decreases the expression of a precursors of germ cell tumours marker by killing precursors of germ cell tumour cells or by interfering with their replication.

Current trends in stem cell research enable a person skilled in the art of various treatments comprising transplanting or regeneration of tissue by the use of e.g. embryonal stem cells.

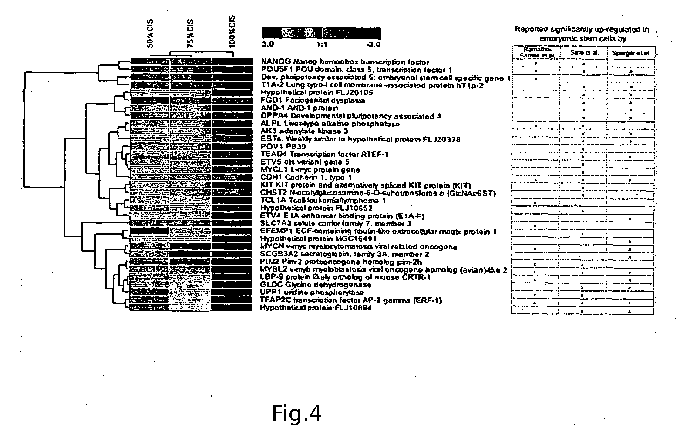

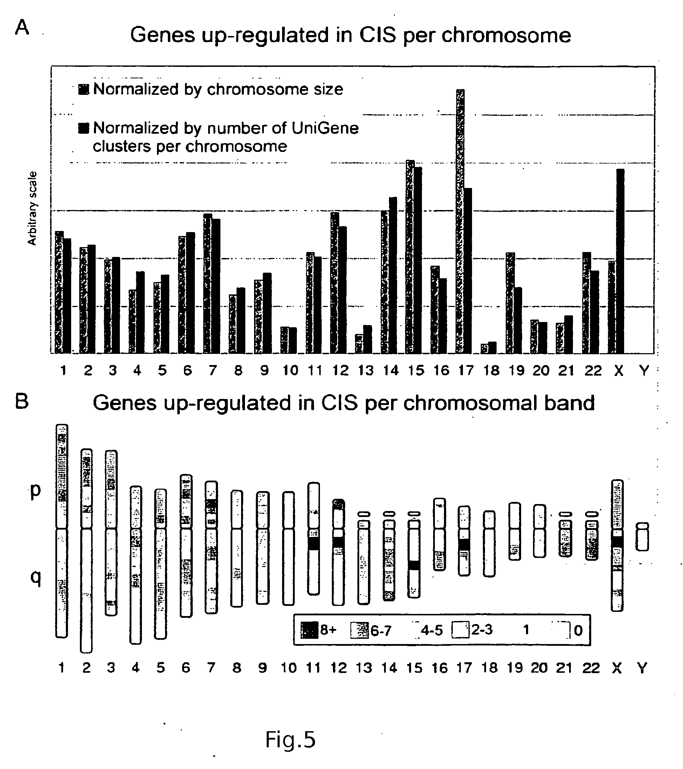

In the present application the present inventors have identified that CIS cells have many features, which are embryonal stem cell like, see figure 4 and figure 5 and the examples below.

CIS cells thus both are embryonal stem cell like and cancer precursor cells. Thus, by use of the present markers a person skilled in the art would be able to judge, if the e.g. tissue derived from embryonal stem cells contains cells, which are likely to become cancer precursor cells, and thus represent valuable information for consideration before transplanting e.g. an embryonal stem cells.

In another aspect, the present invention also relates to a methods for analysing the presence of undifferentiated stem cells highly likely to progress to precursors of germ cell tumours and/or cancer in an individual.

Such methods comprise the following steps in one embodiment:
a) determining the level of transcripts of a marker in a sample, wherein the marker is AP-2γ;
b) comparing said level of transcripts with a reference level of transcripts;
c) identifying whether the level of transcripts is different from said reference level of transcripts and
evaluating whether the sample contains undifferentiated stem cells highly likely to progress to precursors of germ cell tumours and/or cancer in the individual if the level of transcripts is different from the reference level of transcripts.

Alternatively, such methods comprise the following steps in a second embodiment:
a) determining the intensity signal of the protein encoded by a marker in said sample, wherein the marker is AP-2γ,
b) comparing the intensity signal with a corresponding reference intensity signal;
c) identifying whether the intensity signal is different from said corresponding reference intensity signal and
evaluating whether the sample contains undifferentiated stem cells highly likely to progress to precursors of germ cell tumours and/or cancer in the individual if the intensity signal is different from the reference intensity signal.

Testicular carcinoma in situ (CIS) is the common precursor of nearly all testicular germ cell tumors (TGCTs) that occur in young adults. The incidence of TGCTs has increased markedly over the past several decades, and these tumors are now the most common type of cancer in young men, thus representing an increasing health problem. There is a current need in the art of potential screening methods to identify the patients in an early non-invasive stage for the purpose of e.g. treatment strategy.

Thus, in another aspect, the present invention also relates to a method for screening for the presence of precursors of germ cell tumours in an individual of a population, said method comprising determining the level of transcripts and/or the intensity signal(s)of fAP-2γin a sample obtained from said individual, and concluding from the level of transcripts and/or the intensity signal(s) whether the sample contains at least one precursor cell of germ cell tumours.

### Tested Individual Markers

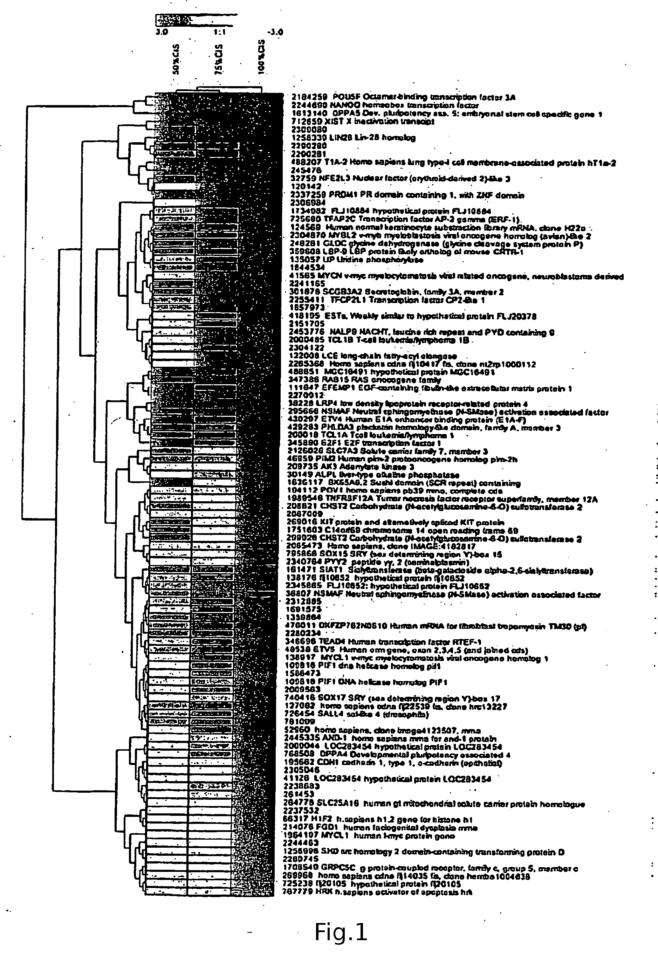

A range of the genes listed in tabel 1 has been further investigated at the protein level. Initially the genes from figure 1 which are highly expressed at the RNA level were studied at the protein level on tissue sections containing CIS using immunohistochemical detection. The antibodies which stained only CIS or tumour cells in testicular tissue sections were further used to stain semen samples spreads (cytospins) on a microscope slide.

The present invention relates to methods according to the present invention, wherein the marker is AP-2γ.

### AP-2γ

AP-2γ is encoded by the *TFAP2C* gene mapped on chromosome 20q13.2 and is a member of a family of DNA-binding transcription factors, which includes AP-2α, AP-2β, AP-2γ, TFAP-2δ and TFAP-2ε. During vertebrate embryogenesis, these proteins play important roles in the development and differentiation of the neural tube, neural crest derivatives, skin, heart and urogenital tissues and show overlapping, but distinct, patterns of expression within these tissues.

AP-2γ is required within the extra-embryonic lineages for early post-implantation development, but apparently does not play an autonomous role within the embryo proper. Mice lacking AP-2γ die at day 7.5-8.5 post coitum due to malformation of extra-embryonic tissues.

In the above genome-wide gene expression profiling study of CIS cells, we have identified a number of genes expressed also in embryonic stem cells and foetal gonocytes but not in adult germ cells, thus providing many possible new markers for CIS. One of such genes was TFAP2C (mapped to chromosome 20q13.2), which encodes the transcription factor activator protein-2 (AP-2γ).

By immunhistochemical detection of the AP-2γ protein the present inventors investigated the protein expression in a large range of tissues including fetal and malignant tissues. The present inventors found that AP-2γ protein expression was very resticted and only found in early gonocytes and other not fully differentiated cells. Also it was found to be very solid nuclear marker of testicular CIS cells and derived neoplastic cells (figure 6).

The present inventors disclose AP-2γ as a novel marker for foetal gonocytes and neoplastic germ cells, including testicular CIS, with a role in pathways regulating cell differentiation and a possible involvement in oncogenesis.

The fact that AP-2γ protein was not expressed in normal adult reproductive tract but was abundant in nuclei of CIS and tumour cells, which are better protected than the cytoplasm from degradation in semen due to a structural strength, prompted us to analyse the value of AP-2γ for detection of CIS and/or tumour cells in ejaculates in a series of patients and controls.

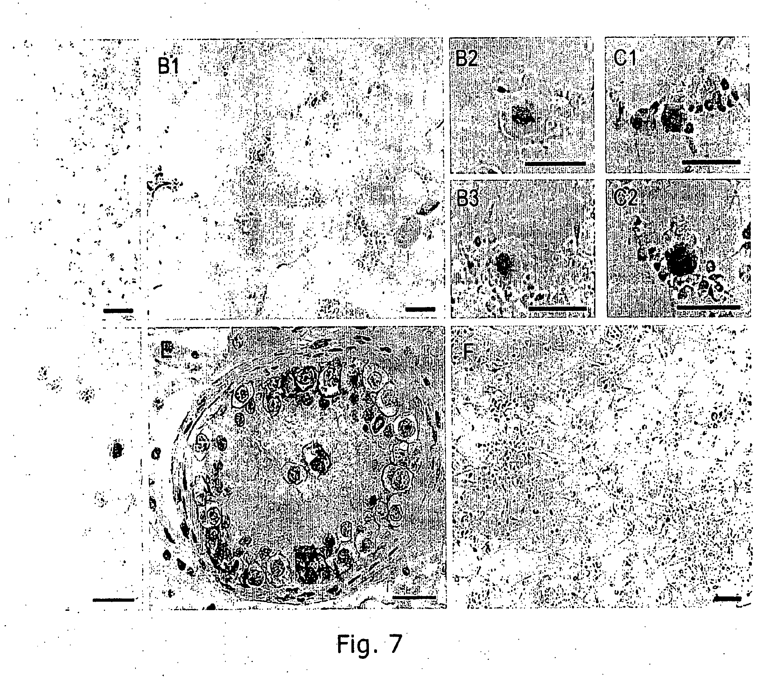

In an investigation of 104 andrological patients, presented in Example 10, cells positive for AP-2γ were found only in semen samples from patients diagnosed a priori with testicular neoplasms and, surprisingly, in a 23-year-old control subject with oligozoospermia and no symptoms of a germ cell tumour (figure 7).

Testicular biopsies performed during the follow-up of the 23-year-old control subject revealed widespread CIS in one testis, thus proving a diagnostic value of the new assay. Optimisation of the method is in progress, but a preliminary assessment suggests that the method may have a place in screening of germ cell neoplasia in at-risk patients. The local ethics committee approved the studies of AP-2γ expression and participants gave written informed consent.

The number of patients that we have screened for the presence of immunoreactive AP-2γ is continuously increasing as we are testing patients as they appear in our clinic. So far we have tested 347 andrological patients (including the 104 mentioned above) and found CIS-like cells positive for Asp-2γ in semen samples of 3 patients, including the first case described above (a 23-year-old subfertile control subject with widespread CIS confirmed by testicular biopsies). Two other patients are currently under clinical follow-up and being considered for testicular biopses.

Thus, in a particular preferred embodiment, the present invention relates to a method according to the present invention, wherein the marker is AP-2γ.

Antibodies directed against the proteins encoded by the genes in table 1 were used for immunohistochemical investigations on sections from different tissues including testicular CIS.

The present application furthermore particularly relates to a method according to the present invention, wherein the intensity signal is determined by immunocytological detection (immuno staining).

As the skilled person will know, any of the methods of the present invention may be used for screening for the presence of precursors of germ cell tumours in individuals of a population, either alone or in any combination.

The present invention also describes to a kit for use in an assay or method as defined in the present application.

In one embodiment, such kit may be a diagnostic array comprising: a solid support; and a plurality of diagnostic agents coupled to said solid support, wherein each of said agents is used to assay the expression level of AP-2γ, wherein each of said diagnostic agents is selected from the group consisting of PNA, DNA, and RNA molecules that specifically hybridize to a transcript from AP-2γ or wherein each of said diagnostic agents is an antibody that specifically binds to a protein expression product of AP-2γ.

The present invention further describes databases of marker genes and information about the marker genes, including the expression levels that are characteristic of precursor of germ cell tumours. The marker gene information is preferably stored in a memory in a computer system. Alternatively, the information is stored in a removable data medium such as a magnetic disk, a CDROM, a tape, or an optical disk. In a further embodiment, the input/output of the computer system can be attached to a network and the information about the marker genes can be transmitted across the network.

Preferred information includes the identity of a predetermined number of marker genes the expression of which correlates with a particular type of precursor of germ cell tumours. In addition, threshold expression levels of one or more marker genes may be stored in a memory or on a removable data medium. A threshold expression level is a level of expression of the marker gene that is indicative of the presence of a particular type or class precursor of germ cell tumours.

In a preferred embodiment, a computer system or removable data medium includes the identity and expression information about a plurality of marker genes for several types or classes of precursor of germ cell tumours disclosed herein. In addition, information about marker genes for normal testis tissue may be included. Information stored on a computer system or data medium as described above is useful as a reference for comparison with expression data generated in an assay of testis tissue of unknown disease status

It should be understood that any feature and/or aspect discussed above in connection with the methods according to the invention apply by analogy to the uses according to the invention.

As will be apparent, preferred features and characteristics of one aspect of the invention may be applicable to other aspects of the invention.

Throughout this specification the word "comprise", or variations such as "comprise" or "comprising", will be understood to imply the inclusion of a stated element, integer or step, or group of elements, integers or steps, but not the exclusion of any other element, integer or step, or group of elements, integers or steps.

The invention will hereinafter be described by way of the following non-limiting Figures and Example.

### Figure legends

### Figure 1

Hierarchical clustering of genes found by filtering data so only genes 4 fold or more up-regulated in the sample with CIS in 100% of the tubules and 2 fold in the samples with 50 and 75% tubules with CIS. Hierarchical clustering was done using Euclidean distances and average linkage. Some redundancy between entries is seen and implies consistency in the results. The image clone number (Unique ID on the array) is reported in front of the gene names and can be used to search the German Resource Center for Genome Research for further sequence information, http://www.rzpd.de.

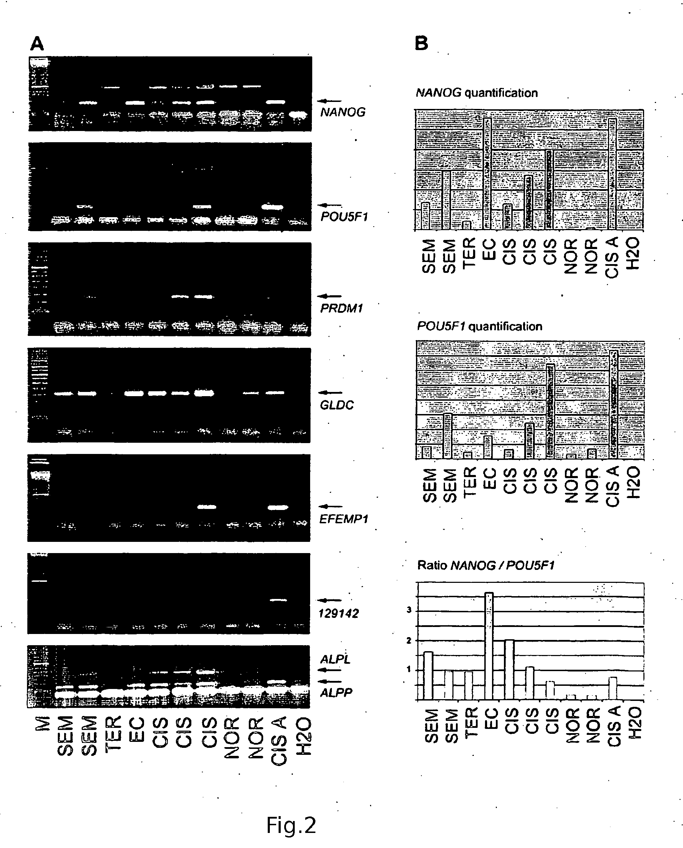

### Figure 2 (not part of the present invention)

Further analysis of selected genes that in microarray study were found up-regulated in CIS. A) Examples of the semi-quantitative RT-PCR on a spectrum of testicular tissue samples. The gene name is on the right side of the panes. In the gels of NANOG, and POU5F1 the beta-actin-marker band of 800 bp is visible. Representative DNA fragments from all RT-PCR amplifications were excised, cloned, and sequence verified. 129142 is an Image clone number. B) Corresponding quantifications of NANOG and POU5F1 and the ratio between the two. Quantification was done by digitalizing the image of the agarose gel. M=100 base pair marker, SEM=seminoma, TER=teratoma, EC=embryonal carcinoma, CIS=testicular tissue showing 100% tubules with carcinoma in situ, NOR=normal testicular tissue, CIS A= CIS amplified for microarray analysis, H20=control. The CIS sample (to the right) is the same sample used in the microarray analyses.

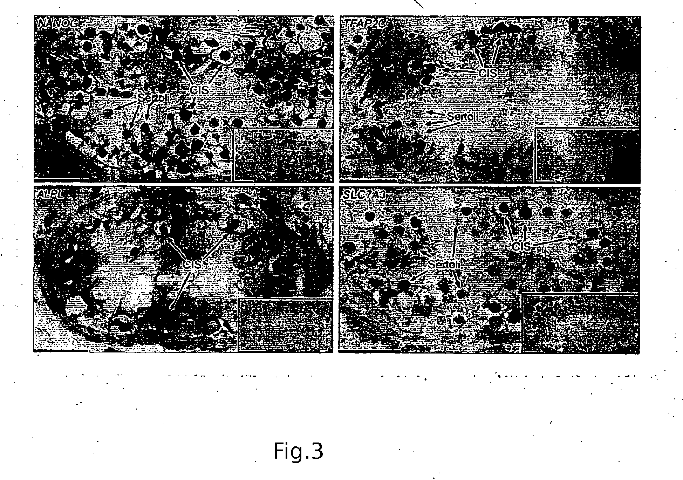

### Figure 3 (not part of the present invention)

Analysis of the cellular expression in tubules with CIS by in situ hybridization of NANOG, SLC7A3, TFAP2C and ALPL showing that the expression was confined to CIS cells (arrows). CIS cells are seen as a stained ring with a stained nucleus in the center, while the rest appears empty, due to large amounts of glycogen washed out during the tissue processing. Staining was in addition seen in Sertoli cells nuclei, probably due to over-staining necessary to visualize low abundance transcripts (arrows), since this was also seen with the sense probe. The sense control is from neighbouring sections of the same tubule as the anti-sense and inserted in the lower right corner. The scale, bar corresponds to 50 µm.

### Figure 4

Genes from figure 1 reported to be up regulated in ESC. Data on mouse ESC are from Ramalho-Santos et al. (Ramalho-Santos, M, et al. Science 2002, 298:597-600) whereas data on human ESC are from Sato et al. (Sato, N, et al. Dev Biol 2003, 260:404-13) and Sperger et al. (Sperger, JM, et al. Proc Natl Acad Sci U S A 2003, 100:13350-5). The overlap between genes reported in these studies and genes identified in CIS (fig. 1) amount to roughly 35% but may be even greater due to differences in gene annotation and RNA references used.

### Figure 5

Chromosomal distribution of genes more than 2 fold up-regulated in the sample with 100 % tubules having CIS. A) Distribution per chromosome and normalized by the size of the chromosomes (blue bars) or by the number of UniGene clusters on each chromosome (red bars). Chromosomal size was acquired from Ensembl, http://www.ensembl.org/, and number of UniGene clusters (Build 34 version 3) from NCBI, http://www.ncbi.nlm.nih.gov/. B) Detailed distribution of the number of up-regulated genes in each chromosomal band. The color-coding is outline in the inserted box.

### Figure 6.

Immuno-staining of transcription factor AP-2γ (TFAP2C) on a cross-section of testicular tissue containing both normal seminiferous tubules without CIS and tubules with CIS lesion. The CIS cells stain darkly and the signal is located to the nucleus of the CIS cells.

### Figure 7

A. Semen sample from a control patient spiked with CIS cells that are strongly stained with AP-2γ.
B1. AP-2γ-positive CIS cells in the semen sample of the 23-years-old subfertile control subject diagnosed with CIS by this assay.
B2, B3. Examples of AP-2γ stained cells in the 23-years-old subfertile control subjectdiagnosed with CIS by this assay, higher magnification.
C1, C2. Examples of AP-2γ stained cells in a patient known a priori to harbour testicular cancer.
D. Histological features of the biopsy of the left testis of the 23-years-old subfertile control subject with AP-2γ staining.
E. placental alkaline phosphatase (PLAP) staining showing CIS cells both adjacent to the tubular membrane and in the lumen. F. Semen sample from a control patient, with no AP-2γ-positive cells. Scale bar =25 µm.

### Figure 8 (not part of present invention)

Immuno-staining of TCL1A on a cross-section of testicular tissue containing tubules with CIS lesion. The CIS cells stain highly red and the signal is located to the nucleus of the CIS cells.

### Figure 9 (not part of present invention)

Immuno-staining of homeobox protein NANOG on a cross-section of testicular tissue containing both normal seminiferous tubules without CIS and tubules with CIS lesion. The CIS cells stain darkly and the signal is located to the nucleus of the CIS cells.

### Figure 10 (not part of present invention)

Immuno-staining of E-cadherin on a cross-section of testicular tissue containing tubules with CIS lesion. The CIS cells stain dark red and the signal is located to the cytoplasm or cellular membrane of the CIS cells.

### Figure 11 (not part of present invention)

Immuno-staining of PIM-2 on a cross-section of testicular tissue containing tubules with CIS lesion. The CIS cells stain dark red and the signal is located to the nucleus of the CIS cells.

**Tables** (SEQ.ID.No 20 is part of the present invention. Other SEQ.ID.No's are there for illustrative purposes only).

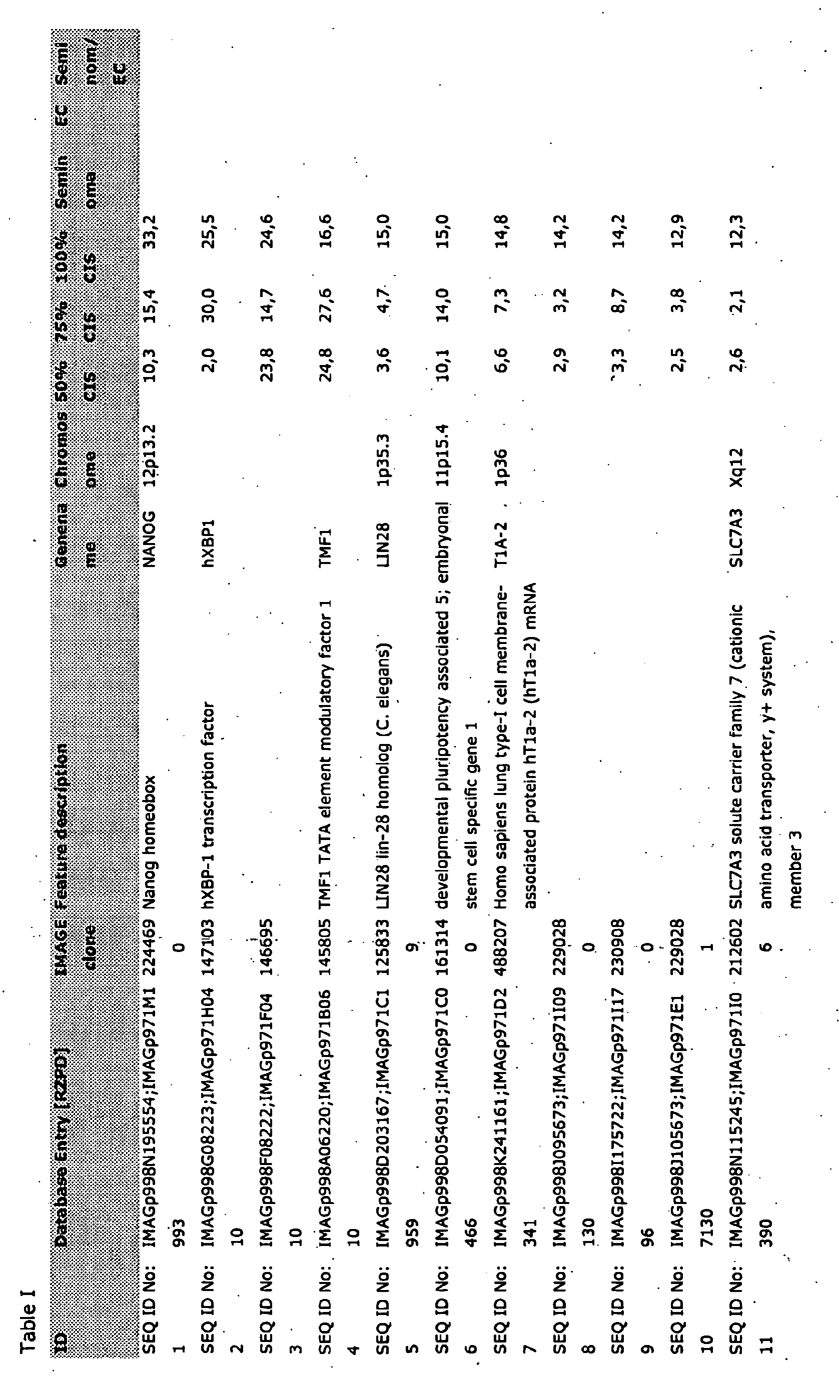

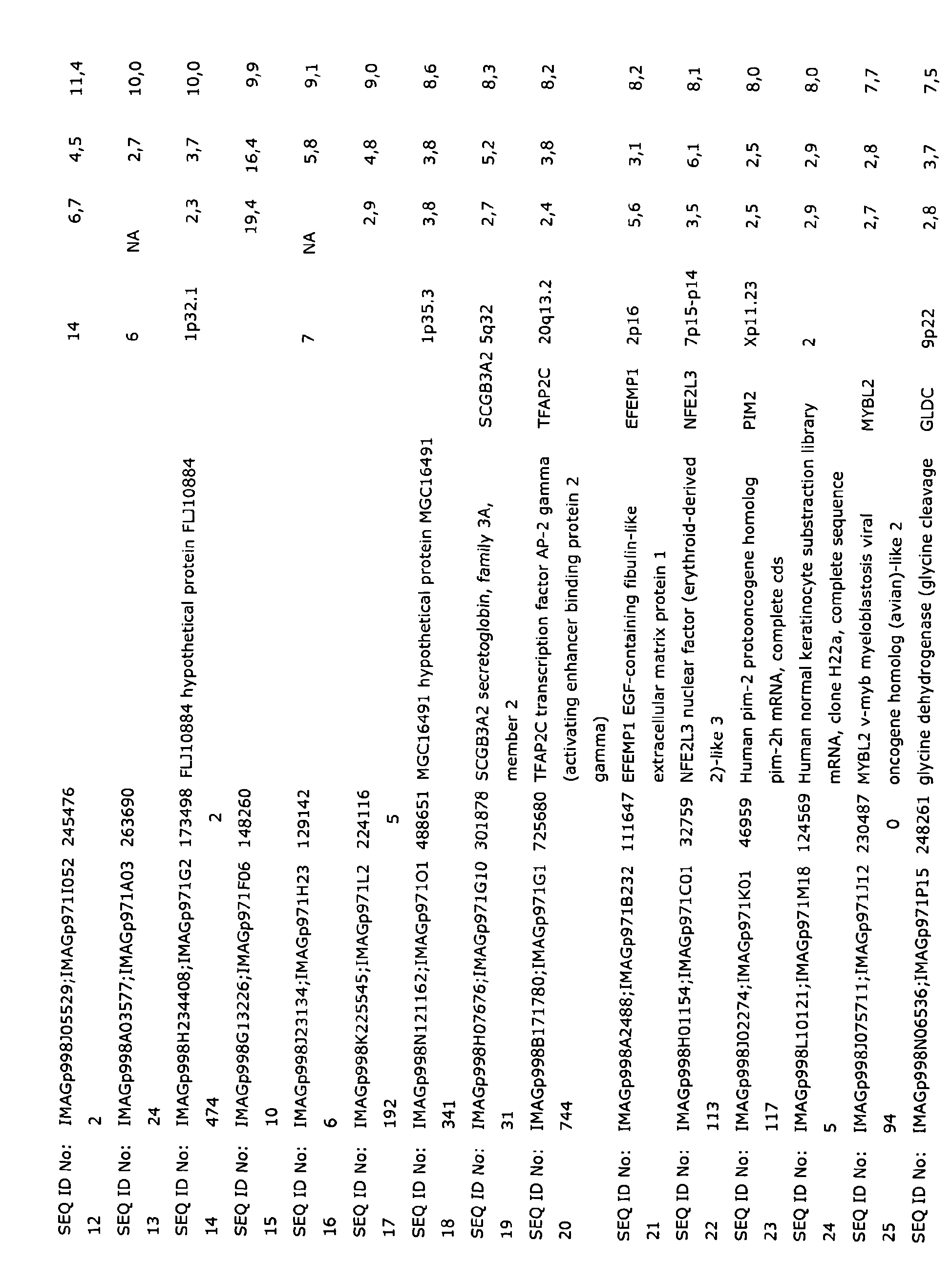

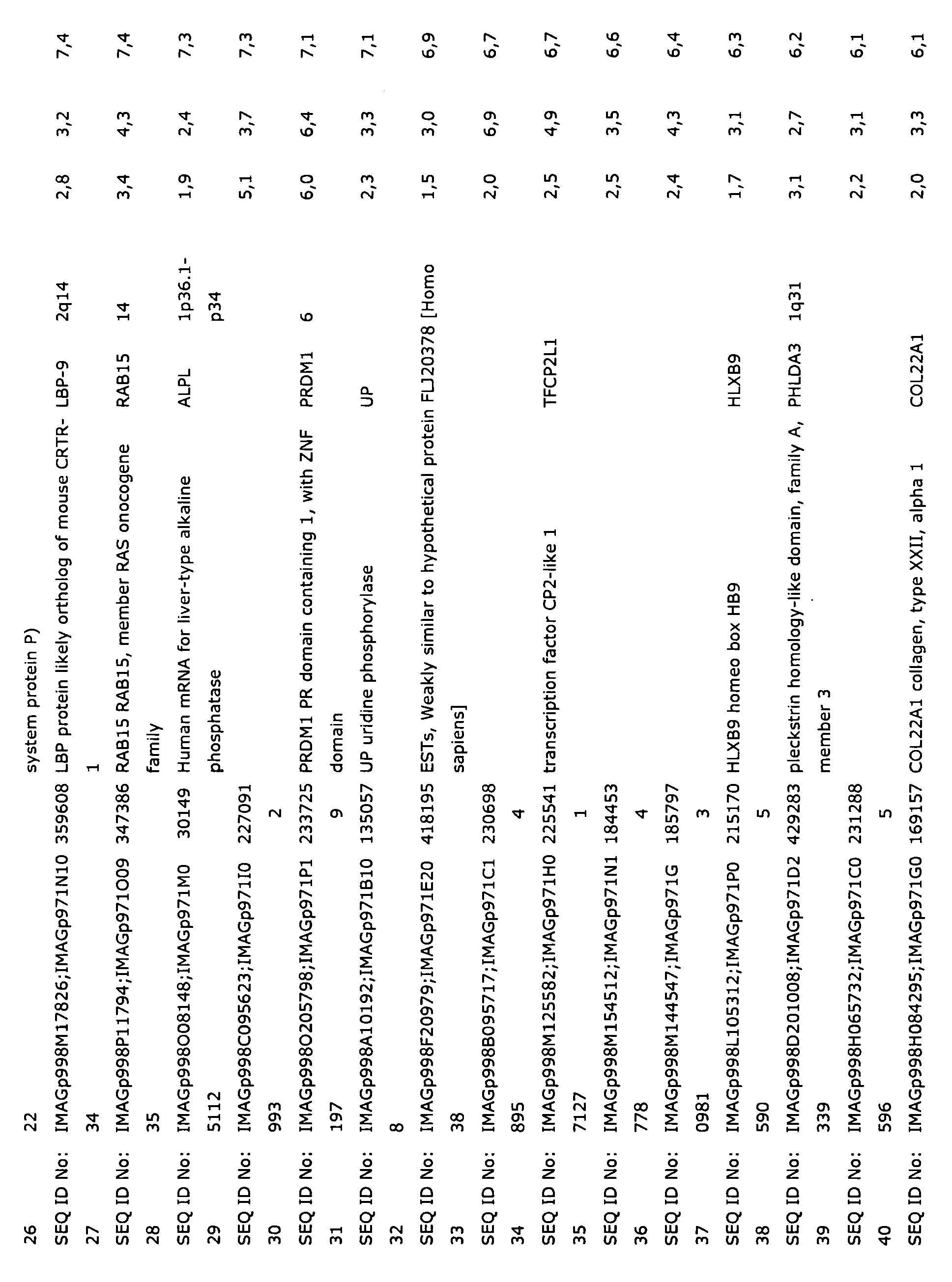

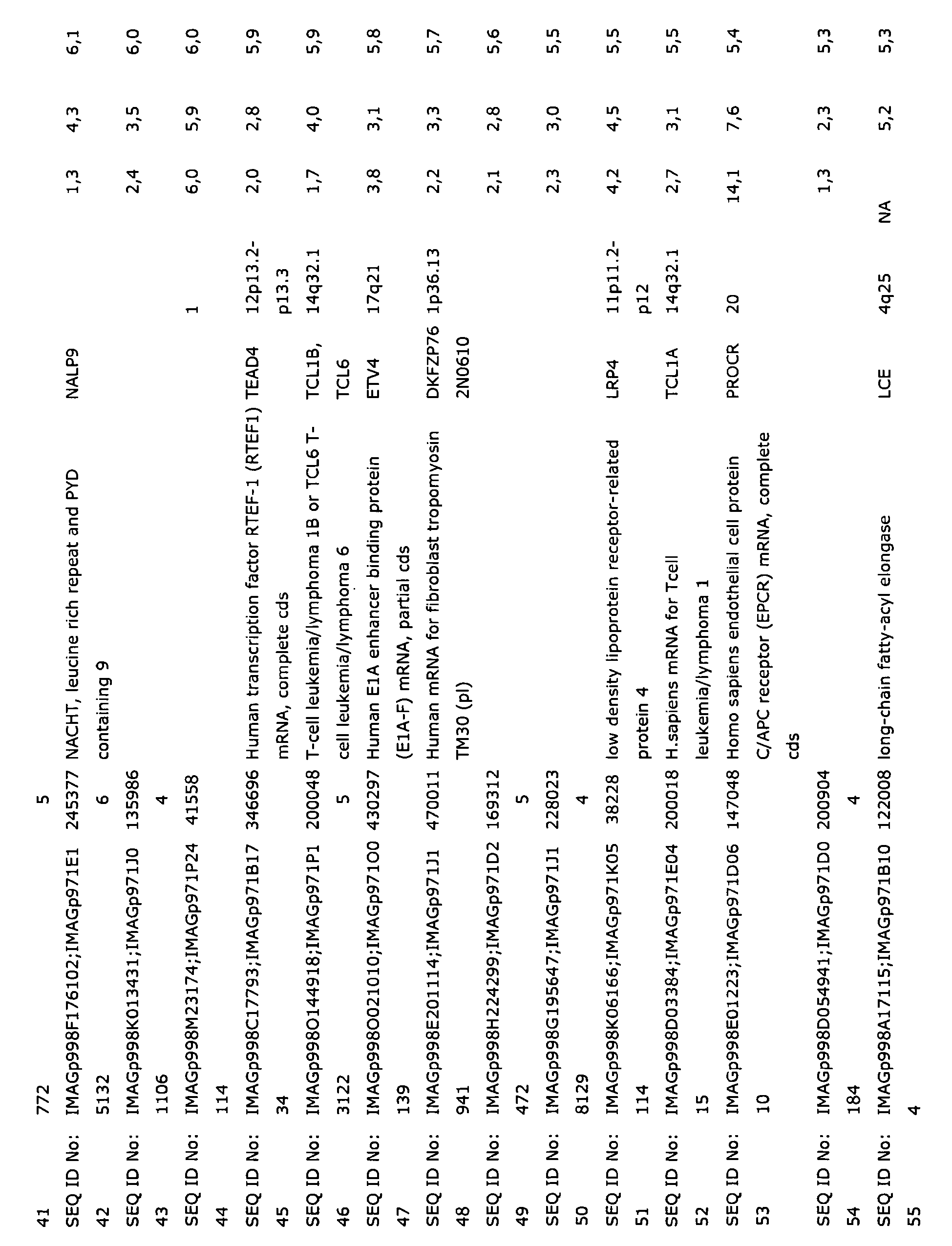

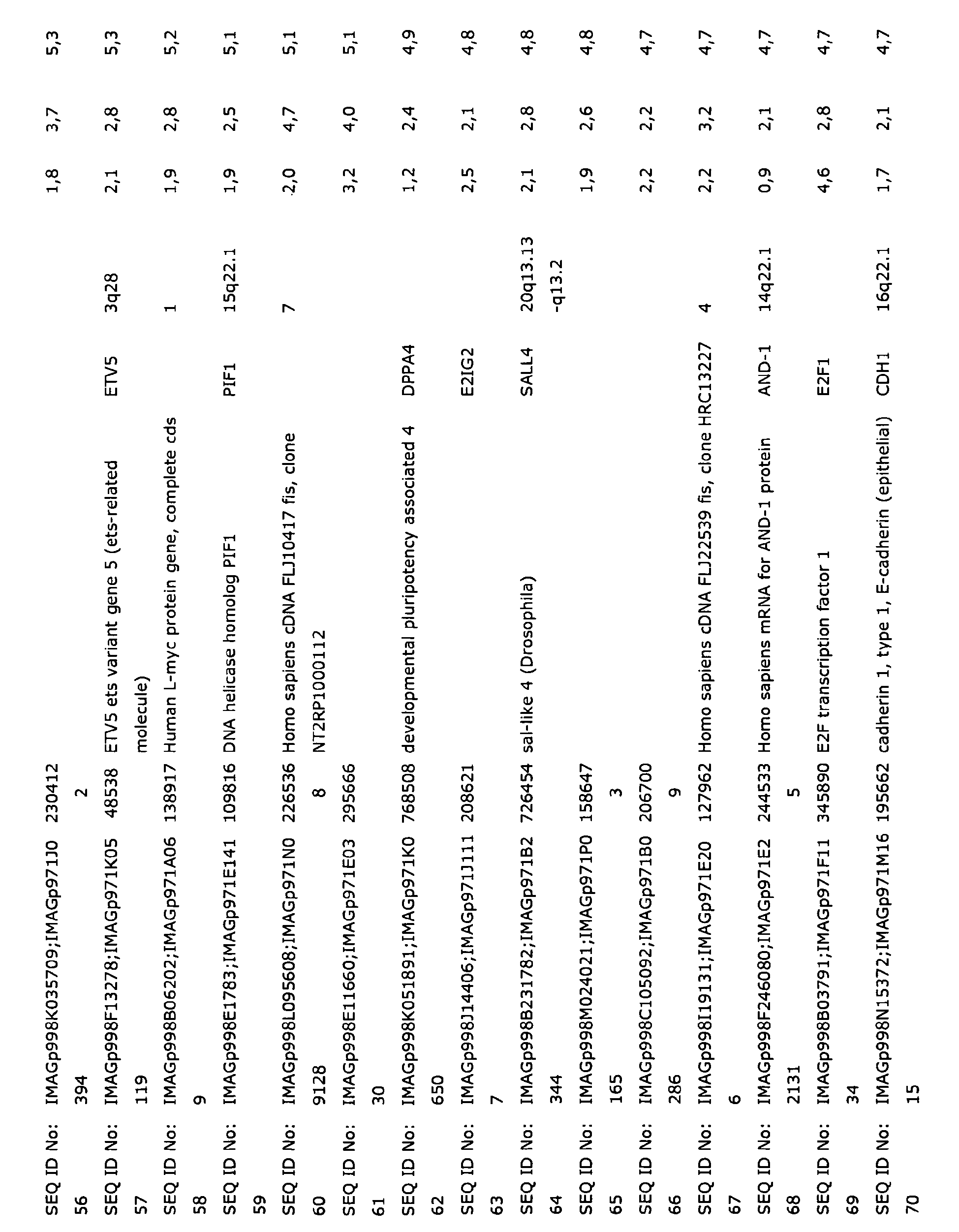

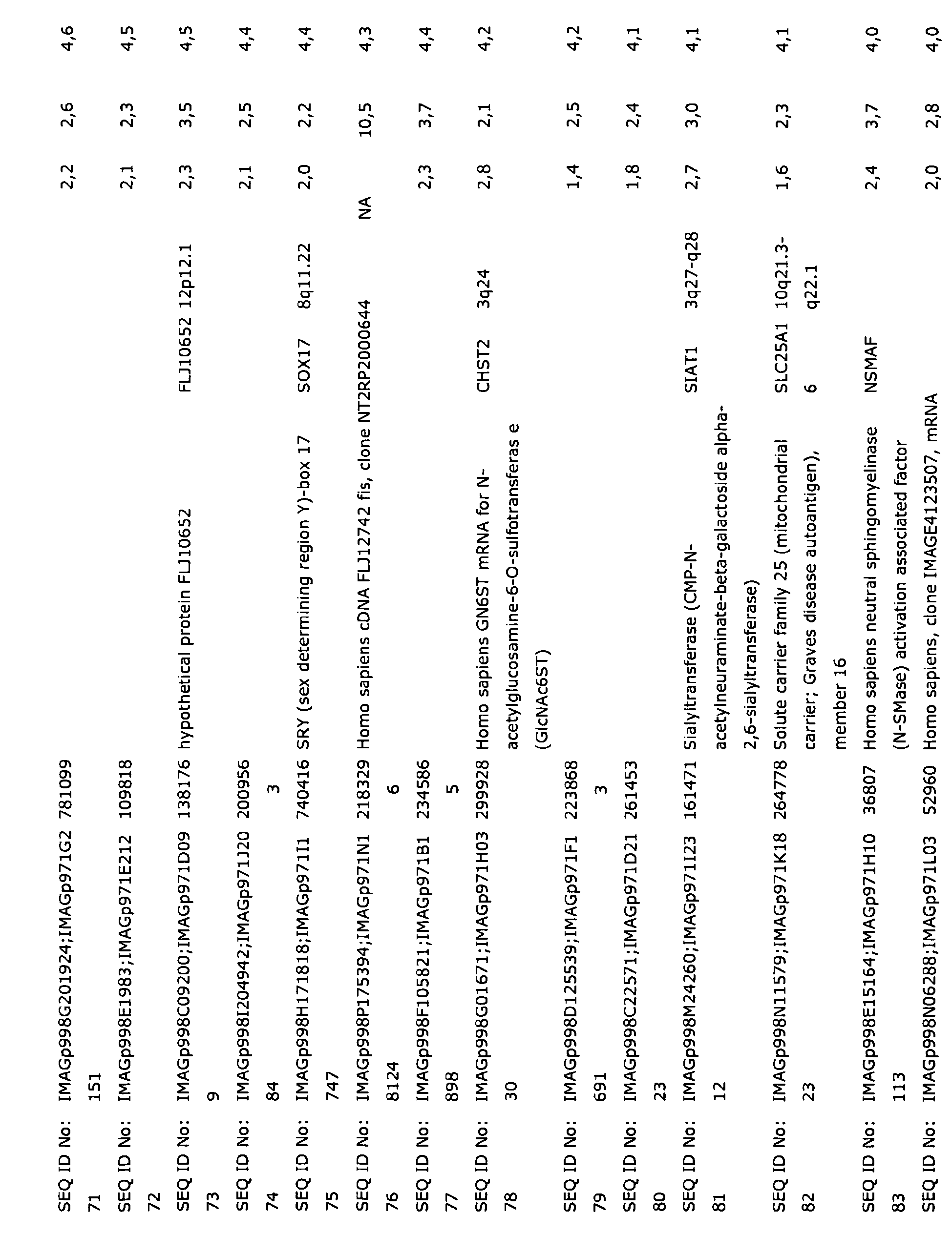

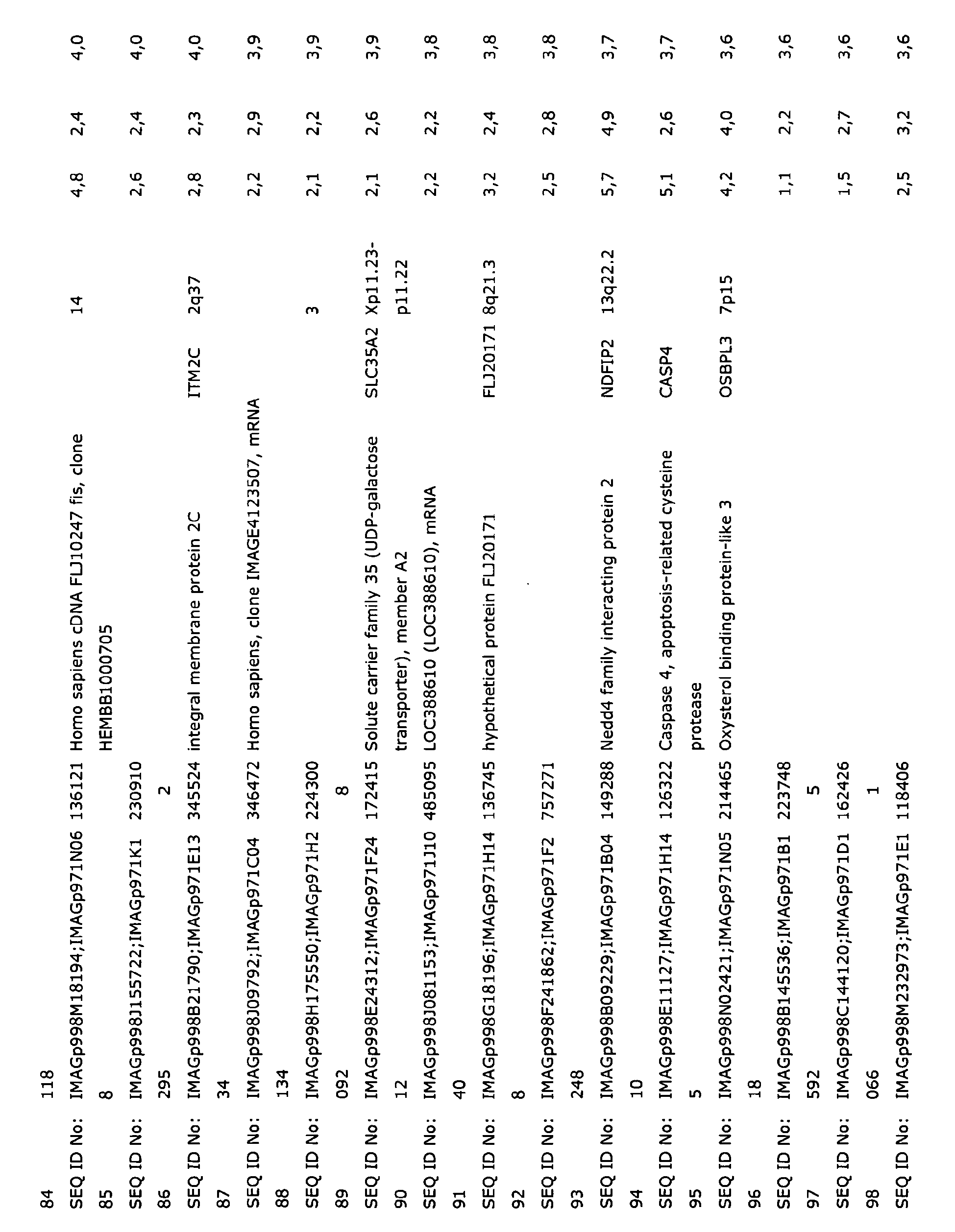

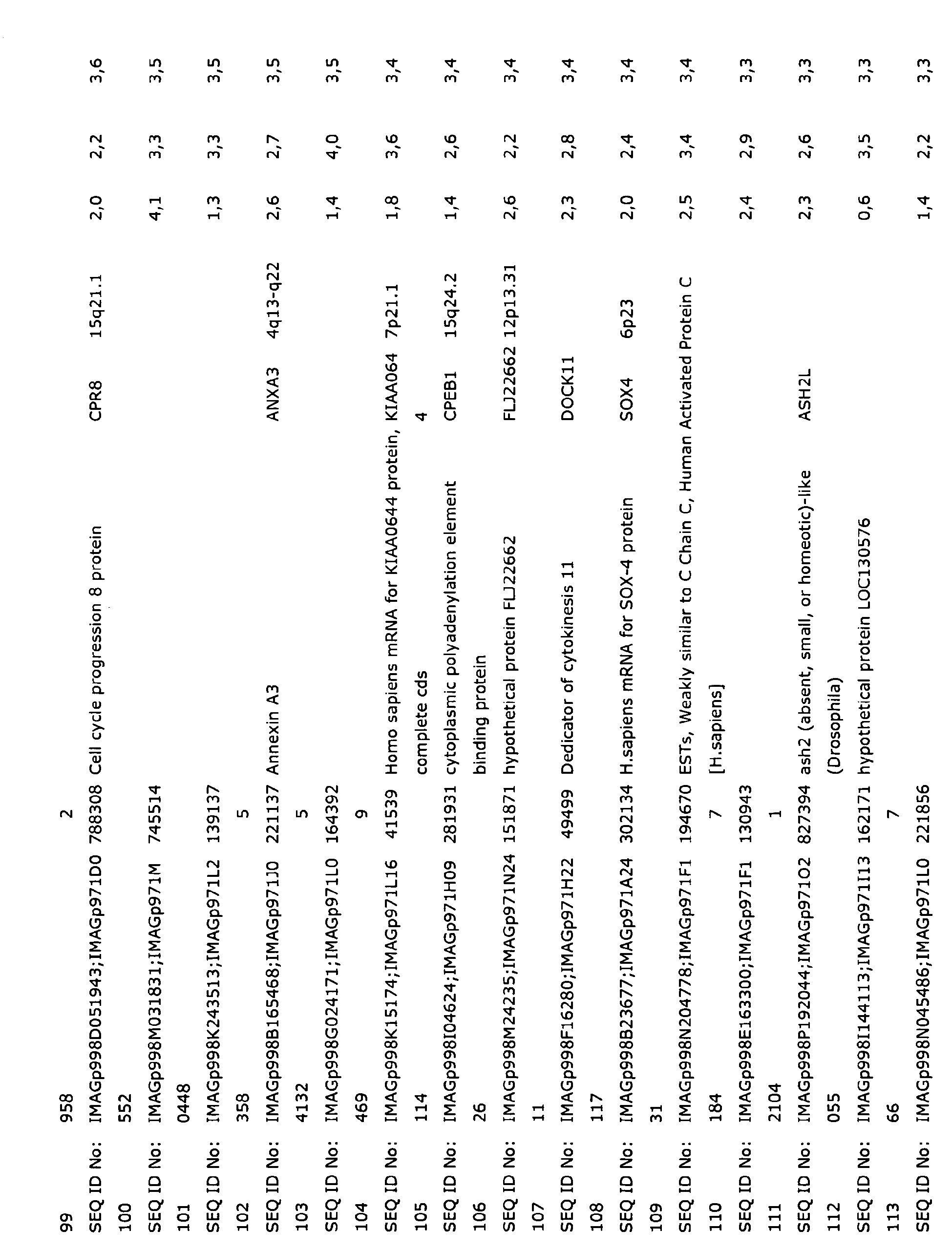

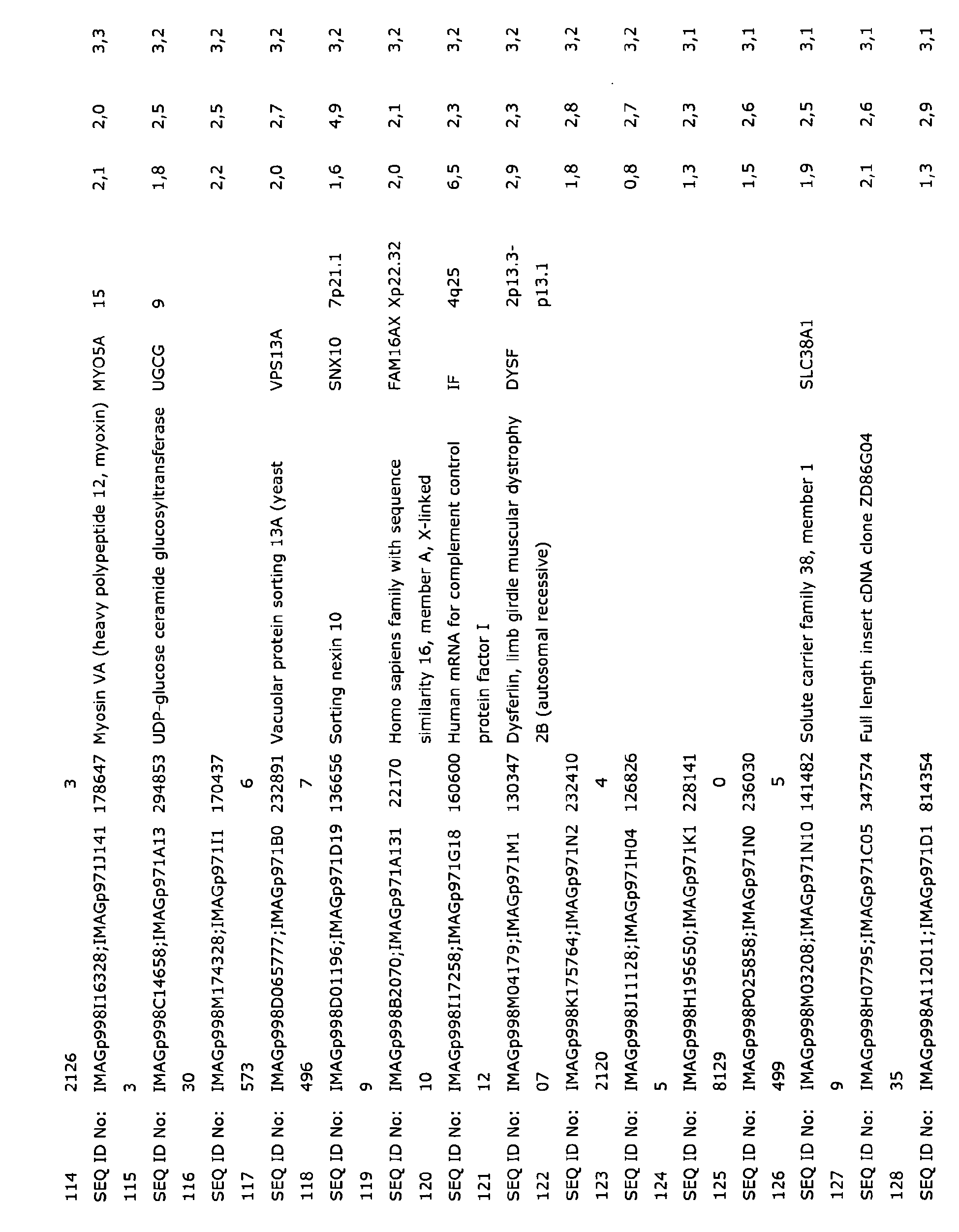

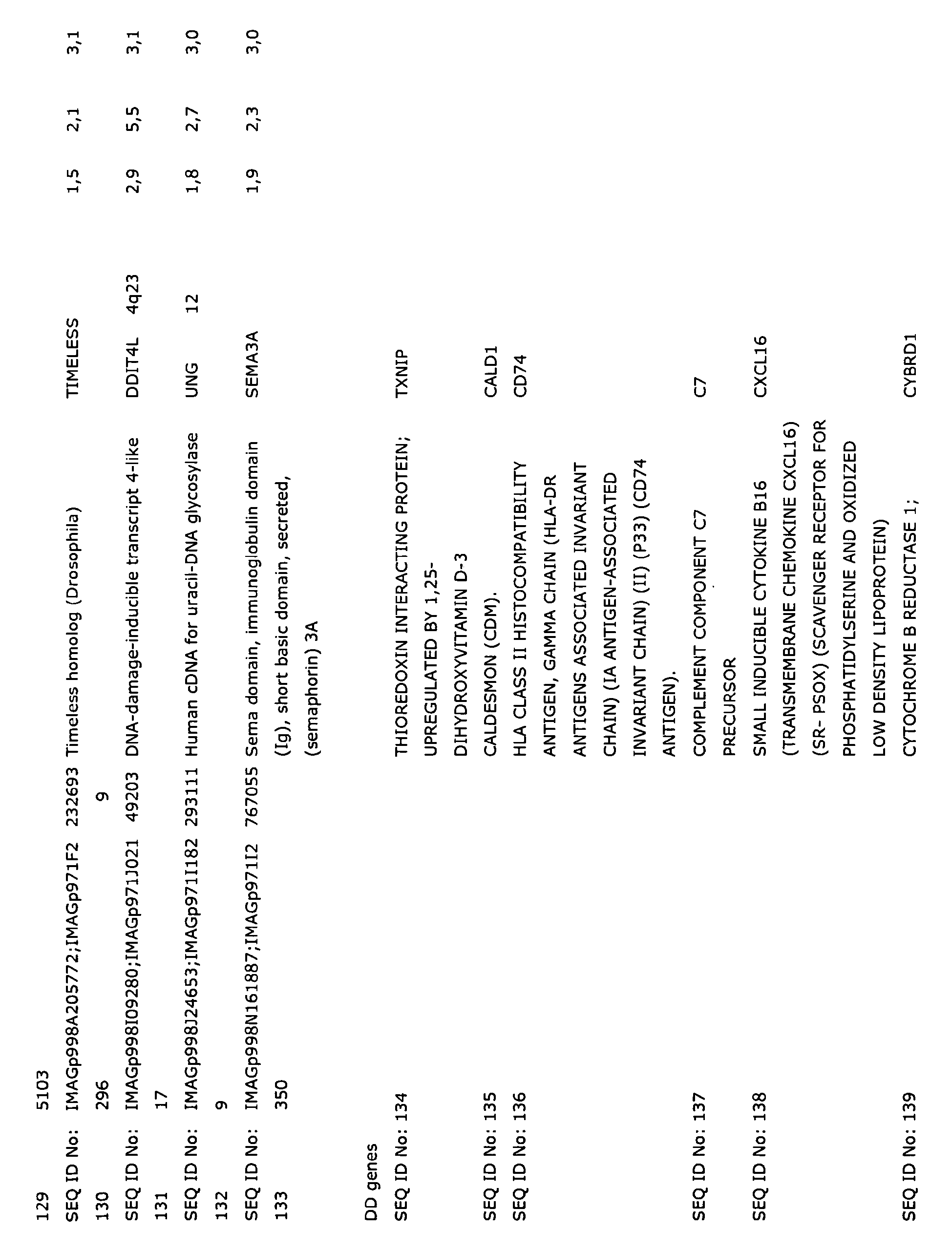

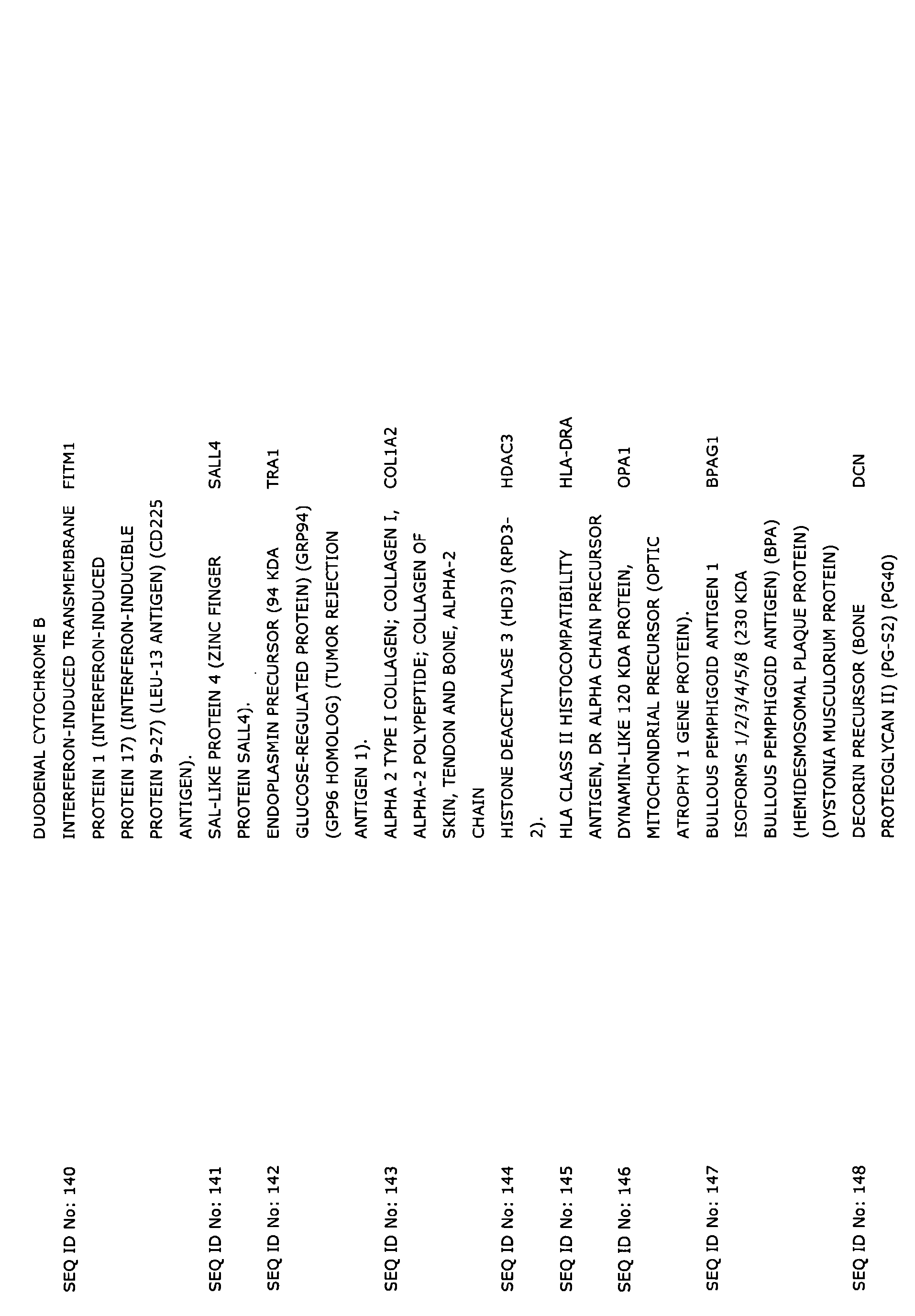

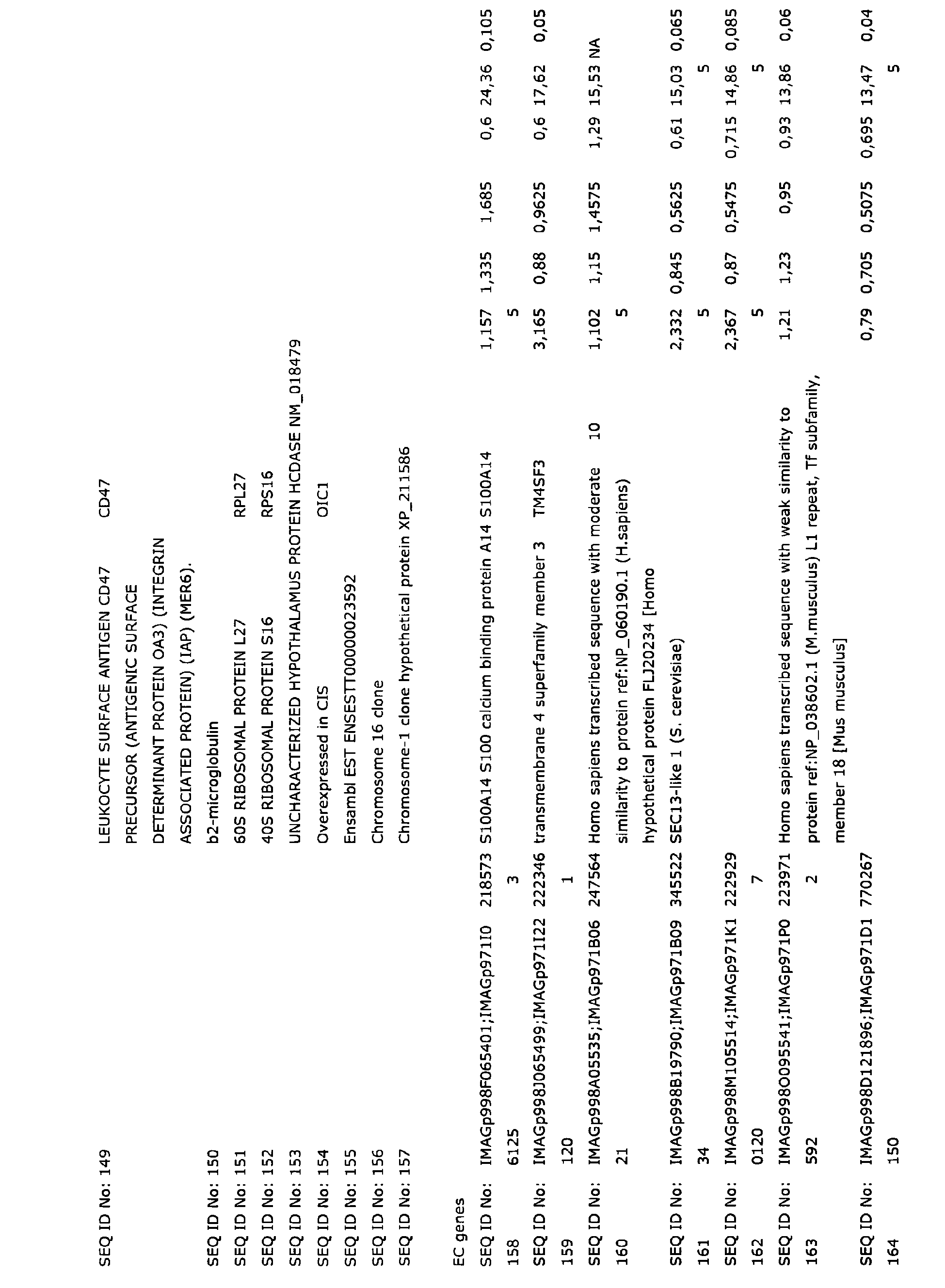

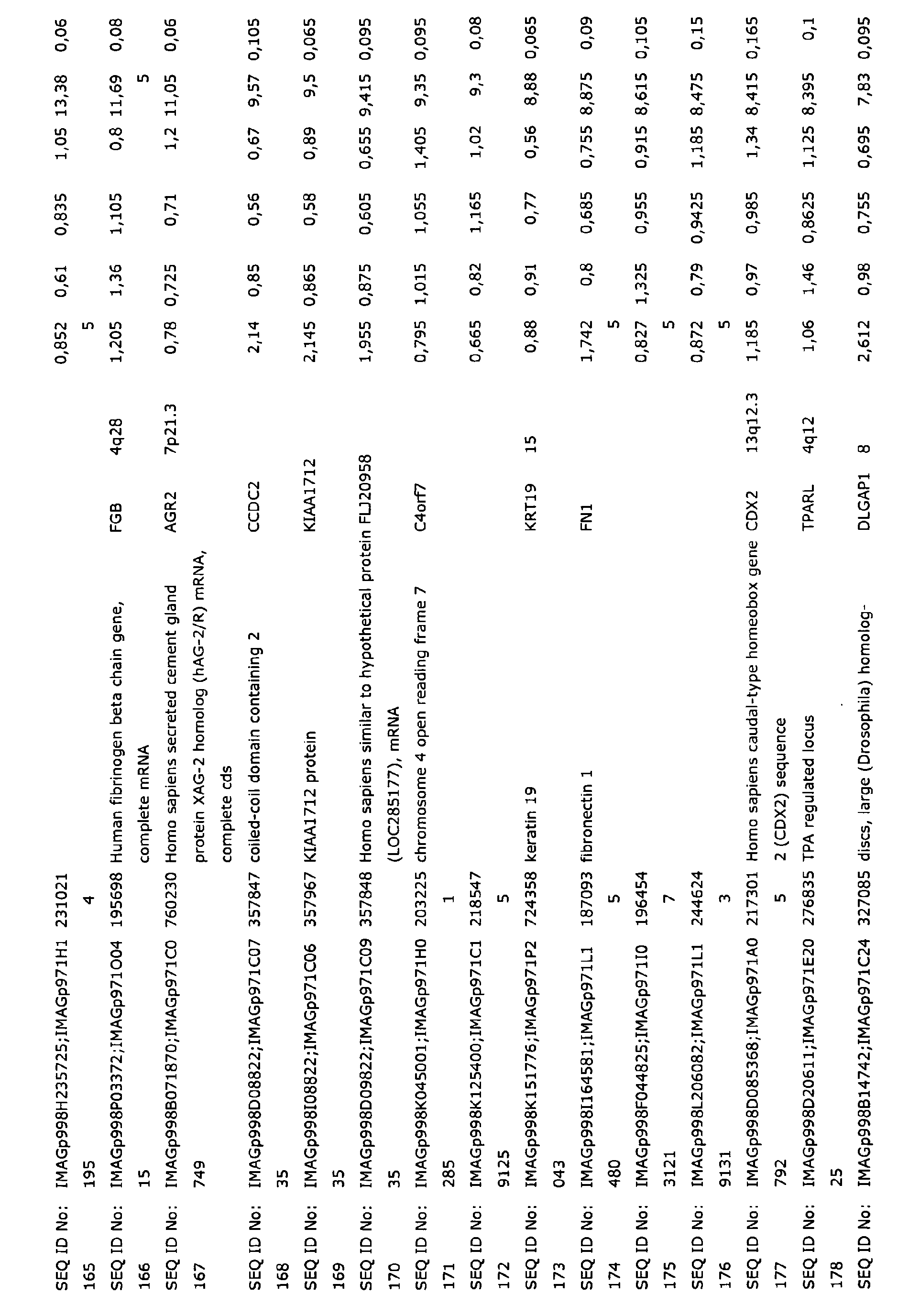

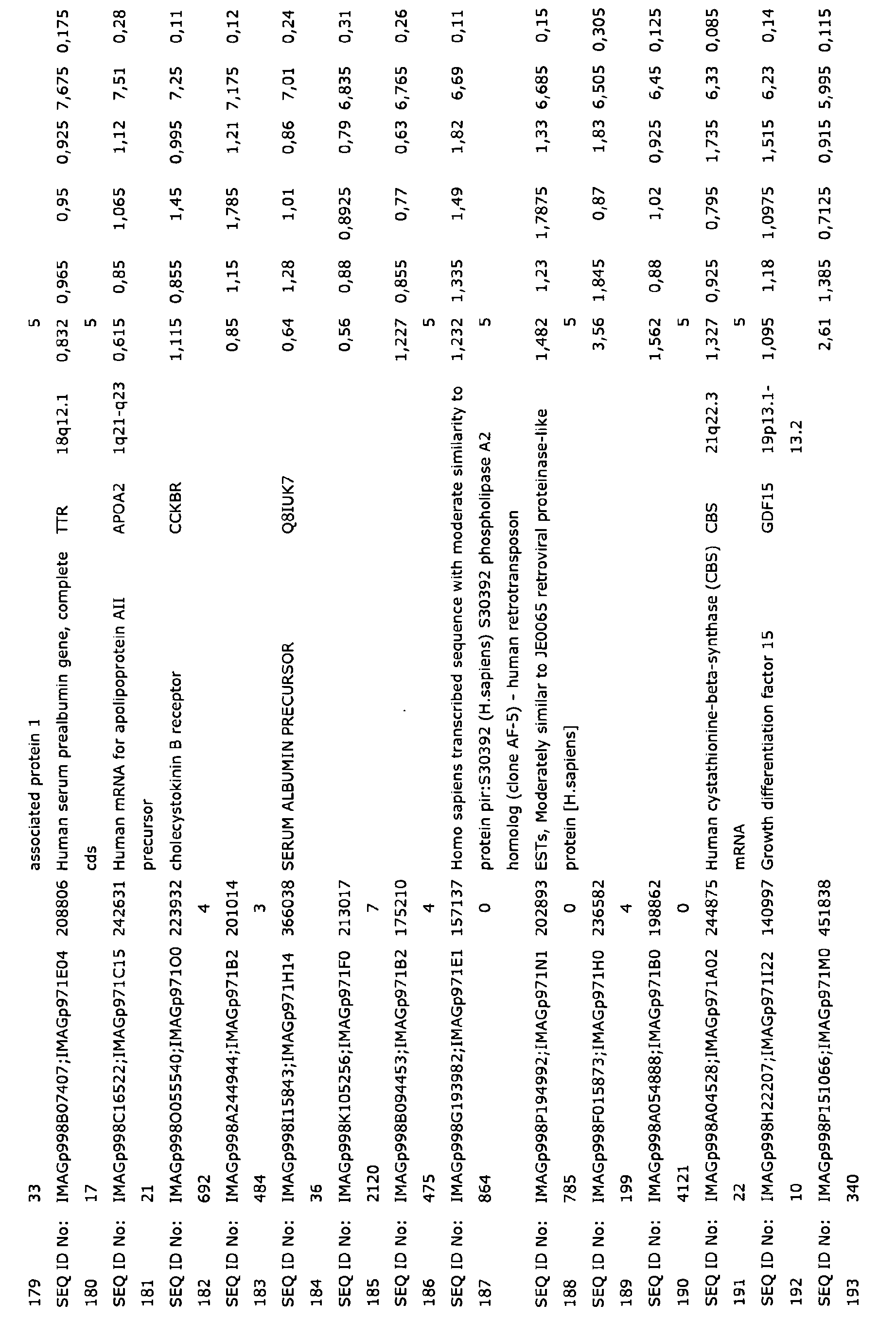

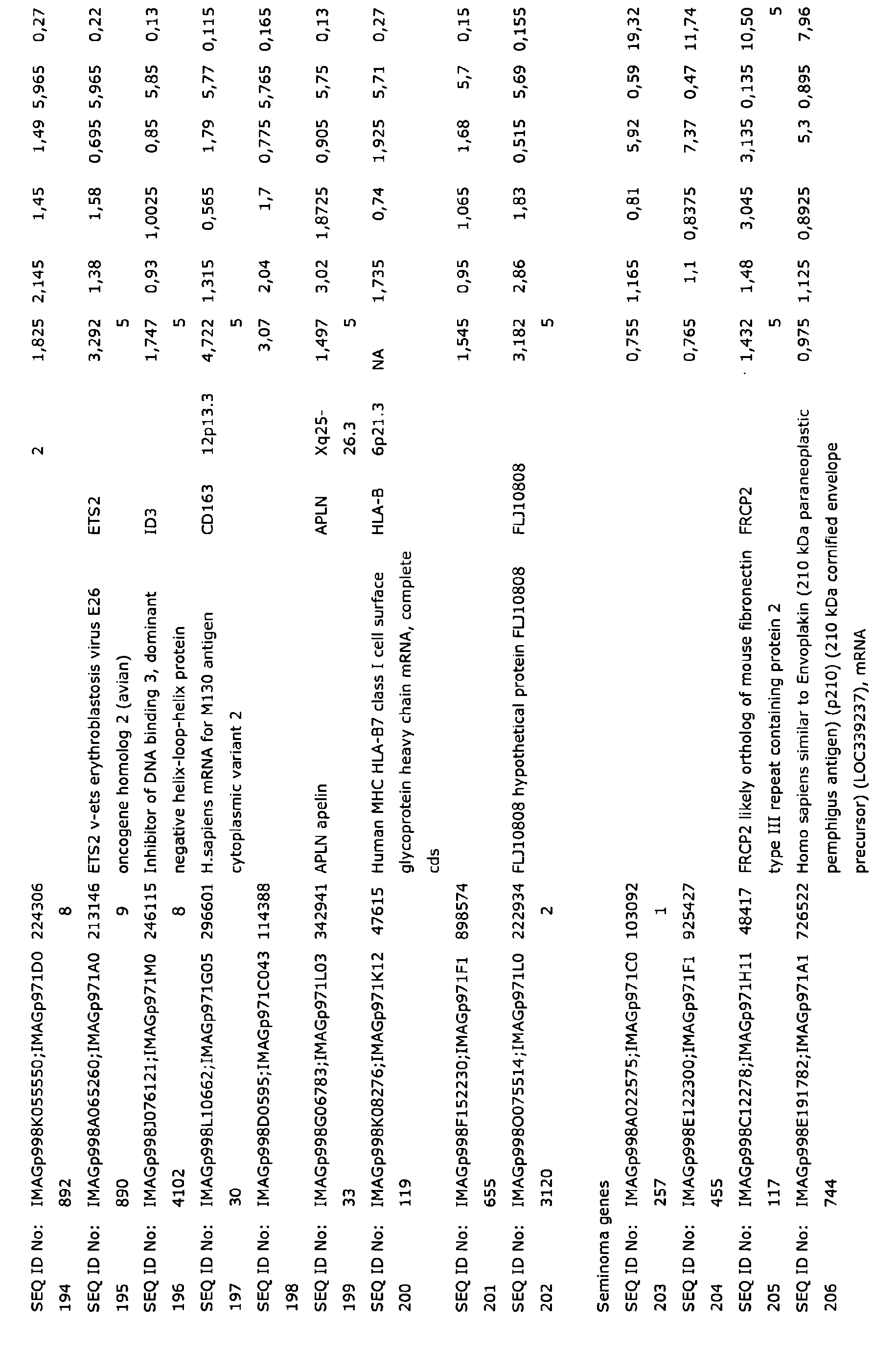

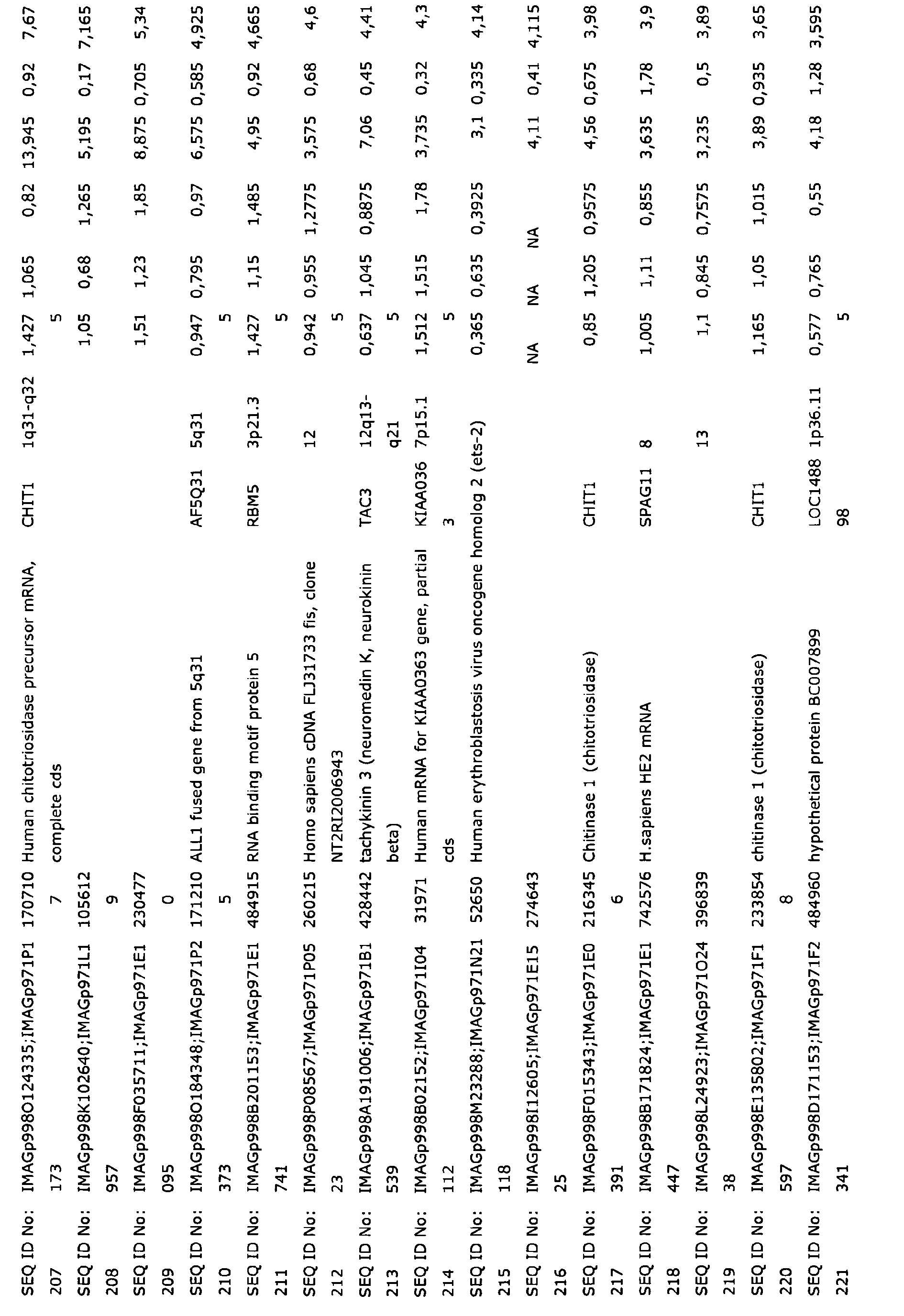

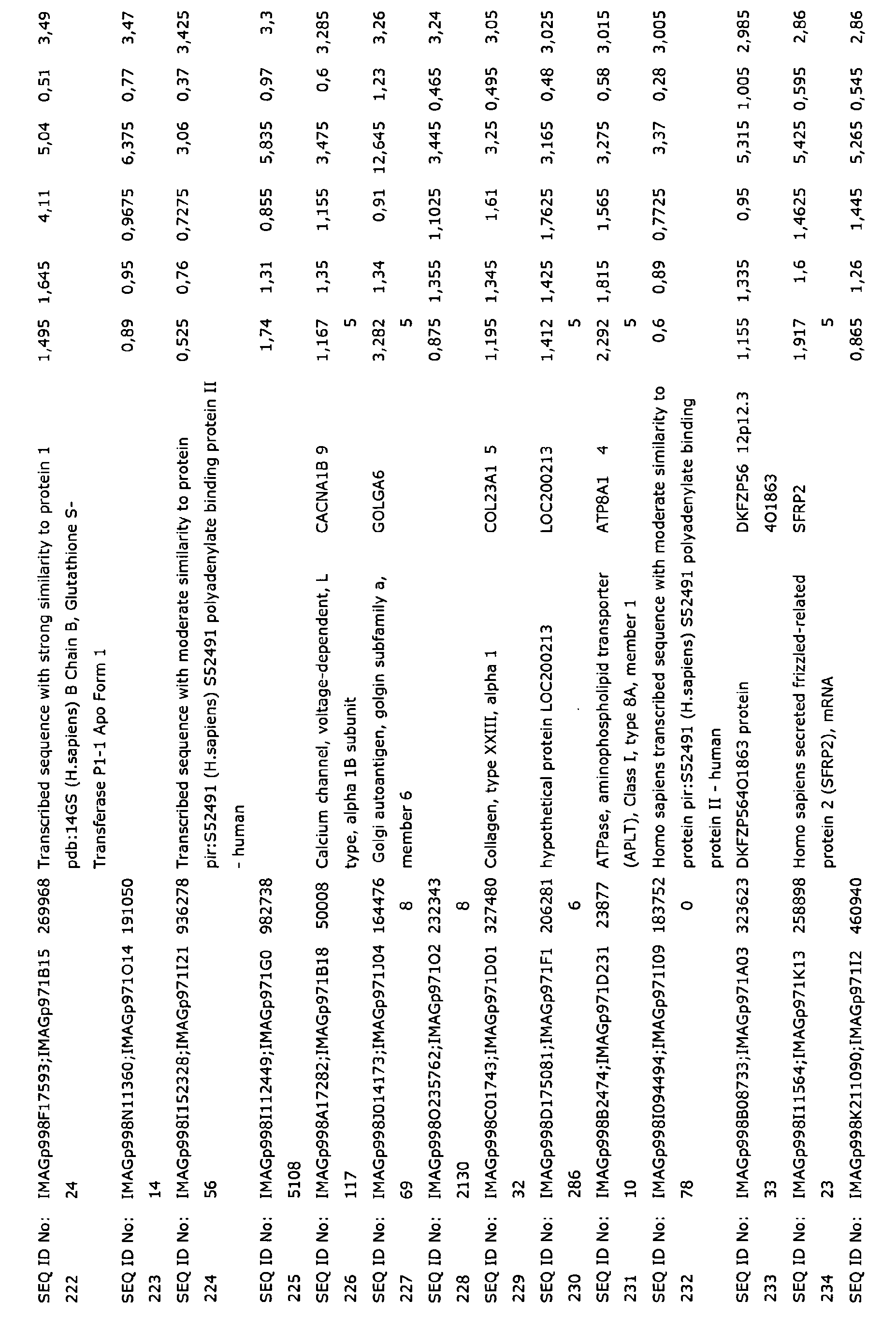

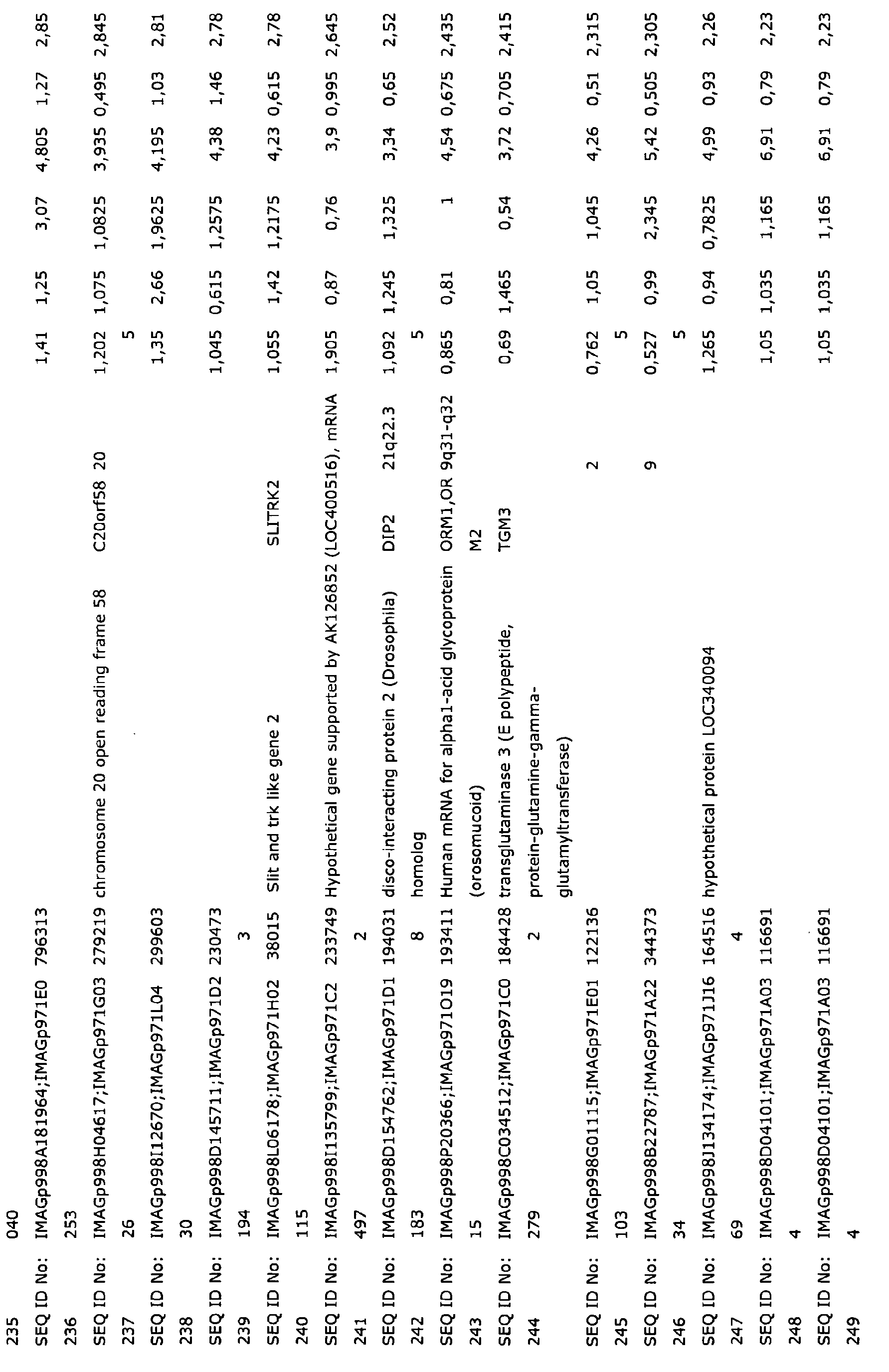

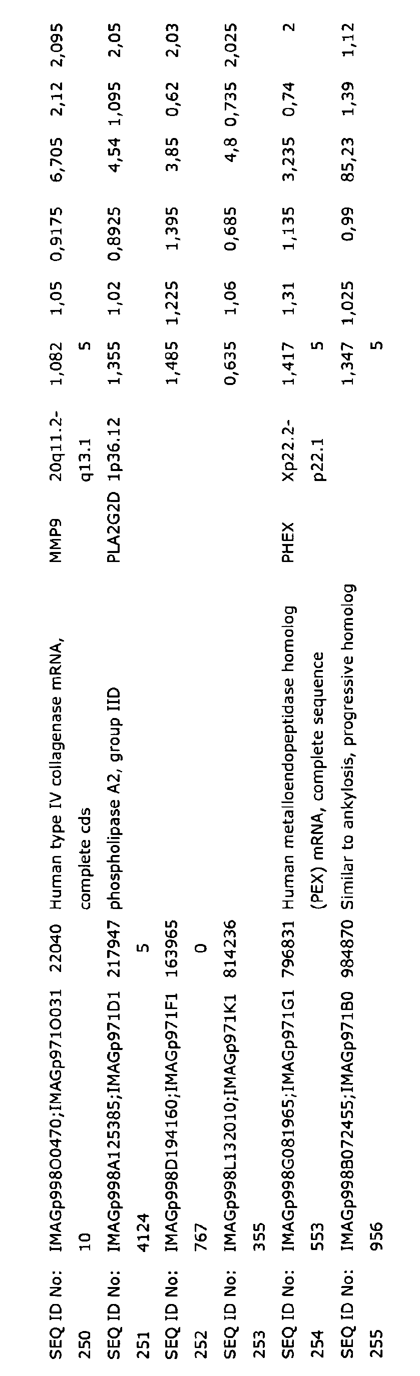

### Table I

This lists features descriptions of all the sequences. In most instances the RZPD (German Resource Center for Genome Research) and IMAGE clone numbers are reported. In addition to that, also the best applicable feature description and gene name as well as chromosomal localization is reported. In the last 6 columns results from the microarray study are reported. 50%, 75%, and 100% CIS reflects analysis on samples with CIS in 50%, 75%, and 100% of the tubules in a given sample. Seminoma reflects a sample with classical seminoma and EC from a sample with embryonal carcinoma. Seminoma/EC reflects an individual experiment with seminoma vs. EC.

### Table II.

Summary and evaluation of the results of the AP-2γ staining of semen samples from the various patient categories. The staining was classified using an arbitrary score 0-5, after a systematical and independent evaluation by two investigators (CHH and ERM), who had no prior knowledge of the diagnosis. The group with testicular neoplasms included patients with the following tumours: 5 seminomas, 4 non-seminomas, 1 mixed GCT and 2 unilateral CIS, which had not proceeded to an invasive tumour, and the subfertile patient diagnosed with CIS in this study. The group of patients with other diseases comprised: 1 benign testis tumour (Leydig adenoma), 1 malignant lymphoma, 2 Hodgkin's disease, 1 glioblastoma, 1 Wegeners granulomatosis, 1 Crohn's disease, 1 bone tumour, 1 torsio testis, 1 pituitary tumour, 1 Klinefelter, 1 XYY male. The healthy controls were recruited among apparently healthy young men attending our department for other projects and 59 subfertile men, of whom 16 had normospermia and 43 had oligospermia. Statistical analysis of data was performed using the Confidence Interval Analysis programme, version 2.0, developed by D.G. Altman, D. Machin, T.N. Bryant and M.J. Gardner.

| | | **Mean age** | **AP-2γ-positive** |
|---|---|---|---|
| **Studied groups** | **N** | | |
| | | | N (%, 95% Cl) |
| | | (years) | |
| Testicular tumour or CIS before operation / irradiation | 13^{#} | 33 | 6^{#} (46.2%, Cl 23-71 %) |
| Testicular cancer after operation, no contralateral CIS | 7 | 32 | 0 (0%, Cl 0-35%) |
| Other diseases | 12 | 29 | 0 (0%, Cl 0-24%) |
| Healthy controls | 14 | 32 | 0 (0%, Cl 0-22%) |
| Subfertile patients with normospermia | 16 | 32 | 0 (0%, Cl 0-19%) |
| Subfertile patients with oligospermia | 43^{#} | 34 | 1^{#} (2.3%, Cl 0.4-12%) |

| | | | |
|---|---|---|---|
| ^{#} including the patient diagnosed with CIS in the course of the study. | | | |

### Table III

Summary of the immunohistochemical staining for AP-2γ. Abbreviations: N=number; wg=weeks of gestation; AIS=androgen insensitivity syndrome; AMH=anti-Müllerian hormone; TDS=testicular dysgenesis syndrome; TGCT=germ cell tumor; CIS=carcinoma *in situ.* Staining intensity: +++: strong staining, ++: moderate staining, +: weak staining, +/-: very weak staining, -: no positive cells detected.

| **Age** | ***N*** | **Histology / Diagnosis** | **AP-2γ** (staining intensity and approximate % of positive cells in a section) | **Remarks** |
|---|---|---|---|---|
| **Normal testes:** | | | | |
| 10-22 wg | 11 | Normal fetal testes | - to ++ (75-100%) | 3 samples negative |
| 23-26 wg | 4 | Normal fetal testes | +/- to + (5-50%) | |
| 27-42 wg | 2 | Normal fetal testes | +1- (< 1 0%) | |
| 0-2½ months | 2 | Normal infantile testes | - to + (25%) | |
| 3-4 months | 5 | Normal infantile testes | +/- (single cells or <10%) | |
| ½-2½ years | 3 | Normal infantile testes | - | |
| 3-11 years | 7 | Normal pre-pubertal testes | - | |
| 13-16 years | 1 | Normal testes with complete spermatogenesis | - | |
| **Normal ovaries:** | | | | |
| 9 wg | 1 | Normal fetal ovary | + (single cells) | Oogonia+ |
| 12-16 wg | 5 | Normal fetal ovaries | +/- to + | Oogonia+, oocytes- |
| 17-26 wg | 7 | Normal fetal ovaries | +/- to ++ | Oogonia+, oocytes- |
| 27-41 wg | 9 | Normal fetal ovaries | + to ++ | Oogonia+, oocytes- |
| Adult | 1 | Normal adult ovary | | |
| **Dysgenetic and intersex gonads:** | | | | |
| 15 wg | 1 | Fetal testis (mosaic isochromosomeY) | - | AMH+, Oct4- |
| 20 wg | 2 | Fetal testes (AIS, 46,XY females) | + (10%) | |
| 4 months | 1 | Infantile testis (AIS, 46,XY females) | + (5%) | |
| 14 months | 1 | Ovotestis (46 XX hermaphrodite) | +/- | Gonocytes positive, oocytes negative |
| 9 months - 6 | 3 | Pre-pubertal testes (AIS, 46 XY females) | + to +++ (2-10%) | Gonocytes with CIS characteristics, |
| years | | | | PLAP+ or -. |
| 2-17 | 6 | Pre-pubertal testes (AIS, 46 XY females) | - | |
| 2½ years | 1 | Infantile testis with decreased number of germ | - | |
| | | cell (idiopathic genital ambiguity) | | |
| 9½ years | 1 | Pre-pubertal testis (Prader-Willy's syndrome) | - | |
| 15 years | 1 | Leydig-cell hyperplasia (adrenogenital | - | |
| | | syndrome) | | |
| 15 years | 1 | 46,XY female, 17βHD mutation | - | |
| 18 years | 1 | Dysgenetic testis with CIS and gonadoblastoma | ++ (90-100%) | |
| | | (45,X/46XY male) | | |
| 25 years | 1 | Klinefelter, bilateral testicular cryptorchidism | - | |
| 20-39 years | 9 | Adult testes with TDS (undifferentiated tubules, | - | |
| | | microliths) but without CIS or TGCT | | |
| 30-48 years | 3 | Adult testes with TDS (undifferentiated tubules, | - | |
| | | microliths) and contralateral TGCT | | |
| **Testicular carcinoma *in situ* and tumour samples:** | | | | |
| Adult | 5 | CIS adjacent to seminomas | + to+++(90-100%) | |
| Adult | 5 | CIS adjacent to non-seminomas | + to+++(90-100%) | |
| Adult | 2 | CIS adjacent to mixed TGCT | ++ (90-100%) | |
| Adult | 2 | CIS without overt TGCT | +++ (90-100%) | |
| Adult | 12 | Classical, homogenous seminomas | + to+++ (90-100%) | |
| Adult | 12 | Non-seminomas | +/-to++ | Various epithelial and stromal |
| | | | | elements positive |
| Adult | 2 | Spermatocytic seminomas | - | |
| Adult / 6 years | 3 | Leydig cell tumors | - to + | Cytoplasmic, background? |
| 14 years | 1 | Gonadoblastoma | +++ | |
| Adult | 1 | Testicular B-cell lymphoma | - | |
| **Other tissue samples:** | | | | |
| Fetal, | 1 of | Liver, heart, thymus, thyroid gland, kidney and | - | |
| 9-20 wg | each | adrenal gland | - | |
| Adult | 11 | Normal breast tissues | + to ++ (90-100%) | Subpopulation of basally situated duct |
| | | | | cells |
| Adult | 47 | Infiltrating ductal breast adenocarcinomas | 7 samples +/- (<10%) | |
| | | | 7 samples + (50%) | |
| | | | 33 samples - | |
| Adult | 1 | Breast mucinous carcinoma | - | |
| Adult | 4 | Breast medullary carcinomas | 2 samples +/- (90-100%) | |
| Adult | 3 | Ductal breast carcinoma in situ | +/- | Single basal cells |
| Adult | 2 | Tubular breast cancers | - | |
| Adult | 1 | Infiltrating nodular breast adenocarcinoma | - | |
| Adult | 4 | Lung | + | Epithelial pneumocytes positive |
| Adult | 7 | Skin | +/- (25%) | Basal cells |
| Adult | 1 | Kidney | +/- (50%) | Single cells in canaliculi, glomeruli- |
| Adult | 1-3 | Skeletal muscle, heart muscle, stomach, | - | |
| | of | esophagus, small intestine, colon, liver, spleen, | | |
| | each | pancreas, salivary gland, pituitary gland, adrenal | | |
| | | gland, thyroid gland, parathyroid gland, thymus | | |
| | | gland, tonsil, epididymis, uterus, cervix, ovarian | | |
| | | serous adenocarcinoma, prostate gland, | | |
| | | prostatic cancer, omentum, peripheral nerve, | | |
| | | cerebral cortex, cerebellum | | |
| **Cell lines** | | | | |
| NT2 cells | Day 0,3,15 of retinoic acid treatment | | -to+ + | In all samples a subpopulation of cells |
| | | | | were stained. |
| 2102Ep | Day 0,1,3,7,10 of retinoic acid treatment | | ++ | In all samples most cells were stained. |
| MCF7 | Exposed to ICI-182,780, ethanol or 1nM | | - to + | In all samples a subpopulation of cells |
| | estradiol for 24 or 48 h. | | | were stained. |

### Examples

### Materials and methods

### Tissue samples

The testicular tissue samples were obtained directly after orchidectomy and macroscopic pathological evaluation. The use of the orchidectomy samples was approved by the Regional Committee for Medical Research Ethics in Denmark. Samples of homogeneous overt tumors (seminoma or EC) and of testicular parenchyma preserved in the vicinity of a tumor were excised. Each testicular sample was divided into several tissue fragments: 2 - 3 fragments were snap-frozen at -80° C for nucleic acid extraction, several adjacent fragments were fixed overnight at 4° C in Stieve's fluid or paraformaldehyde (PFA), and embedded in paraffin. Fixed sections were subsequently stained with haematoxylin and eosin (HE) or with an antibody against PLAP for histological evaluation. Most samples of testicular parenchyma contained variable numbers of tubules with CIS cells. Samples where no tubules with CIS were found and complete spermatogenesis was present, were used as a normal reference. In an attempt to limit the uncertainty about cellular differences between fragments from the same patient, the present inventors also made a direct print of the frozen fragment that specifically was used for RNA isolation. After staining with a PLAP antibody, the present inventors could roughly confirm the approximate proportion of tubules with CIS as established by histology in adjacent tissue fragments. Samples of tissue containing approximately 50%, 75%, and 100% tubules with CIS cells were chosen for microarray analysis along with the above-mentioned samples of overt tumours and the normal testicular tissue (serving as the common reference). Additional samples of teratomas and embryonal carcinomas were also used in the differential display screening and in the RT-PCR.

Labeling of testicular RNA and hybridization to microarrays

Total RNA was purified using the Macherey & Nagel kit, DNase digested, and the RNA quality evaluated using the Agilent Bioanalyzer 2100 (Agilent Technologies Inc., CA, USA), 5 µg of total RNA was linearly amplified using the RiboAmp RNA amplification kit (Arcturus GmbH, Germany) yielding a 400-700 fold amplification of the mRNA. The amplified mRNA was reevaluated on the Bioanalyzer 2100 and 5 µg was then subjected to in-direct labeling with Cy3 and Cy5 (Amersham Biosciences) using the Atlas Glass Fluorescent labeling kit (BD Biosciences Clonetech, CA, USA).

The labeled RNA probes were combined and hybridized at 42° C over night to a 52.000 element cDNA microarray representing the complete Unigene database. The microarray consists of two slides, which are hybridized on top of each other with the labeled probe in-between. Production and handling of the microarray is described elsewhere (http://embl-h3r.embl.de/). Hybridized slides were washed at 42° C for 5 min in 2XSSC, 0.1 % SDS and 10 min in 0.2XSSC, 0.1 % SDS, rinsed, dipped in isopropanol, and dried.

Slides were scanned on a GenePix 4000B scanner (Axon Instruments Inc., Union City, CA, USA) using settings where overall intensity of each channel was equal. The generated images were then analyzed in ChipSkipper (http://chipskipper.embl.de/) and quantified using a histogram segmentation. Flagged spots and controls were taken out and quantified spots were normalized using a framed median ratio centering (frame = 200 genes). The average of dye-swaps was used and spots that did not dye-swap or showed a big standard deviation were sorted out. Data was then imported into software Genesis where hierarchical clustering was done using average linkage and Euclidian distances.

### Differential display competitive PCR screening

Total RNA was prepared using the RNeasy kit from Qiagen as described by the manufacturer (Qiagen, Hilden, Germany). RNA samples were double DNAse digested, first "on-column" and subsequently after the RNA had been purified. cDNA was prepared using one-base-anchored AAGCT11V (V= A, C or G) downstream primers. DDRT-PCR (Liang and Pardee, 1992) was subsequently performed using either one- or two-base-anchored (AAGCT11VN; N = A, C, G or T) primers in combination with different of upstream primers. Bands of interest were cut from the DDRT-PCR gels and reamplified using the up- and downstream primers that displayed the bands, but the downstream primer was extended at the 5'-end with a T7-promotor sequence (taatacgactcactatagggAAGCT11V; T7 promotor sequence in small letters), allowing direct sequencing on ALFexpress sequencers (Amersham-Pharmacia Biotech, Uppsala, Sweden), using a Cy5-labelled T7-promotor complementary primer. The DDRT-PCR method has been described in detail previously (Jorgensen, M, et al. Electrophoresis 1999, 20:230-40).

### Verification by RT-PCR

The expression of a selected set of the regulated genes identified in the microarray analysis was verified with RT-PCR as described previously. In brief, cDNA was synthesized using a dT24 primer. Specific primers targeting each mRNA were designed spanning intron-exon areas to avoid amplification of genomic DNA. PCR was performed in 30 µl of (final concentrations): 12 mM Tris-HCl, pH 8.3; 50 mM KCl; 1.9 mM MgCl2; 0.1 % Triton X-100; 0.005 % Gelatine; 250 µM dNTP; and 30 pmol of each primer. H2O was used as a negative control and β-actin was used as an internal control in all PCR reactions, resulting in an approximately 800 base pair actin fragment. Cycle conditions: 1 cycle of 2 min. at 95° C; 30 - 40 cycles (depending on the intensity of bands) of: 30 sec. at 95° C, 1 min. at 62° C, 1 min. at 72° C and finally 1 cycle of 5 min. at 72° C. PCR products were resolved on 1.5 % agarose gels and visualized by ethidium bromide staining. Digital images of the agarose gels were quantified by the STORM phosphor imager software (ImageQuant, Amersham Biosciences). In a few of the RT-PCR analyses of less abundant transcripts, no bands were detectable after PCR and nested primers were designed. One µl from the first PCR reaction was transferred to a new reaction containing the nested primers and analyzed as above.

### Preparation of biotin-labeled probes and in situ hybridization

In situ hybridization was performed to confirm that the identified transcripts were indeed expressed in CIS cells. Probes for in situ hybridization were prepared by re-amplification of the PCR products using nested primers specific to the individual products and with an added T7- or T3-promotor sequence. PCR conditions were: 5 min. 95° C; 5 cycles of: 30 sec. 95° C, 1 min. 45° C, 1 min. 72° C; 20 cycles of 30 sec. 95° C, 1 min. 65° C, 1 min. 72° C and finally 5 min. 72° C. The resulting PCR product was purified on a 2 % low melting point agarose gel and sequenced from both ends, using Cy5-labelled primers complementary to the added T3 and T7 tags. Aliquots of about 200 ng were used for in vitro transcription labeling, using the MEGAscript-T3 (sense) or MEGAscript-T7 (anti-sense) kits, as described by the manufacturer (Ambion, Houston, TX, USA). The composition of the 10X-nucleotide mix was: 7.5 mM ATP, GTP and CTP, 3.75 mM UTP, 1.5 mM biotin-labeled UTP. To estimate quantity and labeling efficiencies, aliquots of the labeled RNA product were analyzed by agarose gel electrophoresis, and dotted onto nitrocellulose filters and developed as described below.

In situ hybridization was performed essentially as described previously (Nielsen, JE, et al. Eur J Histochem 2003, 47:215-22), except for one additional step needed to remove mercury from sections fixed in Stieve fixative. After deparaffination, mercury was removed by 15 min. treatment with potassium iodide (KI/I ratio 2/1), followed by three washes in DEPC water. The iodine was subsequently removed by incubation in sodium thiosulfate pentahydrate (5 % w/v, 10 min.) followed by washing (4X DEPC water). Subsequently, the procedure was, in brief, as follows: sections were re-fixed in 4 % PFA, treated with proteinase K (Sigma, St. Louis MO USA) (1.0 - 5.0 µg/ml), post-fixed in PFA, pre-hybridized 1 h at 50° C, and hybridized over night at 50° C with biotinylated antisense and - sense control probes. Excess probe was removed with 0,1X SSC (60° C) 3X33 min. Visualization was performed with streptavidin conjugated with alkaline phosphatase (1:1000) (Roche Diagnostics GmbH, Mannheim, Germany) followed by a development with BCIP / NBT (Nielsen, JE, et al. Eur J Histochem 2003, 47:215-22).

### Example 1

### Genes identified by microarray

By analyzing testicular samples containing increasing amounts of tubules with CIS the present inventors identified differentially expressed genes as compared to the normal reference with complete spermatogenesis and no CIS cells present.

From normalized scatter plots of gene expression in CIS-containing-tissue versus normal testis tissue, the present inventors observed that approximately 200-300 genes were significantly up-regulated in CIS-containing tissues. Data was filtered so that only genes that were up-regulated in the CIS samples and had low standard deviation were considered for further analysis. The up-regulated genes were a heterogeneous group including transcription factors, such as TFAP2C (ERF-1), LBP-9, TEAD4 (RTEF-1), NFE2L3 (NRF3) and POU5F1, solute carriers e.g. SLC25A16, SLC7A3, oncogenes e.g. RAB-15, TCLLB (TML1), MYBL2 (B-MYB), PIM2 and many more genes, which are listed in Table I Some of these genes were previously described as CIS markers, e.g. KIT (c-KIT), POV1 (PB39), MYCN (N-MYC) and POU5F1 (OCT-3/4), but the majority were novel, not previously characterized genes.

A hierarchical cluster analysis with average linkage of the top most up-regulated genes (minimum 4 fold increase in the sample with CIS in 100 % of the tubules and more than 2 fold in the other samples) is shown in figure 1. For most of the genes, a gradual increase in expression was observed with increase in the percent of tubules with CIS. This strongly indicates that expression of these genes is linked to the CIS phenotype and indeed to the actual amount of tubules with CIS in the testis. To verify that, the present inventors re-analyzed the expression of selected genes by RT-PCR, in a panel of samples covering a broad spectrum of different testicular neoplasms and normal testicular tissue (fig. 2A). We also investigated the expression of the non-tissue specific alkaline phosphatase (ALPL) simultaneously with the previously known CIS marker PLAP and found that they were expressed in a similar manner (fig. 2A).

Both the microarray and the RT-PCR analysis indicated that the observed up-regulation of genes in the samples with CIS were linked to the CIS phenotype and thus to CIS cells. However, it cannot be excluded that the high expression of some genes (e.g. SIAT1, TCL1A, TCL1B), may be due to the presence of infiltrating lymphocytes, which are commonly seen in patients with CIS, but not present in the normal testis (Jahnukainen, K, et al. Int J Androl 1995, 18:313-20). In addition, some transcripts up-regulated in the tissues with CIS may originate from immature Sertoli cells, which are sometimes present in testes with CIS, but such dysgenetic changes are relatively rare, so this possibility is less likely.

To confirm the cellular localization, the present inventors performed in situ hybridization for some of the identified genes on tissue sections with CIS. All transcripts tested (NANOG, ALPL, TFAP2C, and SLC7A3) were localized to CIS cells. The present inventors also observed in some cases variable staining of Sertoli cell nuclei, which was most probably unspecific, since similar, but lighter staining was observed for the control sense probe. However, in all cases, the strongest signal was from CIS cells.

### Example 2

### Genes identified by Differential Display

To obtain knowledge on gene expression changes between the different stages in the development from non-malignant testicular tissue through CIS to the malignant stage of a germ cell cancer, the present inventors performed a systematic comparison by the DDRT-PCR method (Jorgensen, M, et al. Electrophoresis 1999, 20:230-40). For the initial screening the present inventors analysed six testicular biopsy samples: two with homogenous classical seminoma, two containing almost exclusively CIS tubules, of which one was from an intraabdominal CIS and two with normal spermatogenesis. The undescended testis with CIS was of our interest, because in the hypothesis of Testicular Dysgenesis Syndrome, both testicular cancer and undescended testes are symptoms of maldeveloped genitalia.

We performed 402 primer combinations, and assuming that each competitive PCR reaction gives rise to approximately 100 bands on a DDRT gel and that each band corresponds to one mRNA, this investigation allowed comparison of about 40.000 mRNAs. We excised and sequenced 56 bands, which showed overexpression (ranging from 1-10 fold) in at least one of the CIS samples. Several mRNAs were detected with more than one primer combination; for instance, overexpression of CCND2 was detected with three different primer combinations. The RT-PCR analysis verified overexpression of 24 known genes, 4 transcripts corresponding to ESTs and 1 sequence not annotated as a gene.

### Example 3

### Antibodies against the genes

Several vendors of commercial antibodies were searched for antibodies to the proteins encoded by the identified genes highly expressed in testicular CIS. In addition, new antibodies against protein product of the newly identified CIS markers genes are going to be generated.

### Example 4

### Identification of markers for CIS progression into seminoma or non-seminoma

Besides identification of highly specific CIS markers the present inventors also identified markers for the progression of CIS either into seminoma or the non-seminoma. Currently it is not known why some CIS cells give rise to a seminoma and others non-seminomas. It is however speculated that CIS may be predisposed to the one or the other. By identification of the markers for CIS progression together with the CIS markers, the present inventors will be able to: 1) identify CIS cells in a subject 2) predict whether the subject is predisposed to either a seminoma or a non-seminoma, or may develop a combined tumour 3) evaluate to what degree the CIS in the subject has evolved into a seminoma or a non-seminoma 4) monitor during a possible subsequent treatment whether or not all CIS cells or derived tumours have been successfully eradicated.

### Example 5

### Kit development

A diagnostic kit for screening for a panel of the marker genes can be provided. Such a kit may be in different forms, depending whether the gene expression in a sample will be screened at the RNA or at the protein level. The most obvious form would be a kit based on reverse transcriptase polymerase chain reaction (RT-PCR). By picking out a battery of the most promising CIS marker genes, RNA purified from a human sample can be tested for the presence of CIS marker genes at the transcript level. The present inventors can also take advantage of the high throughput microarray technique for assaying the expression of hundreds of genes simultaneously. This could be in the format of a microarray focused on genes identified here as CIS markers. Additional microarrays e.g. containing markers for the progression into seminoma or nonseminoma can also be developed.

At the protein level, the present inventors can also develop two approaches: either select a limited number of best markers and make an ELISA-based assay using robust and well validated specific antibodies. The present inventors can also develop a protein-microarray using a large number of-antibodies against a broader panel of markers, including those previously known, spotted on a glass slide.

### Example 6

### New nuclear markers

One very important finding from the study of CIS markers at the protein level was to find proteins localized to the nucleus of the CIS cells. This is important because CIS cells shed to seminal fluid are exposed to different pH and high concentrations of proteases that may damage the cell membrane (and the proteins located within, e.g. receptors) before the ejaculation takes place. A nuclear marker will be better retained than a cell surface marker and thus will increase the possibility for detection in semen samples.

### Example 7

### Human samples for detection of CIS

The most convenient and least invasive biological sample to detect CIS cells will be semen (ejaculate). In our earlier studies the present inventors established that some CIS cells are present in semen samples in patients with CIS or overt germ cell tumours in the testes. However, in some cases of early invasion/metastasis, it may also be possible to detect CIS/early tumour cells in a blood sample. Testicular tissue in the form of i.e. biopsies, is nowadays used to detect CIS, and the novel markers can be used to confirm the results of e.g. a CIS array screen, before the final treatment (e.g. orchidectomy) takes place. Yet, other human samples e.g. urine may perhaps be used in the future, e.g. in very young boys, but as only single cells are expected to be detected in urine, careful testing and validation has to be performed.

### Example 8

### CIS cells are embryonic stem cell-like

Among the genes highly expressed in CIS, the present inventors noticed a range of genes known to be expressed primarily in embryonic stem cells (ESC) and/or primordial germ cells (PGC), such as POU5F1 DPPA4, DPPA5, and NANOG. The present inventors therefore performed a systematic comparison of genes highly expressed in CIS with genes recently reported in the literature to be highly expressed in both human and mouse ESC (Ramalho-Santos, M, et al. Science 2002, 298:597-600;Sato, N, et al. Dev Biol 2003, 260:404-13;Sperger, JM, et al. Proc Natl Acad Sci U S A 2003, 100:13350-5). As shown in figure 4, many of the genes found in ESC were also highly expressed in our CIS samples. Among the 100 highest expressed CIS marker genes (listed in figure 1), 34 genes were reported in ESC. The overlap might even be greater, as many of the genes included on the array are yet uncharacterized and thus may be differently annotated on the microarrays used to expression profile ESC. Excluding genes with no functional description (gene name) leads to an approximately 47% overlap. Furthermore, studies where a relative level of expression in ESC cell compared to a reference was reported (Ramalho-Santos, M, et al. Science 2002, 298:597-600;Sperger, JM, et al. Proc Natl Acad Sci U S A 2003, 100:13350-5), the ranking of the expression level seemed to correlate between CIS and ESC, though with some exceptions. The studies the present inventors compared with, in addition all have used different reference samples to explore ESC specific genes and therefore not always agree on the expression in ESC (fig. 4).

Furthermore, the chromosomal distribution of the over-expressed genes in CIS may be associated with a non-random genomic amplification in certain "hot-spot" areas. The present inventors therefore created a map of the chromosomal distribution of the genes highly expressed in CIS. Searching for the chromosomal localization of these genes and normalizing with the length of each chromosome revealed that there was an over-representation of up-regulated genes on chromosomes 7, 12, 14, 15, and especially chromosome 17 (fig. 5) Plotting the density of up-regulated genes in each chromosomal band allowed us to narrow down hot-spots to smaller regions, with 17q as the most pronounced region. Recurrent genomic gain of chromosomes 17q and 12 was recently discovered in cultured human ESCs (Draper, JS, et al. Nat Biotechnol 2004, 22:53-4;Pera, MF Nat Biotechnol 2004, 22:42-3). It was postulated that cultured ESC might gain extra chromosome material in these areas after freezing and thawing, which could inhibit apoptosis and increase their self-renewal in culture etc. (Draper, JS, et al. Nat Biotechnol 2004, 22:53-4). This again indicates that CIS cells may have strong similarities to embryonic stem cells and the identified CIS markers may thus be used to identify transplanted embryonic stem cells how have evolved into CIS cells leading to uncontrolled growth of the embryonic stem cells.

### Example 9

Transcription factor AP-2γ is a developmentally regulated marker of testicular carcinoma *in situ* and germ cell tumors

### Purpose

Transcription Factor Activator Protein-2γ (*TFAP2C,* AP-2γ) was previously reported in extraembryonic ectoderm and breast carcinomas, but not in the testis, the above gene expression study we detected AP-2γ in carcinoma *in situ* testis (CIS, or intratubular germ cell neoplasia, IGCN), precursor of testicular germ cell tumors (TGCT). In this example we aimed to investigate the expression pattern of AP-2γ and to shed light on this factor in germ cell differentiation and the pathogenesis of germ cell neoplasia.

### Experimental Design

We analyzed expression pattern of AP-2γ at the RNA and protein level in normal human tissues and a panel of tumors and tumor-derived cell lines. In the gonads, we established the ontogeny of expression of AP-2γ in normal and dysgenetic samples. We also investigated the regulation of AP-2γ by steroids and retinoic acid.

### Results

We detected abundant AP-2γ in testicular CIS and in TGCTs of young adults, and confirmed differential expression of AP-2γ in somatic tumors. We found that AP-2γ expression was regulated by retinoic acid in an embryonal carcinoma cell-line (NT2). The investigation of ontogeny of AP-2γ protein expression in fetal gonads revealed that it was confined to oogonia/gonocytes and was down-regulated with germ cell differentiation. In some pre-pubertal intersex cases, AP-2γ was detected outside of the normal window of expression, probably marking neoplastic transformation of germ cells.

### Conclusions

AP-2γ is developmentally regulated and associated with the undifferentiated phenotype in germ cells. This transcription factor may be involved in self-renewal and survival of immature germ cells and tissue-specific stem cells. AP-2γ is a novel marker of testicular CIS and CIS-derived tumors.

The aim of this study was to explore the possible role of AP-2γ in the pathogenesis of reproductive tumors, especially in the testis, and to further investigate the timing of neoplastic transformation of germ cells. To that end, we determined a comprehensive expression profile of AP-2γ in a large panel of normal tissues, tumors and tumor-derived cell lines, established the ontogeny of AP-2γ in normal and dysgenetic human gonads and studied regulation of the AP-2γ expression in selected cell lines.

### MATERIALS AND METHODS

### Tissue samples

The Regional Committee for Medical Research Ethics in Denmark approved the use of human tissue samples for the studies of novel genes expressed in germ cell cancer.

The tissue samples from adults with testicular neoplasms were obtained directly after orchidectomy and macroscopic pathological evaluation. Each testicular sample was divided into several tissue fragments, which were either snap-frozen at -80°C for nucleic acid extraction, or fixed overnight at 4°C in Stieve's fluid, buffered formalin or paraformaldehyde (PFA), and subsequently embedded in paraffin.

Tissue sections were stained with haematoxylin and eosin (HE) or with antibodies against placental alkaline phosphatase (PLAP or ALPP) for histological evaluation (Giwercman, A, et al. APMIS 1991, 99:586-94).

A series of 31 of overt testicular tumors were analyzed by immunohistochemistry, including classical seminomas, various non-seminomatous tumor components, spermatocytic seminomas, Leydig cell tumors and a testicular B-cell lymphoma (listed in Table III). Moreover 14 samples of testicular CIS were analyzed: twelve, which were from tissue adjacent to a TGCT, and two, which had not progressed to an overt tumor, but only had signs of micro-invasion. Furthermore, we included 8 samples containing normal testicular tissue from either patients with prostate cancer or from tissue surrounding a GCT. A representative panel of tissues adjacent to the microscopically examined samples (seminomas, nonseminomatous tumors and specimens with CIS) was analyzed by RT-PCR and ISH. RNAs for RT-PCR were isolated from homogeneous tumors and from specimens containing the largest possible number of tubules with CIS, as judged by microscopical examination of adjacent tissue fragments.

The series for immunohistochemical analysis of the ontogeny of expression of AP-2γ in normal and dysgenetic gonads included paraffin embedded specimens from tissue archives of the Rigshospitalet, Copenhagen University Hospital. The 40 normal fetal tissue samples (19 testicular and 21 ovarian specimens) were obtained after induced or spontaneous abortions and stillbirths, mainly due to placental or maternal problems. The developmental age was calculated from the date of the last menstrual bleeding, supported by the foot size of the fetus. Normal postnatal testicular samples (N=16) were obtained either from autopsies of infants who died suddenly of causes unrelated to the reproductive system or as testicular biopsies performed in boys with acute leukemia for monitoring the spread of disease. Pathology specimens comprised a series of 20 overtly dysgenetic gonads from individuals with intersex disorders or other components of the testicular dysgenesis syndrome (TDS)(Skakkebaek, NE, et al. Hum Reprod 2001, 16:972-8), and 12 testicular biopsies with mild dysgenetic features (e.g. undifferentiated tubules and/or microliths) with or without CIS, performed for diagnostic reasons from young adult men with sub-fertility or with contralateral germ cell tumors.

In addition 130 samples from extragonadal tissues were examined. Panels of normal tissues and mammary gland tissues with or without neoplastic lesions were investigated using commercial tissue arrays (MaxArray Human Breast Carcinoma and Human Normal Tissue Microarray slides, Zymed, S. San Francisco, CA, USA). All specimens are listed in Table III.

### Studies in cell lines

The following established cancer cell lines were grown in standard conditions (5% CO₂, 37°C): embryonal carcinoma NT2 and 2102EP lines, and a mammary tumor-derived MCF7 line. Total RNA was isolated for RT-PCR as described below, while for the immunohistochemical staining of the protein, the cells were spun in a cyto-centrifuge or directly grown on microscopic slides.

For analysis of the effect of retinoic acid (RA) on the AP-2γ expression (Andrews, PW Dev Biol 1984, 103:285-93), NT2 cells were grown in DMEM medium (10% FBS, 2 mM L-glutamine, 25 IU/ml penicillin, 25 µg/ml streptomycin) and stimulated with 10 µM RA (Sigma-Aldrich, St. Louis, MO) for 0-15 days to induce differentiation; 2102Ep cells were grown in DMEM medium with added 100 µM β-mercaptoethanol, and stimulated with 10 µM RA for 0-10 days. A possible estrogen regulation of AP-2γ was analyzed in MCF7 cells, which were grown in steroid-depleted DMEM medium (5% dextran-charcoal stripped FBS, 1 x non-essential amino acids, 1 nM insulin, 2 mM L-glutamine, 25 IU/ml penicillin, 25 µg/ml streptomycin) for 6 days and then exposed to 1 nM estradiol, ICI-182,780 (anti-estrogen), or ethanol (vehicle) for 24 or 48 hours.

### RT-PCR

Total RNA was purified using the NucleoSpin RNAII kit as described by the manufacturer (Macherey-Nagel, Düren, Germany). RNA samples were DNAse digested, and cDNA was synthesized using a dT₂₀ primer. Specific primers were designed for *TFAP2C* (TFAP2C-ex6: ATCTTGGAGGACGAAATGAGAT, TFAP2C-ex7: CAGATGGCCTGGCTGCCAA), spanning intron-exon boundaries. RT-PCR was performed in duplex in 30 µl of (final concentrations): 12 mM Tris-HCl, pH 8.3; 50 mM KCI; 1.9 mM MgCl2; 0.1 % Triton X-100; 0.005 % gelatine; 250 µM dNTP; 30 pmol of each primer. For control of PCR load and cDNA synthesis the expression of the marker gene *ACTB* was analyzed with the following primers (ACTB-ex4: ACCCACACTGTGCCCATCTA, ACTB-ex6: ATCAAAGTCCTCGGCCACATT). Cycle conditions for all PCR reactions: 1 cycle of 2 min. at 95°C; 40 cycles of: 30 sec. at 95°C, 1 min. at 62°C, 1 min. at 72°C and finally 1 cycle of 5 min. at 72°C. PCR products were run on 1.5% agarose gels and visualized by ethidium bromide staining. Fragment size was 205 base pairs for *TFAP2C* and 815 for *ACTB.* Representative bands were excised, cloned (with TOPO®Cloning Invitrogen, Carlsbad, CA, USA) and sequenced for verification.

### In situ hybridization (ISH)

Probes for ISH were prepared by use of specific primers (1^{st} primer combination: AAGAGTTTGTTACCTACCTTACT, CATCAATTTGACATTTCAATGGC, 2^{nd} primer combination AATTAACCCTCACTAAAGGGTTAAAGAGCCTTCACT, TAATACGACTCACTATAGGGCTAAGTGTGTGG) containing an added T3- or T7-promotor sequence, respectively (promotor sequences underlined). PCR conditions were: 5 min. 95° C; 5 cycles of: 30 sec. 95°C, 1 min. 45°C, 1 min. 72°C; 20 cycles of 30 sec. 95°C, 1 min. 65°C, 1 min. 72°C and finally 5 min. 72°C. The resulting PCR product was purified on a 2 % low melting point agarose gel and sequenced from both ends, using Cy5-labelled primers complementary to the added T3 and T7 tags. Aliquots of about 200 ng were used for *in vitro* transcription labeling, using the MEGAscript-T3 (sense) or MEGAscript-T7 (antisense) kits, as described by the manufacturer (Ambion, Houston, TX, USA). The composition of the 10x-nucleotide mix was: 7.5 mM ATP, GTP and CTP, 3.75 mM UTP, 1.5 mM biotin-labeled UTP. To estimate quantity and labeling efficiencies, aliquots of the labeled RNA product were analyzed by agarose gel electrophoresis, dotted onto nitrocellulose filters and developed. ISH was performed on 3-6 different samples as described previously (Hoei-Hansen, CE, et al. Mol Hum Reprod 2004, 10:423-31).

### Immunohistochemistry

A commercially available monoclonal anti-AP-2γ antibody (6E4/4:sc-12762, kindly provided for testing by Santa Cruz Biotechnology Inc., Santa Cruz, CA, USA) was used. The antibody was validated by western blots by the manufacturer. In addition, several other antibodies were used as controls, in particular to detect CIS cells (anti-PLAP, a monoclonal antibody from BioGenex, San Ramon, CA, USA; anti-OCT3/4, C-20, sc 8629, Santa Cruz Biotechnology), and to control for integrity of fetal and infantile testis samples obtained at autopsies (anti-AMH, a monoclonal antibody kindly provided by R. Cate, Biogen). The immunohistochemical staining was performed using a standard indirect peroxidase method, as previously described for other antibodies (Giwercman, A, et al. APMIS 1991, 99:586-94;Rajpert-De Meyts, E, et al. Hum Reprod 2004). Briefly, the dewaxed and rehydrated sections were heated in a microwave oven to unmask the antigen. Sections fixed in formalin or PFA were heated in a 5% urea, pH 8.5, whereas sections fixed in Stieve's fluid were heated in TEG buffer, pH 9.0 (TRIS 6.06g/5L, EGTA 0.95g/5L). Subsequently, the sections were incubated with 0.5% H₂O₂ to inhibit the endogenous peroxidase, followed by diluted non-immune goat serum (Zymed, S. San Francisco, CA, USA) to block unspecific binding sites. The incubation with the primary anti-AP-2γ antibody diluted 1:25 - 1:100 was carried out overnight at 4°C. For tissues fixed in Stieve's fluid the primary antibody was diluted in Background Reducing antibody diluent (DakoCytomation, Glostrup, Denmark). For negative control, a serial section from each block was incubated with a dilution buffer. Subsequently, a secondary biotinylated goat-anti-mouse link antibody was applied (Zymed, S. San Francisco, CA, USA), followed by the horseradish peroxidase-streptavidin complex (Zymed, S. San Francisco, CA, USA). Between all steps the sections were thoroughly washed. The bound antibody was visualized using aminoethyl carbazole substrate (Zymed, S. San Francisco, CA, USA). Most sections were lightly counterstained with Mayer's haematoxylin to mark unstained nuclei.

The sections were examined under a light microscope (Zeiss) and scored systematically by two investigators (CHH and ERM). The staining was assessed using an arbitrary semi-quantitative score of the percentage of cells stained in a section and their staining intensity: +++: strong staining, ++: moderate staining, +: weak staining, +/-: very weak staining, neg: no positive cells detected.

### RESULTS

### AP-2γ is a new marker for testicular carcinoma in situ and derived germ cell tumors

After the discovery of the high expression of AP-2γ in testicular tissues harboring CIS cells (5), we extensively mapped the expression at the mRNA level by reverse transcriptase PCR (RT-PCR) in a panel of testicular neoplasms and tumor-derived cell lines. Subsequently, we determined cellular localization of AP-2γ transcripts by *in situ* hybridization (ISH), and at the protein level by immunohistochemistry in a comprehensive panel of normal and neoplastic tissues.

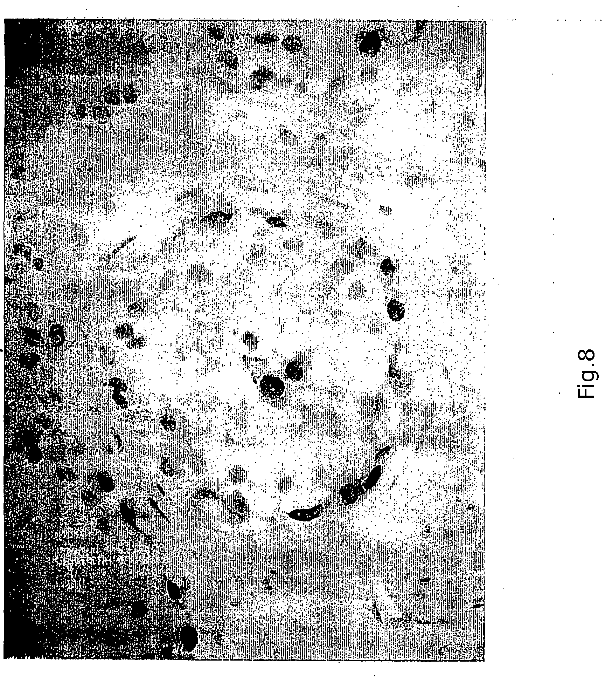

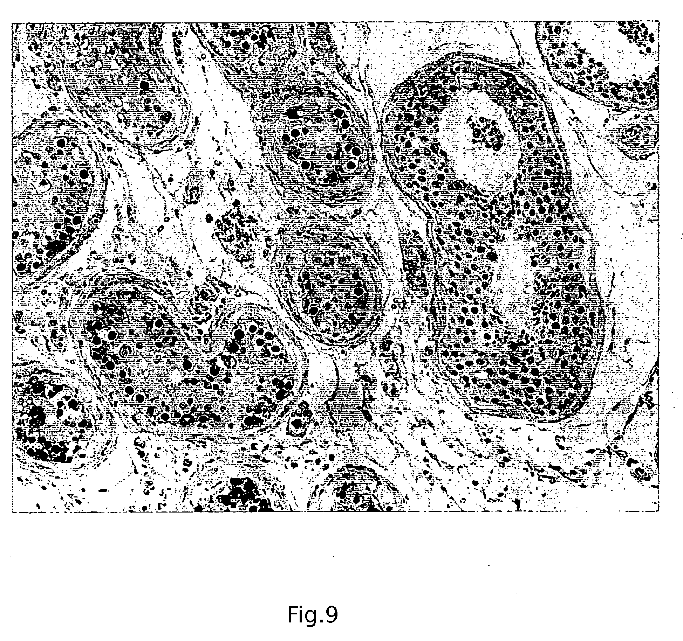

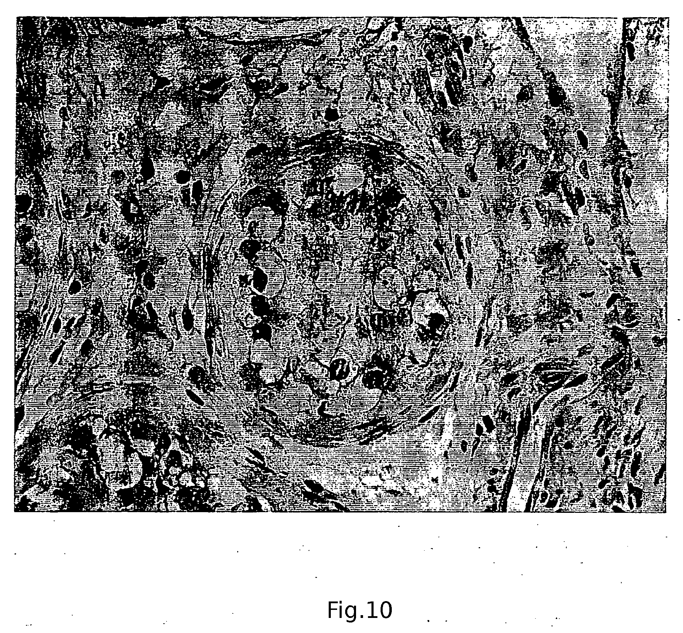

RT-PCR showed a high expression of AP-2γ in samples of seminoma, teratoma, embryonal carcinoma and CIS as compared to normal testicular tissue, which showed only a trace of reaction in one biopsy from a patient with an adjacent TGCT, and could encompass a few CIS or micro-invasive tumor cells (Fig. 8). Expression in CIS cells was approximately equivalent in tissue containing CIS adjacent to overt tumors and in CIS at the micro-invasive stage. For a few CIS and tumor samples the expression was confirmed in the database of our earlier study by differential display PCR (DDRT-PCR) (Hoei-Hansen, CE, et al. Mol Hum Reprod 2004, 10:423-31)(results not shown). ISH revealed cytoplasmic staining in CIS cells, seminoma, embryonal carcinoma and to lesser extent in some components of teratomas (Fig. 9). ISH in normal testicular tissue was not completely conclusive; in contrast to IHC, which did not show any detectable protein in normal germ cells, a reaction was observed in some spermatocytes and spermatids (results not shown). The presence of *TFAP2C* transcripts in the normal germ cells with a simultaneous lack of the protein product cannot, therefore, be excluded. This could be due to translational regulation of AP-2γ in the testis. A similar discrepancy between expression at the mRNA and the protein level was previously described for AP-2α in colon cancer (Ropponen, KM, et al. J Clin Pathol 2001, 54:533-8). Immunohistochemistry demonstrated a high abundance of AP-2γ protein in CIS. CIS cells adjacent to TGCTs were uniformly strongly positive for AP-2γ regardless of the tumor type, and equally strong in CIS present in the testis and as an isolated pre-malignant lesion. Gonadoblastoma, a CIS-like pre-malignant lesion of severely dysgenetic gonads was also strongly positive (Fig. 10). Differential expression was however observed in the overt TGCTs: seminomas were strongly positive, whereas a heterogeneous pattern was detected in the non-seminomas (Fig. 9). Embryonal carcinoma demonstrated strongest staining, teratomas displayed staining in some epithelial and stromal elements but many highly differentiated components were negative. In all TGCT specimens staining was present exclusively in the nucleus. No expression was detected in spermatocytic seminoma. In non-germ-cell-derived testicular tumors no expression was detected in a B-cell lymphoma and two Leydig cell tumors (one testicular and one adrenal). One Leydig-Sertoli cell tumor was unexpectedly positive, however, as the staining was confined to the cytoplasm, this may be an unspecific reaction, which is quite frequently observed for various antibodies in the protein and glycoprotein-filled Leydig cells. A similar but much weaker unspecific staining in Leydig cell cytoplasm was also observed in a few specimens of testicular parenchyma. In general, the intensity of staining varied somewhat depending on the fixative used, with stronger staining in tissues fixed with PFA and formalin compared to those fixed in Stieve's and Bouin. In addition, in samples fixed in Stieve's fluid or in high-percentage formalin, there was a tendency to an unspecific staining of erythrocytes. The complete results of the immunohistochemical staining are listed in Table III and representative examples are shown in Figures 9, 10 and 11.

### The expression of AP-2γ is developmentally regulated in human gonads

To investigate whether the high expression of AP-2γ in CIS cells is linked to their gonocyte-like phenotype, as is the case for a number of other markers for CIS cells (Rajpert-De Meyts, E, et al. APMIS 2003, 111:267-78; discussion 278-9), we established the ontogeny of expression of AP-2γ by immunohistochemistry in a panel of specimens of normal human fetal testis and ovaries during development.

The AP-2γ protein was detected in almost all gonocytes in testes from 10 weeks of gestation (earlier samples were not available) until approximately 22 weeks of gestation (Fig. 10), although a few of our analyzed tissue samples failed to show expression. Expression was gradually reduced after week 22, but persisted in a few cells until approximately 4 months of postnatal age. Afterwards, AP-2γ protein was not detected in spermatogonia in normal pre-pubertal, post-pubertal or adult testes.

In fetal ovaries AP-2γ expression was seen in oogonia in high amounts in early gestational ages and to a lesser extent in third trimester. The number of positive oogonia was decreasing with the increasing number of oocytes that entered the meiotic prophase, which were AP-2γ negative. No expression was seen in adult ovary, but only few samples were analyzed.

### Changes of the expression pattern of AP-2γ in intersex and dysgenetic gonads

Subsequently, we asked the question whether or not the normal developmental pattern of the expression of AP-2γ is changed in testicular dysgenesis and disorders of sex differentiation, conditions with a markedly increased risk of germ cell neoplasia. Expression of AP-2γ in dysgenetic gonads was analyzed only by immunohistochemistry, because the specimens were available only as paraffin-embedded blocks from tissue archives. The pattern of expression in these specimens was in general similar to that in the normal testes, but in some samples expression deviated from the observed pattern. Expression of AP-2γ was not observed in a sample from a male fetus gestational age 15 weeks with mosaic isochromosome Y, similar to previously observed lack of OCT3/4 (Rajpert-De Meyts, E, et al. Hum Reprod 2004). Staining for AMH was positive, suggesting that the integrity of proteins should be preserved in the sample. Prolonged expression of AP-2γ was seen in cells resembling gonocytes in testicular cords in an ovotestis from a 14-month old XX hermaphrodite, but not in the oocytes present in the ovarian part. In a few of the dysgenetic samples neoplastic, pre-invasive CIS or gonadoblastoma was present and these showed a high expression of AP-2γ In three cases staining was observed in foci of germ cells that morphologically were classified as somewhere in between gonocytes and CIS (see Fig. 10). In two of the cases (girls with AIS of age 9 months and 6 years, respectively) PLAP was also positive. The third was from a testis removed from a 2-year old girl with AIS, where a very small focus of AP-2γ positive cells was detected. We were not able to locate this focus morphologically in adjacent sections cut before and after, and stainings with PLAP and OCT3/4 were accordingly negative.

### Expression of AP-2γ is associated with cell differentiation or proliferation and is cell type dependent

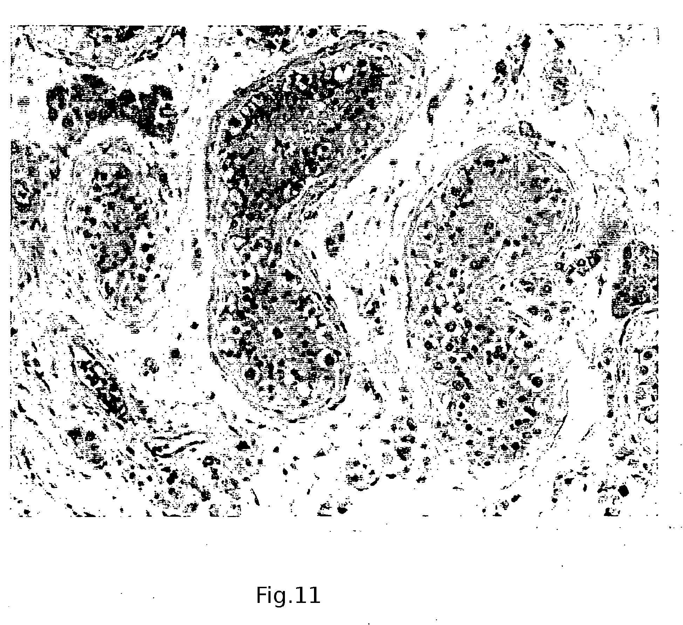

In order to define the extent of expression and a possible link to hormonal stimulation, expression of AP-2γ was analyzed in some tissues that are endocrinologically activated and in a panel of normal somatic tissues. In normal female mammary tissue expression was seen in a subpopulation of basally situated epithelial duct cells, and expression was also detected in some, but not all, premalignant or manifest malignant changes in the female breast (Fig. 11, Table III). In samples from skin, the basal layer of keratinocytes was positive, and weaker staining could be seen in the adjacent layers of epidermis (Fig. 11), which is in concert with the previously reported pattern. In normal adult kidney AP-2γ expression was seen in a subset of cells in proximal tubules. Finally, AP-2γ expression was present in lung tissue, in pneumocytes lining alveoles. All other analyzed tissue samples were negative (Table III). We cannot exclude that the AP-2γ expression may be developmentally regulated in some tissues, in analogy to the testis. We could not gain access to a full spectrum of human fetal tissues at different developmental stages, but we have examined and found no AP-2γ expression in the following fetal tissues from the first or second trimester of gestation: epididymis, liver, heart, thymus, thyroid gland, kidney and adrenal gland.

Finally, regulation of AP-2γ expression was studied *in vitro* in three cell lines. We showed by RT-PCR that AP-2γ was RA-regulated in NT2 cells in a biphasic manner. Strong expression was observed in cells without RA-induced differentiation, after 3 days of RA treatment (which at that point induced cell proliferation) the expression was somewhat stronger and then declined after 15 days when the cells have differentiated (Fig. 8). A heterogeneous staining for AP-2γ protein was seen in NT2 cells by immunocytochemistry in all analyzed samples, regardless of the length of RA treatment. It was not possible to establish the pattern quantitatively, because of differences in intensity from cell to cell. In contrast, in the 2102Ep cells, which do not differentiate with RA treatment, expression was unaffected by RA treatment at the RNA level, and immunocyto-chemical staining was uniformly strong in all analyzed cells. In MCF7 cells, expression was similar in cells exposed to estradiol, the anti-estrogen ICI, or ethanol, both at the RNA level, and at the protein level, where expression was seen in a subset of cells in all analyzed cells, regardless of treatment (Fig. 11). For examples of RT-PCR results, see Figure 8.

### DISCUSSION

In the present study we comprehensively investigated the pattern of expression of the transcription factor AP-2γ and established AP-2γ as a novel marker for neoplastic germ cells, including testicular carcinoma *in situ,* the common precursor of TGCTs. Furthermore, we discovered that AP-2γ was developmentally regulated in human gonads, with marked differences between the testes and ovaries.

Our investigations of the pattern of expression of AP-2γ in extra-gonadal normal and neoplastic tissues were largely in concert with previous studies, thus confirming the specificity of the antibody. The transcription factor was preferentially expressed in proliferating immature cells, which retained tissue-specific stem cell characteristics, suggesting a putative oncogenic function of AP-2γ

In the testis, we found that the AP-2γ protein was expressed in early gonocytes but not in any cell type in the pre- or post-pubertal testis. The expression rapidly declined from approximately 22 weeks of gestation, and was gradually lost while gonocytes differentiated into infantile spermatogonia up to about 4 months of postnatal age, when the final stage of this differentiation takes place, probably stimulated by the hormonal surge at the "mini-puberty" (Andersson, AM, et al. J Clin Endocrinol Metab 1998, 83:675-81; Hadziselimovic, F, et al. J Urol 1986, 136:274-6). Interestingly, in a few pre-pubertal intersex cases, AP-2γ was observed in a subset of gonocytes outside the normal window of expression. This prolonged expression may reflect a delay in differentiation but may perhaps be a marker of the initiation of neoplastic transformation of these immature germ cells into "pre-CIS" cells, which then may further transform to a typical CIS pattern at the onset of puberty. In the ovary, AP-2γ protein was confined to oogonia and was not detectable after meiotic entry in oocytes. This pattern of expression suggests that the protein plays a role exclusively in undifferentiated germ cells. Analyses in cell lines confirmed that AP-2γ was most abundant in undifferentiated cells, as expression of the transcript was down-regulated after a transient induction in AP-2γ-positive NT-2 cells treated with RA for up to 15 days. In cells that do not differentiate (2102Ep) expression was constantly high. Oulad-Abdelghani and colleagues (Oulad-Abdelghani, M, et al. Exp Cell Res 1996, 225:338-47) reported a transient rise in AP-2γ levels with a peak after 12 hours of RA treatment in embryonal carcinoma derived P19 cells, but did not analyze levels beyond 24 hours. An association of AP-2γ with an immature state of germ cells was also observed in overt TGCTs, where classical seminoma and embryonal carcinoma were strongly positive, while highly differentiated somatic elements of teratomas were negative. Spermatocytic seminoma, which is not derived from CIS but from mature spermatogonia, was also AP-2γ-negative. Overall, the pattern of expression of AP-2γ in TGCTs resembles that of KIT and OCT-4, which are also associated with an undifferentiated state and pluripotency, typical for cells retaining some stem-cell-like features. The high expression of AP-2γ in CIS supports our hypothesis that this pre-malignant lesion is derived from an early fetal germ cell. In adult testes AP-2γ was only seen either in CIS or in overt TGCTs, or in rare specimens harboring dysgenetic features, and in the latter only in gonadoblastoma and CIS cells. Thus, AP-2γ can be added to a list of markers for germ cell neoplasms that are also present in gonocytes but not detectable in the normal adult testis, such as PLAP, KIT or OCT-4 (Rajpert-De Meyts, E, et al. APMIS 2003, 111:267-78; discussion 278-9).

Besides an inverse association with cell differentiation, which was also noted by others in other cell systems (Jager, R, et al. Mol Cancer Res 2003, 1:921-9), very little is known concerning the biological function and the mechanism of action of AP-2γ especially in germ cells. The most interesting question is what are the gene targets for AP-2γ in germ cells, and with which other proteins does this transcription factor interact. In other cell types AP-2y has been previously shown to interact with a number of genes that are also highly expressed in CIS cells, e.g. IGF-binding protein 5 (Jager, R, et al. Mol Cancer Res 2003, 1:921-9) or KIT (Rajpert-De Meyts, E, et al. Int J Androl 1994, 17:85-92). KIT appears to be of particular interest, as it was reported to be down-regulated in several types of tumors derived from tissues that express AP-2γ e.g. breast cancer, colorectal cancer or melanoma (Ropponen, KM, et al. J Clin Pathol 2001, 54:533-8).

Estrogen signaling is another possible candidate pathway which could be regulated by AP-2y, as is suggested by its expression in the epithelium of milk ducts and the association with some forms of breast cancer. While the classic estrogen receptor (ERα) is not expressed in the testis (neither in the fetal life or adulthood), the ERβ is expressed in human fetal testis in the second trimester, and gonocytes are potential targets for estrogen action. This is of interest because it has been suggested that inappropriate exposure to estrogens during fetal life may have an impact on the development of CIS and TGCTs (Skakkebaek, NE, et al. Hum Reprod 2001, 16:972-8). Furthermore, the progression from CIS into an overt TGCT occurs after the endocrinological surge of puberty, when the intra-testicular levels of steroid hormones rise markedly. In the current study, we have analyzed the expression of AP-2γ in a breast tumor-derived cell line, MCF7, which is estrogen dependent. We have not observed any changes in expression, neither after treatment with estradiol, nor an anti-estrogen. This is in disagreement with a recent study (Orso, F, et al. Biochem J 2004, 377:429-38), which showed induction of AP-2γ by estrogens in several breast tumor cell lines. Whether AP-2γ is indeed involved in estrogen signaling remains an unresolved issue, since existing reports are partially contradictory and will require further investigations. We need also to keep in mind that the function of AP-2γ may be cell-type specific, as was demonstrated recently for the transcriptional activation of the *ERBB2* gene (Vernimmen, D, et al. Br J Cancer 2003, 89:899-906) and thus may be different in germ cells compared to somatic cell types.

In summary, our investigations suggest a role for AP-2γ in pathways regulating cell differentiation, including in fetal gonocytes, which was not previously known. Frequent expression of AP-2γ in various malignancies suggests that the pathways may be involved in oncogenesis. Finally, developmental expression of this transcription factor in the testis makes AP-2γ a very promising new marker for diagnosis of early stages of germ cell neoplasia. Example 10

### Early diagnosis of pre-invasive testicular germ cell neoplasia by immunocytological detection of AP-2γ-positive cells in semen samples

In the above genome-wide gene expression profiling study of CIS cells, we have identified a number of genes expressed also in embryonic stem cells and foetal gonocytes but not in adult germ cells, thus providing many possible new markers for CIS.

One of such genes was TFAP2C (mapped to chromosome 20q13.2), which encodes the transcription factor activator protein-2 (AP-2γ). We established AP-2γ as a novel marker for foetal gonocytes and neoplastic germ cells, including testicular CIS, with a role in pathways regulating cell differentiation and a possible involvement in oncogenesis. The fact that AP-2y protein was not expressed in normal adult reproductive tract but was abundant in nuclei of CIS and tumour cells, which are better protected than the cytoplasm from degradation in semen due to a structural strength, prompted us to analyse the value of AP-2γ for detection of CIS and/or tumour cells in ejaculates in a series of patients and controls. The local ethics committee approved the study and participants gave written informed consent.

The present example presents a new non-invasive method capable of detecting testicular cancer at the pre-invasive carcinoma in situ (CIS) in a semen sample. The assay is based on immunocytological staining of semen samples for transcription factor AP-2γ previously identified as a marker for neoplastic germ cells.

In an investigation of 104 andrological patients, cells positive for AP-2γ were found only in semen samples from patients diagnosed a priori with testicular neoplasms and in a 23-year-old control subject with oligozoospermia and no symptoms of a germ cell tumour (fig. 7). Testicular biopsies performed during the follow-up of this patient revealed widespread CIS in one testis, thus proving a diagnostic value of the new assay. Optimisation of the method is in progress, but a preliminary assessment gives promise that the method may have a place in screening of germ cell neoplasia in at-risk patients.

The number of patients that we have screened for the presence of immunoreactive AP-2γ is continuously increasing as we are testing patients as they appear in our clinic. So far we have tested 347 andrological patients (including the 104 mentioned above) and found CIS-like cells positive for AP-2γ in semen samples of 3 patients, including the first case described above (a 23-year-old subfertile control subject with widespread CIS confirmed by testicular biopsies). Two other patients are currently under clinical follow-up and being considered for testicular biopses.

### Materials and methods

A semen sample from each patient was diluted if necessary, divided in portions of 100 µl, spun down on a microscope slide with 400 µl PBS buffer, dried, fixed 10 min in formalin, washed and dried again. The immunocytochemical staining was performed with the monoclonal anti-AP-2γ antibody (6E4/4:sc-12762, Santa Cruz Biotechnology Inc., Santa Cruz, CA, USA) using a standard indirect peroxidase method, as previously described (Almstrup, K, et al. Cancer Res 2004, 64:4736-43), except for small modifications tested using control semen samples spiked with AP-2γ-positive seminoma cells (Fig. 7A). For negative control, another cytospin was incubated with a dilution buffer, and for positive control AP-2γ-positive seminoma cells were used. Staining was scored using an arbitrary 0-5 scale based on the intensity and morphological resemblance of stained elements to CIS or tumour cells: Score 0 - no staining, 1 - negative with some unspecific particles stained, 2 - trace reaction in small fragments of nucleus-resembling structures, 3 - clear staining in a part of a nucleus, with correct morphology, 4 - clear staining in a whole nucleus, with correct morphology, 5 - two or more cells with score 4. Scores 0-2 were classified as negative, while 3-5 were classified as positive.

### Results

At the evaluation of one of the first series of semen samples, we detected six AP-2y-positive cells in a sample from one of the control participants (Fig. 7B). He was a 23-year-old man that presented for routine semen analysis due to the infertility problem in the couple. The presence of one small testis and a very poor semen quality in combination with the finding of AP-2γ stained cells constituted a well-justified indication for bilateral open testicular biopsies, which were performed in agreement with the patient. The right-sided biopsy demonstrated normal testicular tissue, whereas the left-sided biopsy revealed numerous tubules containing CIS, which was confirmed with PLAP and AP-2γ staining (Fig.7D, E). The patient was advised to undergo unilateral orchidectomy, prior to which semen was cryopreserved.

A preliminary assessemnet of the value of the AP-2γ immunoassay was subsequently done after analysis of semen samples from 104 patients: 12 patients with CIS or testicular cancer prior to orchidectomy (with a subsequently verified germ cell neoplasm), 7 testicular cancer patients after operation (none had contralateral CIS), 12 patients with other diseases (e.g. referred for semen cryopreservation before treatment for non-testicular cancers and diseases) and 73 healthy or subfertile control patients.

The results are summarised in Table II and examples are shown in Fig. 7. We detected AP-2γ-positive cells in 5 of 12 patients harbouring *a priori* diagnosed testicular neoplasia, giving a sensitivity of our assay of 42%. However, after addition of the newly diagnosed control patient with CIS, the sensitivity is 46% (95% CI, 23 to 71%). It is especially noteworthy that we did not detect false positive staining among the healthy controls and patients with other cancers, suggesting a high specificity of the assay.

The large proportion of negative results in the testicular cancer patients could be due to a small volume of the semen sample used for the test (50-300 µl out of total normal volume 3-5 ml, depending on concentration and number of samples), thus stressing the need for either repeated analysis or larger volume analysed. Negative results could also be due to compression from the tumour causing obstruction of seminiferous tubules resulting in few or no CIS cells shed into semen. However, considering the variability of the number of CIS cells present in the tissue adjacent to a tumour, the sensitivity of our assay is reasonably good, as nearly half of the patients harbouring testicular germ cell neoplasm have been detected in this preliminary study.

The relatively low sensitivity nevertheless leaves room for improvement and further optimisation of the method is currently in progress. The results of planned studies in a wider panel of patients will confirm whether this assay should be offered widely to patients at risk for TGCT as a method of non-invasive screening for CIS. Such screening could be performed, for example, at andrology and fertility clinics in young men with atrophic testes, history of cryptorchidism or in need for an assisted reproduction technique. If diagnosis of testicular cancer is made at the pre-invasive CIS stage, the patient can be offered the most gentle and optimal treatment.

### References

Almstrup, K, Hoei-Hansen, CE, Wirkner, U, Blake, J, Schwager, C, Ansorge, W, Nielsen, JE, Skakkebaek, NE, Rajpert-De Meyts, E and Leffers, H, 2004, Embryonic stem cell-like features of testicular carcinoma in situ revealed by genome-wide gene expression profiling Cancer Res 64:4736-43
Andersson, AM, Toppari, J, Haavisto, AM, Petersen, JH, Simell, T, Simell, O and Skakkebaek, NE, 1998, Longitudinal reproductive hormone profiles in infants: peak of inhibin B levels in infant boys exceeds levels in adult men J Clin Endocrinol Metab 83:675-81
Andrews, PW, 1984, Retinoic acid induces neuronal differentiation of a cloned human embryonal carcinoma cell line in vitro Dev Biol 103:285-93
Draper, JS, Smith, K, Gokhale, P, Moore, HD, Maltby, E, Johnson, J, Meisner, L, Zwaka, TP, Thomson, JA and Andrews, PW, 2004, Recurrent gain of chromosomes 17q and 12 in cultured human embryonic stem cells Nat Biotechnol 22:53-4
Giwercman, A, Cantell, L and Marks, A, 1991, Placental-like alkaline phosphatase as a marker of carcinoma-in-situ of the testis. Comparison with monoclonal antibodies M2A and 43-9F APMIS 99:586-94
Hadziselimovic, F, Thommen, L, Girard, J and Herzog, B, 1986, The significance of postnatal gonadotropin surge for testicular development in normal and cryptorchid testes J Urol 136:274-6
Hoei-Hansen, CE, Nielsen, JE, Almstrup, K, Hansen, MA, Skakkebaek, NE, Rajpert-DeMeyts, E and Leffers, H, 2004, Identification of genes differentially expressed in testes containing carcinoma in situ Mol Hum Reprod 10:423-31
Jager, R, Werling, U, Rimpf, S, Jacob, A and Schorle, H, 2003, Transcription factor AP-2gamma stimulates proliferation and apoptosis and impairs differentiation in a transgenic model Mol Cancer Res 1:921-9
Jahnukainen, K, JorgensenN, Pollanen, P, Giwercman, A and Skakkebaek, NE, 1995, Incidence of testicular mononuclear cell infiltrates in normal human males and in patients with germ cell neoplasia Int J Androl 18:313-20
Jorgensen, M, Bevort, M, Kledal, TS, Hansen, BV, Dalgaard, M and Leffers, H, 1999, Differential display competitive polymerase chain reaction: an optimal tool for assaying gene expression Electrophoresis 20:230-40
Manivel, JC, Jessurun, J, Wick, MR and Dehner, LP, 1987, Placental alkaline phosphatase immunoreactivity in testicular germ-cell neoplasms Am J Surg Pathol 11:21-9
Nielsen, JE, Hansen, MA, Jorgensen, M, Tanaka, M, Almstrup, K, Skakkebaek, NE and Leffers, H, 2003, Germ cell differentiation-dependent and stage-specific expression of LANCL1 in rodent testis Eur J Histochem 47:215-22
Orso, F, Cottone, E, Hasleton, MD, Ibbitt, JC, Sismondi, P, Hurst, HC and De Bortoli, M, 2004, Activator protein-2gamma (AP-2gamma) expression is specifically induced by oestrogens through binding of the oestrogen receptor to a canonical element within the 5'-untranslated region Biochem J 377:429-38
Oulad-Abdelghani, M, Bouillet, P, Chazaud, C, Dolle, P and Chambon, P, 1996, AP-2.2: a novel AP-2-related transcription factor induced by retinoic acid during differentiation of P19 embryonal carcinoma cells Exp Cell Res 225:338-47
Pera, MF, 2004, Unnatural selection of cultured human ES cells? Nat Biotechnol 22:42-3
Rajpert-De Meyts, E, Bartkova, J, Samson, M, Hoei-Hansen, CE, Frydelund-Larsen, L, Bartek, J and Skakkebaek, NE, 2003, The emerging phenotype of the testicular carcinoma in situ germ cell APMIS 111:267-78; discussion 278-9
Rajpert-De Meyts, E, Hanstein, R, Jorgensen, N, Graem, N, Vogt, PH and Skakkebaek, NE, 2004, Developmental expression of POU5F1 (OCT-3/4) in normal and dysgenetic human gonads Hum Reprod
Rajpert-De Meyts, E and Skakkebaek, NE, 1994, Expression of the c-kit protein product in carcinoma-in-situ and invasive testicular germ cell tumours Int J Androl 17:85-92
Ramalho-Santos, M, Yoon, S, Matsuzaki, Y, Mulligan, RC and Melton, DA, 2002, "Stemness": transcriptional profiling of embryonic and adult stem cells Science 298:597-600
Ropponen, KM, Kellokoski, JK, Pirinen, RT, Moisio, KI, Eskelinen, MJ, Alhava, EM and Kosma, VM, 2001, Expression of transcription factor AP-2 in colorectal adenomas and adenocarcinomas; comparison of immunohistochemistry and in situ hybridisation J Clin Pathol 54:533-8
Sato, N, Sanjuan, IM, Heke, M, Uchida, M, Naef, F and Brivanlou, AH, 2003, Molecular signature of human embryonic stem cells and its comparison with the mouse Dev Biol 260:404-13
Skakkebaek, NE, Rajpert-De Meyts, E and Main, KM, 2001, Testicular dysgenesis syndrome: an increasingly common developmental disorder with environmental aspects Hum Reprod 16:972-8
Sperger, JM, Chen, X, Draper, JS, Antosiewicz, JE, Chon, CH, Jones, SB, Brooks, JD, Andrews, PW, Brown, PO and Thomson, JA, 2003, Gene expression patterns in human embryonic stem cells and human pluripotent germ cell tumors Proc Natl Acad Sci U S A 100:13350-5
Vernimmen, D, Gueders, M, Pisvin, S, Delvenne, P and Winkler, R, 2003, Different mechanisms are implicated in ERBB2 gene overexpression in breast and in other cancers Br J Cancer 89:899-906

## Claims

1. A method for determining the presence of precursors of germ cell tumours in a sample, said method comprising:
a) determining the level of transcripts of a marker in said sample, wherein the marker is AP-2γ;
b) comparing said level of transcripts with a reference level of transcripts of said marker in normal testicular tissue;
c) identifying whether the level of transcripts is different from said reference level of transcripts, and
evaluating whether the sample contains precursors of germ cell tumours if the level of transcripts is different from the reference level of transcripts.

2. A method for determining the presence of precursors of germ cell tumours in a sample, said method comprising:
a) determining the intensity signal of the protein encoded by a marker in said sample, wherein the marker is AP-2γ;
b) comparing the intensity signal with a corresponding reference intensity signal of said marker in normal testicular tissue;
c) identifying whether the intensity signal is different from said corresponding reference intensity signal, and
evaluating whether the sample contains precursors of germ cell tumours if the intensity signal is different from the corresponding reference intensity signal.

3. A method according to any of claims 1-2, wherein the marker is set forth in SEQ ID NO 20.

4. A method for monitoring progression from precursors of germ cell tumours to overt cancer cells in an individual, comprising
a) determining the level of transcripts of a marker in a sample, wherein the marker is AP-2γ;
b) comparing said level of transcripts with a reference level of transcripts of said marker in normal testicular tissue;
c) identifying whether the level of transcripts is different from said reference level of transcripts, and
evaluating the development stage of precursors of germ cell tumours to overt cancer cells if the level of transcripts is different from the reference level of transcripts.

5. A method for monitoring progression from precursors of germ cell tumours to overt cancer in an individual, comprising
a) determining the intensity signal of the protein encoded by a marker in a sample, wherein the marker is AP-2γ;
b) comparing the intensity signal with a corresponding reference intensity signal of said marker in normal testicular tissue;
c) identifying whether the intensity signal is different from said corresponding reference intensity signal, and
evaluating the development stage of precursors of germ cell tumours to overt cancer cells if the intensity signal is different from the corresponding reference intensity signal.

6. A method according to any of claims 4 or 5, wherein said method distinguish seminoma from CIS.

7. A method according to any of claims 4 or 5, wherein said method distinguish non-seminoma from CIS.

8. A method of identifying precursors of germ cell tumours in a sample from a mammal, the method comprising
assaying said sample by a quantitative detection assay and determining the level of transcripts and/or the intensity signal of a marker, wherein said marker is AP-2γ;
comparing said level of transcripts and/or the intensity signal with a reference value of said marker in normal testicular tissue;
identifying whether the level of transcripts and/or the intensity signal of the marker from the sample is significantly different from the reference value, and
evaluating whether the sample contains precursors of germ cell tumours if the level of transcripts and/or the intensity signal is different from the reference value.

9. A method for determining the presence of precursors of germ cell tumours in an individual comprising detecting the presence of an expression product, wherein said expression product comprise a nucleotide sequence from AP-2γ in a sample isolated from the individual.

10. A method of assessing the efficacy of a therapy for inhibiting precursors of germ cell tumours in a subject, the method comprising
comparing the level of transcripts of a marker in a first sample obtained from the subject prior to providing at least a portion of the therapy to the subject and the level of transcripts of a marker in a second sample obtained from the subject following provision of the portion of the therapy, wherein a significantly altered level of transcripts of the marker in the second sample, relative to the first sample, is an indication that the therapy is efficacious for inhibiting precursors of germ cell tumours in the subject
wherein the marker is AP-2γ.

11. A method of assessing the efficacy of a therapy for inhibiting precursors of germ cell tumours in a subject, the method comprising
comparing the intensity signal of the protein encoded by a marker in a first sample obtained from the subject prior to providing at least a portion of the therapy to the subject and the intensity signal of the proteins encoded by a marker in a second sample obtained from the subject following provision of the portion of the therapy, wherein a significantly altered intensity signal of the protein encoded by the marker in the second sample, relative to the first sample, is an indication that the therapy is efficacious for inhibiting precursors of germ cell tumours in the subject
wherein the marker is AP-2γ.

12. A method for analysing the presence of undifferentiated stem cells highly likely to
progress to precursors of germ cell tumours and/or cancer in an individual, said method comprising
a) determining the level of transcripts of a marker in a sample, wherein the marker is AP-2γ;
b) comparing said level of transcripts with a reference level of transcripts of said marker in normal testicular tissue;
c) identifying whether the level of transcripts is different from said reference level of transcripts, and
evaluating whether the sample contains undifferentiated stem cells highly likely to progress to precursors of germ cell tumours and/or cancer in the individual if the level of transcripts is different from the reference level of transcripts.

13. A method for analysing the presence of undifferentiated stem cells highly likely to progress to precursors of germ cell tumours and/or cancer in an individual, said method comprising
a) determining the intensity signal of the protein encoded by a marker in said sample, wherein the marker is AP-2γ,
b) comparing the intensity signal with a corresponding reference intensity signal of said marker in normal testicular tissue;
c) identifying whether the intensity signal is different from said corresponding reference intensity signa, and
evaluating whether the sample contains undifferentiated stem cells highly likely to progress to precursors of germ cell tumours and/or cancer in the individual if the intensity signal is different from the reference level of transcripts.

14. A method according to claim 13, wherein the intensity signal is determined by immunocytological detection.

15. Use of a method according to any of claims 1 or 2 for screening for the presence of precursors of germ cell tumours in an individual of a population.

## Patentansprüche

1. Verfahren zur Bestimmung der Gegenwart von Vorläufern von Keimzelltumoren in einer Probe, wobei das Verfahren umfasst:
a) Bestimmen des Gehalts an Transkripten eines Markers in der Probe, wobei der Marker AP-2γ ist;
b) Vergleichen des Gehalts an Transkripten mit einem Referenzgehalt für Transkripte des Markers in normalem Hodengewebe;
c) Feststellen, ob sich der Gehalt an Transkripten vom Referenzgehalt der Transkripte unterscheidet, und
Beurteilen, ob die Probe Vorläufer von Keimzelltumoren enthält, wenn sich der Gehalt an Transkripten vom Referenzgehalt der Transkripte unterscheidet.

2. Verfahren zur Bestimmung der Gegenwart von Vorläufern von Keimzelltumoren in einer Probe, wobei das Verfahren umfasst:
a) Bestimmen des Intensitätssignals des Proteins, für das ein Marker in der Probe codiert, wobei der Marker AP-2γ ist;
b) Vergleichen des Intensitätssignals mit einem entsprechenden Referenzintensitätssignal des Markers in normalem Hodengewebe;
c) Feststellen, ob sich das Intensitätssignal vom entsprechenden Referenzintensitätssignal unterscheidet, und
Beurteilen, ob die Probe Vorläufer von Keimzelltumoren enthält, wenn sich das Intensitätssignal vom entsprechenden Referenzintensitätssignal unterscheidet.

3. Verfahren nach einem der Ansprüche 1-2, wobei der Marker in SEQ ID NR. 20 dargestellt ist.

4. Verfahren zur Überwachung der Progression von Vorläufern von Keimzelltumoren zu offenkundigen Krebszellen bei einem Individuum, umfassend
a) Bestimmen des Gehalts an Transkripten eines Markers in einer Probe, wobei der Marker AP-2γ ist;
b) Vergleichen des Gehalts an Transkripten mit einem Referenzgehalt für Transkripte des Markers in normalem Hodengewebe;
c) Feststellen, ob sich der Gehalt an Transkripten vom Referenzgehalt der Transkripte unterscheidet, und
Beurteilen des Entwicklungsstadiums der Vorläufer von Keimzelltumoren bis zu offenkundigen Krebszellen, wenn sich der Gehalt an Transkripten vom Referenzgehalt der Transkripte unterscheidet.

5. Verfahren zur Überwachung der Progression von Vorläufern von Keimzelltumoren zu offenkundigem Krebs bei einem Individuum, umfassend
a) Bestimmen des Intensitätssignals des Proteins, für das ein Marker in einer Probe codiert, wobei der Marker AP-2γ ist;
b) Vergleichen des Intensitätssignals mit einem entsprechenden Referenzintensitätssignal des Markers in normalem Hodengewebe;
c) Feststellen, ob sich das Intensitätssignal vom entsprechenden Referenzintensitätssignal unterscheidet, und
Beurteilen des Entwicklungsstadiums der Vorläufer von Keimzelltumoren bis zu offenkundigen Krebszellen, wenn sich das Intensitätssignal vom entsprechenden Referenzintensitätssignal unterscheidet.

6. Verfahren nach einem der Ansprüche 4 oder 5, wobei das Verfahren zwischen Seminomen und CIS unterscheidet.

7. Verfahren nach einem der Ansprüche 4 oder 5, wobei das Verfahren zwischen Nichtseminomen und CIS unterscheidet.

8. Verfahren zur Identifizierung von Vorläufern von Keimzelltumoren in einer Probe von einem Säugetier, wobei das Verfahren umfasst
Untersuchen der Probe mit einem quantitativen Detektionsassay und Bestimmen des Gehalts an Transkripten und/oder des Intensitätssignals eines Markers, wobei der Marker AP-2γ ist;
Vergleichen des Gehalts an Transkripten und/oder des Intensitätssignals mit einem Referenzwert des Markers in normalem Hodengewebe;
Feststellen, ob sich der Gehalt an Transkripten und/oder das Intensitätssignal des Markers der Probe signifikant vom Referenzwert unterscheidet, und
Beurteilen, ob die Probe Vorläufer von Keimzelltumoren enthält, wenn sich der Gehalt an Transkripten und/oder das Intensitätssignal vom Referenzwert unterscheidet.

9. Verfahren zur Bestimmung der Gegenwart von Vorläufern von Keimzelltumoren bei einem Individuum, umfassend das Nachweisen der Gegenwart eines Expressionsprodukts, wobei das Expressionsprodukt eine Nukleotidsequenz von AP-2γ in einer Probe umfasst, die von dem Individuum genommen wurde.

10. Verfahren zur Bewertung der Wirksamkeit einer Therapie zur Hemmung von Vorläufern von Keimzelltumoren bei einem Patienten, wobei das Verfahren umfasst
Vergleichen des Gehalts an Transkripten eines Markers in einer ersten Probe, die vom Patienten vor der Durchführung mindestens eines Teils der Therapie des Patienten genommen wurde, mit dem Gehalt an Transkripten eines Markers in einer zweiten Probe, die vom Patienten nach Durchführung des Teils der Therapie genommen wurde, wobei ein im Vergleich zur ersten Probe signifikant geänderter Gehalt an Transkripten des Markers in der zweiten Probe ein Anzeichen dafür ist, dass die Therapie beim Hemmen von Vorläufern von Keimzelltumoren beim Patienten wirksam ist,
wobei der Marker AP-2γ ist.

11. Verfahren zur Bewertung der Wirksamkeit einer Therapie zur Hemmung von Vorläufern von Keimzelltumoren bei einem Patienten, wobei das Verfahren umfasst
Vergleichen des Intensitätssignals des Proteins, für das ein Marker in einer ersten Probe codiert, die vom Patienten vor der Durchführung mindestens eines Teils der Therapie des Patienten genommen wurde, mit dem Intensitätssignal der Proteine, für die ein Marker in einer zweiten Probe codiert, die vom Patienten nach Durchführung des Teils der Therapie genommen wurde, wobei ein im Vergleich zur ersten Probe signifikant geändertes Intensitätssignal des Proteins, für das der Marker in der zweiten Probe codiert, ein Anzeichen dafür ist, dass die Therapie beim Hemmen von Vorläufern von Keimzelltumoren beim Patienten wirksam ist,
wobei der Marker AP-2γ ist.

12. Verfahren zur Analyse der Gegenwart von undifferenzierten Stammzellen, bei denen die Wahrscheinlichkeit einer Progression zu Vorläufern von Keimzelltumoren und/oder Krebs bei einem Patienten sehr hoch ist, wobei das Verfahren umfasst
a) Bestimmen des Gehalts an Transkripten eines Markers in einer Probe, wobei der Marker AP-2γ ist;
b) Vergleichen des Gehalts an Transkripten mit einem Referenzgehalt für Transkripte des Markers in normalem Hodengewebe;
c) Feststellen, ob sich der Gehalt an Transkripten vom Referenzgehalt der Transkripte unterscheidet, und
Beurteilen, ob die Probe undifferenzierte Stammzellen, bei denen die Wahrscheinlichkeit einer Progression zu Vorläufern von Keimzelltumoren und/oder Krebs bei einem Patienten sehr hoch ist, enthält, wenn sich der Gehalt an Transkripten vom Referenzgehalt der Transkripte unterscheidet.

13. Verfahren zur Analyse der Gegenwart von undifferenzierten Stammzellen, bei denen die Wahrscheinlichkeit einer Progression zu Vorläufern von Keimzelltumoren und/oder Krebs bei einem Patienten sehr hoch ist, wobei das Verfahren umfasst
a) Bestimmen des Intensitätssignals des Proteins, für das ein Marker in der Probe codiert, wobei der Marker AP-2γ ist;
b) Vergleichen des Intensitätssignals mit einem entsprechenden Referenzintensitätssignal des Markers in normalem Hodengewebe;
c) Feststellen, ob sich das Intensitätssignal vom entsprechenden Referenzintensitätssignal unterscheidet, und
Beurteilen, ob die Probe undifferenzierte Stammzellen, bei denen die Wahrscheinlichkeit einer Progression zu Vorläufern von Keimzelltumoren und/oder Krebs bei einem Patienten sehr hoch ist, enthält, wenn sich das Intensitätssignal vom Referenzgehalt der Transkripte unterscheidet.

14. Verfahren nach Anspruch 13, wobei das Intensitätssignal durch immunozytologischen Nachweis bestimmt wird.

15. Verwendung eines Verfahrens nach einem der Ansprüche 1 oder 2 zur Untersuchung der Gegenwart von Vorläufern von Keimzelltumoren bei einem Individuum einer Population.

## Revendications

1. Méthode permettant de déterminer la présence de précurseurs de tumeurs germinales dans un échantillon, ladite méthode comprenant les étapes consistant à :
a) déterminer le niveau de transcrits d'un marqueur dans ledit échantillon, où le marqueur est AP-2γ ;
b) comparer ledit niveau de transcrits avec un niveau de référence de transcrits dudit marqueur dans du tissu testiculaire normal ;
c) identifier si le niveau de transcrits est différent dudit niveau de référence de transcrits, et
évaluer si l'échantillon contient des précurseurs de tumeurs germinales si le niveau de transcrits est différent du niveau de référence de transcrits.

2. Méthode permettant de déterminer la présence de précurseurs de tumeurs germinales dans un échantillon, ladite méthode comprenant les étapes consistant à :
a) déterminer le signal d'intensité de la protéine codée par un marqueur dans ledit échantillon, où le marqueur est AP-2γ ;
b) comparer le signal d'intensité avec un signal d'intensité de référence correspondant dudit marqueur dans du tissu testiculaire normal ;
c) identifier si le signal d'intensité est différent dudit signal d'intensité de référence correspondant, et
évaluer si l'échantillon contient des précurseurs de tumeurs germinales si le signal d'intensité est différent du signal d'intensité de référence correspondant.

3. Méthode selon l'une quelconque des revendications 1 à 2, où le marqueur figure dans SEQ ID N° 20.

4. Méthode permettant de surveiller l'évolution de précurseurs de tumeurs germinales en cellules cancéreuses avérées chez un individu, comprenant les étapes consistant à :
a) déterminer le niveau de transcrits d'un marqueur dans un échantillon, où le marqueur est AP-2γ ;
b) comparer ledit niveau de transcrits avec un niveau de référence de transcrits dudit marqueur dans du tissu testiculaire normal ;
c) identifier si le niveau de transcrits est différent dudit niveau de référence de transcrits, et
évaluer le stade de développement des précurseurs de tumeurs germinales en cellules cancéreuses avérées si le niveau de transcrits est différent du niveau de référence de transcrits.

5. Méthode permettant de surveiller l'évolution de précurseurs de tumeurs germinales en cancer avéré chez un individu, comprenant les étapes consistant à :
a) déterminer le signal d'intensité de la protéine codée par un marqueur dans un échantillon, où le marqueur est AP-2γ ;
b) comparer le signal d'intensité avec un signal d'intensité de référence correspondant dudit marqueur dans du tissu testiculaire normal ;
c) identifier si le signal d'intensité est différent dudit signal d'intensité de référence correspondant, et
évaluer le stade de développement des précurseurs de tumeurs germinales en cellules cancéreuses avérées si le signal d'intensité est différent du signal d'intensité de référence correspondant.

6. Méthode selon l'une quelconque des revendications 4 ou 5, où ladite méthode fait la distinction entre séminome et carcinome *in situ.*

7. Méthode selon l'une quelconque des revendications 4 ou 5, où ladite méthode fait la distinction entre non-séminome et carcinome *in situ.*

8. Méthode permettant d'identifier des précurseurs de tumeurs germinales dans un échantillon provenant d'un mammifère, la méthode comprenant les étapes consistant à :
tester ledit échantillon en réalisant un test de détection quantitative et déterminer le niveau de transcrits et/ou le signal d'intensité d'un marqueur, où ledit marqueur est AP-2γ ;
comparer ledit niveau de transcrits et/ou le signal d'intensité avec une valeur de référence dudit marqueur dans du tissu testiculaire normal ;
identifier si le niveau de transcrits et/ou le signal d'intensité du marqueur provenant de l'échantillon est significativement différent de la valeur de référence,
et
évaluer si l'échantillon contient des précurseurs de tumeurs germinales si le niveau de transcrits et/ou le signal d'intensité est différent de la valeur de référence.

9. Méthode permettant de déterminer la présence de précurseurs de tumeurs germinales chez un individu comprenant la détection de la présence d'un produit d'expression, où ledit produit d'expression comprend une séquence nucléotidique d'AP-2γ dans un échantillon prélevé sur l'individu.

10. Méthode permettant d'évaluer l'efficacité d'un traitement destiné à inhiber les précurseurs de tumeurs germinales chez un sujet, la méthode comprenant l'étape consistant à :
comparer le niveau de transcrits d'un marqueur dans un premier échantillon prélevé sur le sujet avant d'administrer au moins une partie du traitement à ce sujet au niveau de transcrits d'un marqueur dans un deuxième échantillon prélevé sur le sujet après l'administration d'une partie du traitement, où un niveau de transcrits du marqueur significativement altéré dans le deuxième échantillon, par comparaison au premier échantillon, indique que le traitement permet d'inhiber efficacement les précurseurs de tumeurs germinales chez le sujet
où le marqueur est AP-2γ.

11. Méthode permettant d'évaluer l'efficacité d'un traitement destiné à inhiber les précurseurs de tumeurs germinales chez un sujet, la méthode comprenant l'étape consistant à :
comparer le signal d'intensité de la protéine codée par un marqueur dans un premier échantillon prélevé sur le sujet avant d'administrer au moins une partie du traitement à ce sujet au signal d'intensité des protéines codées par un marqueur dans un deuxième échantillon prélevé sur le sujet après l'administration d'une partie du traitement, où un signal d'intensité de la protéine codée par le marqueur significativement altéré dans le deuxième échantillon, par comparaison au premier échantillon, indique que le traitement permet d'inhiber efficacement les précurseurs de tumeurs germinales chez le sujet
où le marqueur est AP-2γ.

12. Méthode permettant d'analyser la présence de cellules souches indifférenciées hautement susceptibles d'évoluer en précurseurs de tumeurs germinales et/ou en cancer chez un individu, ladite méthode comprenant les étapes consistant à :
a) déterminer le niveau de transcrits d'un marqueur dans un échantillon, où le marqueur est AP-2γ ;
b) comparer ledit niveau de transcrits avec un niveau de référence de transcrits dudit marqueur dans du tissu testiculaire normal ;
c) identifier si le niveau de transcrits est différent dudit niveau de référence de transcrits, et
évaluer si l'échantillon contient des cellules souches indifférenciées hautement susceptibles d'évoluer en précurseurs de tumeurs germinales et/ou en cancer chez l'individu si le niveau de transcrits est différent du niveau de référence de transcrits.

13. Méthode permettant d'analyser la présence de cellules souches indifférenciées hautement susceptibles d'évoluer en précurseurs de tumeurs germinales et/ou en cancer chez un individu, ladite méthode comprenant les étapes consistant à :
a) déterminer le signal d'intensité de la protéine codée par un marqueur dans ledit échantillon, où le marqueur est AP-2γ ;
b) comparer le signal d'intensité avec un signal d'intensité de référence correspondant dudit marqueur dans du tissu testiculaire normal ;
c) identifier si le signal d'intensité est différent dudit signal d'intensité de référence correspondant, et
évaluer si l'échantillon contient des cellules souches indifférenciées hautement susceptibles d'évoluer en précurseurs de tumeurs germinales et/ou en cancer chez l'individu si le signal d'intensité est différent du niveau de référence de transcrits.

14. Méthode selon la revendication 13, où le signal d'intensité est déterminé par détection immunocytologique.

15. Utilisation d'une méthode selon l'une quelconque des revendications 1 ou 2 pour dépister la présence de précurseurs de tumeurs germinales chez un individu d'une population.


### SEQUENCE LISTING

<110> Rigshospitalet
   Almstrup, Kristian
   Ansorge, Wilhelm
   Hei-Hansen, Christina E.
   Rajpert-De Meyts, Ewa
   Leffers, Henrik
   Skakketwk, Niels Erik
<120> Panel of gene expression for diagnosis of testicular cancer
<130> 36243PC01
<150> DK-PA 200402032
   <151> 2004-12-30
<150> DK-PA 200400647
   <151> 2004-04-23
<160> 255
<170> FastSEQ for Windows Version 4.0
<210> 1
   <211> 483
   <212> DNA
   <213> unknown·
<220>
   <223> >sr|IMAGp998N195554.2|cl|IMAGp998N195554|gb|BX1087 31|oid|234551841 BX108731 NCI_CGAP_GC6 Homo sapiens cDNA clone IMAGp998N195554 ; IMAGE: 2244690, mRNA sequence
<400> 1
   tggagagctg cgggcaagct cagcctcgaa acacacacac ccacacgaga tgggcacgga 60
   gtagtcttga aagacatttt aataaccttg gctgctaagg acaacattga tagaagccgt 120
   ctctggctat agataagtag atctaatact agtttggata tctttagggt ttagaatcta 180
   acctcaagaa taagaaatac aagtacgaat tggtgatgaa gatgtattcg tattgtttgg 240
   gattgggagg ctttgcttat tttttaaaaa ctattgaggt aaagggttaa gctgtaacat 300
   acttaattga tttcttaccg tttttggctc tgttttgcta tatcccctaa tttgttggtt 360
   gtgctaatct ttgtagaaag aggtcttgta tttgctgcat cgtaatgaca tgagtactac 420
   tttagttggt ttaagttcaa atgaatgaaa caaatatttt tcctttagtt gattttaccc 480
   tga                                                               483
<210> 2
   <211> 683
   <212> DNA
   <213> unknown
<220>
   <223> >ImageID=147103 IMAGP971H0410_S1 CC FromEXP of ImAGp971HO410_s1.exp April 26, 2002 13:06
<221> misc_feature
   <222> 675
   <223> n=a, t, c or g
<400> 2
   caaatggtct aagattgaca gtagcacagt tttactctgt gaaggtaatg ttcaggacaa 60
   atttcaagaa aactagaaaa ccattcttta cagctgaaat ctttccctaa ccattgttat 120
   ttccactttt aagtcctcaa gagatgagaa aagggaggta aggcttcctt atacatttcc 180
   tgcacaatga aacatttttc ctcctccagg caaagattca agcagaactg gcaaatatct 240
   tatcttgctc ttctcaataa taataatgtt gttagataat aaagttctat agcaatttaa 300
   ccctagaatc tttttgaaaa gtaattcttt aaagttgaga atcacagctg tctagcaagc 360
   atttccttgg gcacttgaag ctgtttattc actttggtct ttcctcccag gggaatgaag 420
   tgaggccagt ggccgggtct gctgagtccg cagcactcag actacgtgca cctctgcagc 480
   aggtgcaggc ccagttgtca cccctccaga acatctcccc atggattctg gcggtattga 540
   ctcttcagat tcagaggtag ggatcattct gacttattaa agagctatat aaccagttaa 600
   ttccatctgt ttgatgcttg acatccctaa ctagacagat gagggttgaa gttagttttt 660
   ggtggggttt ggganggtga aca                                         683
<210> 3
   <211> 718
   <212> DNA
   <213> unknown
<220>
   <223> >sr|IMAGp998F08222.1|cl|IMAGp998F08222|gb|BX091352 |oid|234517071 BX091352 Soares placenta Nb2HP Homo sapiens cDNA clone IMAGp998F08222 ; IMAGE:146695, mRNA sequence.
<400> 3
   atttttttaa aagactatcc ccttcatcac tgtgctttgt ccctttgtct aaagtcggtt 60
   gttcatatat gtgggtctgt tccgtagact ttattcagtt ctattgatct tttttgatat 120
   gatgccagta ccatactctg ttagtaacga tagtgtttta ataatacatc ttgaaattag 180
   gaagtataga gttattttgc ctatagtaga tcctttattt tgcattttag aatcagctta 240
   agagtttctg caaaaatacc tgcaggaatt ttgatggaga ttgcattgat tctctagatc 300
   aatttgggga gaattttaac aatattgagt attttgacat atgagtgcaa tatgcctgtc 360
   attcattcag gtctttaatt tctctcagga ttgatctttt taaaagtaaa ccctttattt 420
   tgggataatt ttcatttttt tcccccttca tgtcacagtt tttcatttta tttttccccc 480
   tccatgtcac agttttgatc atggaataat ttttcaagat ctgagagcta cagtgaaaag 540
   gaaaaaaaga aagaaagaaa acatgatact atagtccctg atgattgatt ttttttaatc 600
   ccaatcatct gtaattcttc ccagagataa gaaatggttg tagtatgtta tctcctagga 660
   attttttttg gaattcaaac agataaataa aatatactca tagaaaaaaa aaaaaaaa   718
<210> 4
   <211> 461
   <212> DNA
   <213> unknown
<220>
   <223> >gi|855247|sr|IMAGp998AO6220s1|cl|IMAGp998A06220|g b|R78966|R78966 yi83b03.r1 Homo sapiens cDNA clone 145805 5'.
<221> misc_feature
   <222> 359, 371, 383, 406, 428, 442, 452, 460
   <223> n=a, t, c or g
<400> 4
   agccaactga acctgctaat tgccagaatt aggcatattc ggctgatctg tcacaggaaa 60
   tgctgggctg gagcaggcct cccatgtttt acaacctgcc tacataagac ataagcatct 120
   aggagcagag aatccctcct cctttcaagc agctctttcc caacagaata aagggaagtc 180
   actgagctgt gaatgcctgt tctattcaga tttacctctt agataagttg ttccagctac 240
   cccgggaagg aggggtaaag gggatgagat gtttgaactg taacaccctg tacatgggag 300
   gagacaacat gagttgttga tgggagcatc cttccattct tacctttttg ggcttttana 360
   aaaactttta ntggcagtgc agnaaaatat ttgaaaattg tttttnacag ggtttttgaa 420
   ggccattncc caggctgctg gnaaatcctg gnaaaattgn t                     461
<210> 5
   <211> 305
   <212> DNA
   <213> unknown
<220>
   <223> >sr|IMAGp998D203167.2|c1|IMAGp998D203167|gb|BX1016 98|oid|234537773 BX101698 NCI_CGAP_GC4 Homo sapiens cDNA clone IMAGp998D203167 ; IMAGE:1258339, mRNA sequence
<400> 5
   aaccaaagct ttgtgtaaaa tcttgaattt atggggcggg agggtaggaa agcctgtacc 60
   tgtctgtttt tttcctgatc cttttccctc attcctgaac tgcaggagac tgagcccctt 120
   tgggctttgg tgaccccatc actggggtgt gtttatttga tggttgattt tgctgtactg 180
   ggtacttcct ttcccatttt ctaatcattt tttaacacaa gctgactctt cccttccctt 240
   ctcctttccc tgggaaaata caatgaataa ataaagactt attggtactc aaaaaaaaaa 300
   aaaaa                                                             305
<210> 6
   <211> 628
   <212> DNA
   <213> unknown
<220>
   <223> >sr|IMAGp998D054091.2|c1|IMAGp998D054091|gb|BX0925 81|oid|234519530 BX092581 NCI_CGAP_GC3 Homo sapiens cDNA clone IMAGp998DOS4091 ; IMAGE:1613140, mRNA sequence
<221> misc_feature
   <222> 600
   <223> n=a, t, c or g
<400> 6
   ataagatggg aactctcccg gcacgtagta catatcccgc cgtgggtgaa agttcccgaa 60
   gacctgaaag atccagaggt gttccaggtc cagacgcggc tgctgaaagc cattttcggc 120
   ccggacggat ctcgaatccc ttacatcgag caggtgagca aggccatgct cgagctgaag 180
   gctctggagt cttcagacct caccgaggtc gtggtttacg gctcctattt gtacaagctc 240
   cggaccaagt ggatgctcca gtccatggct gagtggcacc gccagcgcca ggagcgaggg 300
   atgctcaaac ttgccgaagc catgaatgcc ctcgaactag gcccttggat gaagtgaacc 360
   agtttccagc caatgcaatg aagccgggtt gcagagatta ggttgtggcc agagctagag 420
   tgattcctta agcttgtttt aaaatctgct ccagcctaaa gagttaaggg aaaaccattt 480
   gttcccttaa agagttaagg gaaaaccctt ggctctgagt cttgttgtga atatttcttt 540
   gatgattgtt aataaaaagt gttttttctt ttttcccatt tttaaaaata acaataaagn 600
   tttaaataag ttgaaaaaaa aaaaaaaa                                    628
<210> 7
   <211> 545
   <212> DNA
   <213> unknown
<220>
   <223> >gi|1526341|gb|AA046430|sr|IMAGp998K241161.1|c1|IM AGp998K241161|AA046430 zk70f12.s1 Soares_pregnant_uterus_NbHPU Homo sapiens cDNA clone IMAGE:488207 3' similar to PIR:A54560 A54560 TPA-induced protein OTS-8 - mouse ;contains element PTR5 repetitive element ;, mRNA sequence
<221> misc_feature
   <222> 414
   <223> n=a, t, c or g
<400> 7
   tttttcacat ggttacagca tgtaatatcg atccaatggt ccagtgttag ttacaaatca 60
   gtttatacag ttttgctcca ttttctatga attttgtctt tcaaataatc ttttatagaa 120
   tgtatattca aaaacatgtt tgaacagcaa atgtttagag gagccaagtc tggtgaacgg 180
   tctttccttc ggttattttg gcaagcggag aggtcaccgt ggattctgag tgggccaggc 240
   aagtgttcca cgggtcatct gctcccacga gctcagggac agggcacaga gtcagaaacg 300
   gtcttttttt aagcacgttg gcagcagggc gtaacccttc agctctttag ggcgagtacc 360
   ttcccgacat ttttcgcata accacaacga tgattccacc aatgaagccg atgntagtaa 420
   gaccccaact atgattccaa ccagggtcac tgttgacaaa ccatctttct caactgttgt 480
   ctgtgtgtct ccatccactt tctccgtgga gtgactggtg gccacgtttg aggctgcctc 540
   gtgcc 545
<210> 8
   <211> 223
   <212> DNA
   <213> unknown
<220>
   <223> >gi|4682406|gb|AI631076|sr|IMAGp998J095673.1|cl|IM AGp998J0956731A|631076 tz32c05.x1 NCI_CGAP_Ut2 Homo sapiens cDNA clone IMAGE:2290280 3', mRNA sequence.
<400> 8
   tttttttttt tttttttttt tttttttttt tttttttttt tttttttttt tttttattgg 60
   gaaaggttta tttaaaggtt tgggggcccc ttttaccccc ccccaaaaag aaaaaaaaaa 120
   attttttttt tgccgtccca aacagggaag tcccttcaaa gaggagctgg cgggtgggtt 180
   caaaccccaa aagggggacc cctgccaggg ggtttttccc ggc 223
<210> 9
   <211> 491
   <212> DNA
   <213> unknown
<220>
   <223> >sr|IMAGp998I175722.2|cl|IMAGp998Il75722|gb|BX0978 70|oid|234530113 BX097870 NCI_CGAP_GC6 Homo sapiens cDNA clone IMAGp998I175722; IMAGE:2309080, mRNA sequence
<400> 9
   gcacccaggc agcctgcgcg gagaaatcgg atcggctagg acggcctgca gcccctgcgc 60
   gcggggaaag ggaagaagtc ctcccctaca aagcgagttc acaaacttgg aagaagccac 120
   tttcacagga tgcacagatc tcaacggaag gacacagaaa acatgaaaaa gcaaggaaat 180
   ggggcgcctc caacggaacc cagtaattct ccagcagcag atccccatcc aaaagaaaca 240
   gaagacattt tggagagaga attgaatata catattatac agttgtgggc ataatacaag 300
   catcatttgg aaaagaacag agaaaaaaga tggaaggagt taccgtactg caggaaaaaa 360
   ttatcaactc cagtgatatt attaaataca acctaacgac acttctgaag ctgaagaaat 420
   cactggatga aatacaaagt aaactcaaaa gcttcagtga tagactagaa caagaagaag 480
   aaagactctc a 491
<210> 10
   <211> 296
   <212> DNA
   <213> unknown
<220>
   <223> >gi|4682412|gb|AI631082|sr|IMnGp998J105673.1|cl|IM AGp99BJ105673|AI631082 tz32d05.x1 NCI_CGAP Ut2 Homo sapiens cDNA clone IMAGE:2290281 3', mRNA, sequence.
<400> 10
   tttttttttt tttttttttt tttttttttt tttttttttt tttttttttt tttttattgg 60
   gaaaggttta tttaaagggt tggggtcccc ttttaacccc ccccaaaaag agaaaaaaaa 120
   attttttttt tgccgttcca aacaggaaag tcctttcaaa gaggagctgg cgtgtgggtt 180
   caaagcccaa gagggggacc cctgccaggg ggtttttccc ggcctgggcc accctgggta 240
   ggagggccac ccaggccccg gtttcctggg accctggggg ttttggaggg agggag 296
<210> 11
   <211> 550
   <212> DNA
   <213> unknown
<220>
   <223> >gi|4293562|gb|AI436660|sr|IMAGp998N115245.1|cl|Im AGp998N115245|AI436660 th91c06.x1
   Soares NSF_FB_9W_OT_PA_P_S1 Homo sapiens cDNA clone IMAGE:2126026 3' similar to TR:P70423 P70423 CATIONIC AMINO ACID TRANSPORTER. ;, mRNA sequence.
<400> 11
   ttttattttt ctactttctt ttaatatcat tttttaaagt tggtaagcag ctagacatca 60
   tttagaagca gacgggttaa aatagacaag aaatagcaaa gacacatcct tcacatcgta 120
   cagaactgta ttagtatcca ccaccaccat cacaggggag ggctagctgt cactggggtc 180
   aggagtactc tccattattg tgcaggggac cagacagcat ttaggtgtga cgatgtcaaa 240
   ctgagtggac atagagagtg ccgggatcaa ggtctacagt tttggctcta gacttgcgtg 300
   agggttggtt actcttaatc tcttccaggc tgtgctggat cccatagccg aagtagatag 360
   caaagccaat cagcatccag accccaaatc gggcccaggt accagctgtc atctgcatca 420
   taaggtaaat attcacaaag atgctcatta gtgggaggag aggcaaagca ggcaccttaa 480
   agtgaagggg agtggaactc tgtggctgtc ttcagatgac cacaatgatc ccaataatga 540
   gcagcaggag 550
<210> 12
   <211> 618
   <212> DNA
   <213> unknown
<220>
   <223> >sr|IMAGp998J05529.1|cl|IMAGp998J05529|gb|BX091496 |oid|234517359 BX091496 Soares fetal liver spleen 1NFLS Homo sapiens cDNA clone IMAGp998J05529; Image:245476, mRNA sequence.
<400> 12
   agtcattgac ggtgaataca ggtctggaag tctgaggtcc aatataataa aaaatctagt 60
   cttcaccttc tatcctaggg atagacatta ggaatatttg aggatgttgt tcttggtctt 120
   cacgttcatg cattttccct ctatttttat aattggatca atgaatcaat aagcaaatat 180
   attttaactg cataaagata aaaataggcg ttgtccaatt atggaaatat atggtgaaat 240
   aagaaatact attcacattt attaaaaaca ctgaggtctt ctttctatta acaaataaat 300
   gactcagagg tattactcgc ttgctgcagg atggcatata cagtaaaaat aataataacg 360
   tatgtgaggt catttgggtc aggaccatgt tcatggttgt ttgttttctc aagaaaaaat 420
   agtttcacac atacttacat gactaataga atgaaaggag aaatgtgtgg ataaatggac 480
   agaatatttc catcttttta tcttttttgc tttgtactct cacaatgaac accctcaagt 540
   tgacttaagc acccttccat ggtcatgagg tttctggttg cctctgtgtg ttagaagctt 600
   ggagaccaat gccttctg 618
<210> 13
   <211> 713
   <212> DNA
   <213> unknown
<220>
   <223> >sr|IMAGp998AO3577.1|cl|IMAGp99BA03577|gb|BX102059 |oid|234538498 BX102059 Soares melanocyte 2NbHM Homo sapiens cDNA clone IMAGp998A03577 ; IMAGE:263690, mRNA sequence
<400> 13
   ttaaatccag tgtggaatag ctgccttagg accttgaccc acacttactt taagaaatcg 60
   ttctagttct taaatttcaa tggtaaagcc agaggctggt gcttgaaaag tatttacagt 120
   gctcaggcag cactttctgc agttacagat tctttactgc ccctttttat gtctgaactt 180
   tacatactgg attactcgtg tcaataggag aaggaacagt gccttgattt aacacgtcct 240
   tgtaatagtc gtggtccttc tcttctggga aaggagaaga aatagtgccc ctggagtccc 300
   attgccttgg gaggcatatg gctcgtttca gtgattcctt gagaattctg gtaagaagtc 360
   aaaaataagc atttaaataa aaagaatagt aaaaaccaca gtaaggaaag agcttctgat 420
   ccatttagta aaataaaaca tacattttat aaggtagaaa tattaaatag aaccatttat 480
   tactctaaat tctatcctta tttatctctc aaaataatag aacagtgcca cttttgataa 540
   tttttctatt tgcacaccca aaatgcctgt tttaaaatat ataatagaga atgaagaaat 600
   ttggggaatt tcagaccaac aatgtgttta tattcatgta agtctgattt tcaaatcagt 660
   gtaaattgtc agtaatttcc ataatacata ttggcctagt tctgtatgct tac        713
<210> 14
   <211> 335
   <212> DNA
   <213> unknown
<220>
   <223> >gi|3756508|gb|AI203902|sr|IMAGp998H234408.1|cl|IM AGp99BH234408|AI203902 qd71g12.x1
   Soares_testis_NHT Homo sapiens cDNA clone IMAGE:1734982 3' similar to contains L1.t2 L1 repetitive element ;, mRNA sequence.
<400> 14
   tttttttttt tttttaaaca taccctttat taacatctag gtaatatctg taatattcct 60
   tgctcctcat ccccaagtac gctattagct gtccatcctt ctgggtagaa gtgtattttc 120
   gttttacttg ttgatttttg gatgcatgct gggggaggaa agcatattgt ttgtagtcac 180
   cctggcgtgc taaggtatat tattccccag taattctctc aaggtgggca tatgcaaaac 240
   ataatctcta aattcttcaa tactaagaaa tacctttgtt ttacccctaa aatcaaatgc 300
   cattttggct ggatatagga ttctaggatt aaagc                            335
<210> 15
   <211> 565
   <212> DNA
   <213> unknown
<220>
   <223> >sr|IMAGp998GI3226.1|cl|IMAGp998Gl3226|gb|BX093404 |oid|234521175 BX093404 Soares placenta Nb2HP Homo sapiens cDNA clone IMAGp99BG13226 ; IMAGE:148260, mRNA sequence
<400> 15
   tttttttata taacagaggt tatcttggaa gagagatagc aaacaatttc tgtgtgtctt 60
   tgtgaccttt aaattcaaaa caaaaaaaat aagccactct tttaaaagtg ctcagtctaa 120
   aaaataaaca cgtagtctag aggcggcaag gcccgtgtgt gaagcctggc tcttcctcgg 180
   ctaagctgtg tgacggtcgg caagttgctt accttcttga agctgcattc tcctcattct 240
   taaaataaaa atggtaataa caggaccgcc tcattgagct tgtgtgaaga gtaacagaca 300
   actgcagcca agtccccaga aggtgcctgg cacatagtga gggcttgata tgaggtagct 360
   attatcagga catgattcct gtaatcttca cattttttta ctttgacaat caaaaagcca 420
   atacagataa ttgaaaagac aagaaaagtg acatcagcag gctccttagc ccaaaattca 480
   ccccctccct ctctcgaccc agagctccac ttgagaaggg cgggtggcag acacacctct 540
   tatatttagg aatattcctg cggcc 565
<210> 16
   <211> 580
   <212> DNA
   <213> unknown
<220>
   <223> >sr|IMAGp998J23139.1|cl|IMAGp998J23134|gb|BX092461 |oid|234519290 BX092461 Soares fetal liver spleen INFLS Homo sapiens cDNA clone IMAGp998J23134 ; IMAGE:129142, mRNA sequence.
<221> misc_feature
   <222> 17
   <223> n=a, t, c or g
<400> 16
   caactatttg ccccagnaca actgtttttc ccccttcctt ctatggattg accagccagt 60
   tctgctttag atggtattgg atggagccct ggaatggctg aaaagtccaa actggcctga 120
   cttgctcagc cagcaatgcg gtgcaggctc ctagctgggg ccttagttct cctgcacctg 180
   cggctctcca tatggctgct tggacttcct catagcatgg cagatggatt acgagaagga 240
   gcattcccaa ataaaggacc acataagcta gatctctgga gggctagcct cagaagtcac 300
   ccagtgtcac atgggccaca ttttattggt tacagggcca gccagtttga aggggaagag 360
   aaatatgtcg ctgtttatgc tgtgtccagt gctagcttgc tacctgctct cccagttcca 420
   gtgctcaggg cagcactggc agaacagatg tacttactga gttaaaaaca gcaacatcca 480
   agacaattgt taacttttaa aactgtctcc catcccagaa ggtataacta aaaaactaac 540
   aataaaaata atagtaataa ataataaaaa aaaaaaaaaa 580
<210> 17
   <211> 306
   <212> DNA
   <213> unknown
<220>
   <223> >gi|4987550|gb|AI699650|sr|IMAGp998K225545'.|cl|IM AGp998K225595|AI699650 tt18f11.x1 NCI_CGAP_GC6 Homo sapiens cDNA clone IMAGE:2241165 3', mRNA sequence.
<400> 17
   gtcacgcacc gcacgttcgt ggggaacctg gcgctaaacc gacagattta acaaagggag 60
   tgatgtgtga tcacatatgt gttcttaaaa gattgttatg catgtttcat gtagaataga 120
   ttagggaaga tcagggaaga tacagggaga caatatactc ctgctgtagt agtcaagcca 180
   gaaatcctgt tgcctggatc cagggttgtg gcagtagaaa aatagatcaa aagtagataa 240
   aatttgcagt agtaattaac tgtattggag catttgaaaa ggatgattta aaaaaaaaaa 300
   aaaaaa 306
<210> 18
   <211> 443
   <212> DNA
   <213> unknown
<220>
   <223> >gi|525834|gb|AA045940|sr|IMAGp998N121162.2|cl|IM AGp998N121162|AA045940 zk75d06.r1 Soares_pregnant_uterus_NbHPU Homo sapiens cDNA clone IMAGE:488651 5', mRNA sequence
<221> mise_feature
   <222> 351
   <223> n=a, t, c or g
<400> 18
   ggaccagcat gccggtgggg tgcaggcgcc atttgtcttg gaagtcaatt tccttcagga 60
   gacaaagcaa gtttggggtg gtagccttaa tgcccagcac cttggattga ttcccatggt 120
   ctaagagggt ctttttgact aaggccagcc tgtggtgatc ccagcagctt tggagggcct 180
   gcagccacac actgactgat acatggtatg ttacagggac ttgtcacttt ggatcaggct 240
   gggcactaca gggcctgatg ggcaaacttg ggtaatcact gggaggaggg aaactgaggc 300
   ccgaggcaga tacctgggtt tggggagggc tgggtttagc ccggttctct naactacata 360
   cccccagctg ttaagccttc ctcttgaggg gttgaggtgg gcagtataca ggagccaagc 420
   agcccagggt ggggtcgcag ccc                                         443
<210> 19
   <211> 509
   <212> DNA
   <213> unknown
<220>
   <223> >sr|IMAGp99SH07676.3|cl|IMAGp99SH07676|gb|BX100963 |oid|234536302 BX100963 Soares_fetal_lung_NbHL19W Homo sapiens cDNA clone IMAGp998H07676 ; IMAGE:301878, mRNA sequence.
<400> 19
   gaaccctggc ttggtggatt ttgctagatt tttctgattt ttaaactcct gaaaaatatc 60
   ccagataact gtcatgaagc tggtaactat cttcctgctg gtgaccatca gcctttgtag 120
   ttactctgct actgccttcc tcatcaacaa agtgcccctt cctgttgaca agttggcacc 180
   tttacctctg gacaacattc ttccctttat ggatccatta aagcttcttc tgaaaactct 240
   gggcatttct ttgagcacct tgtggagggg ctaaggaagt gtgtaaatga gctgggacca 300
   gaggcttctg aagctgtgaa gaaactgctg gaggcgctat cacacttggt gtgacatcaa 360
   gataaagagc ggaggtggat ggggatggaa gatgatgctc ctatcctccc tgcctgaaac 420
   ctgttctacc aattatagat caaatgccct aaaatgtagt gacccgtgaa aaggacaaat 480
   aaagcaatga atacaaaaaa aaaaaaaaa                                   509
<210> 20
   <211> 775
   <212> DNA
   <213> unknown
<220>
   <223> >sr|IMAGp99OB171780.3|cl|IMAGp9988171780|gb|BX1084 62|oid|234551303 BX108462 Soares ovary tumor NbHOT Homo sapiens cDNA clone IMAGp998B171780 ; IMAGE:725680, mRNA sequence
<400> 20
   cctgattgtc atagacaaat cctacatgaa ccctggagac cagagtccag ctgattctaa 60
   caaaaccctg gagaaaatgg agaaacacag gaaataaaat tggaacgaag aaaggttagg 120
   agagtaggga aggaacagga ctgcaaaaat ccttctccac cgcacagact gggaacccct 180
   cctggcctgg gggaagagtt tgttacctac cttactattt aaagagcctt cactggttct 240
   gcatcacccg cccctggact tcttagttgt ttctctagcg ctgagctatc tcctaacttt 300
   ggacctatta tcagaaggtg acaagtactg gctctttatt cattaagctt tttttttttg 360
   aaccccattc tttccttctc tgaaagtggt gctataagtt ttagaatctt ttaaatacat 420
   tccctgggcc aacagaccca cacacttagc cattgaaatg tcaaattgat gtgccctaga 480
   tcaacagatc aacaatacct tttttttcag tgttaaggta atggttggtt tttgtgtccg 540
   ctaaatattt accttgaaaa aaagaaaagt gtgtatctag cttcttcaga gatcaagtcc 600
   tctggtagga ggcaaaggtt ctatctgctt agcaactagt taataagtgg tatctgacac 660
   actctaaacc ccgtgttcaa acgggggcct tctggtttta ggaaacttgt agaaacgaag 720
   cctgctgatt gatttttttc tccttttttt tttttttttt ttttaacttt ggaag 775
<210> 21
   <211> 773
   <212> DNA
   <213> unknown
<220>
   <223> >sr|IMAGp998A2488.1|cl|IMAGp998A2488|gb|Bx098995|o id|234532364 BX098995 Soares fetal liver spleen 1NFLS Homo sapiens cDNA clone IMAGp99BA2488 ; IMAGE:111 647, mRNA sequence
<221> misc_feature
   <222> 20
   <223> n=a, t, c or g
<400> 21
   atgaatgcag aacctcaagn ctacctgtgt caatatcaat gtgtcaatga acctgggaaa 60
   ttctcatgta tgtgccccca gggataccaa gtggtgagaa gtagaacatg tcaagatata 120
   aatgagtgtg agaccacaaa tgaatgccgg gaggatgaaa tgtgttggaa ttatcatggc 180
   ggcttccgtt gttatccacg aaatccttgt caagatccct acattctaac accagagaac 240
   cgatgtgttt gcccagtctc aaatgccatg tgccgagaac tgccccagtc aatagtctac 300
   aaatacatga gcatccgatc tgataggtct gtgccatcag acatcttcca gatacaggcc 360
   acaactattt atgccaacac catcaatact tttcggatta aatctggaaa tgaaaatgga 420
   gagttctacc tacgacaaac aagtcctgta agtgcaatgc ttgtgctcgt gaagtcatta 480
   tcaggaccaa gagaacatat cgtggacctg gagatgctga cagtcagcag tatagggacc 540
   ttccgcacaa gctctgtgtt aagattgaca ataatagtgg ggccattttc attttagtct 600
   tttctaagag tcaaccacag gcatttaagt cagccaaaga atattgttac cttaaagcac 660
   tattttattt atagatatat ctagtgcatc tacatctcta tactgtacac tcacccataa 720
   ttcaaacaat tacaccatgg tataaagtgg gcatttaata tgtaaagatt caa 773
<210> 22
   <211> 623
   <212> DNA
   <213> unknown
<220>
   <223> >sr|ILMAGp998H01159.1|cl|IMAGp998H01154|gb|BX102894 loid|234540168 BX102894 Soares infant brain 1NIB Homo sapiens cDNA clone IMAGp998H01154 ; IMAGE:32759, mRNA sequence
<400> 22
   tttttttttt ttttaaaggg aaagttaatt tttaatacaa atacaaaatt tatttccata 60
   gaaagcaaaa tttataagtt aaatataaag attaaaatag tgtttgaaaa gctcactata 120
   gtgctaacaa actaaatgaa aataaggcta ttttgcattt aaaactgaaa tacctcttaa 180
   aataatttga tccccagtgt ttgctctttt tgaagtaacc aacttactct taaaaaggat 240
   ggctgccaag atggaaagtc ttactgggtt ttcatgttaa cctattcttt ggacataact 300
   atgaattttg tatacaatgc acttcatgaa aagttgtggc tcccccagat tgcccacaag 360
   tgtgatcttg aagtcctaaa catttgtcca tgtaagcttc aaaacagcgt taactgagtt 420
   attcaagtag cagtacttaa agatacaatt cttgaagcag tttcaatggt ttctgatcca 480
   aataatcagt ttctgaacat tactacttca cataatagag tccatcttca gtttcttctc 540
   actttctctt tcccttttgg gtttcctttt tgtggcctga ggccaccagt tctttgggta 600
   ctatcaagat acttccatca tgg                                          623
<210> 23
   <211> 531
   <212> DNA
   <213> unknown
<220>
   <223> >sr|IMAGp998J02274.1|cl|IMAGp998J02274|gb|BX107144 |oid|234548669 BX107144 Soares infant brain 1NIB Homo sapiens cDNA clone IMAGp998J02274 ; IMAGE:46959, mRNA sequence
<221> misc_feature
   <222> 14, 430, 431, 434, 435, 521
   <223> n=a, t, c or g
<400> 23
   tttttttttt tttnctgatt ctattacttt tattaaatag tgggtttcca cacatggctt 60
   tttaaataat ccaggcagga gaagagagga gggcacactt ggaactcccc tccccacaat 120
   acgtgattat ttacatttta gtaattggac aatcccggct caggaggagg ttgcaagaat 180
   ctgcaaaagt tggagggagc gccccaggag aacaaacagc aagccttatt tcccctagcc 240
   catcccccaa aaaaccatcc atcccatcct agtgtctggt ggtgtccggt ggtgtccatc 300
   ttccattcct tcccaaatta tggaagtaag gttcttctca ccagaataag agcacttggg 360
   ataacagagt agggtcccct cacccaaaaa aaaaaaaaaa aaaaagaagc cttggggtaa 420
   caacagggcn nttnncctcc cccagaataa agaatcctgg gctgaggcag ctaagcagct 480
   tggcccaata tgggacccta ggctagggga aagggtcccc nttactaaaa t          531
<210> 24
   <211> 742
   <212> DNA
   <213> unknown
<220>
   <223> >sr|IMAGp998L10121.1|cl|IMMAGp998L10121|gb|BX111578 - |oid|234557533 BX111578 Soares fetal liver spleen 1NFLS Homo sapiens cDNA clone IMAGp998L10121 ; IMAGE:124569, mRNA sequence
<221>misc_feature
   <222> 721
   <223> n=a, t, c or g
<400> 24
   acctggcctg aatctatgta ctttcatcag cgcggccagt aagtttgcac agctccagtg 60
   tctgtggaag acgagagact caggaactgt gagtgcgctg acttggagcc tctcttccta 120
   tacctgtgca acaagaataa tcacaacctt aatgaccacc aatgatttta caattcttct 180
   acgttttgtg atcatgctgg ctttaaatat atgggtaaca gtgacagtac ttcgctaccg 240
   gaagaccgct ataaaggctg aatgatggat acattattcc ttcacacagt ggattttgag 300
   taactgaacc aaaggaaaaa gaagctcttt gctaaattaa ggtcttttat aaatttagta 360
   aatcagttta taatctttaa agccaaaggt ttttttagac ttgaaagaaa gagccactta 420
   aattcttgtt taaaaatacc aatttgcctc ctccttcctc acttcgttag gttatggtag 480
   tgctcagaca tctgcagtgt tgagaaggcc agtcactgtt ggaagtcatc caagaagccc 540
   attttgagcc ttactcttaa gttctctatg aagaactaca ttgatttgtt ggctttcaga 600
   atcttttagg aaataaatcc tctccaggac aaaaatgaac atgaatggag tggcattttg 660
   ttccaagtca gaggtgggca cctataataa atgactaggg ttcactttct gggactgatg 720
   nttaattgta acacagatac aa                                         742
<210> 25
   <211> 455
   <212> DNA
   <213> unknown
<220>
   <223> >gi|4735957|gb|AI6519781sr1IMAGp998J075711.|cl|IM AGp998J0757111A|651478 wb06c04.x1 NCI_CGAP_GC6 Homo sapiens cDNA clone IMAGE:2304870 3', mRNA sequence.
<400> 25
   tttttttttt attttttaca tatgtaatta taatgaactg aaagatatat atatatatca 60
   aaaggaacaa aaaaatgact tcaaattcag actagaattg ctcaactatc aattgctact 120
   gatcaatcat ctttttaata acatcaaata attaggtcta ataactacat acaactaaac 180
   agctgtctaa aggagatgct gttaaagaac tgttccaggt caaaatgacc aagctgttaa 240
   attatactca aagtaaaaaa caaacacaat aaaaaatatt taaaaaaaca aacaaacaca 300
   atagtagata ctgaaataat tatcaatccc ctgcaagtag atggatgtac ttaaggagac 360
   tcaaaattct cgggtgtggg tgggatacat tgggaaggct gttttaacac tggagccttg 420
   catggatttc aggaggttcc tgaatctact gaaat                           455
<210> 26
   <211> 704
   <212> DNA
   <213> unknown
<220>
   <223> >sr|IMAGp998N06536.1|cl|IMAGp998N06536|gb|BX103225 |oid|234540830 BX103225 Soares fetal liver spleen 1NFLS Homo sapiens cDNA clone IMAGp998N06536 ; IMAGE:248261, mRNA sequence
<400> 26
   gtcttaaaca agccacggaa actgcgatat taaatgccaa ctacatggcc aagcgattag 60
   aaacacacta cagaattctt ttcaggggtg caagaggtta tgtgggtcat gaatttattt 120
   tggacacgag acccttcaaa aagtctgcaa atattgaggc tgtggatgtg gccaagagac 180
   tccaggatta tggatttcac gcccctacca tgtcctggcc tgtggcaggg accctcatgg 240
   tggagcccac tgagtcggag gacaaggcag agctggacag attctgtgat gccatgatca 300
   gcattcggca ggaaattgct gacattgagg agggccgcat cgaccccagg gtcaatccgc 360
   tgaagatgtc tccacactcc ctgacctgcg ttacatcttc ccactgggac cggccttatt 420
   ccagagaggt ggcagcattc ccactcccct tcgtgaaacc agagaacaaa ttctggccaa 480
   cgattgcccg gattgatgac atatatggag atcagcacct ggtttgtacc tgcccaccca 540
   tggaagttta tgagtctcca ttttctgaac aaaagagggc gtcttcttag tcctctctcc 600
   ctaagtttaa aggactgatt tgatgcctct ccccagagca tttgataagc aagaaagatt 660
   tcatctccca ccccagcctc aagtaggagt tttatatact gggt                 704
<210> 27
   <211> 468
   <212> DNA
   <213> unknown
<220>
   <223> >sr|IMAGp99SH17826.1|cl|IMAGp998M17626|gb|BX108828 |oid|234552035 BX108828 Soares_fetal_heart_NbHH19W Homo sapiens cDNA clone IMAGp998M17826 : IMAGE:359608, mRNA sequence
<400> 27
   tttttttttt acttagtctg tgtttggttc cccagtttta tgctgccttt ctactctgtt 60
   cttgctgtct ccctctttac ctgagtcaac ggtactgagt cctgtctctc tctgatgttc 120
   cccagtcttc cttggtgcat gttctagctc cacacactag tccttggagg aaggttgaga 180
   ccaatgattt cctgttatga gtcatgagga aactgaatca cctagaagtg gaataatgtg 240
   ctcagggtca ccatagccca ttagtggaag gaccaggact agacctttag tcttctgagg 300
   tccagcccct taggctgtct gtcatcactg tacccaagtg atgtcactac caaggccaaa 360
   tgatggtggg ctaaatttta attctcaaaa gtgtaggagg ctaatattgt cttctaagtt 420
   ccaaaagaag atgtaataaa agtctgttac cttaaaaaaa aaaaaaaa             468
<210> 28
   <211> 756
   <212> DNA
   <213> unknown
<220>
   <223> >sr|IMAGp998P11794.1|cl|IMAGp99BP11794|gb|BX093757 |oid|234521882 BX093757 Soares_fatal_heart_NbHH19W Homo sapiens cDNA clone IMAGp998P11794 ; IMAGE:347386, mRNA sequence
<400> 28
   aggcctagga agagggtcat tcttaagcca cacattagct gcactgcgtg gctgcagcca 60
   aaacaaagaa ctgggtgttg agtattcatc aactaagaac caaaatccag ggcactcata 120
   tgtgaaggat aagaacctca cttccttact cctccaaaaa gaagtgggga aagaaccatc 180
   aaacctttcc tcctgactta ccaaaccagg aaaacagcag gagagggtgg ctcaggactt 240
   agggacaggg tatagcttag atggtggaaa gcaaaggaga gcaggaagtt gtaaatcact 300
   ggctaatgag aaaaggagac agctaactct aggatgaagc tgtgactagg ctggagttgc 360
   ttccttgaag atgggactcc ttgggtatca agacctatgc cacatcacac tggggctagg 420
   gaagtaggtg atgccagccc tcaagtctgt cttcagccag ggacttgaga agttatattg 480
   ggcagtggct ccaatctgtg gaccagtatt tcagctttcc ctgaagatca ggcagggtgc 540
   cattcattgt ctttctctcc tagccccctc aggaaagaag gactatattt gtactgtacc 600
   ctaggggttc tggaagggaa aacatggaat caggattcta tagactgata ggccctatcc 660
   acaagggcca tgactgggaa aaggtatggg agcagaagga gaattgggat ttttaagggt 720
   gcagctacgc tcaccctaaa cttttggtgg cctggg                         756
<210> 29
   <211> 363
   <212> DNA
   <213> unknown
<220>
   <223> >gi|772089|sr1|IMAGp99800814|cl|IMAGp998008148|gb| R18479|R18479 yf95f04.r1 Homo sapiens cDNA clone 30149 5' similar to gb:X14174 ALKALINE PHOSPHATASE, TISSUE-NONSPECIFIC ISOZYME PRECURSOR (HUMAN);
<221> misc_feature
   <222> 85, 346
   <223> n=a, t, c or g
<400> 29
   acgtgatcat ggggggtggc cggaaataca tgtaccccaa gaataaaact gatgtggagt 60
   atgagagtga cgagaaagcc agggnaccga ggctggacgg cctggacctc gttgacacct 120
   ggaagagctt caaaccgaga tacaagcact cccacttcat ctggaaccgc acggaactcc 180
   tgacccttga cccccacaat gtggactacc tattgggtct cttcgagcca ggggacatgc 240
   agtacgagct gaacaggaac aacgtgacgg acccgtcact cttccgagat ggtggtggtg 300
   gccatccaga tcctgcggaa gaaccccaaa ggcttcttct ttgctngttg gaagggaggc 360
   aga 363
<210> 30
   <211> 449
   <212> DNA
   <213> unknown
<220>
   <223> >gi|5367341|gb|AI801869|sr1|MAGp998CO95623.1|cl|IM AGp998C0956231A|801869 tx28e05.x1 NCI_CGAP_Lu24 Homo sapiens cDNA clone IMAGE:2270912 3', mRNA sequence
<400> 30
   tttgttgttg atccattcat aagcatttat tgagggctga ctggatgccc agcactaggg 60
   aaacggcaga tggcacaaag ggtggggtcc ccaaggacag ggcgatttca gctcttccct 120
   tgccaacccc gggtcatccc cagggctcaa tcccaggctg gggggagggt ctgggagtgg 180
   gggcagtcgt catagggtgg gtcttcctgg aaatccagcc gagcccgggt gcccttgccc 240
   aggtgtaggc agcctcctag gtggacatgg agggagagca ggagggcgaa ggctcagact 300
   tcccggcacc tctgaaactc ggtgtttctc tacgtctaac tctccttctt gctgcaacgc 360
   atttgccggg gcgggcggcc tctgcattcc tgaactgggt cagaccagga gagaggattc 420
   ctggatcctg gcccagtggc caatccgcc 449
<210> 31
   <211> 525
   <212> DNA
   <213> unknown
<220>
   <223> >gi|99698901gb|AI6925501sr1|IMAGp9980205798.1|cl|IM AGp998020579B|AI692550 wd73f10.x1 NCI_CGAP_Lu24 Homo sapiens cDNA clone IMAGE:2337259 3'. mRNA sequence
<400> 31
   atgttaaaat tgcacaaaca tggtgctcta ccaggaagga ttcgaggtag ataggctcag 60
   gccacacttt aaaaacaaac acacaaacaa caaaaaacgg gtattctagt catcttgggg 120
   taaaagcggg taatgaacat tcctatcccc aacacatcaa ttgtattttt tctgtaaaac 180
   tcagattttc ctcagtattt gtgtttttac attttatggt taatttaatg gaagatgaaa 240
   gggcattgca aagttgttca acaacagtta cctcattgag tgtgtccagt agtgcaggaa 300
   atgatgtctt atctaatgat ttgcttctct agaggagaaa ccgagtaaat gtgctccagc 360
   aagatagact ttgtgttatt ctatctttta ttctgctaag cccaaagatt acatgttggt 420
   gttcaaagtg tagcaaaaaa tgatgtatat ttataaatct atttatacca ctatatcata 480
   tgtatatata tttataacca cttaaattgt gagccaagcc atgta 525
<210> 32
   <211> 461
   <212> DNA
   <213> unknown
<220>
   <223> >gi|788859|sr|IMAGp998A10192|cl|IMAGp998A10|92|gb1 R33016|R33016 yh70b05.s1 Homo sapiens cDNA clone 135057 3' similar to SP:ZK783.2 CE00841 TYROSINE PHOSPHATASE ;._gi|343426|gb|G23100|G23100 human STS WI-11994
<221> misc_feature
   <222> 199, 217, 311, 390, 456
   <223> n=a, t, c or g
<400> 32
   ggacaatgct tttaatggca agtcatcaca gcaggtctgc ggaggtgcag ggcagcgctc 60
   aggccttgct cagtttcttc ttgatgaagt agctcaccag ccgctgcggc ctctgctggt 120
   actcgctgag cacattgcga gggctgctga tctggtcccc ttccaggcgg ttcaggaggg 180
   tgacacacac cacggccgnt tggaggccct agggctngca catggcggca aacaccgagg 240
   actccatctc gatattgcgg acgccggctg cataggctgc ctccagatac gcctgcttgt 300
   ccttctccgt ntaggagcag agagccccat ccagacggcc ttgcccttca taggaagtcc 360
   aagggtgcac atgggttttc cccaccactn ttggttgaac tcgttcagtt ttgcagaaca 420
   cagcaacagt ttctgcacca gttttttttt aaaggncctt t                      461
<210> 33
   <211> 500
   <212> DNA
   <213> unknown
<220>
   <223> >sr|IMAGp998F20979.1|cl|IMAGp998F20979|gb|BX115560 |oid|234565495 BX115560
   Soares_fetal_liver_spleen_1NFLS_S1 Homo sapiens cDNA clone IMAGp998F20979 ; IMAGE:418195, mRNA sequence
<400> 33
   taatcagtga tgatactttg gtgtatgttc aggactgaaa ccttcttatt gtgctaactt 60
   gatggtttct ttaaaaataa tagtcttgcc ataaattatg aataagactt gtttttttag 120
   ccatatactc agaagaacac tagagttctc caactggcta cgcttagttc ttgctttgcc 180
   aggtagtccc acgcttacta taatgttcaa tttagagttc taagtataaa atatttttac 240
   ttatttattt atttttgtga cagagtcttg cactgttgtc caggctggag tgcagtggtg 300
   tgatctcggc tcactgcagc ctccacttcc tgggttcaag tgatcctcac gcctcaacct 360
   cctgagtagc tgggattaca ggtgtgagcc atggtgcctg gctaattttt ttttcttttt 420
   ttccttttct gagacggagt ctcccactgt cactccagtt tgggcaacag agtgagaccc 480
   tgtttaaaaa aaaaaaaaaa                                              500
<210> 34
   <211> 565
   <212> DNA
   <213> unknown
<220>
   <223> >gi|14736233|gb|AI652254|sr|IMAGp998H095717.1|cl|IM AGp990B095717|AI652254 wb28c05.x1 NCI_CGAP_GC6 Homo sapiens cDNA clone IMAGE:2306984 3', mRNA sequence
<400> 34
   cacagtatga atgtatttta ttttgtgaac cgggaaggac accaaccaaa acataattga 60
   aacaaaagtt tcacagcaca atagttacct tagtacatgt gatacacaat ggactctatt 120
   cttgtccaca ctgttccatt aaagaaaaat atgacacact tactgatttc acagtggggc 180
   cttgaaagtg cccacaatag gctgtcaggt gagaggaaac acagaaggga ggtagcttac 240
   tacagaatga tcatggccta ataacatctg ggtgattata tgggttgcac ctcagctgtc 300
   tataaacttt agcgtcagca gatctgtcca tggctgtctc tgcttttcca gttggttggg 360
   catatctccg gcctgagatg gtcacccgtc agaatccttt gagtatcttg ccagaattca 420
   gcagtttttc tgtgcaccag gaggatgctg cagggaggct gagattctct gaagatacgt 480
   gtattgctga aagtagactt atgattatgg aatcataatt tccttgtact tttatctaaa 540
   attatacatg agcccgctga aggag                                       565
<210> 35
   <211> 518
   <212> DNA
   <213> unknown
<220>
   <223> >gi|4889|49|gb|A16789671sr1|IMAGp998M125582.|cl|IM AGp998M125582|AI678967 tu60b06.x1 NCI_CGAP_Gas4 Homo sapiens cDNA clone IMAGE:225541-1 3', mRNA sequence
<400> 35
   tttttttttt ttttttctta caaatgggag ttttatttgc attctaaccc aagcacagat 60
   cccacataca gacttctcgt ccgcactatg gtacagtaac aagtgcaaaa tatgctttat 120
   ttcaggtaca aaaacatggc agtaggacaa ctttgagctc aacgcccacc tccccaggac 180
   ctttcacacc cacctgaccc atccaagggc cacaccaccc cgacagatac tcccaccacc 240
   tttagaaaag agtcacccaa tctggagaaa ggtgtggagg ttacatcttt agaaagaaat 300
   cattttaaat acatgaacat tagagaacac agtaaccgtg cttccaccca gcacggggag 360
   gctgcagaga ggcaccccaa acagtcccct tctcttctgt ggttaaccaa gcaagccccg 420
   caaagctttt cccagcagag cacacccagc cagcaatgag gcctcaggac agagccagca 480
   ctgtgacaaa agaacttgtc ccacctatct gagagcat                         518
<210> 36
   <211> 430
   <212> DNA
   <213> unknown
<220>
   <223> >gi|3801639|gb|A12194361sr1IMAGp998MIS4512.1|cl|IM AGp998M1545121A|219436 qh13a08.x1
   Soares_NFL_T_GBC_S1 Homo sapiens cDNA clone IMAGE:1844534 3' similar to contains Alu repetitive element;contains element MER33 repetitive element ; mRNA sequence
<400> 36
   tttgagacaa gagtctgact ttattgccta gtggagtggc atgatctctg ctcactgcaa 60
   cctctgtctc ccgggttcag gcaattctca tgcctcaacc tcctgagtag ctgggattac 120
   agacacatgc caccacgccc gactataaaa ttgagttctt atagttcaat aaagttaatt 180
   tattatctct ttatgctagt attatagaat tttggagcag gtagtatctt agattcccta 240
   cattagcagt gttatataaa agagaaataa ttcaagtcac atatgtaatt taaaatattt 300
   tagtagccat attaaaaaag caaaaagaaa caggtgaaat tacttttgat tgtacatttt 360
   tttaacccag tatatcagaa atactgtgtc aacatgtaat caatgtaaaa ttattagaga 420
   tagtttgcac                                                        430
<210> 37
   <211> 493
   <212> DNA
   <213> unknown
<220>
   <223> >sr|IMAGp998M144547.2|c1|IMAGp998M144547|gb|BX1071 17|oid|234548615 BX107117 Soares_NhHMPu_S1 Homo sapiens cDNA clone IMAGp998MI44547 ; IMAGE:1857973, mRNA sequence
<400> 37
   aggacatagg ctgctaatga acagccccat ctgatttaat cactgtgcaa tgaccgtgaa 60
   ctgtacagtt acctccgtgt gtcacaggaa gacttttttg acttaagcac ccttccatgg 120
   tcatgaggtt tctggttgcc tctgtgtgtt agaagcttgg agaccaatgc cttctgctgt 180
   gatcgcgttc tccacagttg cctctgggta ggagcctgga cactaataga aaatggatgc 240
   acttgagccg acctaagcat tcatggtcat gagatttgtg gttgccactg ttggttagga 300
   gcatagagat cagtaatgga cacacctggg ctggcagaac atcttgcctt gatctggagc 360
   cccatgtctg cccctctgta gaggtgcaca tgggccaatg atggatgcaa tgagctgatc 420
   attttgctgt gatcaaggct atgttgcctc tgtgttaggc caatgtgagt aatggttctt 480
   gtcttggttt agg                                                   493
<210> 38
   <211> 294
   <212> DNA
   <213> unknown
<220>
   <223> >gi|4312796|gb|AI4599151sr1IMAGp998L105312.1|cl|IM. AGp998L105312|AI459915 ar81h05.x1 Barstead colon HPLRB7 Homo sapiens cDNA clone IMAGE:2151705 3', mRNA sequence
<400> 38
   tttccagtac aaaaccaatt tattttacat tccatcgtgg gcgatgggag aagctaaata 60
   ataatttagc atgcatacgt agacgtttct ttttatatat aaatacaata tacaaaaata 120
   aagacagtac agttttgaga tttccagcag tttgaacgct cgtgacataa tccccccgac 180
   cccagagacg taagcataaa cccttttttc tttttccaaa tactctgcag aatggcggct 240
   ccagaggcgg tttcaagttt cataagtcag gtaacactgt gggtttccgc cttc      294
<210> 39
   <211> 434
   <212> DNA
   <213> unknown
<220>

   <223> >sr|IMAGp998D201008.1|cl|IMAGp998D201008|gb|BK1092 26|oid|234552831 BX109226
   Soares_fetal_liver_spleen_INFLS_S1 Homo sapiens cDNA clone IMAGp998D201008 ; IMAGE:429283, mRNA sequence
<400> 39
   tgaagatgca gcccctggat ggtcccagac tctcaggaca tgcccagctc aggggcttcg 60
   agccacaggc ctggcctcat atggcatgag ggggatctgg cataggagcc agactgctct 120
   tagccccctg gctttgtgcc aggcctggag gagggcagtc ccccatgggg tgccgagcca 180
   acgcctcagg aatcaggagg ccagcctggt accaaaagga gtacccaggg cctggtaccc 240
   aggcccactc cagaatggcc tctggactca ccttgagaag ggggagctgc tgggcctaaa 300
   gcccactcct gggggtctcc tgctgcttag gtccttttgg gacccccacc catccaggcc 360
   ctttctttgc acacttcttc ccccacctct atgcatcttc cccccactgc ggtgttcggc 420
   ctgaaggtgg tggg                                                  434
<210> 40
   <211> 513
   <212> DNA
   <213> unknown
<220>
   <223> >gi|5397100|gb|A1810534|sr1|IMAGp998H065732.1|cl|IM AGp998H065732|IA810534 wb89h03.x1 NCI_CGAP_Pr28 Homo sapiens cDNA clone IMAGE:2312885 3', mRNA sequence
<221> misc_feature
   <222> 7
   <223> n=a, t, c or g
<400> 40
   ataatanttt aatttttttt tttttttttt taacttttcg ccttatttaa tactgggata 60
   tggggtcagg gagtttgccc cacttggaga gatgacttcc cttggaaaac atggggtttc 120
   cttctgaagg ggaatcctga ttccttcctt aatgttgggg ggatccaaaa gtgtggccag 180
   atggaacatc tcattggcgg tttctttctt gcttgcttgg ggcttctctg tttggccttt 240
   tccccattct cctttgtgtc cggcaggggg agaggcggct tcgtataagg gcctttttga 300
   ggtttattat ttccttggcc atatacttga tgctcttcat tggcttgtct gggacctgcc 360
   ttaggttctc cgaggcataa aagggccgga cagcccccga gttgggggaa ctctgaagct 420
   tcttggtggc tggaaccttg gtcatcttaa aaatccttca ggttttagcc tgtgccccca 480
   agacaaggat ttttccagaa tcttctactt cag 513
<210> 41
   <211> 489
   <212> DNA
   <213> unknown
<220>
   <223> >sr1|IMAGp998H084295.2|cl|IMAGp998H084295|gb|BX1047 34|oid|234543848 BX104734
   Soares_fetal_heart_NbHH19W Homo sapiens cDNA clone IMAGp998H089295 ; IMAGE:1691575, mRNA sequence
<400> 41
   ctttgtgagt tcttttccct cctttaaagt tgggaaaact tgattcgtgg ggcaggagat 60
   tgtttcttca ttcttctgac agcccccatc tgacgcgtaa ctgcccattt taaggaaact 120
   cttggtgcta caaaaccctg accagacact tggcaaattt acctctttct tcaaaagaaa 180
   aactttaaga aaatgagcca atgggcttca ttctcagtca tgcccggaga tcacccagga 240
   gaaataatac aaacaccacc actgtccaga gagagtaaag aagcagaaag agaaagaatt 300
   tgcaaccatg aggaatgttc ccacctcccg acgggacgtg catttggaaa acacagaatc 360
   agccctcagg gtgcactcca gccacctcag tgctctaagc tcacagaagt gaaataatgt 420
   ctgtgggttg gcaatggctt tgtgggatca tatgtcttgg ccaaagatgg gaaaacctat 480
   gttgaagag 489
<210> 42
   <211> 467
   <212> DNA
   <213> unknown
<220>
<223> >sr|IMAGp998F176102.2|cl|IMAGp998F176102|gb|BX1072 49|oid|234548879 BX107249 NCI_CGAP_Ut1 Homo sapiens cDNA clone IMAGp998F176102 ;
   IMAGE:2453776, mRNA sequence
<400> 42
   tttttttttt ttttaagcaa gcacattata agaagcaata gatgtgccag ggcatatgaa 60
   catcagatga atggaagtta tcctgtgttt ttctttttaa cttaaattca tcttttttga 120
   attaaatctt aattgataca tcctgtatat gaaatttcac tttatggtat aaatcagagg 180
   cttcctatcc tataggaaag attcatgctt gtttttcaaa ttcagtacaa ttctcccagt 240
   gatttctagg aaagaataaa gaggagagga gaggtagaca tcagaatgta gagaggcaat 300
   actgtgtaaa aatgaaaaac aaaaaaaaaa actttatttc tattttccca ggctgcacaa 360
   atctggcttt gatgaagaaa ctcagaagat cctgatgtct gtggaagaaa aaattcccca 420
   tctgaccatt tcacatggac cttggattga cgaggaatac aagatca 467
<210> 43
   <211> 275
   <212> DNA
   <213> unknown
<220>
   <223> RZPD CloneID : IMAGp998K013431
<400> 43
   tttttttttt tttttttttt tttttttttt tttttccaaa agccaaaaag tttatggcca 60
   gaaaagcaaa cttcataaag accccttaaa gtacatcgaa taggacaagc aaaataaaca 120
   gaaaactttg cccaaagaaa agatggccgc ggtcaggcac agtcaaatta ataccaaccc 180
   aaacagggtc ctcgaagggt ataggggtta tacaacccac cctcggaaac taagggggcc 240
   ccattccaaa atccttggtt ttgggttggg ggttt 275
<210> 44
   <211> 359
   <212> DNA
   <213> unknown
<220>
   <223> >sr|IMAGp998M23174.2|cl|IMAGp998M23174|gb|BX113547 |oid|234561471 BX113547 Soares infant.brain INIB Homo sapiens cDNA clone IMAGp998M23174 ;
   IMAGE:41558, mRNA sequence
<221> misc_feature
   <222> 39, 43, 93, 96, 101, 106, 125, 141, 156, 227, 248, 271, 276, 316, 323, 328, 334, 352.
   <223> n=a, t, c or g
<400> 44
   tttttttttt ttttaagcag aaaaaagaga atttttaang gcncaacaaa aaagatttac 60
   agcctacttt tctacagaaa tgatggaagt canaanacaa nggagngaca tcttcaggta 120
   aagtnttaaa agaaaataag ngccaattta taattntata ccagcaaaaa tatgcttcaa 180
   aaatgaaagt aaaataaaga tgtctactag gcaaacaaaa cagaganttt atcaccagca 240
   aacctgtnct gaaagaaatg ctaaagaatt ntttanacac aaggaaaatg atcccagagg 300
   gaaacatgaa attatncaaa gtnttganca ccancagaaa cggtaaatat gngaataaa 359
<210> 45
   <211> 713
   <212> DNA
   <213> unknown
<220>
   <223> >sr|IMAGp998c17793.3|cl|IMAGp998c17793|gb|BX089984 |oid|234514327 BX089984 Soares_fetal_heart_NbHH19W Homo sapiens cDNA clone.IMAGp998C17793 ;
   IMAGE:346696, mRNA sequence
<221> misc_feature
   <222> 696
   <223> n=a, t, c or g
<400> 45
   acatccgcca aatctatgac aaattcccgg agaaaaaggg tggactcaag gatctcttcg 60
   aacggggacc ctccaatgcc ttttttcttg tgaagttctg ggcagacctc aacaccaaca 120
   tcgaggatga aggcagctcc ttctatgggg tctccagcca gtatgagagc cccgagaaca 180
   tgatcatcac ctgctccacg aaggtctgct ctttcggcaa gcaggtggtg gagaaagttg 240
   agacagagta tgctcgctat gagaatggac actactctta ccgcatccac cggtccccgc 300
   tctgtgagta catgatcaac ttcatccaca agctcaagca cctccctgag aagtacatga 360
   tgaacagcgt gctggagaac ttcaccatcc tgcaggtggt caccaacaga gacacacagg 420
   agaccttgct gtgcattgcc tatgtctttg aggtgtcagc cagtgagcac ggggctcagc 480
   accacatcta caggctggtg aaagaatgag agactcgggg agcagggagg gggggaagag 540
   acgtgtgtgc aggaaacggg gacgggggga aggggaccct gcaggggcag ccccctgaag 600
   tgccaagaga gctgagagga gcagttgtga ctctacccag gaacaaactg tgcctgaacc 660
   tgaggtgccc aaccccaaat aaacccaaga tgctgngtaa aaaaaaaaaa aaa        713
<210> 46
   <211> 432
   <212> DNA
   <213> unknown
<220>
   <223> >sr|IMAGp9980144918.2|c1|IMAGp9980144918|gb|BX1000 64|oid|234534504 SX100064 NCI_CGAP_Lym12 Homo sapiens cDNA clone It4AGp9980144918 ;
   Image:2000985, mRNA sequence
<400> 46
   tttttttttt ttttgacagt gagcaataaa gtttaattaa ccagtactgt gaggtcacct 60
   tcacggacta gcatcccttc cgtgagctga aggactgaat aagcagaagc acaggccaaa 120
   cccatccctc catgctcccc actgtgcaga tgggctcact gaggctccgg tgtacattgc 180
   ttgctcagca ctggatctat ttcgggccaa agtgtcagca gaactgatac ccacagggca 240
   tccaagtcca catccagact gggctccctc taatgccatc tcttaaaccg aaccactgcc 300
   tgccgatggt gagtttccaa tcacgagctg cactgggggt gctggaggcc tggctgggca 360
   cacatcctgg tgggtatgaa ggggatttgg ggagtggcat ctagaagatc agcatgaggg 420
   gcacagagga ga                                                    432
<210> 47
   <211> 710
   <212> DNA
   <213> unknown
<220>
   <223> >sr|IMAGp9980021010.1|cl|IMAGp9980021010|gb|BX1155 10|oid|234565395 Bx115510
   Soares_fetal_liver_spleen_1NFLS_S1 Homo sapiens cDNA clone IMAGp9960021010 ; IMAGE:430297, mRNA sequence
<221> misc_feature
   <222> 547, 591
   <223> n=a, t, c or g
<400> 47
   ctggtggcct tgctggatga cccaacaaat gcccatttca ttgcctggac gggccgggga 60
   atggagttca agctcattga gcctgaggag gtcgccaggc tcttgggcat ccagaagaac 120
   cggccagcca tgaattacga caagctgagc cgctcgctcc gatactatta tgagaaaggc 180
   atcatgcaga aggtggctgg tgagcgttac gtgtacaagt ttgtgtgtga gcccgaggcc 240
   ctcttctctt tggccttccc ggacaatcag cgtccagctc tcaaggctga gtttgaccgg 300
   cctgtcagtg aggaggacac agtccctttg tcccacttgg atgagagccc cgcctacctc 360
   ccagagctgg ctggccccgc ccagccattt ggccccaagg gtggctactc ttactagccc 420
   ccagcggctg ttccccctgc cgcaggtggg tgctgccctg tgtacatata aatgaatctg 480
   gtgttgggga aaccttcatc tgaaacccac agatgtctct ggggcagatc cccactgtcc 540
   taccagntgc cctagcccag actctgagct gctcaccgga gtcattggga nggaaaagtg 600
   gagaaatggc aagtctagag tctcagaaac tcccctgggg ggtttcacct gggccctgga 660
   ggaattcagc tcagcttctt cctaggtcca agccccccac accttttccc           710
<210> 48
   <211> 666
   <212> DNA
   <213> unknown
<220>
   <223> >sr|IMAGp998E201119.1|cl|IMAGp998E2011191|gb|BX1095 81|oid|234553541 BX109581
   Soares_pregnant_uterus_NbHPU Homo sapiens cDNA clone IMAGp998E201114 ; IMAGE:470011, mRNA sequence
<400> 48
   cttgggtgga tgggaaagtt aaatattggt ctcttcaagt tttttttttc ttttgctttg 60
   ttaccacttg tcactgtctc catgttaaaa tgccaaaaat gatgtagttg ttgttgcttt 120
   tttccctatt ttccacccca gtcgctcctt accgtgactc ctgcccttgg agggcatgta 180
   gcagtgtctg tcctgccagt cccaaggccc tgtgggagga gactggcctg catctctcta 240
   agacttagtc tgacgccacg cgcatctctt gttctgtgtt caatcagtag tccaggggag 300
   aagcttctgc tacttcagag ctttgctaaa ctaacctaat ttgtccaaat caccccaaaa 360
   ccaccatctc tgacgtaagc ttccatgcga cagcctgatc cgtttccctg gacaggtctc 420
   tttcctggaa tgcagcccag gcacctgtgc tcctggcacc cttgaggtct ctcctttgag 480
   ccgtggtcac cgagagggtt gaggacgcag cacccgaggt cccagccttt gcaggagcct 540
   ccctgggctt agctggactt agatcttcgg tggcctcatg taaacgtggc agccagcctc 600
   ttctagaacc ctagcccagg gactggagca ggaaagggac cttcaaagtg aagactgcct 660
   tgtccc                                                            666
<210> 49
   <211> 1515
   <212> DNA
   <213> unknown
<220>
   <223> Homos sapiens|chromosome:NCBI34:2:43429015:43430529:1
<400> 49
   ggctttgcta atcacatcac aagaccttga gccagggcca aaggcgcgga gtggcgcggg 60
   acgtcccggg cgggggagct gactctgagg acaggaggaa aggccgggag agccgtgcgg 120
   gcttgaaggc gacttagcgc cacctttccg tctgctggtc gggccggcct tccccagccc 180
   tctggggctg ggggtgcagc tgcctgccga ggctccccgc ccggcacagc ctgggcctgt 240
   tgcgtcctcc cagcctcttc cctccccggc agtgggggcg ccgcgcccag cctttgctcg 300
   atgagggctg caggatccgg ctgagaccaa cccgcggcgc aggtggggct gcacagacgc 360
   tgatggtggg cgcgccaccc tctgttttcc ctcactcgcc agccccgcac cccaacagct 420
   tgcgctggaa aatgcgcttt gccagcgggc gccccgcctt cgtccgggta cggaggggac 480
   actcccatct agcgcccagt gaggctaaag ccgctgacag ccccactagg aacgatctgg 540
   agtctggtct agggcacttc tccctccgcg ttcatacagg ttgccacaaa aagcttgggc 600
   ctgaagctcg aaggccccgg acccgctggg gaaagaacta ggcctgccac tcagggattc 660
   gagtccctgg agtgtatcct gcatttccca ctactgtact cccctcatgg cccagggagc 720
   cagagtccag ggtaaggatc aggaatgctg ggctggagtt caggtcctgc cccagagaaa 780
   tcatgtggtc ttaggcaaat cacatcatct ctttggtact tgggtttcct tgttaaacga 840
   agataatatt cttgctttat tcatctctca cctttggcaa ggtgcaaatg atatctgtga 900
   aggtgtttaa aagacaagca agaactaaac catctttatg tattaaacca aattgacaaa 960
   agcgtaaggg tggagaaagc ctggcttctg aactgtggcc agatggacag agagccacag 1020
   aatcatgagt gcagagctgt gggggcaccc aagtcctgga ccctaggact cacagccaag 1080
   agcaggtgac ctcagaggga ccagtttcac ttttagcacc taccctacac tgaccccttg 1140
   acatacctgg gcaactgtgg tctggtttgt tctttggtga gatgacccca aacatctcac 1200
   caaactgtga aacagccact tccctaggca ctcctattag gttagtaaca agatttcaga 1260
   tacagaacca gtggaaacat ctgtttgaaa caacgtccaa atctggggtg agtcccagtc 1320
   aacagctgtg agaacaccca ctttgcagat taggattctg gagataacat gtggacatgt 1380
   tacgggacat gcttgtgtac agagtacctg ctagtataaa atgtctaatg tagctgcttt 1440
   tatttcattt taaatggaga aaaaaagaag tgctggccat ttccaatgag ctgggctttg 1500
   ttgggccagt ttcat                                                  1515
<210> 50
   <211> 302
   <212> DNA
   <213> unknown
<220>
   <223> >gi|4851404 |gb|AI6716731sr1|IMAGp998G195647.1|cl|IM AGp998G195647|AI671673 ty26e10.x1 NCI_CGAP_Ut3 Homo sapiens cDNA clone IMAGE:2280234 3', mRNA sequence
<400> 50
   tttttttttt tttttttttt tttttttttt tttttttttt tttttttttt tttttttttt 60
   caaaaaaacc ggggaatttt tttaaaggcc ggggggcccc agaacccaag ggggttgggg 120
   gttttaaagg acccgaaaaa aaaagggtaa aacccccgaa tttccccccg gcccccaaaa 180
   tttggggggg gaaaaaaggg gttttggaaa aaaacccccc gccaaaccca accaaaaaaa 240
   aaatcgggag ggggtttttt tcccccttgg ggtttaaaaa aacccccaaa attaggggcc 300
   ca                                                                 302
<210> 51
   <211> 538
   <212> DNA
   <213> unknown
<220>
   <223> >sr1|IMAGp99SK06166.1|cl|IMAGp998K06166|gb|BX092114 |oid|234518596 BX092114 Soares infant brain 1NIB Homo sapiens cDNA clone IMAGp998K06166 ;
   IMAGE:38228, mRNA sequence
<221> misc_feature
   <222> 531
   <223> n=a, t, c or g
<400> 51
   tttttttttt ttttgattaa gaaagctaaa tttatattaa attatcataa agtcctaaaa 60
   tactgaacat agtggttaaa taactccaga aagtccaatc tctccagtga gtaacgttaa 120
   aaccattaca catgagcatg ggagaatcgc ttccattagt ttaggacaga gagattttgc 180
   tttttacaga gtaaatcagt gctcaaatag atacttcctc aaatatgtcc tttctacatt 240
   ctgaacagcc caagtgcaat aagatccttc ccccttccca atcaagaaaa tgccactttt 300
   ctacttgctc ttcctcccca gacatgagtc taaggaccca aagtgctcac tcctttactg 360
   cttgttaagt gtaatgtggg gaggctcaga actggggctg acgctactga gagcaaggct 420
   aagggcctgg tatctctctc tagcagtctt agaactgact agaaaagaat tgatcttgtc 480
   ctttttcact ttggtagctg atttctcaat accagtaacc acaggagaag nttcaact   538
<210> S2
   <211> 680
   <212> DNA
   <213> unknown
<220>
   <223> >sr|IMAGp998D03384.3|cl|IMAGp998D03384|gb|BX111866 |oid|234558109 BX111866 Soares fetal liver spleen 1NFLS Homo sapiens cDNA clone IMAGp998D03384 ;
   IMAGE:200018, mRNA sequence
<221> misc_feature
   <222> 3, 13, 33
   <223> n=a, t, c or g
<400> 52
<210> 53
   <211> 713
   <212> DNA
   <213> unknown
<220>
   <223> >sr1|IMAGp998E01223.1|cl|IMAGp998E012231|gb|BX103782 |oid|234541945 BX103782 Soares placenta Nb2HP Homo sapiens cDNA clone IMAGp998E01223 ; IMAGE:147048, mRNA sequence
<400> 53
   cacgtgtggt accagggcaa cgcgtcgctg gggggacacc taacgcacgt gctggaaggc 60
   ccagacacca acaccacgat cattcagctg cagcccttgc aggagcccga gagctgggcg 120
   cgcacgcaga gtggcctgca gtcctacctg ctccagttcc acggcctcgt gcgcctggtg 180
   caccaggagc ggaccttggc ctttcctctg accatccgct gcttcctggg ctgtgagctg 240
   cctcccgagg gctctagagc ccatgtcttc ttcgaagtgg ctgtgaatgg gagctccttt 300
   gtgagtttcc ggccggagag agccttgtgg caggcagaca cccaggtcac ctccggagtg 360
   gtcaccttca ccctgcagca gctcaatgcc tacaaccgca ctcggtatga actgcgggaa 420
   ttcctggagg acacctgtgt gcagtatgtg cagaaacata tttcctcgga aaacacgaaa 480
   gggagccaaa caagccgctc ctacacttcg ctggtcctgg gcgtcctggt gggcagtttc 540
   atcattgctg gtgtggctgt aggcatcttc ctgtgcacag gtggacggcg atgttaatta 600
   ctctccagcc ccctcagaag gggctggatt gatggaggct ggcaagggaa agtttcagct 660
   cactgtgaag ccagactccc caactgaaac accagaaggt ttggagtgac agc 713
<210> 54
   <211> 317
   <212> DNA
   <213> unknown
<220>
   <223> >gi | 4073985 | gb | AI337058 | sr | IHAGp998D054941.1 | cl | IM AGp998D054941 | AI337058 qx82g03.x1 NCI_CGAP_GC6 Homo sapiens cDNA clone IMAGE:2009044 3', mRNA sequence.
<400> 54
   aagtcttcat taagagagga aaagatgaaa actgccttta catgataaat tatatatgtc 60
   aaaaatctag tatcaagttt aatggaacaa tgtcgcagcc tgatgggttc ttctttccca 120
   ctgccctgaa aaaagactaa tgagaccagc aggagttgca acaaagaatg taataatcac 180
   aggcctgctg agcaaggaga acagggagaa agttctcaaa cctgtctccc ccagaatttg 240
   gaggctgggg ttttgtaaaa gtacttttta aaaagtactt tcccagcagg gttctgggaa 300
   actggaacag atgattg 317
<210> 55
   <211> 832
   <212> DNA
   <213> unknown
<220>
   <223> >sr | IMAGp998A17115.1 | cl | IMAGp998A17115| gb | BX097937 | oid | 234530247 BX097937 Soares fetal liver spleen 1NFLS Homo sapiens cDNA clone IMAGp998A17115 ;
   IMAGE:122008, mRNA sequence
<400> 55
   acaaaggcta ttaacacata ggaaaaaatg tatttactaa gtgtcacatt tctctaagat 60
   gaaagatttt tactctagaa actgtgcgag cacaacacac acaatccttt ctaactttat 120
   ggacactaaa ctggagccaa tagaaaagac aaaaatgaaa gagacacagg gtgtatatct 180
   agaacgataa tgcttttgca gaaactaaag cctttttaag aaatgccagc tgctgtagac 240
   cccatgagaa aagatgtctt aatcatcctt atgaaaacag atgtaaacaa ctatatttca 300
   actaacttca tcttcactgc atagcctcag gctagtgagt ttgccaaaac caaagggggt 360
   gaatacttcc ccaagattct tcctgggagg atggaaacag tgcagcccag gtcccatggg 420
   ggcagctcca tcccagagca tttctgatag ttgaactgta atttctactc ttaagtgaga 480
   tatgaagcat tatccttttg ttcagttgcc ccgggctttt gaacagaaga gtaaatacag 540
   aattgaaaaa gataaacact caaccaaaca atgtgaaaac gggttctgta gtatttgtaa 600
   aaaggcccgg cccaggacca ctgtgagctg gaaaagggag aaaggcagtg ggaaaagagg 660
   tgagccgaag atcaattcga cagacagacg gtgtgtatgc ccctccctgt ttgacttcac 720
   acacactcat aactttccaa atgaaacccc acagtatagc gcatattttc gatatttttg 780
   tgaattccaa aagggaaatc acaggggctg gtcgaaatat tgggggaaca ct 832
<210> S6
   <211> 455
   <212> DNA
   <213> unknown
<220>
   <223> >gi | 4735095 | gb | AI651116 | sr| IMAGp998K035709.1 | cl | IM AGp998K035709 | AI651116 wa97e02.x1 NCI_CGAP_GC6 Homo sapiens cDNA clone IMAGE:2304122 3', mRNA sequence
<400> 56
<210> 57
   <211> 480
   <212> DNA
   <213> unknown
<220>
   <223> >sr | IMAGp998F13278.1 | cl | IMAGp998F13278 | gb | BX092118 | oid | 234518604 BX092118 Soares infant brain 1NIB Homo sapiens cDNA clone IMAGp998F13278 ;
   IMAGE:48538, mRNA sequence
<400> 57
   tttttttttt ttttaaacga aaagcaaact tctttattgt acacagtaca gaatataacg 60
   cagcttggca ggatgcatac ggccctgcgc aggggaaagt atttcaaatc agctggcagg 120
   ttcaagcctt tctgcactgt agactttcca cactctggaa aagaagcaaa caaacaaacc 180
   ccaaagaacc cccgaaaaaa acaaaaacca tccgggaggt gcatgagtcc aatgggaatg 240
   caaccgtgat gccgctgtcc tatgcccagt gacagcacag gtcacgtaag ttacagcagg 300
   ggaggggtag ctcaagctac agaggattat tgtcatattg ctaagacagc ataaatccat 360
   tcaaaaaaaa aaaaaaaaaa atccaaacca gggtaagtaa agaaaggaaa accaaatcta 420
   tacagcattt acaacaaata aatctctagc cagctggggg taaaatatgc atctatgtat 480
<210> 58
   <211> 786
   <212> DNA
   <213> unknown
<220>
   <223> >sr | IMAGp998BO6202.4 | cl | IMAGp998B06202 | gb | BX092972 | oid | 234520310 BX092972 Soares placenta Nb2HP Homo sapiens cDNA clone IHAGp998B06202 ; IMAGE:138917, mRNA sequence
<221> misc_feature
   <222> 789
   <223> n=a, t, c or g
<400> 58
   ctttgtctca gagccctctg ggctctctcc tctgagggag ggacctttct ttcctcacaa 60
   gggacttttt tgttccatta tgccttgtta tgcaatgggc tctacagcac cctttcccac 120
   aggtcagaaa tatttcccca agacacaggg aaatcggtcc tagcctgggg cctggggata 180
   gcttggagtc ctggcccatg aacttgatcc ctgcccaggt gttttccgag gggcacttga 240
   ggcccagtct tttctcaagg caggtgtaag acacctcaga gggagaactg tactgctgcc 300
   tctttcccac ctgcctcatc tcaatccttg agcggcaagt ttgaagttct tctggaacca 360
   tgcaaatctg tcctcctcat gcaattccaa ggagcttgct ggctctgcag ccacccttgg 420
   gccccttcca gcctgccatg aatcagatat ctttcccaga atctgggcgt ttctgaagtt 480
   ttggggagag ctgttgggac tcatccagtg ctccagaagg tggacttgct tctggtgggt 540
   tttaaaggag cctccaggag atatgcttag ccaaccatga tggattttac cccagctgga 600
   ctcggcagct ccaagtggaa tccacgtgca gcttctagtc tgggaaagtc acccaaccct 660
   agcagttgtc atgtgggtaa cctcaggcac ctctaagcct gtcctggaag aaggaccagc 720
   agcccctcca gaactctgcc caggacagca ggtgcctgct ggctctggtt tggaagttgg 780
   gggnaa                                                            786
<210> 59
   <211> 476
   <212> DNA
   <213> unknown
<220>
   <223> >sr | IMAGp99BE1783.1 | cl | IMAGp998E1783 | gb | BX109827 | o id | 234554033 BX109827 Soares fetal liver spleen 1NFLS Homo sapiens cDNA clone IMAGp998E1783 ;
   IMAGE:109816, mRNA sequence
<400> 59
   ctttgccctg ggtaagctgc tgtagggtca gaagtaaccc tttctgtgtc agttgagaat 60
   gagcctgtgt ggtagctgat gtcagaggac aaagctctct gcaagggctg gacacagagc 120
   tgcagagtcc tgaacatccc tcctttcagg ctgcagaagg gagaggcaat gaagacagct 180
   gctccggaag cagcatcagg gctcttggag gggactggtg gggactcagg ctgggtgcag 240
   cctccaaaca gagaacggaa cttaggtgtg tctctacagc taggcccagc ctagcccagc 300
   ccagaacaaa cacccttcag agcctaacca aagaacataa gctgcaaaat gtgcacccat 360
   attttaagct gctttttcag gggataaata gtgtttgggg acattgaaat ggatgttctc 420
   aggttgtatt tatttcggac aaataaacta gagaattgtg taaaaaaaaa aaaaaa     476
<210> 60
   <211> 457
   <212> DNA
   <213> unknown
<220>
   <223> >sr | IMAGp998L095608.2 | cl | IMAGp998L095608 | gb | BX1059 35 | oid | 234546251 BX105935 NCI_CGAP_Ut3 Homo sapiens cDNA clone IMA6p998L095608 ;
   IMAGE:2265368, mRNA sequence
<400> 60
   tttttttttt ttttaaaaat aatttacttt attacaaaga tagtaataca atttctagaa 60
   aatacatgca tgtacaaaga ttgaaataaa aaaaacactc ataaatatcc agatttaaat 120
   gtcagcatat accttcctga tctttttcta tcatactgta tctatcaaca tctaacctgc 180
   ttttttaact ataagtcata cattttgcca tatcagtaca cttttttctg taacattatt 240
   tttagagcat atcaaaccat agtttattta accaatcccc aattaattca taattagaat 300
   atttcgtgtt tttccagtat aacagttcta taatctttga ttataacctt tgtgtaaatt 360
   cctataagta ttgagtcaaa ggagacacac atcttttcag gcattacata taaaataaat 420
   actaaactag actccaggaa agtggtatca attcaca                          457
<210> 61
   <211> 517
   <212> DNA
   <213> unknown
<220>
   <223> >gi | 1274428 | sr | IMAGp998E1160s1 | cl | IMAGp998E11660 | gb | WO2449 | WO2949 za47a06.r1 Soares fetal liver spleen lNFLS Homo sapiens cDNA clone 295666 5'
<221> misc_feature
   <222> 481
   <223> n=a, t, c or g
<400> 61
   tctttagaga aaaatatttt taatgtagaa aagatgaata gctgtaatga aatacaaacc 60
   gttcctatac acagtaaact tactaagaac tgctaatcct tctccaggtt cttagaatct 120
   gggtacagca ttatagaaag taaccacgct atcgaaaaca cacacccaaa acccgatggg 180
   tggcaattgt gatagattag gaagacaaaa cagattcaga agtagcagag aatactgcac 240
   attttcttgg aaaatttctt aattgttcag tgctttctga caagtttccc cagcccaatt 300
   tatctcttag ctattctctg ctacttagtc ctgtcttctg acaatcatca tacggggacg 360
   atcatctgcc ttacagtgta acatgttttt cctaacacag ccgcatttta tctggcccta 420
   agagaatagc aactaatggg ctttcaatga agtcaccatg atttaggaaa gtttaaaacc 480
   ncaaattttt ccatggtggg taaaacttct aattacc                          517
<210> 62
   <211> 343
   <212> DNA
   <213> unknown
<220>
   <223> >gi | 2229312 | gb | AA495991 | sr | IMAGp998K051891.2 | cl | IM AGp998K051891 | AA495991 zw06e03.s1 Soares_NhHMPu_S1 Homo sapiens cDNA clone IMAGE:768508 3', mRNA sequence
<400> 62
   tttttttttt ttttttaaga aaaaatgtct attatttatt acacaataat tctgaccacc 60
   aacaaccaac ggcgggggcg ggcaggagag aagaacatct tgcttctcaa caaactttcc 120
   tcccttgctt taacattttt gaggattctt tcccaaacct attacacctg tattatgatg 180
   gttacaaatt ttccaactct tccactcctt ccagtgcatt atttttagta tcttcattaa 240
   acgggcaaaa aaaaagatcc cctacttgta ataacaaaac aatgttggaa actgtcatta 300
   aatcaggata agtgaaaaac agatctgttc ccagactcgc agg                   343
<210> 63
   <211> 330
   <212> DNA
   <213> unknown
<220>
   <223> >gi | 1018091 | sr | IMAGp998J14406 | cl | IMAGp998J14406 | gb | H63290 | H63290 yr49d07.r1 Homo sapiens cDNA clone 208621 5'
<221> misc_feature
   <222> 198, 235, 252, 268, 282, 292
   <223> n=a, t, c or g
<400> 63
   atgttcaacc tcagtccctc aaggccatac ctggacccaa cggtgaagaa agacgatgag 60
   gaggaggacc cgctggacca gctgatctcc cgctctggct gtgctgcctc ccactttgca 120
   gtgcaggagt gcatggccca gcaccaggac tggcggcaat gcagccacag gtgcaggcgt 180
   tcaaggattg catgagtnaa cagcaggcga ggcggcaaga ggagctgcag aggangcaag 240
   aacaagccgg tntccaccac tgagaccnca aaccacctat cnccagtaga tnggcccttc 300
   aagaccagca tcctagcaag attattagag 330
<210> 64
   <211> 430
   <212> DNA
   <213> unknown
<220>
   <223> >sr | IMAGp998B231782.3 | cl | IMAGp998B231782 | gb | BX0898 18 | oid | 234513990 BX089818 Soares_testis_NHT Homo sapiens cDNA clone IMAGp998B231782 ; IMAGE:726454, mRNA sequence
<400> 64
   ctcaatggcg gtctggccgt gaagaccaat gagatctctg tgatccagag tgggggggtt 60
   cctaccctcc cggtttcctt gggggccacc tccgttgtga ataacgccac tgtctccaag 120
   atggatggct cccagtcggg tatcagtgca gatgtggaaa aaccaagtgc tactgacggc 180
   gttcccaaac accagtttcc tcacttcctg gaagaaaaca agattgcggt cagctaaggg 240
   agaacttgcg tggaaggagc aatgcagaca cagtgaaatc tctagaatct gctttgtttt 300
   gtaagaactc atctcctcct gttttctttt tcttactgat atgcaaatga tgtttactac 360
   gttggttgtg accacaacct caggcaagtg ctacaatcac gattgttgct atgctgcttt 420
   gcaaaaagtt 430
<210> 65
   <211> 365
   <212> DNA
   <213> unknown
<220>
   <223> >gil | 3148939 | gb | AA973759 | sr | IMAGp998M024021. 1 | cl | IM AGp998M024021 | AA973759 oq16b01.s1 NCI_CGAP_GC4 Homo sapiens cDNA clone IMAGE:1586473 3' similar to contains element MSR1 repetitive element mRNA sequence
<400> 65
   tcactttgca attctttatt agaactcaag tttgtgtttg caaccatgca acagaaaggg 60
   taaaacaaaa aatccacatc ctctccccaa ccccagcttc tgggcggaaa tacgggcttt 120
   cactttaacc cctgactggc tctggctcca gggcctgaga tgccttcata atctagtaac 180
   tgggtcccgg gcatcctcgc gaaaccgctg ggtcactgcc ttgctggcag cttcggggga 240
   tcgctgggtc cccgacttgt tggcagcctg gagagactgc tgggtcccgg cctcctgcac 300
   ctccaccgga gaaccctgtg tcccgacctc ctggacttcc accgaacgct gggtcccggc 360
   ctccc 365
<210> 66
   <211> 507
   <212> DNA
   <213> unknown
<220>
   <223> >gi | 53688311gb | AI803437 | sr | IMAGp998C105092.1 | cl | IM AGp99SC105092 | AI803437 tc39f05.x1 Soares_total_fetus_Nb2HF8_9w Homo sapiens cDNA clone IMAGE:2067009 3', mRNA sequence.
<400> 66
   tttgtttata aatggttatt ttattcagag tggttgcttt ttaatgctgc tctcttaatt 60
   tcctgttaaa tctgtttcat ctatagccat ccaatattag tatttatttt gttcatttaa 120
   ctttaaagta ttcttaaatt aaatctatta caaatattct actgctggtg aagctaattc 180
   agcataggca ctaccacaaa aatatggaaa acatgatcaa tgtattatag atgtaatctg 240
   gcttcagaaa cacaatacta aaaactattt tagtaaatta agaaataagc attctagatt 300
   gtgttataaa atccatttct aaatataccc ctttttaaac ctttatgctt ctttaccttg 360
   gaattcttac cagcgtagct agattttgta ttgcaatggt aattatactt aagaatcatt 420
   gtgtgttaaa atattcccca cgtacagatt atttttatca tcttttccct aaaataaaat 480
   tctcagcatt taacctatgc atgttgg                                     507
<210> 67
   <211> 638
   <212> DNA
   <213> unknown
<220>
   <223> >sr | IMAGp998I19131.1 | cl | IMAGp998I191311 | gb | BX10791 | oid | 234555961 aX110791 Soares fetal liver spleen 1NFLS Homo sapiens cDNA clone IMAGp998I19131 ;
   IMAGE:127962, mRNA sequence
<400> 67
   tgtggaagtt gttctagaag ttagatggac agtgggagca cattcgtgag tggggcttgg 60
   gagagctcaa aagtggcact ttgattttag gctgggcagg cagaagtctc acagtaaaat 120
   atatttcact agacgtccaa accaacactg cattgtagta acctcttctg aagtaactgg 180
   ttaaattaat aactggtccc ataagtcagg gtcaagccac atttataacc cattcgggcc 240
   ggccatgaag ggttctgact cgacattggt atttaaatgc tgttactttc ccaatgctta 300
   gtagcacttg agactttatt tccatcgaca aaaaccaaga aggccacaga agtagcctaa 360
   cagtcctccc tggtcatact cgtttcaaac aggtctgcct gtctagacat attcaggcag 420
   gaggttgctt gctgctatct tgaaaaccac tggaagattc agcttcttgg ggacctcaga 480
   gcccatattc atatggaagc ccacccatat aagcctcaca gagcaaatca cagcaatgtt 540
   gaagttgtta ttttcaagtg cttatttcac gacattaaaa aaaatatttt ttcctggtgt 600
   attaaaatta aaaataaaat cataactttt gatttaaa                         638
<210> 68
   <211> 445
   <212> DNA
   <213> unknown
<220>
   <223> >sr | IMAGp998E246080.2 | cl | IMAGp998F246080 | gb | BX1049 07 | oid | 234544194 BX104907 MCI_CGAP_Gas4 Homo sapiens cDNA clone IMAGp998F246080 ;
   IMAGE:2445335, mRNA sequence
<400> 68
   tttttttttt ttttaatgca caaaggtttt aataccttgg ctttaatgat ttttcaaggt 60
   taagaaacaa attcaaattg gttggagctt caactcagta attacaatca caatgcatct 120
   ctgaaaggcc ctgcatttgg aggcagagta atctgcaaag atgatagttt ttacatatgt 180
   cctgttacct acaccaatat aattactaca ttatcttata aagacaaaca gttgcttcaa 240
   actctttaaa aaatatatat ataatgagtt tcccaaagac tcgagtctat attcaaagat 300
   gagtaaaaaa aaatccatta cttccctagg gtcactttct tcctttactc ctgcttaaat 360
   gcaaaagctg atagtttctg atttgtagaa aaatctaaag gtttctgctt tttagacaaa 420
   ttcaggttct cttttgcttt ttctt                                       445
<210> 69
   <211> 464
   <212> DNA
   <213> unknown
<220>
   <223> >gi | 3483725 | gb | AF086380 | sr | IHAGp998B03791.1 | cl | IMA Gp998B03791 | AF086380 Homo sapiens full length insert cDNA clone ZD69C02
<400> 69
   gtttggaaac atttaattta tacccctctc ctctgtctcc agaagcttct agctctgggg 60
   tctggctacc gctaggaggc tgagcaagcc aggaagggaa ggagtctgtg tggtgtgtat 120
   gtgcatgcag cctacaccca cacgtgtgta ccgggggtga atgtgtgtga gcatgtgtgt 180
   gtgcatgtac cggggaatga aggtgaacat acacctctgt gtgtgcactg cagacacgcc 240
   ccagtgtgtc cacatgtgtg tgcatgagtc catgtgtgcg cgtggggggg ctctaactgc 300
   actttcggcc cttttgctct gggggtccca caaggcccag ggcagtgcct gctcccagaa 360
   tctggtgctc tgaccaggcc aggtggggag gctttggctg gctgggcgtg taggacggtg 420
   agagcacttc tgtcttaaag gttttttctg attgaaaaaa aaaa                   464
<210> 70
   <211> 727
   <212> DNA
   <213> unknown
<220>
   <223> >s | IMAGp998N15372.1 | cl | IMAGp998N15372 | gb | BX105335 | ioid | 234S45051 BX105335 Soares fetal liver spleen 1NFLS Homo sapiens cDNA clone IMAGp998N15372 ;
   IMAGE:195662, mRNA sequence
<221> misc_feature
   <222> 725
   <223> n=a, t, c or g
<400> 70
   caatccttct gccttggccc cccaagtgct gggattgtgg gcatgagctg ctgtgcccag 60
   cctccatgtt ttaatatcaa ctctcactcc tgaattcagt tgctttgccc aagataggag 120
   ttctctgatg cagaaattat tgggctcttt tagggtaaga agtttgtgtc tttgtctggc 180
   cacatcttga ctaggtattg tctactctga agacctttaa tggcttccct ctttcatctc 240
   ctgagtatgt aacttgcaat gggcagctat ccagtgactt gttctgagta agtgtgttca 300
   ttaatgttta tttagctctg aagcaagagt gatatactcc aggacttaga atagtgccta 360
   aagtgctgca gccaaagaca gagcggaact atgaaaagtg ggcttggaga tggcaggaga 420
   gcttgtcatt gagcctggca atttagcaaa ctgatgctga ggatgattga ggtgggtcta 480
   cctcatctct gaaaattctg gaaggaatgg aggagtctca acatgtgttt ctgacacaag 540
   atccgtggtt tgtactcaaa gcccagaatc cccaagtgcc tgcttttgat gatgtctaca 600
   gaaaatgctg gctgagctga acacatttgc ccaattccag gtgtgcacag aaaaccgaga 660
   atattcaaaa ttccaaattt ttttcttagg agcaagaaga aaatgtggcc ctaaaggggg 720
   ttagntg                                                           727
<210> 71
<220>
   <223> gi | 2113210 | gb | AA430036 | sr | IMAGp998G201924. | cl | IMA Gp998G201924 | AA430036 zw65f10.sl Soares_testis_NHT Homo sapiens cDNA clone IMAGE:781099 3', mRNA sequence
<400> 71
   000
<210> 72
   <211> 428
   <212> DNA
   <213> unknown
<220>
   <223> >gib | 713479 | sr | IMAGp998E1983 | cl | IMAGp998E1983 | gb | T8 5127 | T85127 yd31a10.r1 Homo sapiens cDNA clone 109818 5'
<221> misc_feature
   <222> 191, 306, 365, 390, 417
   <223> n=a, t, c or g
<400> 72
   taagcccaga atgaagctgc tgggtcatcg tagggcactt gcatgcttta tgatttaatg 60
   actgcctagt tgtgcacaga ggcctgccca gtctgtttcc accagtatat ccagtaaagg 120
   gtgctgtaca ccacctctgc ccacacttgg cctttcccac tctctggtgg ttgccagtcc 180
   acaggcgtag nagctgcctt gctgttcttc aggcacatcc ctctgggcac ccacataaca 240
   gaggtgtctc tgtgtgctct gcctgctgtt cattcccagc taattcgggg agggccgagg 300
   gcaggnaaaa ttcgctttaa acccggggag ggcaggaggt ttggcaagtt gaggccgagg 360
   atggncaacc atttgcggtt ttcagttttn ggggcaaaca ggaggttagg atttccnttt 420
   ttcaaaaa                                                          428
<210> 73
   <211> 379
   <212> DNA
   <213> unknown
<220>
   <223> >sr | IMAGp998C09200.1 | cl | IMAGp998C09200 | gb | BX105022 | oid | 234544425 BX105022 Soares placenta Nb2HP Homo sapiens cDNA clone IMAGp998C09200 ; IMAGE:138176, mRNA sequence
<221> misc_feature
   <222> 335, 371
   <223> n=a, t, c or g
<400> 73
   tttaaatgca ctgaacgtga aagcatttct ctcaccaaat tagaaagttc acccaggaag 60
   cttcataaag ataagagaca ggaaaataaa cataagacct ttttaccggt gaaaggtaac 120
   acagaaaaat caaacatgct ggagtttaaa ttatgtccag atatcttact aaagaataca 180
   aactctgtgg aagaacggaa ggatgtaaag cctcatccta ggaaggagca agcccctctg 240
   caagtttcag gaataaaaag tacaaaagaa gactggttaa aatttgttgc tacaaagaaa 300
   aggacacaga aagacagcca agagagagat aatgntaatt caagactctc gaagagaagc 360
   ttcagtgcag ntggatttg                                              379
<210> 74
   <211> 607
   <212> DNA
   <213> unknown
<220>
   <223> gi | 14074023 | gb | AI337096 | sr | IMAGp998I204942.1 | cl | IM AGp998I204942 | AI337096 qx88b10.x1 NCI_CGAP_GC6 Homo sapiens cDNA clone IMAGE:2009563 3', mRNA sequence
<400> 74
   ccagttgtta caaatttatt ttctatagtg gacttcacat gtaatttttt ccaacaaagt 60
   attcaattga atggaagcag gaaattgaaa atcactgata ttgaagatga tgtactggcg 120
   gataaaggca cctgccctcc tgtaatattc agtagaaatt ttgagatctt gaaaacatat 180
   gaaaaatgtg ggctatgtta tcactcctga cagtgatggc tttgatggag tgggatgact 240
   ttggacccat ctcttggcca gtgtatctgt gccagggtca aaggagaagg cactgggttt 300
   ggactcactc aatccaaatt gccttgctct gagattttat gtagatcagt gaaaacttga 360
   gacttcctga agaccagaag gaggcttcca ttccatctga tctcacctag atctggagca 420
   aaggcaacat gataggtaaa atttgcacat cagatggtca aataggaatc agataataaa 480
   aatgtggtcc tcgctgtctg ttcagcatgg tgagtgtgtg cctgtgctcc ctgattcttc 540
   ttggctgagg tgttgagctg atactggtga gtggcaagga gtcctttgcc ttctgagctt 600
   tgagcac                                                           607
<210> 75
   <211> 495
   <212> DNA
   <213> unknown
<220>
   <223> >gi | 5439998 | gb | AI820919 | sr | IMAGp998H171818.2 | cl | IM AGp998H171818 | AI820919 zu39g09.y5 Soares ovary tumor NbHOT Homo sapiens cDNA clone IMAGE:740416 5', mRNA sequence
<221> misc_feature
   <222> 483
   <223> n=a, t, c or g
<400> 75
   actatcctga cgtgtgacag gtccctgatc cgccccagcc tgcaggccag aagcagtgtt 60
   acacacttcc tggaggagct aaggaaatcc tcagactcct gcgtttttgt tgttgctgtt 120
   gatgtttttt aaaaggtgtg ttggcatata atttatggta atttattttg tctgccactt 180
   gaacagtttg ggggggtgag gtttcattta aaatttgttc agagatttgt ttcccatagt 240
   tggattgtca aaaccctatt tccaagttca agttaactag ctttgaatgt gtcccaaaac 300
   agcttcctcc atttcctgaa agtttattga tcaaagaaat gttgtcctgg gtgtgttttt 360
   tcaatcttct aaaaaataaa atctggaatc ctgctttttt gctctactag tacctctgtc 420
   acactagtct tatcaaaaac cagttcttaa gaatcaatgt aagtttatta gttaatgtaa 480
   atntctcatc ctcga                                                  495
<210> 76
   <211> 719
   <212> DNA
   <213> unknown
<220>
   <223> >gi | 4435381 | gb | AI521246 | sr | IMAGp998P175394.11 | cl | IM AGp998P175394 | AI521246 to66g09.x1 NCI_CGAP_Gas4 Homo sapiens cDNA clone IMAGE:2183296 3' similar to gb:U02570 !!!! ALU CLASS C WARNING ENTRY !!!! (HUMAN);, mRNA sequence.
<400> 76
   aacttattaa aaagagtttt atttagcact tggttctgtt ggctgggaaa ttcaggatca 60
   ggcagctgta tctggtgggt tctcatgact gcctcatgct gcatcaaaac atcgtagaga 120
   aacagaaggg gaccgagttt gtgcaaacaa aaagcgcaaa atagaagagg cagcattgtt 180
   ttataacaac tcactctctt gggaactaac cattcccagg agaacccagt ctcagtgtca 240
   agagaaagat gttaatccat cttagcaacc taattacctc ttaaaaatac catctcccaa 300
   cactattaca ttggcaattg aactacaaca tgagttttgg aggggccaaa caacatgcaa 360
   accatagcag caggtttaaa ggtaaaagga tgggagaaaa gtacacagaa agatattctg 420
   gagaaaaggc acagagtgag ccaaggcaca aggtaaaatg tttgactggt tagagacgct 480
   agattatttt gtgtggctaa gacaaaacaa agctggtggg gagtggcagg aggtaagtct 540
   agaatggcac aatggggcca gactgcgaat gcaaacaagt atgtgatctt ttatttctaa 600
   agaaatgtag tatgattagc acacacacac acaaaaatta ttggccgggc gcagtggcca 660
   tgcctgttat ccagcacttt gggaggccga gagggtggac atgaggtcag agttcagac 719
<210> 77
   <211> 420
   <212> DNA
   <213> unknown
<220>
   <223> >gi | 4834825 | gb | AI670051 | sr | IMAGp998F105821.1 | cl | IM AGp998F105821 | A1670051 we64d05.x1 Soares_thymus_NHFTh Homo sapiens cDNA clone IMAGE:2345865 3', mRNA sequence
<400> 77
   tttttttttt tttttttttt tttttttttt tttttttttt tttttttttt tttttttttt 60
   tttttttttt ttggggtttt aaaaaaaaaa agggtttttc caggggggaa agggaggggt 120
   aaacccgggc ccccaaatgg gggcccgttc ccccgggtaa aaaaaagccc ccggaaaaaa 180
   gggtccccat tgggtttaaa ccccctaaaa aattttttgg cccccccccc ccccgggggc 240
   cttttggaaa aaaaaatttg gctaatatgg caaaaaaccc caaccctttt tcccgccccc 300
   tttaaaaaaa ccggccccta ccggggggcc ggcccccccc caaaaggggg gaaattccaa 360
   atgggttggg taaacccccc cctttaaaaa aaggggaaac cggggccaaa aaaggaaaaa 420
<210> 78
   <211> 708
   <212> DNA
   <213> unknown
<220>
   <223> >sr | IMAGp998G01671.1 | cl | IMAGp998G01671 | gb | BX109981 | oid | 234554341 BX109981 Soares_fetal_lung_NbHL19W Homo sapiens cDNA clone IMAGp998G01671 ; IMAGE:299928, mRNA sequence
<221> misc_feature
   <222> 654, 690, 707
   <223> n=a, t, c or g
<400> 78
   catctgcaat agtatggcta agacgctgca gacagccctg cagccccctg actggctgca 60
   gggccactac ctggtggtgc ggtacgagga cctggtggga gaccccgtca agacactacg 120
   gagagtgtac gattttgtgg gactgttggt gagccccgaa atggagcagt ttgccctgaa 180
   catgaccagt ggctcgggct cctcctccaa gcctttcgtg gtatctgcac gcaatgccac 240
   gcaggccgcc aatgcctggc ggaccgccct caccttccag cagatcaaac aggtggagga 300
   gttttgctac cagcccatgg ccgtcctggg ctatgagcgg gtcaacagcc ctgaggaggt 360
   caaagacctc agcaagaccc tgcttcggaa gccccgtctc taaaaggggt tcccaggaga 420
   cctgattccc tgtggtgata cctataaaga ggatcgtagt gtgtttaaat aaacacagtc 480
   cagactcaaa cggaggaagc ccacatattc tattatagat atataaataa tcacacacac 540
   acttgctgtc aatgttttga gtcagtgcat ttcaaggaac agccacaaaa tacacacccc 600
   taagaaaagg caagacttga acgttctgac caggtgcccc ctcttcttct ttgncttctc 660
   ttggcctctt tctcctattt cttaccctgn cctccacctg cctttcnt 708
<210> 79
   <211> 398
   <212> DNA
   <213> unknown
<220>
<223> >gi | 4687900 | gb | AI636570 | sr | IMAGp998D125539.1 | cl | IM AGp998D125539 | AI636570 ts91h06.x1 NCI_CGAP_GC6 Homo sapiens cDNA clone IMAGE:2238683 3', mRNA sequence
<400> 79
   tttttttttt tttttttagg ggaagagaag caaggcccct cagagacacc tttattcttg 60
   ctctttctcc tttcaagaca tggttgctcc ctaggcagtc acatttctct ggtgatggag 120
   agaaaggagg gcagttggag attgagggga cagcatggga tagccgagac atgctgatag 180
   ctggagggga ggctgcctgg gacctgaaga agcagtgatg tcccagctcc agtcatctga 240
   actcccctaa taggcctctc catctctcca gacattagat cttgtagaga aagaaactgc 300
   tagaatccaa atcttttcac acagtgaggc ccccaaacag cccagccttc acacacaagc 360
   cagcctgctt ctcctgactg acttgatcaa tacctcaa 398
<210> 80
   <211> 615
   <212> DNA
   <213> unknown
<220>
   <223> >gi | 1123701 | sr | IMAGp998C22571 | c1 | IMAGp998C22571 | gb | H99033 | H99033 yx11f11.s1 Homo sapiens cDNA clone 261453 3'
<221> misc_feature
   <222> 1, 397, 434, 452, 490, 563
   <223> n=a, t, c or g
<400> 80
   ntttttaaag ttggtctctg ctttttatta tcatgtgctg acaagtctaa acaatgtcaa 60
   tgctaaaata tttcttcttg ggcagaccat tcctaccttc tcggtaccac accgtaccac 120
   aagtagctta cccattcctc tagtgataga catttgggtt gtttccagca tgggtttatt 180
   agaaagagca ctgtagccgg gcatggtggt tcatgcctgt taagtgagct gaggtaaaat 240
   atattaatac atgaaatcta tcattttaac catttttatg tgtacaattc agtgcattaa 300
   gtacattcac aatgttatgc aaccaccacc actctccatt tccagaacat cttcatcatc 360
   ccagagaatc tgtttgcagc ctcactccct attccancct tcccctagcc ctggcaacct 420
   ctaccttctt ggcnctgaaa ttggcctatt cnagatgcct catctaaatg ggatcaaatt 480
   ggccctttgn ggctggctaa ttcattaaca ggtttcaagg tcatccacgt ggagctggca 540
   cagacttatg cagcccaaac canccctacg gatcaatccc cggagaaggg gcaggccagc 600
   agatccctgg caccg 615
<210> 81
   <211> 759
   <212> DNA
   <213> unknown
<220>
   <223> >sr | IMAGp998M24260.1 | cl | IMAGp998M24260 | gb | BX097294 | oid | 234528960 BX097294 Soares breast 3NbHBst Homo sapiens cDNA clone IMAGp998M24260 ; IMAGE:161471, mRNA sequence
<400> 81
   tgcacaggat cccaagtcat gctgtagagc cggattccta gacccagggc tctgcactct 60
   caaggctggc cccatgtgct caagggggtc taatgtttgg gctccaaact aaccatctcg 120
   gagctgggct cctcatttac tgccaaaccc tcagcttatg tagctagaaa gggccctgga 180
   gtgagaaagc ctggattttc aaattgatgc tcccctactg actagctgtg ccactctggg 240
   caaatgctct tccttgagcc tgtttccaca cctgtaaagt ggggatgatg atcctatctc 300
   actgcttttg tgaggattac aggaaagcac ctgtcctggc tctgtacctg gcacgtagta 360
   ggtgctcagt tcatgctggt ttccttcctg cctttagtag ggacctgctc tgtgctcaca 420
   cctcggctgc atgcaccctg ctgtgacgga ggctagtgtg gaagaggtcc tgtcctcagg 480
   gaattaactg tcttattggg agacaacaac tgtcctcctt ggaacaccca agaaaccatg 540
   caaagcagtg gacaacacag aacacgccct cctcctcgct gcctgcagct ccaatctgat 600
   tctgcttggg aatgggcgga gcacgtgggc tgcttaactg ctgtatagga caagcccctt 660
   acccctctct gggcccatga attcctggct tggtttatgt tctgatttga cacactgatt 720
   ttaatcttcg aatcatgaca ctgagtgcag aggaggtgg 759
<210> 82
   <211> 751
   <212> RNA
   <213> unknown
<220>
   <223> >sr | IMAGp998N11579.1 | cl | IMAGp998N11579 | gb | BX118177 | oid l 234570730 BX118177 Soares melanocyte 2NbHM Homo sapiens cDNA clone IMAGp998N11579 ;
   IMAGE:264778, mRNA sequence
<221> misc_feature
   <222> 702, 745
   <223> n=a, t, c or g
<400> 82
   gtttctttgg attttacaga ggtctgatgc ctactatttt aggaatggct ccatatgcag 60
   gtgtttcatt ttttactttt ggtaccttga agagtgttgg gctttcccat gctcctaccc 120
   ttcttggcag accttcatca gacaatccta atgtcttagt tttcaaaact catgtaaact 180
   tactttgtgg tggtgttgct ggagcaatag cgcagacaat atcctaccca tttgatgtga 240
   ctcgtcggcg aatgcaatta ggaactgttc tgccggaatt tgaaaagtgc cttaccatgc 300
   gggatactat gaagtatgtc tatggacacc atggaattcg aaaaggactc tatcgtggtt 360
   tatctcttaa ttacattcgc tgtattccct ctcaagcagt ggcttttaca acatacgaac 420
   ttatgaagca gttttttcac ctcaactaaa aaaaaattat ggttggtttt tcttaataca 480
   ttctcagagg gagaaatgaa acattactat aattgtgggg ggaacattac ttgaatgggg 540
   atatttaccc tgtcacaaga gccactggta ttttagtact tgattatttt ttctttagtc 600
   acaaatcaga actgcttacc atactttttg atgccaaaca ttatacctta gaacattgaa 660
   gaaaatattc ctaagctgat gctggctaaa ccgctttaaa gntttatttg gaagtagaaa 720
   ctagctttaa aacggggttc aagangttgc c 751
<210> 83
   <211> 401
   <212> DNA
   <213> unknown
<220>
   <223> >ImageID=36807 IMAGP971H10113_S1 CC FromEXP of IMAGp971H10113_s1.exp April 26, 2002 13:08
<400> 83
   agagaaaaat atttttaatg tagaaaagat gaatagctgt aatgaaatac aaaccgttcc 60
   tatacacagt aaacttacta agaactgcta atccttctcc aggttcttag aatctgggta 120
   cagcattata gaaagtaacc acgctatgaa aacacacacc caaaacccga tgggtggcaa 180
   ttgtgataga ttaggaagac aaaacagatt cagaagtagc agagaatact gcacattttc 240
   ttggaaaatt tcttaattgt tcagtgcttt ctgacaagtt tccccagccc aatttatctc 300
   ttagctattc tctgctactt agtcctgtct tctgacaatc atcatatggg gacgatcatc 360
   tgccttacag tgtaacatgt ttttcctaac acagccgcat t 401
<210> 84
   <211> 406
   <212> DNA
   <213> unknown
<220>
   <223> >gi | 900602 | sr | IMAGp998N06288sl | cl | HIMAGp998N06288 | g b | H29692 | H29692 ym61d03.rl Homo sapiens cDNA clone 52960 5'
<400> 84
   tcagtccttc cccaggtggg ccaacccaca gggctgcttg agtgtcttca caatatggcg 60
   ctcggcttcc ccccagagca agagattcaa gggcccaggg taaaagccaa cgtgttattt 120
   ttatccctag cctcagaatt cacacgccgt tgcctccacc atgctctggt ttgatacagc 180
   ccagctctga ttggaagggg ctggggctgc ccgtgctgac tcttcaaagg catcccatcc 240
   tgcagatggt gttcacaggg agagtttgtg ggggccggca ctccctcatc tactggggct 300
   cattctggaa gaaggtccag aagaattggg agaccccttg ccccttcacc caaactttgg 360
   gaggttggca gggtgaacag caggccaagt tcaggttcca agacag 406
<210> 85
   <211> 646
   <212> DNA
   <213> unknown
<220>
   <223> >sr | IMAGp998M18194.1 | cl | IMAGp998M18194 | gb | BX114372 | oid | 234563121 BX114372 Soares placenta Nb2HP Homo sapiens cDMA clone IMAGp998M18194 ; IMAGE:136121, mRNA sequence
<221> misc_feature
   <222> 8, 14
   <223> n=a, t, c or g
<400> 85
   ctttattnag tatnatatat gtggtcggca agaaatatta ataataaatg ttgaaaacac 60
   tattcccatt aagaaataag tggatcaaag tgttaccctt aatgatcata tagacactga 120
   taaataatag tcaactttat tactgtggtt caaaccaagt ggaggttgta tgttttgtct 180
   agaaaatata taaactacaa aaagttaata aacacctaag tgaatgagta aattaaatgt 240
   ggggagtaag attcaattaa gataaagtcc taattttctt ttaatcaaat gctctgcttt 300
   gtactcctta gaatggatgc actggagctg atttaaacat tctttggtgg tcacaagatg 360
   tatagctgcc tctgtatgtt tggcgcatgg aaaaaaatat gagtttttgt ggacatacac 420
   tctttatttg aaaagtatga aatataatcc aagaaagcca gccatggctc agtgataaat 480
   atggaattga tatgaaacat tatattgatt aatcaccttt gaggatgatt aataaaagag 540
   agaaagtgat tctatataga gatgcaggac tatttcaggc tcattcatga tctaggtttt 600
   tgtgcttcat ggttatttta ttatcatcaa taaaaaaaaa aaaaaa 646
<210> 86
   <211> 437
   <212> DNA
   <213> unknown
<220>
   <223> >sr | IMAGp998J155722.2 | cl | IMAGp998J155722 | gb | BX1121 14 | oid | 234558605 BX112114 NCI_CGAP_GC6 Homo sapiens cDNA clone IMAGp998J155722 ; IMAGE:2309102, mRNA sequence
<400> 86
   agtaacttca ttggcttcta ctcagtagat gccaatgttg accctcctcc cagtgtgacg 60
   atcaaacatt tcttcagact ttggcaaatt tcatctgtgg ggggcaacat tgtccccagt 120
   tgagaaccac tgaggtacaa catggactga agaggagcat gtgttaaaaa cgtgtgaatt 180
   atttatcctg tagtatctta ctgatgttca gataaagaaa aagcaaaaca aaagacctaa 240
   aactaggctc ttcccccacg gggtcaggtt catgtgtata gttgcattta attgtattac 300
   gattctgggt tgttgatggg ctgatcatac tgtgttttct ccgaaaaaac aaagaattgt 360
   tcatgagtac ttactaaatg aataagtgaa atgacaaatg aataaagaaa cacataaagg 420
   agaaaaaaaa aaaaaaa 437
<210> 87
   <211> 236
   <212> DNA
   <213> unknown
<220>
   <223> >sr | IMAGp99B821790.1 | cl | IMAGp998B21790 | gb | BX114745 | oid | 234563867 BX114745 Soares_fetal_heart_NbHH19W Homo sapiens cDNA clone IMAGp998B21790 ;
   IMAGE:345524, mRNA sequence
<221> misc_feature
   <222> 232, 235
   <223> n=a, t, c or g
<400> 87
   agaagtatct gcacaattag aaaagtcctc agtaagcttt ttcttggagg gtacactttc 60
   ttcactgtcc ctattcctag acctggggct tgagctgagg atgggacgat gtgcccaggg 120
   agggacccac cagagcacaa gagaaggtgg ctacctgggg gtgtcccagg gactctgtca 180
   gtgccttcag cccaccagca ggagcttgga gtttggggag tggggatgag tncgnc     236
<210> 88
   <211> 506
   <212> DNA
   <213> unknown
<220>
<223> >sr | MAGp998J09792.1 | c1 | IMAGp998J09192 | gb | BX088778 | oid | 234511653 BX088778 Soares_fetal_heart_NbHH19W Homo sapiens cDNA clone IMAGp998J09792 :
   IMAGE:346472, mRNA sequence
<400> 88
   acccacaggg ctgcttgagt gtcttcacaa tatggcgctc ggcttccccc cagagcaaga 60
   gattcaaggg cccagggtaa aagccaacgt gttattttta tccctagcct cagaattcac 120
   acgccgttgc ctccaccatg ctctggtttg atacagccca gctctgattg gaaggggctg 180
   gggctgcccg tgctgactct tcaaaggcat cccatcctgc agatggtgtt cacagggaga 240
   gtttgtgggg gccggcactc cctcatctac tggggctcat tctggaagaa ggtccagaag 300
   aattggagac ccctgcccct cacccaaact ttggaggtgg cagggtgaac agcaggccaa 360
   gttcaggtcc caagacaggc caaggccagt gcggtttccc ttccactgcc tcagtttacc 420
   tgtattcaga agacagtcta ggaagagttg agcagagttc cctctaaaag agtagggagc 480
   tgataacagt ccccaagccc tcctct                                      506
<210> 89
   <211> 371
   <212> DNA
   <213> unknown
<220>
   <223> >sr | IMAGp998H175550.2 | cl | IMAGp998H175550 | gb | BX1148 20 | oid | 234564017 BX114820 NCI_CGAP_GC6 Homo sapiens cDNA clone IMAGp998H175550 ;
   IMAGE:2243008, mRNA sequence
<400> 89
   acagccaggt gggaaggact ccttggcaaa actccaacca gcctgtacac tgggaggaat 60
   gtgcactggg atggagccat agaagtttgt gtcgtttgca gtggggagga gcctggtccc 120
   tcctcttcct gtgaggaacc tggaattcaa tctgtgaggt tgttctggag atgttctggg 180
   gagactgcat taaacacagc ttcgcaccat tgaataaact cagcaacaag ccaatgcata 240
   aaagtaatct atgcttcagg tcacagaagc ttcaagggga aaaaaacaga atactctagg 300
   gccattgttc acaaactcat ctgaaaacat cctggaaaaa ttttcccaaa cacatgaaaa 360
   aaaaaaaaaa a                                                      371
<210> 90
   <211> 784
   <212> DNA
   <213> unknown
<220>
   <223> >sr | IMAGp99BE24312.1 | cl | IMAGp998E24312 | gb | BX099422 | oid | 234533220 BX099422 Soares adult brain N2bSHB55Y Homo sapiens cDNA clone IMAGp998E24312 ;
   IMAGE:172415, mRNA sequence
<400> 90
   cttctggagg ggtgtgggac tagagctggg tgtcacgtga acccttcctg gtagggtgac 60
   ccccttcccc tggagggggt tttagagctg ccgcctctgc tccctctaac ctctttggag 120
   gcagggttgg gggtattgtc attcaaggcc ttttttttgt ctgctccctc cccgaccctg 180
   tgccctcttc tggaggtttc tcgtctggga gagtccctcc cagcagtccc tccacctcca 240
   taaggacaca ctggacaaaa ctcccgcagc tcttcaggaa tgaccgatgc ctacctgtgg 300
   ggttcagttg cccatagttt gaggccttct ctcctcccct accaccgctc tggatcatgt 360
   tactagttcc gtcttttgtg tggccttggg ccagcttcct tgataccttg aagatgggct 420
   tcttgtgagt ccccagggag aaagggacaa gagctaagat ttttgcatca gcccttctgg 480
   cagaaggtgt ggtaggggcc atttgttttt tttagtggac ttgggatttg tggtgtaatc 540
   atatcattaa tgatccaggg tgtgggaaaa atggaggtcc ttgaagtggc tgaatctcat 600
   tgtatttaag acactgtcag ttgccagatg taggcttatt tttggagatg tctaggagag 660
   gaaaaagcta ccaatcatac tcttgatatc cgtctggctg tgtgaggcac ccctacctca 720
   tggggggtgt cttgggattg atgaactgtg gaacctgcct cctgcgctcc ccaaagctta 780
   ttaa                                                              784
<210> 91
   <211> 579
   <212> DNA
   <213> unknown
<220>
   <223> >sr | IMAGp998J081153.1 | cl | IMAGp998J081153 | gb | BX1000 23 | oid | 234534422 BX100023
   Soares_pregnant_uterus_NBHPU Homo sapiens cDNA clone IMAGp998J0811S3 ; IMAGE:485095, RNA sequence
<221> misc_feature
   <222> 19
   <223> n=a, t, c or g
<400> 91
   ttgctacagg tccctgagna tgcaatcaga ttaagcgtag caagcattgc caatgggaaa 60
   gtcaaaataa tttatttttt ttccctttcc ccctacccca tccccagcca agaatttctt 120
   ttcaagatat cgtcatcatt cttaaacaac attcttaacc cccagctggg gtccccattt 180
   taatagatgt cattgcttca agtctaacgg cgccgggagg cctgtttgag ggaaaacatt 240
   agtttgaaaa atccccgttc ccttcatcca ctgcccttgt tctccacgtg ggagtgtgct 300
   tgtggcccct cagaaagata gtctgctggc tcctaggggt tggggtgggg gacacacctt 360
   tttctcagga agaggtgatg gcaatgtaaa acatctaagc aaagttttaa atgaaaaaaa 420
   ggaaacacat ttaaacatcc tgataatgga gggaaggggg gcacatttac acatagccca 480
   gaacttgtag aattctgcat agtgaatgta tattgaatta gtctcctgcc ttatacattc 540
   aggaggaata aatttccata atgtaaaaaa aaaaaaaaa                        579
<210> 92
   <211> 628
   <212> DNA
   <213> unknown
<220>
   <223> >sr | IMAGp998G18196.1 | cl | IMAGp998G18196 | gb | BX091497 | oid | 234517361 BX091497 Soares placenta Nb2HP Homo sapiens cDNA clone IMAGp998G18196 ; IMAGE:136795, mRNA sequence
<400> 92
   tatgttgaag tctttcagtg ttcagctgag gagatgaact ttgtgttaat ggggggcact 60
   ttaaatcgaa atggcttatc cccaccgcca tgtaagttac catgtaagtt tttcttgggt 120
   cttggcgcta ttctacgcta tatgctggta ggtgcttaag ctgctttcgt aactttctgt 180
   gcccctggtt ctttctgagg caggtgaggt ggttatataa ggctctccca tctgtaatca 240
   gtagtacctg gtaatcattt aagaccttgg ttgtggacac ccataattag agatgctaca 300
   aatacctcct ttcaaattat attcctgctt ttccagtgat aagcacttga tttctgaagc 360
   ttagtgtgct ccagataagc actgactctg ggaggcaacc tggctatata gataatttta 420
   gtacgcattt aaaggcaaaa taaaagtatg gctggattct ttttatttta ttttttaaaa 480
   cgtattaacc tttaagaagg gcagtttgat gcacatgttg catttaactt tttgagcatc 540
   aagtattttt ttaacatttt tgagcatttc tgttgctctc caggatatct ggaatgagca 600
   agtgagcaaa aacaaaaaaa aaaaaaaa                                    628
<210> 93
   <211> 260
   <212> DNA
   <213> unknown
<220>
   <223> >gi | 2106587 | gb | AA426115 | sr | IMAGp998F241862.1 | cl | IM AGp998F241862 | AA426115 zv52d12.s1
   Soares_testis_NHT Homo sapiens cDNA clone IMAGE:757271 3', mRNA sequence
<400> 93
   tttttttcca actggttaga gaggcaagtg atttattctc cggctcatgt ttctatatgg 60
   aggcattttc caagactaac aatcctggct tctctctttt ggctttcctt gtcctccgtg 120
   gggcaaagtt atctggaaag tctggaaggg accaggcaca cgcagctcag cctttgctgc 180
   tctcccctct cttcacactt gtgttatctg gagggagatg gaggcaggtg tccgtagccg 240
   ggtcctgagc ccctcgtccc                                            260
<210> 94
   <211> 723
   <212> DNA
   <213> unknown
<220>
   <223> >sr | IMAGp998B09229.1 | cl | IMAGp990B09229 | gb | BX112820 | oid | 234560017 BX112820 Soares placenta Nb2HP Homo sapiens cDNA clone IMAGp998B09229 ; IMAGE:149288, mRNA sequence
<400> 94
   tatttcttgt acttggcctg ctccttttct tcgaggattt gttaattatc taaaagtcag 60
   aaacatgtct gaaagtatgg cagctgctca tagaacaagg tatttcttct tattgtagag 120
   actgcatcaa cccgacattc ctttcttata ccaatgtgaa atttccagat catctgtaaa 180
   cctacaactt taatagaaga ctactaataa cagaagacaa attagtgaag aaaagactga 240
   gtttcgaaat tgaatggcag ggtggttttt gcttacaagc catttctgtt cattctttaa 300
   gtatctatat ttcatttgtt ttgcacatat gcatatgtgc ccatttaaga tatttgcata 360
   tacttgatag aaaccataaa gttgtagcag ttaagtccag tcacatttgg ttaatcagtg 420
   tttgatataa ttgaaagagt tgagtggata aacagtcttc cagcttgtaa atgccattga 480
   cttctgacct gacatttagt ataataaaaa tgaaattctt aaccatgtca aatgatttag 540
   tttctggctc ttagactcat ctggcagttc tacacatgaa acatcttttg ttatataagg 600
   tgtattgaaa cctgcagtgc tgattattag aaaggatttg tcagattttt gaacatgata 660
   tttacattat tatttaggaa aactcttcct gtaaataacc atgcataact tactttctgc 720
   aat                                                              723
<210> 95
   <211> 450
   <212> DNA
   <213> unknown
<220>
   <223> >gi | 757038 | sr | IMAGp998E11127 | cl | IMAGp998E11127 | gb | R06418 | R06418 yf09a06.sl Homo sapiens cDNA clone 126322 3' similar to SP:I1BC_HUMAN P29466 INTERLEUKIN-1 BETA CONVERTASE PRECURSOR
<221> misc_feature
   <222> 7, 50, 61, 78, 156, 182, 189, 196, 249, 312, 316, 348, 378, 415, 423, 436
   <223> n=a, t, c or g
<400> 95
   tttgttngtc ttcactttta ttgaaataca aaatgttaaa tatgcaagcn gtactaatga 60
   nggtgctcct tgaagttnat taaggagggc tgggctgctt gtggcttcca ttttcaattg 120
   ccaggaaaga ggtagaaata tcttgtcatg gacagncgtt ctatggtggg catttgagct 180
   tnggccctnc gagttncaaa tgattgctgt accttccgaa atacttcctc taggtggcag 240
   caccaagant atttctggga agcatgtgat gagtcgtgtg atgatgatag agcccattgt 300
   gctgtctctc cngggncacg tgtgtgtggg cgttgaagga ggcagtanag ccaatgaagt 360
   cccttctcca cgtggggnct ttgttaaaca gcatcttccc ctagggtgct cagancactt 420
   ttnaagacgc cacttnccag ggtgctggga                                  450
<210> 96
   <211> 125
   <212> DNA
   <213> unknown
<220>
   <223> >sr | IMAGp998N02421.1 | cl | IMAGp998N02421 | gb | HX110042 | oid | 234554463 BX110042 Soares fetal liver spleen 1NFLS Homo sapiens cDNA clone IMAGp998N02421 ;
   IMAGE:214465, mRNA sequence
<400> 96
<210> 97
   <211> 335
   <212> DNA
   <213> unknown
<220>
   <223> >gi | 5637283 | gb | AI917428 | sr | IMAGp998B14S536.1 | cl | IM AGp998B145536 | AI917428 ts79d07.x1 NCI_CGAP_GC6 Homo sapiens cDNA clone IMAGE:2237485 3' similar to contains element MER22 repetitive element ;, mRNA sequence
<400> 97
   tttttttttt tttttttttt tttttttttt tttttttttt tttttttttt tttttttttt 60
   tttttttttt ttaaaaaggg gccccttaaa ttttccttgg gaaaaaactt ttcaaaaatc 120
   cctttatttt tcccaattcc tttgggggga aaccgggggg ggggggggaa aaaaccaaaa 180
   accaattttt ttaaaccccc ggccccccaa aaaagccttt tggttcaaaa ccccaaaaaa 240
   aagtttttgg tctttttggg gccccccccg gggggggggg ggaacccaac cggccacccc 300
   gggggaaaat ttaaaaaacc cccccccggg gggaa 335
<210> 98
   <211> 464
   <212> DNA
   <213> unknown
<220>
   <223> >gi | 3179587 | gb | AA993042 | sr | IMAGp998C144120.1 | cl | IM AGp998C144120 | AA993042 ot92f07.s1 Soares_total_fetus_Nb2HF8_9w Homo sapiens cDNA clone IMAGE:1624261 3', mRNA sequence
<400> 98
   tttcctttat tttattcact gacatctctc agacctagac cagtgcctgg catatcctag 60
   gcatacatta tattgaatgc atgaatttaa acatttaata attttaattt catagttgtc 120
   ttttaatatt tgagattttt accagatctt gcacattctg ttgctactag aaaagcccct 180
   tttctatggg cttgaaggat agaaaaatct tagcctgact tcactaacca gtataaatcc 240
   agtggcccca ggaaggctgg gaacatccta tgtgaccatc cttgattttg gaccctaaaa 300
   cgaaagctca acttcacact catctggaaa catctaatat cttttcaaac acatgtattt 360
   tgttccccca actagacatg aaacacattg acagaaattg cacaaagtat tcagcacagt 420
   cctgaatact tcataaagac ataaaacctg gcttttgatg gact 464
<210> 99
   <211> 232
   <212> DNA
   <213> unknown
<220>
<223> >gi | 2583451 | gb | AA651799 | sr | IMAGp998M232973.1 | cl | IM AGp998M232973 | AA651799 ns19a12.s1 NCI_CGAP_GCB1 Homo sapiens cDNA clone IMAGE:1184062 3' similar to contains Alu repetitive element;contains LTRS.b3 LTRS repetitive element :, mRNA sequence.
<400> 99
   ctttgcacaa atttaggatc aattttgtta ggaaagcaag cattgaagtg taggccgatg 60
   cgggaggatc acttggggtc aggaattcaa gatcagtgtg ggaaacatac caagagccca 120
   tctatacaaa aaacattttt aattagctgg gcatggtggt gttcacctgt agacccaatt 180
   acttggaaag ctgagacagg aggattactt gaaccatgat tgcaccactg ca 232
<210> 100
   <211> 680
   <212> DNA
   <213> unknown
<220>
   <223> >sr | IMAGp998D051943.3 | cl | IMAGp998D051943 | gb | BX1034 10 | oid | 234541200 BX103410
   Scares_total_fetus_Nb2HF8_9w Homo sapiens cDNA clone IMAGp998D051943 ; IMAGE:788308, mRNA sequence
<221> misc_feature
   <222> 38, 114, 638
   <223> n=a, t, c or g
<400> 100
   ataaggatgg atgaatttag acagataatt caaaggtnca tgttaaaaga actggatact 60
   ttttgtcact ggaacgaact tgatcagttc atcaataagt ttttcctaaa cggngtcttt 120
   atacatgatc agaagctctt cactgacttt gttaatgatg ttaaagatta tcttagaaac 180
   atgaaggaat atgaagtaga taatgatgga gtatttgaga agttggatga atatatatat 240
   agacacttct ttggtcacac tttttcccct ccatatggac ccaggtcggt ttacataaaa 300
   ccgtgtcatt acagtagttt gtaacatttg tagattggat agcattttta tgatttgatg 360
   agtttcttgt aaggttaccg tttctaagag ttgtgcttta tggccactga gagaattcag 420
   aataaattga aagatggagt ctaaaaatta ttagctgtta caaatggaac atttcattat 480
   aacgtgatca ctttgacttg agcaaatggt ttaattttta tcttaaaaat cagttaagaa 540
   tatataaaat cctactttgg ccaagtttgt ttcttttcat tatagtttat atgaaaagat 600
   caccttaagt gaaattattt tcctttaatc ttttatgnat ttattcactt ttggaagcta 660
   ggaatgagca acacaaattt                                             680
<210> 101
   <211> 402
   <212> DNA
   <213> unknown
<220>
   <223> >gi | 2538624 | gb | AA626237 | sr | IAGp998M031831.1 | cl | IM AGp998M031831 | AA626237 zu93a02.s1
   Soares_testis_NHT Homo sapiens cDNA clone
   IMAGE:745514 3' similar to SW:POL_MLVMO P03355 POL POLYPROTEIN ;, mRNA sequence.
<400> 101
   tttaaagtct tctcctggtt agcctcctag aggaggggct ctttctttcc ttctctttgt 60
   ggcttagcca taagctagaa atttgggatc taaatgcagc agaaccttgc tgcccttagg 120
   aactctctta tttgacacca ggtggttgga gtaggaagtg cacaaacaga ttgcttttgt 180
   ttactactaa gccatctttc ctcttggctt atataaaggc ttaaatactg gagactttta 240
   gagcagattt gagctttttt ctgacacttt atgtcctgcc tttcacagca ggtgcaggga 300
   gtcttgggtt tctcctgggt gcagtagccc ttggctggag ggtttttttt tataccatgg 360
   ccttgaccat tctccttatt ttatttattt attttttggg gc                    402
<210> 102
   <211> 593
   <212> DNA
   <213> unknown
<220>
   <223> >sr | IMAGp998K243513.1 | cl | IMAGp998K243513 | gb | BX0890 19 | oid | 234512315 BX089019 Soares_testis_NHT Homo sapiens cDNA clone IMAGp998K243513 ;
   IMAGE:1391375, mRNA sequence
<400> 102
   agaggatgtt aaagtctctc tttgataaac ttaaattatg aaatagccca gttgttcagg 60
   gtcacttcta ttgactcaat ttgtgtgaga ctttgcactc actatctcgc cggccggact 120
   gcatgcccag ggtcagtagt cagcagagct aaaggtgccc gaatttagca cagctggctg 180
   tggtgcctga catagggcag taccggggga ttcttcagag gtcagccaag tcggtaacat 240
   tctgtgaagc agattaaata cagtaaaatc aaacaaacgg aggtctttgg aagtgatgct 300
   gggcccaggc agtgaaaatc aagaaaagag gagaaagacc aagacctgga tgtcctggac 360
   gacctgaccc aaaaaggggc acaaagcagc agagcttcac caggtgactg ggtggcttct 420
   ttgtcctcat taaatgtaca tctttgcatg aaaaggacct tgactggtct ggactttaac 480
   aaagtaaaat atgaagaaac gtaaaattat gcttctgaaa aatatactga aataaaattt 540
   tgtaaatagc taaaagaaaa ataaataaat ataataacaa aaaaaaaaaa aaa 593
<210> 103
   <211> 671
   <212> DNA
   <213> unknown
<220>
   <223> >gi | 4511234 | gb | AI560893 | sr | IMAGp998B165468.1 | cl | Im AGp998B165468 | AI560893 tq4ld08.x1 NCI_CGAP_Ut1 Homo sapiens cDNA calorie IMAGE:2211375 3' similar to gb:M20560 ANNEXIN III (HUMAN);, mRNA sequence.
<221> misc_feature
   <222> 634
   <223> n=a, t, c or g
<400> 103
   ttaacaaaag caacaatttt tattatcttg ctttatattt aatggattag aactataaag 60
   attcttaact ttgaaagcag aaatataagt tggatagtag ttgcagatct ttaataccat 120
   tttcaatttc atttatgagc tgctacatta taaatgagat gctctaaaat aataatcgct 180
   tttgttgttg ttgttataga acaatgaaaa ttcctgttag gaacacaagt tgctgtttat 240
   atttgcttgt tctcttaaat agtatgagaa gaagtaaggt ggagctgttg gaaaagccca 300
   tcgtggacct ttggagatta tcttcttggt tcagtcatct ccaccacaga tttttaagag 360
   tgtgatttca tagtctccag aagtatccga tttaattgct gaatataggg aatagccata 420
   atgcttcttg aactctgttc gaatgtccaa aaggtcaatt tctgatctgg acaccattat 480
   tcggttcaga gtaaactcat cagttccaat acccttcaag gctcgatgca gtctttcggc 540
   taaaaaaggc cggcgtgttc tcacacaatt aactatggcc cacagtagtc ttcaaaatgc 600
   cagatatttc tctttatgct ggccacatgt cctnttggct gatattctga ttcatcaaat 660
   gtagttttaa t 671
<210> 104
   <211> 467
   <212> DNA
   <213> unknown
<220>
   <223> >sr | IMAGp998G024171.2 | cl | IMAGp998G024171 | gb | BX0897 65 | oid | 234513883 BX089765 Soares_testis_NHT Homo sapiens cDNA clone IMAGp998G024171 ;
   IMAGE: 1693929, mRNA sequence
<400> 104
   tatgccaggt actttgcttt ctttgtatta actcttattc ctcacaacaa ttctgaagta 60
   ttactgtaac taccaacatg ttatagatga ggaaacaaag agaagttaag caacttgctc 120
   cagtgacgca gctggtaagc agcagagctg ggattaaaac ccaggcattc tgattccacc 180
   acctacacac ttagccattc cgccccactg agaatgaaaa tgtcaaaaaa tactaggcca 240
   gtaagtgtta gcaaggcaag aaatttccac accaaaggtg agtatgttgt ttggaaagat 300
   tgggatggaa atgagtaaag tatgtttctt tttaaaagtc ttttagtaag atttaaagct 360
   ctatatgtaa gaagcagatg aaaataccaa gtgaattttt agatttgatg ttatgtaagg 420
   ttttgttttg ttaattaaaa tttggtcatt ttaaaaaaaa aaaaaaa 467
<210> 105
   <211> 510
   <212> DNA
   <213> unknown
<220>
   <223> >sr | IMAGp998K15174.1 | cl | IMAGp998K15174 | gb | BX094991 | oid | 234524351 BX094991 Soares infant brain 1NIB Homo sapiens cDNA clone IMAGp998K15174 ;
   Image:41539, mRNA sequence.
<400> 105
   tttttttttt ttttatctct attagtgggt ttattgacat aactggacat aacaatgtat 60
   aacatttatt catctcccac tgaaagactt cccagtgaaa ctaaagtaga acacagatcc 120
   aggcattagg cattttcagt gatgttctat ggctgaaaag gtgggtggtg aggagaacca 180
   gaggccttgt ccatacttgc aatgggactc aatattaccc tgctgggcaa aagaaatagc 240
   taggagatgc aatagtgcta tgattgaaaa aagccaggag cacaaaaggt gccagatagc 300
   tccctggttg aaaataagag caaacaacac tactgctctc atgtaccagt atgctctcaa 360
   tctaaaataa gccctggcag caaactaaga atgacagtct atttcctgtg gtttattaag 420
   ttttggcccc gcagacgaga ctgccagaaa agcagctgta gttttaagtg gtctggacat 480
   ttactcattt gaacataaac aacgatcagc 510
<210> 106
   <211> 268
   <212> DNA
   <213> unknown
<220>
<223> >sr | IMAGp998I04624.1 | cl | IMAGp998I04624 | gb | BX116135 | oid | 234566646 BX116135
   Soares_multiple_sclerosis_2NbHMSP Homo sapiens cDNA clone IMAGp998I04624 ; IMAGE:281931, mRNA sequence
<400> 106
   tctgcagttc tcagcctggt cctttcttct gtcgagatca ggtctgcttc aaatacttct 60
   gccggagctg ctggcactgg cggcacagca tggagggcct gcgccaccac agccccctga 120
   tgcggaacca gaagaaccga gattccagct agaggagctg gccttgccca gtggcctgtg 180
   gcgcccaaag ctggcaggtc aggcaagcag cctgtaccac cctgccactg gcgaccaggg 240
   agctggcttc ccaaggacaa gggaaaat 268
<210> 107
   <211> 828
   <212> DNA
   <213> unknown
<220>
   <223> >sr | IMAGp998M24235.1 | cl | IMAGp998M24235 | gb | BX116782 | oid | 234567940 BX116782 Soares placenta Nb2HP Homo sapiens cDNA clone IMAGp998M24235 ; IMAGE:151871, mRNA sequence
<221> misc_feature
   <222> 720
   <223> n=a, t, c, or g
<400> 107
   ttacccaggg tttttggagt ctctggatga tttttacatt cttagcagtg gattgatatt 60
   gctgcagacc acaaacagtg tgtttaataa aaccctgcta aagcaggtaa tacccgagac 120
   tctcctgtcc tggcaaagag tccgtgtggc caatatgatg gcagatagtg gcaagaggtg 180
   ggcagacatc ttttcaaaat acaactctgg cacctataac aatcaataca tggttctgga 240
   cctgaagaaa gtaaagctga accacagtct tgacaaaggc actctgtaca ttgtggagca 300
   aattcctaca tatgtagaat attctgaaca aactgatgtt ctacggaaag gatattggcc 360
   ctcctacaat gttcctttcc atgaaaaaat ctacaactgg agtggctatc cactgttagt 420
   tcagaagctg ggcttggact actcttatga tttagctcca cgagccaaaa ttttccggcg 480
   tgaccaaggg aaagtgactg atacggcatc catgaaatat atcatgcgat acaacaatta 540
   taagaaggat ccttacagta gaggtgaccc ctgtaatacc atctgctgcc gtgaggacct 600
   gaactcacct aacccaagtc ctggaggttg ttatgacaca aaggtggcag atatctacct 660
   agcatctcag tacacatcct atgccataag tggtcccaca gtacaaggtg gcctccctgt 720
   ntttcgctgg gaccgtttca acaaaactct acatcagggc atggcagagg tctacaactt 780
   tgattttatt accatgaaac caattttgaa acttgatata aaatgaag 828
<210> 108
   <211> 565
   <212> DNA
   <213> unknown
<220>
   <223> >sr|IMAGp998F16280.1|cl|IMAGp998F16280|gb|BX102345 |oid|234539070 BX102345 Soares infant brain 1NIB Homo sapiens cDNA clone IMAGp998F16280 ; IMAGE:49499, mRNA sequence
<400> 108
   tttttttttt ttttacaaat aagttttacc ttttattgta ttccattata caattttaca 60
   tagtaccatt attttaagca ataattaagc aaattgaact actgtttttg agctctactt 120
   ttcagcatat agatttacct aaaaaagtac aaaccaagca cagtggtgca attttccagg 180
   cactttcaag ttgcttattt acaaatatgt aagcaccctg ttggaacaca tgaaaatttt 240
   ggtgtcgaga agaaacactc catgtatatc aaatctttaa attttaacat ttgcacagct 300
   ccaaatattc ctgagaaagg tcagtctcat tgtcacgtac atctgcattt cctcacactt 360
   cagcgtatct tggggaacca taacctcggt cacttgatgt accactaatt gcacaaaata 420
   catgtaatgt gttgctcatc cagggagaat gcattgtgtc ttcttgtaat atcgacttaa 480
   gtcttcaaag ctgctcatgg ataatgtcag ataattcttt taccatgtct ctgaaatttg 540
   actttagccc ttcatggtac tcaac 565
<210> 109
   <211> 453
   <212> DNA
   <213> unknown
<220>
   <223> >gi|12908931|sr|IMAGp998B23677s1|cl|IMAGp998B23677| gb|W16493|W16493 zb15c12.rl Soares fetal lung NbHL19W Homo sapiens cDNA clone 302134 5'

<221> misc_feature
   <222> 304, 390, 412, 417, 450
   <223> n=a, t, c, or g
<400> 109
   gagaaactgt gtgtgagggg aagaggcctg tttcgctgtc gggtctctag ttcttgcacg 60
   ctctttaaga gtctgcactg gaggaactcc tgccattacc agctcccttc ttgcagaagg 120
   gagggggaaa catacattta ttcatgccag tctgttgcat gcaggctttt tggcttccta 180
   ccttgcaaca aaataattgc accaactcct tagtgccgat tccgcccaca gagagtcctg 240
   gagccacagt cttttttgct ttgcattgta ggagagggac taagtgctag agactatgtc 300
   gctntcctga gctaccgaga gcgctcgtga actggaatca actgcttcag ggaaaaagaa 360
   aaaaaaaaaa aaaaagactt gcctgggagn cgcgagaaac ttgcattgga anttcanaac 420
   cagcattcga gaaactcctc tctactttan cac                              453
<210> 110
   <211> 490
   <212> DNA
   <213> unknown
<220>
   <223> >sr|IMAGp998N204778.2|cl|IMAGp998N204778|gb|BX0924 50|oid|234519268 BX092450 NCI_CGAP_GC4 Homo sapiens cDNA clone IMAGp998N204778 ; IMAGE:1946707, mRNA sequence
<400> 110
   cctttggcct tttataaaaa ggtatttata attcgttgat aaaaaccaat atttgaagct 60
   gttgattacc aacaaaaaag tttcacactc tttatagtgc ttcaggatac aactttttca 120
   gggccttatt agagaaaaac tgattcacat gttattcttc taaagctaaa ttattaaaat 180
   gtaggttgaa agattggatt gattgtgaat ctacactaaa gatatggttc caggcagacc 240
   tccttagaga cctccaggca gacctcctta ctgtcttcag tgatgtttaa tttcattact 300
   gttactgggt gccaagtgtc tttcatttgg aagtgaactt actccagtta ttgaatgttt 360
   atcatatcat aatttgcatt tttccaacat tgaaggtatt ttaaaaagtc actaagagat 420
   tcattctttt attattcaac tacaaaaaaa tagtgtaatg atcagaattt aaaatgtgtg 480
   attctttttc                                                         490
<210> 111
   <211> 493
   <212> DNA
   <213> unknown
<220>
   <223> >ImageID=1309431 IMAGP971F12104_S1 CC FromEXP of IMAGp971F12104_sl.exp April 26, 2002 12:57
<400> 111
   agtttttttt tagaatttag agcattatga gactattgta gaaattttaa ctaccaggac 60
   ctacaggatg gagagtgggg gaggggagaa gaccagaaga gaggcaggaa aacagatgcc 120
   tttgctgatg tcctttgtag tataaaactt aacagcacca cagcacctaa tcattttctc 180
   aggtgtggct ttgagaaata aacgttttat gttaaaaatg aaaatacttc acaatatgag 240
   acagctggca tttgaatact tttcccagta tgtttgtaaa cattttggat tcatatttca 300
   tattaaaatg gacatttagt aggattatta gatgaaaagg gtcccccagg ggatatctaa 360
   tatcaaatta tctgcaggac atcttctcca gaaagcagac tgactgtaat atgaactaat 420
   tttcccccct ttgagttgct tgcaagaatc tctgagatct gacactgatg ggctacagta 480
   atcagcagtt ctt                                                    493
<210> 112
   <211> 496
   <212> DNA
   <213> unknown
<220>
   <223> >sr|IMAGp998I144113.2|cl|IMAGp998I144113|gb:BX0934 51|oid|234521269 BX093451 Soares_testis_NHT Homo sapiens cDNA clone ImaGp998I144113 ;
   IMAGE:1621717, RNA sequence
<221> misc_feature
   <222> 22
   <223> n=a, t, c, or g
<400> 112
   cggctacata gctgtgacta cngggggtgc agtctctggg gaccaccact accgaggctg 60
   ggtaccatga ttatcgcgga acacctgccc attaatcttg accctgttaa aacaataact 120
   gcctatccag atatgccaca cttctgcctg ctgttggcaa ccctcctgga ctaggctgct 180
   cttgttaatc acatggatgt tgctgattac tctgagtgca aaccttttca ctgttccaga 240
   gaggagcctg acaaccacat tctccttctc aagatgtgaa aggcattgta cagaagaaga 300
   tgagaatcac agtaaaccaa acaaaggaaa tgcagaagag atattttcag ctgaatattg 360
   gaaagtaaga ttccctgcga gcaggatctc aggcaacgtc aatgaacaac atgaagcctg 420
   acacattgta ggtgccccgt aaatattggt tgagtaactg aataaacgta tttagtaact 480
   taaaaaaaaa aaaaaa                                                 496
<210> 113
   <211> 496
   <212> DNA
   <213> unknown
<220>
<223> >sr|IMAGp998I144113.2|cl|IMAGp998I144113|gb|BX0934 51|oid|234521269 BX093451 Soares_testis_NHT Homo sapiens cDNA clone ImGp998I144113 ; IMAGE:1621717, mRNA sequence
<221> misc_feature
   <222> 22
   <223> n=a, t, c, or g
<400> 113
   cggctacata gctgtgacta cngggggtgc agtctctggg gaccaccact accgaggctg 60
   ggtaccatga ttatcgcgga acacctgccc attaatcttg accctgttaa aacaataact 120
   gcctatccag atatgccaca cttctgcctg ctgttggcaa ccctcctgga ctaggctgct 180
   cttgttaatc acatggatgt tgctgattac tctgagtgca aaccttttca ctgttccaga 240
   gaggagcctg acaaccacat tctccttctc aagatgtgaa aggcattgta cagaagaaga 300
   tgagaatcac agtaaaccaa acaaaggaaa tgcagaagag atattttcag ctgaatattg 360
   gaaagtaaga ttccctgcga gcaggatctc aggcaacgtc aatgaacaac atgaagcctg 420
   acacattgta ggtgccccgt aaatattggt tgagtaactg aataaacgta tttagtaact 480
   taaaaaaaaa aaaaaa                                                 496
<210> 114
   <211> 392
   <212> DNA
   <213> unknown
<220>
   <223> >sr|IMAGp998N095486.2|cl|IMAGp998N045986|gb|BX0998 82|oid|234534140 BX099882 NCI_CGAP_Ov23 Homo sapiens cDNA clone IMAGp998N045486 ;
   IMAGE:2218563, mRNA sequence
<400> 114
   tttttttttt ttttatagct taaattttca actccaaaat tttatgattt tattattcta 60
   gttagccaaa tatttttcag agtccacgga tgtctctgtg gacattccag aaaactaaga 120
   gtctattaaa ctccaaatct tgatctaacc attttacact gtcaaattat ttattcctta 180
   cagtttttga taataaaatg gtcgaggatt ttgagaacat gtgggcagca gattcctgcc 240
   tctaattggg attcgtgatg ccagggcagg aaaatatcac ttcccagtcc ctgccagtga 300
   aaactcaaaa aaagctgttc agctcatttc atcatgctgt cactaccctc tttctaaaaa 360
   ctgcaaaact cttttcttgg aaatcattat cc                               392
<210> 115
   <211> 615
   <212> DNA
   <213> unknown
<220>
   <223> >sr|IMAGp998I16328.1|cl|IMAGp998I16328|gb|BX104592 |oid|234543564 BX104592 Soares adult brain N2b5HB55Y Homo sapiens cDNA clone IMAGp998I16328 ;
   IMAGE:178647, mRNA sequence
<221> misc_feature
   <222> 580
   <223> n=a, t, c, or g
<400> 115
   tttttgtggg atcctgcagt aaaattgact tttttgtgtc tttgggagat ttaaattgcg 60
   ctaacagtgt tgcgcaaaaa tgagttcatg ccatttaaca tattgtattt taattattaa 120
   ctgtattaat ttactatgaa atggacatcc ttttaactaa aatggaattg aacattgcag 180
   ttttcaaata tttttccttg ttgggtctgg aaaaggaatt ctactttgat ctgcatagaa 240
   aattttgata caattttttg aaagttctta ggtgaaacat ttacccatta aaaaggaagc 300
   agaaatactg agacatgaaa ggcattatca actaactcta gactctagaa cccattctag 360
   catatctcac gtgcaatttt taaaaataag ttaataattc atctcatatc aacaaaagcc 420
   tttgaaacat gggttttcac tagatatcac ctagtgctaa gataaaaacc aaaacaatat 480
   cagaattaca tttatgctct aaatttgtag ttgtccattg ttgtgcttag taaatgtgtg 540
   tcattaatgc tgtattctcc tagctattat ggaaacttgn ttaaataaag atatggatat 600
   aaaaaaaaaa aaaaa                                                   615
<210> 116
   <211> 508
   <212> DNA
   <213> unknown
<220>
   <223> >sr|IMAGp998C14658.1|cl|IMAGp998C14658|gb|BX106304 |oid|234546989 BX106304 Soares fetal liver spleen 1NFLS Homo sapiens cDNA clone IMAGp998C19658 ;
   IMAGE:294853, mRNA sequence
<221> misc feature
   <222> 93
   <223> n=a, t, c, or g
<400> 116
   tctagtattt atatgaatct gtgagagata atttttttgg tctcactgca atgaaccaaa 60
   agcggcctga gtttggtctt ttaattgtag ccntgtattg aaggcatctt tttgaccaac 120
   tcttgttggt tctgtcttga accattgtta cgtcactgtg ctgtaattag tatagctaaa 180
   tctttccttt ccttgctcct cccccagccc accccgtctt cccttaacat tttttcaggg 240
   ggggttggga gtggtttcat tttagtgtga gtggatgttt tgatagttgt aaggaaaaaa 300
   tgcatttcag acacatttca cacatgagct attttcttac acagtatgtc ttattgttaa 360
   taagaatgta atttcatgat acaagtattt aatacctttt ttaagtcagt aaatattcta 420
   gccatcaata aattgcagta gagtattaag aggggacagg atgagtttac tcttaaaatt 480
   aatcagtatc aaactaagtc tactgtgt 508
<210> 117
   <211> 707
   <212> DNA
   <213> unknown
<220>
   <223> >ImageID=1704376 IMAGP971I1573_S1 CC FromEXP of IMAGp971I1573_sl.exp April 26, 2002 13:15
<400> 117
   ccacctttaa attcctgcaa tttatgctgc aaccaacatc ttagtgtttg ctctctccgg 60
   gaagaggcca ggtgggggag aacatacggt ccagagcctg agatcgcctg gctggggtgc 120
   aggcaagcag gaacccagtc tagatagaat gcagtgttct ctaaagctat ggatggtttc 180
   agtgaaatgt ctgtgtctga caatcgtcac gcaagagtac ttactgtctt cccttgtaca 240
   tacattgtct gtatttagct tttcctagta ttgtgaggtt tcaaataggg gttccatgta 300
   aatactcttt ctgctactga aatgccggcc cccttgagta tgcatcaccc ccacagatgg 360
   ttttgccttt ctctatgcta acactttttg agccactgac gtgcagatcg gtggttagtt 420
   tcttgggctt ttattttgca attttatata tatacacata ttatatatat atatatattt 480
   atgggttggt tttgtatagt tttctgtttg aatctctgag caccaaagct gccctttgat 540
   tttcacgaga actttccaaa gccacttcct gagccctcct gctgctttgg tgtctgacgg 600
   tgatgctgcg gtggcatctc tgcccgtgaa tttctgtatg tgtgtgctgt tacctcacct 660
   gcagacatga ccctagtaaa tctaacgtgc ttggcaaaaa aaaaaaa 707
<210> 118
   <211> 390
   <212> DNA
   <213> unknown
<220>
   <223> >ImageID=2328917 IMAGP971B0496_S1 CC FromEXP of IMAGp971B0496_s1.exp April 26, 2002 12:37
<221> misc_feature
   <222> 41, 285, 312, 346, 372
   <223> n=a, t, c, or g
<400> 118
   tgagttgtcc aatcagtttt caattgatac tgttggtagt natggagctg ttaaatgtaa 60
   aggcctgaaa atggactatc aagttggtgt cactatagac ctgagcagtt ttaacattac 120
   tagaattgtg acatttaccc ctttttatat gattaaaaac aaaagcaaat accatatatc 180
   agtggctgaa gaaggaaatg ataaatggct ctctcttgat ttggagcagg tgggtagatg 240
   aatttcaaaa atatacctct ttggattcta ttttctcctt gaagntagat acctaaagtc 300
   accatgtaat gntttaactc tccccttact ccaattttgt tttagnttca tgaatcttta 360
   ttgaatattt gngccctgct ctagatagat 390
<210> 119
   <211> 385
   <212> DNA
   <213> unknown
<220>
<223> >sr|IMAGp998D01196.1|cl|IMAGp998D011961|gb|BX106181 |oid|234546743 BX106181 Soares placenta Nb2HP Homo sapiens cDNA clone IMAGp998D01196 ; IMAGE:136656, mRNA sequence
<221> misc_feature
   <222> 5, 14, 324
   <223> n=a, t, c, or g
<400> 119
   tttgntttct tatnttgtat aatcaggtta atgatttatt ttttgactaa atgtgcaatt 60
   tcttatcact agataacttt cagtatcagt ggtggttact tattacttaa atcagaggaa 120
   ggattttata aagattaata aatttaattt taccaataaa tattcccata atttagaaaa 180
   ggatgtcgac ttgctaattt cagaaataat tattcatttt taaaaagccc cttttaaagc 240
   atctacttga agattggtat aattttcata aaatgtcttt ttttttagtg tcccaaagat 300
   atcttagata aactattttg aagntcagat ttcagatgag gcaacatttt cttgagataa 360
   ttacccaagc ttcatccatg ttgaa 385
<210> 120
   <211> 501
   <212> DNA
   <213> unknown
<220>
   <223> >sr|IMAGp998B2070.1|cl|IMAGp99882070|gb|BX112318|o id|234559013 BX112318 Soares infant brain 1NIB Homo sapiens cDNA clone IMAGp998B2070 ;
   IMAGE:22170, mRNA sequence
<221> misc_feature
   <222> 29
   <223> n=a, t, c, or g
<400> 120
   tttttttttt ttttaacatg tgtcaaagna ttttattaaa ctaattcaat ttgttaggga 60
   aatggtaaga tgttacaact ggttaagagg agcattaaaa gtgcagacat atattcaggc 120
   catcaagaat gaggaaatga atttgctaaa ttgatgcaac atcaatcaat agccagggcc 180
   tgtcattcac agacaatcac tccaccacac gcacacatac attcctacgg tttgtccaca 240
   acttccagag ctgaagagta gctcaaggac atcaaagggc agaggcccct aggaagggct 300
   gagccacaat gaagcagtgc tccttcactc ccgatttcca tggctaccac cgtttttctc 360
   tggcaaatca gagacgaatt ttaaagactg agtaaatcta taaaagcgat taatcacaga 420
   agttcaatgt ctgataaatt attccaaaag tgttcctggg cactgagact caccaagcat 480
   acattctgaa atcaaattct a 501
<210> 121
   <211> 764
   <212> DNA
   <213> unknown
<220>
   <223> >sr|IMAGp998I17258.1|cl|IMAGp998I17258|gb|BX109425 |oid|234553229 BX109425 Soares breast 3NbHBst Homo sapiens cDNA clone IMAGp998I17258 ; IMAGE:160600, mRNA sequence
<221> misc_feature
   <222> 647
   <223> n=a, t, c, or g
<400> 121
   gtggctgttg gattctgact gctgcacatt gtctcagagc cagtaaaact catcgttacc 60
   aaatatggac aacagtagta gactggatac accccgacct taaacgtata gtaattgaat 120
   acgtggatag aattattttc catgaaaact acaatgcagg cacttaccaa aatgacatcg 180
   ctttgattga aatgaaaaaa gacggaaaca aaaaagattg tgagctgcct cgttccatcc 240
   ctgcctgtgt cccctggtct ccttacctat tccaacctaa tgatacatgc atcgtttctg 300
   gctggggacg agaaaaagat aacgaaagag tcttttcact tcagtggggt gaagttaaac 360
   taataagcaa ctgctctaag ttttacggaa atcgtttcta tgaaaaagaa atggaatgtg 420
   caggtacata tgatggttcc atcgatgcct gtaaagggga ctctggaggc cccttagtct 480
   gtatggatgc caacaatgtg acttatgtct ggggtgttgt gagttggggg gaaaactgtg 540
   gaaaaccaga gttcccaggt gtttacacca aagtggccaa ttattttgac tggattagct 600
   accatgtagg aaggcctttt atttctcagt acaatgtata aaattgngat ctctctcttc 660
   attctattct ttttctctca agagttccat ttaatggaaa taaaaacggt ataattaata 720
   attctctagg ggggaaaaat gaagcaaatc tcattggata tttt 764
<210> 122
   <211> 457
   <212> DNA
   <213> unknown
<220>
   <223> >gi|7765081|sr|IMAGp998M04179|cl|IMAGp998M04179|gb| R21727|R21727 yh21b02.sl Homo sapiens cDNA clone 130347 3'
<221> misc_feature
   <222> 19, 130, 326, 357, 372, 392, 435, 455
   <223> n=a, t, c, or g
<400> 122
   ttttttgtgg ttccaactnt ttttatactg aaatatgtct gagctgatcc aaaaaagcgt 60
   tgggttgggg gagaaggaaa tgatggaagt ggggtgatct cagagctcca tgttagcaac 120
   tctgggagtn tgggccggtc catctgtctg tctgcccacc ctggcaggac aatcaggcct 180
   aggaggtctg gaggagctcg ccgagctggg cggaggaggc aggccagtcc catgctggag 240
   gggaccccac ggccccttct acagggcagg agagtcctca gctgaagggc ttcaccagct 300
   tcatggcagc atagttcggg aaggcntaga tgaagatggc cagggaacag cagcagnctg 360
   aagaggatga tnaagaggat gatggcccac cngaaacgcc gccacaggat gaactttcat 420
   ggtcttgtat tgggnaagta aaccacagga aggangt 457
<210> 123
   <211> 376
   <212> DNA
   <213> unknown
<220>
   <223> >gi|5631530|gb|AI911675|sr|IMAGp998K175764.1|cl|IM AGp998K175764|AI911675 wc71e09.x1 NCI_CGAP_Pan1 Homo sapiens cDNA clone IMAGE:2324104 3' similar to contains element MER22 repetitive element ;, mRNA sequence
<221> misc_feature
   <222> 374, 375
   <223> n=a, t, c, or g
<400> 123
   ttgacacaaa atatgttatt tattgtcaat ttctgcaaag acacatttaa gcaacaaaat 60
   atacatttgt caacctttta gaatttgttt tgtacattaa caaatacaat gtgctaccat 120
   agcaataaat taaatgtaca ttatttacat tacataggca gttgggatct acgaatcacc 180
   tcctcactaa gaggaaagaa agcaaagtcg tcgtcacatg gagcatcact tccaagccac 240
   agttcttagg ttcgaccaca caggggaatc tgcacactca cacgccagca gtccccgagc 300
   tggagtgtcc aaggggaaca cccaaaacag gagagagctg cacgcatgct aaggagcagg 360
   aagccggggg gggnng                                                 376
<210> 124
   <211> 501
   <212> DNA
   <213> unknown
<220>
   <223> >sr|IMAGp998J11128.1|cl|IMAGp998J11128|gb|BX090520 |oid|234515407 BX090520 Soares fetal liver spleen 1NFLS Homo sapiens cDNA clone IMAGp998J11128 ;
   IMAGE:126826, mRNA sequence
<400> 124
   gatacagtgt gacattttaa tacatttaca tattgtgtaa tgatcaaatt aggataatta 60
   gcttatgtat cacttcaaaa acataatttc tttgtgatga gaacattcaa aatcttctag 120
   tattttgaaa tacataatgc aatattgtta accacaatca ccctactgtg caatagaaca 180
   ccagagttta ctcctccttt ccatctgtag cttcagaccc attgaccaat ctctcccctt 240
   cgcccccacc accccccccc cgctgtcacc actctcctcc cctccccagc aagaggcttg 300
   aatcttgaag gagcacatca gatagaaaga gggaaatcaa ctgggacatg gtttgttgga 360
   agtggggtgg aatctggttt gtttacagtt tcagagtttg aggcatcaaa agtgtatgtc 420
   attgcccaaa agtatttaac agctttctat gttttaggaa ttcactagaa ataaggcatg 480
   ctttttaaaa aaaaaaaaaa a                                           501
<210> 125
   <211> 293
   <212> DNA
   <213> unknown
<220>
   <223> gi|4628611|gb|AI619485|sr|IMAGp998H195650.1|cl|IMA Gp998H195650|AI619485 ty38g10.×1 NCI_CGAP_Ut2 Homo sapiens cDNA clone IMAGE:2281410 3' similar to contains MSR1.t1 MSR1 repetitive element ;, mRNA sequence
<400> 125
   tttttttttt tttttttttt tttttttttt tttttttttt tttttttttt tttttttttt 60
   tttttgggga aaagggatta aaaaaccttt ttcccaaaaa ggaaggcccc cccccccccc 120
   aaatcccttt gggacaagaa ccgacccccc ccggaaaccc caaccattgt ccccccctcc 180
   cacccctggt ctttcccaag gaaaaaaggc cattcaaaaa tggggggggg gggttagagg 240
   gggggggccc cttcccttta aatgggggaa aaaaaaaaaa gcccccgggt ggc        293
<210> 126
   <211> 519
   <212> DNA
   <213> unknown
<220>
   <223> gi|5111779|gb|AI743491|sr|IMAGp998P025858.1|cl|IMA Gp998P025858|AI743491 wf63h01.×2 Soares_NFL_T_GBC_S1 Homo sapiens cDNA clone IMAGE:2360305 3', mRNA sequence
<400> 126
   tttaaactcc acactgtaac caggagtctt ttcaaagcaa tatcctggtc ttgtctggca 60
   ttcccttggt ctaccttgga ggcatttatc caggtgagaa ttgtggttaa tgaagctcca 120
   taaaacccac acaaagggtc aaccctgcat tatttggtaa agtgatgggg ttaccataac 180
   cagcagcaca aagcacagca cttagacctg tgccagagag cttaactgta caatgtgggc 240
   caccctagcc atgataaaac aatgactgat gaagactaac tcgacaaaga attgtttgag 300
   agtcacgcat tctacccact tacctactct gtattttatt ctggcgttaa actggactgt 360
   cagaaacaaa atgctttggc ttcactaccc aacccaacta gtctcccaac acaaccccat 420
   cccgctaaga aaactgctat cactgagtca tgtttttttg tcatgccctc accttccagc 480
   aagggttgct gtaagaaaca gcctcgagaa tactaaggc 519
<210> 127
   <211> 370
   <212> DNA
   <213> unknown
<220>
   <223> >gi|846987|sr|IMAGp998M03208|cl|IMAGp998M03208|gb| R72955|R72955 yi38a02.rl Homo sapiens cDNA clone 141482 5'.
<221> misc_feature
   <222> 331, 360
   <223> n=a, t, c, or g
<400> 127
   cgagataaag ctttcaggta caagctgaag gtggggtgtc taacaactaa aaactatcac 60
   taaatctcaa agagaaagtt cttgcaaaat atgtaaagtt cacaaggtgc agacattttc 120
   cttctttagg cttttatcta aggaagggct atgaaactgg gcccatctgt atacaggctc 180
   aaatttacgt ttttaaagga agaatctatg cagctgaggc ttattgcagg aaatacttca 240
   tttgtatgta aatattattg taaataaata gggggctgta tatttttgaa gttgttgatc 300
   tgcacttgaa ataggaagtc tctaggggtc ntgcctataa accataaatg tttccccggn 360
   aattagttgg 370
<210> 128
   <211> 283
   <212> DNA
   <213> unknown
<220>
   <223> >gi|34838111|gb|AF086466|sr|IMAGp998H07795.1|cl|IMA Gp998H07795|AF086466 Homo sapiens full length insert cDNA clone ZD86G04
<400> 128
   caagactcca tctcaagaaa aaaaaaaaaa ttagctgggt gtggtggcac atgcctgtag 60
   tcccagctac ttgggaggct gaggtgaaag gatcgcttgg gcctgggaag cggtgattgc 120
   aacgctgcac atcagcctgg gtgaccaagc gggactctgt ctcagaacaa aacaaaacaa 180
   aaatacatat tcccagatcc tggaatgtcc tgggaagcta tatgtgtgtc atgccctccc 240
   agctgcacct gaacaatgag catagcaaac atcaaaaaaa aaa 283
<210> 129
   <211> 451
   <212> DNA
   <213> unknown
<220>
   <223> >gi|2183927|gb|AA459020|sr|IMAGp998A112011.2|cl|IM AGp998A112011|AA459020 aa26a06.rl NCI_CGAP_GCB1 Homo sapiens cDNA clone IMAGE:814354 5', mRNA sequence
<400> 129
   agaatccctg tgcaattctg taaaagaata ccaggttctc tggcatgtag aagtgaactt 60
   gggcaggtgg aaatggttaa caaagaattc taggagtaga gaataaggtc tgaatcctct 120
   taggacagtt gggagttaca aaaaattaac aaacagaaac attgagattg gtgctttaga 180
   acttggagta aattaagaat aaagggaagt agacaccttt aaaaaacaaa gaaaaagaac 240
   ttggtgagta aaaatagaaa agacaaatta tggagtagga aataaaaact caccagaaaa 300
   gttgtctttt acctcctaag ggaaaagcta aaggaattag agttgttttg tctaaagaaa 360
   ggcaagggaa gactcaattt gttgttgtca gttattaaca atttatataa agaatagtgt 420
   actatttctt ttctgtctgc agagattgag c                                 451
<210> 130
   <211> 424
   <212> DNA
   <213> unknown
<220>
   <223> >sr|IMAGp998A205772.2|cl|IMAGp998A205772|gb| BX0975 89|oid|234529551 BX097589 NCI_CGAP_Co3 Homo sapiens cDNA clone IMAGp998A205772 ; IMAGE:2326939, mRNA sequence
<221> misc_feature
   <222> 424
   <223> n=a, t, c, or g
<400> 130
   agtgaaatag agaaagcggg cctggcatcc ccagtaggag gaagacgctg ttggtaaaga 60
   gccgctgaag gcagcaccca agaaacgaca attgctggac agcgacgagg aacaggaaga 120
   agatgagggc aggaacagag caccagagtt gggagctcca ggaatccaaa agaagaaacg 180
   ataccagatt gaggtgggtc gcagcaccag atggggagct ttatgacatg acaggcggga 240
   gttttgtacc acttttagaa gataccagat gcctctgtcc tcattttgtt tcctatgatc 300
   atcctctaga taccagatag aggcccattc ctgcatttga gatagggttg gaatatagta 360
   ctctagatga tgaatgtgtt tgctttgtgt ttggtattaa acatgtaaga ttagaagttc 420
   tcan                                                              424
<210> 131
   <211> 472
   <212> DNA
   <213> unknown
<220>
   <223> >sr|IMAGp998I09280.1|cl|IMAGp998I09280|gb|BX091207 |oid|234516781 BX091207 Soares infant brain 1NIB Homo sapiens cDNA clone IMAGp998I09280 ; IMAGE:49203, mRNA sequence
<221> misc_feature
   <222> 27, 186, 204, 238, 376
   <223> n=a, t, c, or g
<400> 131
   tttttttttt ttttaaaaat atataangtt ttattgtcaa aaatagacaa actttaattt 60
   cctttaacag gaatattaat ttaacagcct tccataagcc atcaccattt tgtaagcata 120
   acaggcaaga gagtcaaaga taactgttag tggaaaaagg acaacagttc tacatcgatg 180
   cccaanaagc cttgcccagt cagnggtgac aactccagga cagcggcaga aacacagnga 240
   acctttggag cttaacaata gacatgcaaa acaacattga tttatcttgg cccaattcta 300
   taaagattgg ctttgtagta tctttccaag catttgaaga gtttagtttg ttagaacact 360
   ggctaatttg aacagngaca ttttaggtca cttatagtat cagtaacagg atcacacctg 420
   tttttcagta acaccaggat gaatacatct gtccctacgc cggtctccag ca        472
<210> 132
   <211> 666
   <212> DNA
   <213> unknown
<220>
   <223> >sr|IMAGp998J24653.1|cl|IMAGp998J24653|gb|BX098770 |oid|234531914 BX098770 Soares fetal liver spleen lNFLS Homo sapiens cDNA clone IMAGp998J24653 ; IMAGE:293111, mRNA sequence
<400> 132
   ttctttggat gtagacactt ttcaaagacc aatgagctgc tgcagaagtc tggcaagaag 60
   cccattgact ggaaggagct gtgatcatca gctgaggggt ggcctttgag aagctgctgt 120
   taacgtattt gccagttacg aagttccact gaaaattttc ctattaattc ttaagtactc 180
   tgcataaggg ggaaaagctt ccagaaagca gccatgaacc aggctgtcca ggaatggcag 240
   ctgtatccaa ccacaaacaa caaaggctac cctttgacca aatgtctttc tctgcaacat 300
   ggcttcggcc taaaatatgc agaagacaga tgaggtcaaa tactcagttg gctctcttta 360
   tctcccttgc ctttatggtg aaacagggga gatgtgcacc tttcaggcac agccctagtt 420
   tggcgcctgc tgctccttgg ttttgcctgg ttagactttc agtgacagat gttggggtgt 480
   ttttgcttag aaaggtcccc ttgtctcagc cttgcagggc aggcatgcca gtctctgcca 540
   gttccactgc ccccttgatc tttgaaggag tcctcaggcc cctcgcagca taaggatgtt 600
   ttgcaacttt ccagaatctg gcccagaaat tagggctcaa tttcctgatt gtagtagagg 660
   ttaaga                                                            666
<210> 133
   <211> 430
   <212> DNA
   <213> unknown
<220>
   <223> >gi|2103286|gb|AA424316|sr|IMAGp998N161887.1|cl| IM AGp998N161887|AA424316 zv90d08.r1 Soares_NhHMPu_S1 Homo sapiens cDNA clone IMAGE:767055 5', mRNA sequence
<400> 133
   caagaaaata agaaaggtag aaacaggagg acccaccgaa tttgagaggg cacccaggag 60
   tgtctgagct gcattacctc tagaaacctc aaacaagtag aaacttgcct agacaataac 120
   tggaaaaaca aatgcaatat acatgaactt ttttcaaagc attatgtgga tgtttacaat 180
   ggtgggaaat tcagctgagt tcaccaatat aaattaaatc catgagtaac tttcctaata 240
   ggcttttttt cctaatacca ccacctaaca gagaacacag gtgaatgcag atgttcactt 300
   tagcagactt aatgtttcct atgagatttc actgtacagg tttgtctttc ttctttgcct 360
   gagaaataaa aatgtcattt gccatattgc catctaaagg agaaaaactg catcagcaaa 420
   gccattgtat                                                        430
<210> 134
   <211> 2698
   <212> DNA
   <213> unknown
<220>
   <223> >TXNIP (HUGO ID) THIOREDOXIN INTERACTING PROTEIN; UPREGULATED BY 1,25-DIHYDROXYVITAMIN D-3. [Source: RefSeq (NM_006472)]
<400> 134
   gcttagtgta accagcggcg tatatttttt aggcgccttt tcgaaaacct agtagttaat 60
   attcatttgt ttaaatctta ttttattttt aagctcaaac tgcttaagaa taccttaatt 120
   ccttaaagtg aaataatttt ttgcaaaggg gtttcctcga tttggagctt tttttttctt 180
   ccaccgtcat ttctaactct taaaaccaac tcagttccat catggtgatg ttcaagaaga 240
   tcaagtcttt tgaggtggtc tttaacgacc ctgaaaaggt gtacggcagt ggcgagaagg 300
   tggctggccg ggtgatagtg gaggtgtgtg aagttactcg tgtcaaagcc gttaggatcc 360
   tggcttgcgg agtggctaaa gtgctttgga tgcagggatc ccagcagtgc aaacagactt 420
   cggagtacct gcgctatgaa gacacgcttc ttctggaaga ccagccaaca ggtgagaatg 480
   agatggtgat catgagacct ggaaacaaat atgagtacaa gttcggcttt gagcttcctc 540
   aggggcctct gggaacatcc ttcaaaggaa aatatgggtg tgtagactac tgggtgaagg 600
   cttttcttga ccgcccgagc cagccaactc aagagacaaa gaaaaacttt gaagtagtgg 660
   atctggtgga tgtcaatacc cctgatttaa tggcacctgt gtctgctaaa aaagaaaaga 720
   aagtttcctg catgttcatt cctgatgggc gggtgtctgt ctctgctcga attgacagaa 780
   aaggattctg tgaaggtgat gagatttcca tccatgctga ctttgagaat acatgttccc 840
   gaattgtggt ccccaaagct gccattgtgg cccgccacac ttaccttgcc aatggccaga 900
   ccaaggtgct gactcagaag ttgtcatcag tcagaggcaa tcatattatc tcagggacat 960
   gcgcatcatg gcgtggcaag agccttcggg ttcagaagat caggccttct atcctgggct 1020
   gcaacatcct tcgagttgaa tattccttac tgatctatgt tagcgttcct ggatccaaga 1080
   aggtcatcct tgacctgccc ctggtaattg gcagcagatc aggtctaagc agcagaacat 1140
   ccagcatggc cagccgaacc agctctgaga tgagttgggt agatctgaac atccctgata 1200
   ccccagaagc tcctccctgc tatatggatg tcattcctga agatcaccga ttggagagcc 1260
   caaccactcc tctgctagat gacatggatg gctctcaaga cagccctatc tttatgtatg 1320
   cccctgagtt caagttcatg ccaccaccga cttatactga ggtggatccc tgcatcctca 1380
   acaacaatgt gcagtgagca tgtggaagaa aagaagcagc tttacctact tgtttctttt 1440
   tgtctctctt cctggacact cactttttca gagactcaac agtctctgca atggagtgtg 1500
   ggtccacctt agcctctgac ttcctaatgt aggaggtggt cagcaggcaa tctcctgggc 1560
   cttaaaggat gcggactcat cctcagccag cgcccatgtt gtgatacagg ggtgtttgtt 1620
   ggatgggttt aaaaataact agaaaaactc aggcccatcc attttctcag atctccttga 1680
   aaattgaggc cttttcgata gtttcgggtc aggtaaaaat ggcctcctgg cgtaagcttt 1740
   tcaaggtttt ttggaggctt tttgtaaatt gtgataggaa ctttggacct tgaacttatg 1800
   tatcatgtgg agaagagcca atttaacaaa ctaggaagat gaaaagggaa attgtggcca 1860
   aaactttggg aaaaggaggt tcttaaaatc agtgtttccc ctttgtgcac ttgtagaaaa 1920
   aaaagaaaaa ccttctagag ctgatttgat ggacaatgga gagagctttc cctgtgatta 1980
   taaaaaagga agctagctgc tctacggtca tctttgctta agagtatact ttaacctggc 2040
   ttttaaagca gtagtaactg ccccaccaaa ggtcttaaaa gccatttttg gagcctattg 2100
   cactgtgttc tcctactgca aatattttca tatgggagga tggttttctc ttcatgtaag 2160
   tccttggaat tgattctaag gtgatgttct tagcacttta attcctgtca aattttttgt 2220
   tctccccttc tgccatctta aatgtaagct gaaactggtc tactgtgtct ctagggttaa 2280
   gccaaaagac aaaaaaaatt ttactacttt tgagattgcc ccaatgtaca gaattatata 2340
   attctaacgc ttaaatcatg tgaaagggtt gctgctgtca gccttgccca ctgtgacttc 2400
   aaacccaagg aggaactctt gatcaagatg ccgaaccctg tgttcagaac ctccaaatac 2460
   tgccatgaga aactagaggg caggtcttca taaaagccct ttgaaccccc ttcctgccct 2520
   gtgttaggag atagggatat tggcccctca ctgcagctgc cagcacttgg tcagtcactc 2580
   tcagccatag cactttgttc actgtcctgt gtcagagcac tgagctccac ccttttctga 2640
   gagttattac agccagaaag tgtgggctga agatggttgg tttcatgttt ttgtatta   2698
<210> 135
   <211> 4609
   <212> DNA
   <213> unknown
<220>
   <223> CALD1 (HUGO ID) CALDESMON (CDM). [Source: SWISSPROT (Q05682)] <400> 135
<210> 136
   <211> 1501
   <212> DNA
   <213> unknown
<220>
   <223> >CD74 (HUGO ID) HLA CLASS II HISTOCOMPATIBILITY ANTIGEN, GAMMA CHAIN (HLA-DR ANTIGENS ASSOCIATED INVARIANT CHAIN) (IA ANTIGEN-ASSOCIATED INVARIANT CHAIN) (II) (P33) (CD74 ANTIGEN). [Source: SWISSPROT (P04233)]
<400> 136
   cagggtccca gatgcacagg aggagaagca ggagctgtcg ggaagatcag aagccagtca 60
   tggatgacca gcgcgacctt atctccaaca atgagcaact gcccatgctg ggccggcgcc 120
   ctggggcccc ggagagcaag tgcagccgcg gagccctgta cacaggcttt tccatcctgg 180
   tgactctgct cctcgctggc caggccacca ccgcctactt cctgtaccag cagcagggcc 240
   ggctggacaa actgacagtc acctcccaga acctgcagct ggagaacctg cgcatgaagc 300
   ttcccaagcc tcccaagcct gtgagcaaga tgcgcatggc caccccgctg ctgatgcagg 360
   cgctgcccat gggagccctg ccccaggggc ccatgcagaa tgccaccaag tatggcaaca 420
   tgacagagga ccatgtgatg cacctgctcc agaatgctga ccccctgaag gtgtacccgc 480
   cactgaaggg gagcttcccg gagaacctga gacaccttaa gaacaccatg gagaccatag 540
   actggaaggt ctttgagagc tggatgcacc attggctcct gtttgaaatg agcaggcact 600
   ccttggagca aaagcccact gacgctccac cgaaagtact gaccaagtgc caggaagagg 660
   tcagccacat ccctgctgtc cacccgggtt cattcaggcc caagtgcgac gagaacggca 720
   actatctgcc actccagtgc tatgggagca tcggctactg ctggtgtgtc ttccccaacg 780
   gcacggaggt ccccaacacc agaagccgcg ggcaccataa ctgcagtgag tcactggaac 840
   tggaggaccc gtcttctggg ctgggtgtga ccaagcagga tctgggccca gtccccatgt 900
   gagagcagca gaggcggtct tcaacatcct gccagcccca cacagctaca gctttcttgc 960
   tcccttcagc ccccagcccc tcccccatct cccaccctgt acctcatccc atgagaccct 1020
   ggtgcctggc tctttcgtca cccttggaca agacaaacca agtcggaaca gcagataaca 1080
   atgcagcaag gccctgctgc ccaatctcca tctgtcaaca ggggcgtgag gtcccaggaa 1140
   gtggccaaaa gctagacaga tccccgttcc tgacatcaca gcagcctcca acacaaggct 1200
   ccaagaccta ggctcatgga cgagatggga aggcacaggg agaagggata accctacacc 1260
   cagaccccag gctggacatg ctgactgtcc tctcccctcc agcctttggc cttggctttt 1320
   ctagcctatt tacctgcagg ctgagccact ctcttccctt tccccagcat cactccccaa 1380
   ggaagagcca atgttttcca cccataatcc tttctgccga cccctagttc cctctgctca 1440
   gccaagcttg ttatcagctt tcagggccat ggttcacatt agaataaaag gtagtaatta 1500
   g 1501
<210> 137
   <211> 4009
   <212> DNA
   <213> unknown
<220>
   <223> >C7 (HUGO ID) COMPLEMENT COMPONENT C7 PRECURSOR. [Source: SWISSPROT (P10643)]
<400> 137
   cagggagagg cagagaggca ggcagcctgc tgggctcttc ctgctgttga aaacttaccc 60
   ggcccttaca gaggaaatct tcctcctctc ttctgccctg aatgttttcc caaacatgaa 120
   ggtgataagc ttattcattt tggtgggatt tataggagag ttccaaagtt tttcaagtgc 180
   ctcctctcca gtcaactgcc agtgggactt ctatgcccct tggtcagaat gcaatggctg 240
   taccaagact cagactcgca ggcggtcagt tgctgtgtat gggcagtatg gaggccagcc 300
   ttgtgttgga aatgcttttg aaacacagtc ctgtgaacct acaagaggat gtccaacaga 360
   ggagggatgt ggagagcgtt tcaggtgctt ttcaggtcag tgcatcagca aatcattggt 420
   ttgcaatggg gattctgact gtgatgaaga cagtgctgat gaagacagat gtgaggactc 480
   agaaaggaga ccttcctgtg atatcgataa acctcctcct aacatagaac ttactggaaa 540
   tggttacaat gaactcactg gccagtttag gaacagagtc atcaatacca aaagttttgg 600
   tggtcaatgt agaaaggtgt ttagtgggga tggaaaagat ttctacaggc tgagtggaaa 660
   tgtcctgtcc tatacattcc aggtgaaaat aaataatgat tttaattatg aattttacaa 720
   tagtacttgg tcttatgtaa aacatacgtc gacagaacac acatcatcta gtcggaagcg 780
   ctcctttttt agatcttcat catcttcttc acgcagttat acttcacata ccaatgaaat 840
   ccataaagga aagagttacc aactgctggt tgttgagaac actgttgaag tggctcagtt 900
   cattaataac aatccagaat ttttacaact tgctgagcca ttctggaagg agctttccca 960
   cctcccctct ctgtatgact acagtgccta ccgaagatta atcgaccagt acgggacaca 1020
   ttatctgcaa tctgggtcgt taggaggaga atacagagtt ctattttatg tggactcaga 1080
   aaaattaaaa caaaatgatt ttaattcagt cgaagaaaag aaatgtaaat cctcaggttg 1140
   gcattttgtc gttaaatttt caagtcatgg atgcaaggaa ctggaaaacg ctttaaaagc 1200
   tgcttcagga acccagaaca atgtattgcg aggagaaccg ttcatcagag ggggaggtgc 1260
   aggcttcata tctggcctta gttacctaga gctggacaat cctgctggaa acaaaaggcg 1320
   atattctgcc tgggcagaat ctgtgactaa tcttcctcaa gtcataaaac aaaagctgac 1380
   acctttatat gagctggtaa aggaagtacc ttgtgcctct gtgaaaaaac tatacctgaa 1440
   atgggctctt gaagagtatc tggatgaatt tgacccctgt cattgccggc cttgtcaaaa 1500
   tggtggtttg gctactgttg aggggaccca ttgtctgtgc cattgcaaac cgtacacatt 1560
   tggtgcggcg tgtgagcaag gagtcctcgt agggaatcaa gcaggagggg ttgatggagg 1620
   ttggagttgc tggtcctctt ggagcccctg tgtccaaggg aagaaaacaa gaagccgtga 1680
   atgcaataac ccacctccca gtgggggtgg gagatcctgc gttggagaaa cgacagaaag 1740
   cacacaatgc gaagatgagg agctggagca cttgaggttg cttgaaccac attgctttcc 1800
   tttgtctttg gttccaacag aattctgtcc atcacctcct gccttgaaag atggatttgt 1860
   tcaagatgaa ggtacaatgt ttcctgtggg gaaaaatgta gtgtacactt gcaatgaagg 1920
   atactctctt attggaaacc cagtggccag atgtggagaa gatttacggt ggcttgttgg 1980
   ggaaatgcat tgtcagaaaa ttgcctgtgt tctacctgta ctgatggatg gcatacagag 2040
   tcacccccaa aaacctttct acacagttgg tgagaaggtg actgtttcct gttcaggtgg 2100
   catgtcctta gaaggtcctt cagcatttct ctgtggctcc agccttaagt ggagtcctga 2160
   gatgaagaat gcccgctgtg tacaaaaaga aaatccgtta acacaggcag tgcctaaatg 2220
   tcagcgctgg gagaaactgc agaattcaag atgtgtttgt aaaatgccct acgaatgtgg 2280
   accttccttg gatgtatgtg ctcaagatga gagaagcaaa aggatactgc ctctgacagt 2340
   ttgcaagatg catgttctcc actgtcaggg tagaaattac acccttactg gtagggacag 2400
   ctgtactctg cctgcctcag ctgagaaagc ttgtggtgcc tgcccactgt ggggaaaatg 2460
   tgatgctgag agcagcaaat gtgtctgccg agaagcatcg gagtgcgagg aagaagggtt 2520
   tagcatttgt gtggaagtga acggcaagga gcagacgatg tctgagtgtg aggcgggcgc 2580
   tctgagatgc agagggcaga gcatctctgt caccagcata aggccttgtg ctgcggaaac 2640
   ccagtaggct cctggaggcc ctggtcagct tgcttggaat ccagcaggca gctggggctg 2700
   agtgaaaaca tctgcacaac tgggcactgg acagcttttc cttcttctcc agtgtctacc 2760
   ttcctcctca actcccagcc atctgtataa acacaatcct ttgttctccc aaatctgaat 2820
   cgaattactc ttttgcctcc tttttaatgt cagtaaggat atgagccttt gcacaggctg 2880
   gctgcgtgtt cttgaaatag gtgttacctt ctctgggcct tggtttttta aaatctgtaa 2940
   aattagagga ttgcactaga gaaacttgaa tgctccattc aggcctatca ttttattaag 3000
   tatgattgac acagcccatg ggccagaaca cactctacaa aatgactagg ataacagaaa 3060
   gaacgtgatc tcctgattag agagggtggt tttcctcaat ggaaccaaat ataaagagga 3120
   cttgaacaaa aatgacagat acaaactatt tctatcctga gtagtaatct cacacttcat 3180
   cctatagagt caaccaccac agataggaat tccttattct ttttttaatt tttttaagac 3240
   agagtctcac tttgttgccc aggctggagc gcagtggggt gatctcatct ccctgcaacc 3300
   tccgcctcct gggttcaagc gattcttgtg cctcagcttc ccaagcagct gggattacag 3360
   gtgcccgcca ccacgcccag ctaatttttg catttttagt agagatgggg tttcaccatg 3420
   ttggccacgc tcgtctccaa ctcctgacct caggtaatcc gcctgccttg gcctcccaaa 3480
   gtgctgggat tacagacatg aaccaccacg cctggctgga atacttactc ttgtcgggag 3540
   attgaaccac taaaatgtta gagcagaatt cattatgctg tggtcacagg ggtgtcttgt 3600
   ctgagaacaa atacaattca gtcttctctt tggggtttta gtatgtgtca aacataggac 3660
   tggaagtttg cccctgttct tttttctttt gaaagaacat cagttcatgc ctgaggcatg 3720
   agtgactgtg catttgagaa tagttttccc tattctgtgg atacagtccc agagttttca 3780
   gggagtacac aggtagatta gtttgaagca ttgacctttt atttattcct tatttctctt 3840
   tcatcaaaac aaaacagcag ctgtgggagg agaaatgaga gggcttaaat gaaatttaaa 3900
   ataagctata ttatacaaat actatctctg tattgttctg accctggtaa atatatttca 3960
   aaacttcaga tgacaaggat tagaacactc attaaagatg ctattcttc 4009
<210> 138
   <211> 2240
   <212> DNA
   <213> unknown
<220>
   <223> >CXCL16 (HUGO ID) SMALL INDUCIBLE CYTOKINE B16 (TRANSMEMBRANE CHEMOKINE CXCL16) (SR- PSOX) (SCAVENGER RECEPTOR FOR PHOSPHATIDYLSERINE AND OXIDIZED LOW DENSITY LIPOPROTEIN). [Source: SWISSPROT (Q9H2A7)]
<400> 138
   catgcagcgc ggctgggtcc cgcggcgccc ggatcgggga agtgaaagtg cctcggagga 60
   ggagggccgg tccggcagtg cagccgcctc acaggtcggc ggacgggcca ggcgggcggc 120
   ctcctgaacc gaaccgaatc ggctcctcgg gccgtcgtcc tcccgcccct cctcgcccgc 180
   cgccggagtt ttctttcggt ttcttccaag attcctggcc ttccctcgac ggagccgggc 240
   ccagtgcggg ggcgcagggc gcgggagctc cacctcctcg gctttccctg cgtccagagg 300
   ctggcatggc gcgggccgag tactgagcgc acggtcgggg cacagcaggg ccggggggtg 360
   cagctggctc gcgcctcctc tccggccgcc gtctcctccg gtccccggcg aaagccattg 420
   agacaccagc tggacgtcac gcgccggagc atgtctggga gtcagagcga ggtggctcca 480
   tccccgcaga gtccgcggag ccccgagatg ggacgggact tgcggcccgg gtcccgcgtg 540
   ctcctgctcc tgcttctgct cctgctggtg tacctgactc agccaggcaa tggcaacgag 600
   ggcagcgtca ctggaagttg ttattgtggt aaaagaattt cttccgactc cccgccatcg 660
   gttcagttca tgaatcgtct ccggaaacac ctgagagctt accatcggtg tctatactac 720
   acgaggttcc agctcctttc ctggagcgtg tgtgggggca acaaggaccc atgggttcag 780
   gaattgatga gctgtcttga tctcaaagaa tgtggacatg cttactcggg gattgtggcc 840
   caccagaagc atttacttcc taccagcccc ccaatttctc aggcctcaga gggggcatct 900
   tcagatatcc acacccctgc ccagatgctc ctgtccacct tgcagtccac tcagcgcccc 960
   accctcccag taggatcact gtcctcggac aaagagctca ctcgtcccaa tgaaaccacc 1020
   attcacactg cgggccacag tctggcagct gggcctgagg ctggggagaa ccagaagcag 1080
   ccggaaaaaa atgctggtcc cacagccagg acatcagcca cagtgccagt cctgtgcctc 1140
   ctggccatca tcttcatcct caccgcagcc ctttcctatg tgctgtgcaa gaggaggagg 1200
   gggcagtcac cgcagtcctc tccagatctg ccggttcatt atatacctgt ggcacctgac 1260
   tctaatacct gagccaagaa tggaagcttg tgaggagacg gactctatgt tgcccaggct 1320
   gttatggaac tcctgagtca agtgatcctc ccaccttggc ctctgaaggt gcgaggatta 1380
   taggcgtcac ctaccacatc cagcctacac gtatttgtta atatctaaca taggactaac 1440
   cagccactgc cctctcttag gcccctcatt taaaaacggt tatactataa aatctgcttt 1500
   tcacactggg tgataataac ttggacaaat tctatgtgta ttttgttttg ttttgctttg 1560
   ctttgttttg agacggagtc tcgctctgtc atccaggctg gagtgcagtg gcatgatctc 1620
   ggctcactgc aacccccatc tcccaggttc aagcgattct cctgcctcct cctgagtagc 1680
   tgggactaca ggtgctcacc accacacccg gctaattttt tgtattttta gtagagacgg 1740
   ggtttcacca tgttgaccag gctggtctcg aactcctgac ctggtgatct gcccacccag 1800
   gcctcccaaa gtgctgggat taaaggtgtg agccaccatg cctggcccta tgtgtgtttt 1860
   ttaactacta aaaattattt ttgtaatgat tgagtcttct ttatggaaac aactggcctc 1920
   agcccttgcg cccttactgt gattcctggc ttcatttttt gctgatggtt ccccctcgtc 1980
   ccaaatctct ctcccagtac accagttgtt cctcccccac ctcagccctc tcctgcatcc 2040
   tcctgtaccc gcagcgaagg cctgggcttt cccaccctcc ctccttagca ggtgccgtgc 2100
   tgggacacca tacgggttgg tttcacctcc tcagtccctt gcctacccca gtgagagtct 2160
   gatcttgttt ttattgttat tgcttttatt attattgctt ttattatcat taaaactcta 2220
   gttcttgttt tgtctctccg                                             2240
<210> 139
   <211> 4234
   <212> DNA
   <213> unknown
<220>
   <223> CYBRD1 (HUGO ID) CYTOCHROME B REDUCTASE 1; DUODENAL CYTOCHROME B. [Source: RefSeq (NM_024843)]
<400> 139
   ggccactacc cagagggctg ccgccgcctc tccaagttct tgtggccccc gcggtgcgga 60
   gtatggggcg ctgatggcca tggagggcta ctggcgcttc ctggcgctgc tggggtcggc 120
   actgctcgtc ggcttcctgt cggtgatctt cgccctcgtc tgggtcctcc actaccgaga 180
   ggggcttggc tgggatggga gcgcactaga gtttaactgg cacccagtgc tcatggtcac 240
   cggcttcgtc ttcatccagg gcatcgccat catcgtctac agactgccgt ggacctggaa 300
   atgcagcaag ctcctgatga aatccatcca tgcagggtta aatgcagttg ctgccattct 360
   tgcaattatc tctgtggtgg ccgtgtttga gaaccacaat gttaacaata tagccaatat 420
   gtacagtctg cacagctggg ttggactgat agctgtcata tgctatttgt tacagcttct 480
   ttcaggtttt tcagtctttc tgcttccatg ggctccgctt tctctccgag catttctcat 540
   gcccatacat gtttattctg gaattgtcat ctttggaaca gtgattgcaa cagcacttat 600
   gggattgaca gagaaactga ttttttccct gagagatcct gcatacagta cattcccgcc 660
   agaaggtgtt ttcgtaaata cgcttggcct tctgatcctg gtgttcgggg ccctcatttt 720
   ttggatagtc accagaccgc aatggaaacg tcctaaggag ccaaattcta ccattcttca 780
   tccaaatgga ggcactgaac agggagcaag aggttccatg ccagcctact ctggcaacaa 840
   catggacaaa tcagattcag agttaaacag tgaagtagca gcaaggaaaa gaaacttagc 900
   tctggatgag gctgggcaga gatctaccat gtaaaatgtt gtagagatag agccatataa 960
   cgtcacgttt caaaactagc tctacagttt tgcttctcct attagccata tgataattgg 1020
   gctatgtagt atcaatattt actttaatca caaaggatgg tttcttgaaa taatttgtat 1080
   tgattgaggc ctatgaactg acctgaattg gaaaggatgt gattaatata aataatagca 1140
   gatataaatt gtggttatgt tacctttatc ttgttgagga ccacaacatt agcacggtgc 1200
   cttgtgcaga atagatactc aatatgtgaa tatgtgtcta ctagtagtta attggataaa 1260
   ctggcagcat ccctggcctg ttgtcatgca gtcatttcct gttaattctg ggagacaatg 1320
   atttcacaac tagcgggaag cagtcctaaa agtttaaaat ccgataagga atatctggga 1380
   cagggtttag atcatgactc tacacagata ccatgatgag agtatattaa agaaatttag 1440
   gaaagcacct ggttcctttc tccccatgcc tgccttctgc tccctcccca gctggtttgg 1500
   gctcaaattg tccctggaga ctagggttta tgttagggta ttgatagatt agagcaggtg 1560
   gttgaagaga tcttctctgg tcagacttgg aagaatttcc aaaactgaag ttagccccaa 1620
   gacttcccta gggttgatgt actttatgat ccagatgcta aacttcttag aatgaaaata 1680
   tgcttcaaca cttaagtagc atacactgcc ctacaaacct cagagagcac ttttccccaa 1740
   gttcttgttt ttatttttga aagtactcac acagcactta ctatgctcca aacactcctc 1800
   taagcacttt acacatatta gctcattcag tccccagaca gacgggatga agtaggtatt 1860
   gttactgttc ccattttaca ggtgagagat ttgaagcctg gggaggctag taactcaccc 1920
   caaggtcaca cggctcatac atggtgggac tgagactcag atgcaggcag tctggcacct 1980
   cagtctggat tctaaccatt tcactaagct atttttgtct tgtactactt tgacccaccc 2040
   ctgaataaac ctcaattgct ggagtggggt gtagttatta aagggatgct tttacctttt 2100
   tgctgtttgc tgtggcagat tccccagata accaaggaaa aggggccacc catacctgga 2160
   aataggccat agggccccta ctactgccaa caagccatgg cctaccttga cacttgtttg 2220
   atcttaaaat tgtgtcttgg taacaaaaga tttggacagg catatctgta gctttcaagt 2280
   taattaattg caatattttt ttcttcagga ttttagctgc tgaacaactt tcagtttgga 2340
   gctaaaagag acctgtctca tggtctgccc ttccctgggg caatagctag ggtctttcct 2400
   gatttttatg gaattttagg ggatattttg agctttgggt tctcagtagt gaattgagac 2460
   ttggaggtga cttttcatgt ttggagtatc atctctgtct gggatctggg ctgacaaatt 2520
   aaaacctaga gtagtgctta tgctgaaatg atacttttca ttttttggtt gatttttttg 2580
   ccttcccttc aattttaaac tgaagcattt taatatgggt agaaactcta caccaaatac 2640
   actaaacatt ttggtgctta gtggatttct ttttaggtaa ctggtactta cttccaaaga 2700
   ctgaatacaa gccacactcc atcatatccc ttaaacttca tgaaaaacca ttcaagatcc 2760
   ccttgctgca acactgttct cttcttctct actaaattct atttccaaaa ttggtaatag 2820
   agccagaagg atccccagta cccagccctc tgcctggcac aaagtggtag cacaattaaa 2880
   ttcagtatgg gtggagcatg gtacagtctt ggtgccatag aaggagtagt tgcatagtca 2940
   cacatcattt gataagttgg atgttccatt acatagagga acacaaaatt ccagggtttt 3000
   tggaggaagg gattagatag tgactaagcc gccagaattg aggtggccat tcctttttgt 3060
   ataggctaag aaacaggtta tcagtgaaaa gttaattatg gctttggcac tagaatagca 3120
   ctgttgcaaa gtatttaagc accccccatc tcagcccttt attttatctt tcatgtgggc 3180
   taatgtgagg ataatcttac agatattata ggaatttctt ttctatcttt atgaaaacaa 3240
   cgtatataaa atatatctag aaaacctttg tttgagactc ttatttaatg ggcttttgat 3300
   tctaatgata attgtacctt tatctttcaa aagctgatat ttcctaccta agcatctccc 3360
   gagaaaaata tctcattaaa aagcccataa ataatagggg agaagaaagc cttaggtatc 3420
   aattccaaaa cagtgattga aatttcccaa aataattatg gcttctgtca tctccagaga 3480
   taatctggct tggtttaccc cataatctaa tttcagaaaa gaaagcttta ttttaacact 3540
   catctgaatc aacattaaag ccttttctct caaagcgttt attgagaaac tcaaatgaat 3600
   atactttttg aattactgtc atcaaaagtg tacggcttcc tgtgctgctt gtgtcaaatg 3660
   gaacctgccc tctaaagcac tttctttcct ttacttgcgt ggtttcatgt aagctgtgct 3720
   gtttagaaac aacatctcag actttacaaa gaaatgacaa agaaggcaat tgcacttttt 3780
   aagggatatc gacaagcagt ttctgttttc taaaggacaa aatacagagt gtgtgtcatt 3840
   tttaattaga ttctttcccc tgctgagttg gaaattccag tgcagcactg attgaccaca 3900
   gttgccaatc taaaagcaca aagacagaag taaagcttta tgctaatttt atttcaatat 3960
   gatagaaaat ttatcttggt atgtcctttt ttagataact ccagcaggaa actgtaactg 4020
   ctatgtcttt aggaaaatgt agaagaaaga acattattgt tctttaattc ctacaaggta 4080
   ctcgaaaacc ttaagtgaaa aagatttcta tctttttatc ttagcgcatt tatggaaaaa 4140
   atattaacta tcctgaatat tttataattt tgtaggaaaa atatgcatct attttttctt 4200
   gacttctttt atatagtaat aaaagttatt ttgg                            4234
<210> 140
   <211> 606
   <212> DNA
   <213> unknown
<220>
   <223> >IFITM1 (HUGO ID) INTERFERON-INDUCED TRANSMEMBRANE PROTEIN 1 (INTERFERON-INDUCED PROTEIN 17) (INTERFERON-INDUCIBLE PROTEIN 9-27) (LEU-13 ANTIGEN) (CD225 ANTIGEN). [Source: SWISSPROT (P13164)]
<400> 140
   cccttcgctc cacgcagaaa accacacttc tcaaaccttc actcaacact tccttcccca 60
   aagccagaag atgcacaagg aggaacatga ggtggctgtg ctggggccac cccccagcac 120
   catccttcca aggtccaccg tgatcaacat ccacagcgag acctccgtgc ccgaccatgt 180
   cgtctggtcc ctgttcaaca ccctcttctt gaactggtgc tgtctgggct tcatagcatt 240
   cgcctactcc gtgaagtcta gggacaggaa gatggttggc gacgtgaccg gggcccaggc 300
   ctatgcctcc accgccaagt gcctgaacat ctgggccctg attctgggca tcctcatgac 360
   cattggattc atcctgttac tggtattcgg ctctgtgaca gtctaccata ttatgttaca 420
   gataatacag gaaaaacggg gttactagta gccgcccata gcctgcaacc tttgcactcc 480
   actgtgcaat gctggccctg cacgctgggg ctgttgcccc tgcccccttg gtcctgcccc 540
   tagatacagc agtttatacc cacacacctg tctacagtgt cattcaataa agtgcacgtg 600
   cttgtg                                                           606
<210> 141
   <211> 3423
   <212> DNA
   <213> unknown
<220>
   <223> SALL4 (HUGO ID) SAL-LIKE PROTEIN 4 (ZINC FINGER PROTEIN SALL4). [Source: SWISSPROT (Q9UJQ4)]
<400> 141
   aactccagga atttgtggcg gagagggcaa ataactgcgg ctctcccggc gccccgatgc 60
   tcgcaccatg tcgaggcgca agcaggcgaa accccagcac atcaactcgg aggaggacca 120
   gggcgagcag cagccgcagc agcagacccc ggagtttgca gatgcggccc cagcggcgcc 180
   cgcggcgggg gagctgggtg ctccagtgaa ccacccaggg aatgacgagg tggcgagtga 240
   ggatgaagcc acagtaaagc ggcttcgtcg ggaggagacg cacgtctgtg agaaatgctg 300
   tgcggagttc ttcagcatct ctgagttcct ggaacataag aaaaattgca ctaaaaatcc 360
   acctgtcctc atcatgaatg acagcgaggg gcctgtgcct tcagaagact tctccggagc 420
   tgtactgagc caccagccca ccagtcccgg cagtaaggac tgtcacaggg agaatggcgg 480
   cagctcagag gacatgaagg agaagccgga tgcggagtct gtggtgtacc taaagacaga 540
   gacagccctg ccacccaccc cccaggacat aagctattta gccaaaggca aagtggccaa 600
   cactaatgtg accttgcagg cactacgggg caccaaggtg gcggtgaatc agcggagcgc 660
   ggatgcactc cctgcccccg tgcctggtgc caacagcatc ccgtgggtcc tcgagcagat 720
   cttgtgtctg cagcagcagc agctacagca gatccagctc accgagcaga tccgcatcca 780
   ggtgaacatg tgggcctccc acgccctcca ctcaagcggg gcaggggccg acactctgaa 840
   gaccttgggc agccacatgt ctcagcaggt ttctgcagct gtggctttgc tcagccagaa 900
   agctggaagc caaggtctgt ctctggatgc cttgaaacaa gccaagctac ctcacgccaa 960
   catcccttct gccaccagct ccctgtcccc agggctggca cccttcactc tgaagccgga 1020
   tgggacccgg gtgctcccga acgtcatgtc ccgcctcccg agcgctttgc ttcctcaggc 1080
   cccgggctcg gtgctcttcc agagcccttt ctccactgtg gcgctagaca catccaagaa 1140
   agggaagggg aagccaccga acatctccgc ggtggatgtc aaacccaaag acgaggcggc 1200
   cctctacaag cacaagtgta agtactgtag caaggttttt gggactgata gctccttgca 1260
   gatccacctc cgctcccaca ctggagagag acccttcgtg tgctctgtct gtggtcatcg 1320
   cttcaccacc aagggcaacc tcaaggtgca ctttcaccga catccccagg tgaaggcaaa 1380
   cccccagctg tttgccgagt tccaggacaa agtggcggcc ggcaatggca tcccctatgc 1440
   actctctgta cctgacccca tagatgaacc gagtctttct ttagacagca aacctgtcct 1500
   tgtaaccacc tctgtagggc tacctcagaa tctttcttcg ggactaatcc ccaaggacct 1560
   cacgggtggc tccttgcccg gtgacctgca gcctgggcct tctccagaaa gtgagggtgg 1620
   acccacactc cctggggtgg gaccaaacta taattcccca agggctggtg gcttccaagg 1680
   gagtgggacc cctgagccag ggtcagagac cctgaaattg cagcagttgg tggagaacat 1740
   tgacaaggcc accactgatc ccaacgaatg tctcatttgc caccgagtct taagctgtca 1800
   gagctccctc aagatgcatt atcgcaccca caccggggag agaccgttcc agtgtaagat 1860
   ctgtggccga gccttttcta ccaaaggtaa cctgaagaca caccttgggg ttcaccgaac 1920
   caacacatcc attaagacgc agcattcgtg ccccatctgc cagaagaagt tcactaatgc 1980
   cgtgatgctg cagcaacata ttcggatgca catgggcggt cagattccca acacgcccct 2040
   gccagagaat ccctgtgact ttacgggttc tgagccaatg accgtgggtg agaacggcag 2100
   caccggcgct atctgccatg atgatgtcat cgaaagcatc gatgtagagg aagtcagctc 2160
   ccaggaggct cccagcagct cctccaaggt ccccacgcct cttcccagca tccactcggc 2220
   atcacccacg ctagggtttg ccatgatggc ttccttagat gccccaggga aagtgggtcc 2280
   tgcccctttt aacctgcagc gccagggcag cagagaaaac ggttccgtgg agagcgatgg 2340
   cttgaccaac gactcatcct cgctgatggg agaccaggag tatcagagcc gaagcccaga 2400
   tatcctggaa accacatcct tccaggcact ctccccggcc aatagtcaag ccgaaagcat 2460
   caagtcaaag tctcccgatg ctgggagcaa agcagagagc tccgagaaca gccgcactga 2520
   gatggaaggt cggagcagtc tcccttccac gtttatccga gccccgccga cctatgtcaa 2580
   ggttgaagtt cctggcacat ttgtgggacc ctcgacattg tccccaggga tgaccccttt 2640
   gttagcagcc cagccacgcc gacaggccaa gcaacatggc tgcacacggt gtgggaagaa 2700
   cttctcgtct gctagcgctc ttcagatcca cgagcggact cacactggag agaagccttt 2760
   tgtgtgcaac atttgtgggc gagcttttac caccaaaggc aacttaaagg ttcactacat 2820
   gacacacggg gcgaacaata actcagcccg ccgtggaagg aagttggcca tcgagaacac 2880
   catggctctg ttaggtacgg acggaaaaag agtctcagaa atctttccca aggaaatcct 2940
   ggccccttca gtgaatgtgg accctgttgt gtggaaccag tacaccagca tgctcaatgg 3000
   cggtctggcc gtgaagacca atgagatctc tgtgatccag agtggggggg ttcctaccct 3060
   cccggtttcc ttgggggcca cctccgttgt gaataacgcc actgtctcca agatggatgg 3120
   ctcccagtcg ggtatcagtg cagatgtgga aaaaccaagt gctactgacg gcgttcccaa 3180
   acaccagttt cctcacttcc tggaagaaaa caagattgcg gtcagctaag ggagaacttg 3240
   cgtggaagga gcaatgcaga cacagtgaaa tctctagaat ctgctttgtt ttgtaagaac 3300
   tcatctcctc ctgttttctt tttcttactg atatgcaaat gatgtttact acgttggttg 3360
   tgaccacaac ctcaggcaag tgctacaatc acgattgttg ctatgctgct ttgcaaaaag 3420
   ttg                                                               3423
<210> 142
   <211> 2412
   <212> DNA
   <213> unknown
<220>
   <223> TRA1 (HUGO ID) ENDOPLASMIN PRECURSOR (94 KDA GLUCOSE-REGULATED PROTEIN) (GRP94). (GP96 HOMOLOG) (TUMOR REJECTION ANTIGEN 1). [Source: SWISSPROT (P14625)]
<400> 142
   atgagggccc tgtgggtgct gggcctctgc tgcgtcctgc tgaccttcgg gtcggtcaga 60
   gctgacgatg aagttgatgt ggatggtaca gtagaagagg atctgggtaa aagtagagaa 120
   ggatcaagga cggatgatga agtagtacag agagaggaag aagctattca gttggatgga 180
   ttaaatgcat cacaaataag agaacttaga gagaagtcgg aaaagtttgc cttccaagcc 240
   gaagttaaca gaatgatgaa acttatcatc aattcattgt ataaaaataa agagattttc 300
   ctgagagaac tgatttcaaa tgcttctgat gctttagata agataaggct aatatcactg 360
   actgatgaaa atgctctttc tggaaatgag gaactaacag tcaaaattaa gtgtgataag 420
   gagaagaacc tgctgcatgt cacagacacc ggtgtaggaa tgaccagaga agagttggtt 480
   aaaaaccttg gtaccatagc caaatctggg acaagcgagt ttttaaacaa aatgactgaa 540
   gcacaggaag atggccagtc aacttctgaa ttgattggcc agtttggtgt cggtttctat 600
   tccgccttcc ttgtagcaga taaggttatt gtcacttcaa aacacaacaa cgatacccag 660
   cacatctggg agtctgactc caatgaattt tctgtaattg ctgacccaag aggaaacact 720
   ctaggacggg gaacgacaat tacccttgtc ttaaaagaag aagcatctga ttaccttgaa 780
   ttggatacaa ttaaaaatct cgtcaaaaaa tattcacagt tcataaactt tcctatttat 840
   gtatggagca gcaagactga aactgttgag gagcccatgg aggaagaaga agcagccaaa 900
   gaagagaaag aagaatctga tgatgaagct gcagtagagg aagaagaaga agaaaagaaa 960
   ccaaagacta aaaaagttga aaaaactgtc tgggactggg aacttatgaa tgatatcaaa 1020
   ccaatatggc agagaccatc aaaagaagta gaagaagatg aatacaaagc tttctacaaa 1080
   tcattttcaa aggaaagtga tgaccccatg gcttatattc actttactgc tgaaggggaa 1140
   gttaccttca aatcaatttt atttgtaccc acatctgctc cacgtggtct gtttgacgaa 1200
   tatggatcta aaaagagcga ttacattaag ctctatgtgc gccgtgtatt catcacagac 1260
   gacttccatg atatgatgcc taaatacctc aattttgtca agggtgtggt ggactcagat 1320
   gatctcccct tgaatgtttc ccgcgagact cttcagcaac ataaactgct taaggtgatt 1380
   aggaagaagc ttgttcgtaa aacgctggac atgatcaaga agattgctga tgataaatac 1440
   aatgatactt tttggaaaga atttggtacc aacatcaagc ttggtgtgat tgaagaccac 1500
   tcgaatcgaa cacgtcttgc taaacttctt aggttccagt cttctcatca tccaactgac 1560
   attactagcc tagaccagta tgtggaaaga atgaaggaaa aacaagacaa aatctacttc 1620
   atggctgggt ccagcagaaa agaggctgaa tcttctccat ttgttgagcg acttctgaaa 1680
   aagggctatg aagttattta cctcacagaa cctgtggatg aatactgtat tcaggccctt 1740
   cccgaatttg atgggaagag gttccagaat gttgccaagg aaggagtgaa gttcgatgaa 1800
   agtgagaaaa ctaaggagag tcgtgaagca gttgagaaag aatttgagcc tctgctgaat 1860
   tggatgaaag ataaagccct taaggacaag attgaaaagg ctgtggtgtc tcagcgcctg 1920
   acagaatctc cgtgtgcttt ggtggccagc cagtacggat ggtctggcaa catggagaga 1980
   atcatgaaag cacaagcgta ccaaacgggc aaggacatct ctacaaatta ctatgcgagt 2040
   cagaagaaaa catttgaaat taatcccaga cacccgctga tcagagacat gcttcgacga 2100
   attaaggaag atgaagatga taaaacagtt ttggatcttg ctgtggtttt gtttgaaaca 2160
   gcaacgcttc ggtcagggta tcttttacca gacactaaag catatggaga tagaatagaa 2220
   agaatgcttc gcctcagttt gaacattgac cctgatgcaa aggtggaaga agagcccgaa 2280
   gaagaacctg aagagacagc agaagacaca acagaagaca cagagcaaga cgaagatgaa 2340
   gaaatggatg tgggaacaga tgaagaagaa gaaacagcaa aggaatctac agctgaaaaa 2400
   gatgaattgt aa                                                     2412
<210> 143
   <211> 4757
   <212> DNA
   <213> unknown
<220>
   <223> >COL1A2 (HUGO ID) ALPHA 2 TYPE I COLLAGEN; COLLAGEN I, ALPHA-2 POLYPEPTIDE; COLLAGEN OF SKIN, TENDON AND BONE, ALPHA-2 CHAIN. [Source: RefSeq (NM_000089)]
<400> 143
   atgctcagct ttgtggatac gcggactttg ttgctgcttg cagtaacctt atgcctagca 60
   acatgccaat gtaagtgcct tcagcttgtt tgggggagac tgggtagaga gggtccacca 120
   ggccccccag gcagagatgg tgaagatggt cccacaggcc ctcctggtcc acctggtcct 180
   cctggccccc ctggtctcgg tgggaacttt gctgctcagt atgatggaaa aggagttgga 240
   cttggccctg gaccaatggg cttaatggga cctagaggcc cacctggtgc agctggagcc 300
   ccaggccctc aaggtttcca aggacctgct ggtgagcctg gtgaacctgg tcaaactggt 360
   cctgcaggtg ctcgtggtcc agctggccct cctggcaagg ctggtgaaga tggtcaccct 420
   ggaaaacccg gacgacctgg tgagagagga gttgttggac cacagggtgc tcgtggtttc 480
   cctggaactc ctggacttcc tggcttcaaa ggcattaggg gacacaatgg tctggatgga 540
   ttgaagggac agcccggtgc tcctggtgtg aagggtgaac ctggtgcccc tggtgaaaat 600
   ggaactccag gtcaaacagg agcccgtggg cttcctggtg agagaggacg tgttggtgcc 660
   cctggcccag ctggtgcccg tggcagtgat ggaagtgtgg gtcccgtggg tcctgctggt 720
   cccattgggt ctgctggccc tccaggcttc ccaggtgccc ctggccccaa gggtgaaatt 780
   ggagctgttg gtaacgctgg tcctgctggt cccgccggtc cccgtggtga agtgggtctt 840
   ccaggcctct ccggccccgt tggacctcct ggtaatcctg gagcaaacgg ccttactggt 900
   gccaagggtg ctgctggcct tcccggcgtt gctggggctc ccggcctccc tggaccccgc 960
   ggtattcctg gccctgttgg tgctgccggt gctactggtg ccagaggact tgttggtgag 1020
   cctggtccag ctggctccaa aggagagagc ggtaacaagg gtgagcccgg ctctgctggg 1080
   ccccaaggtc ctcctggtcc cagtggtgaa gaaggaaaga gaggccctaa tggggaagct 1140
   ggatctgccg gccctccagg acctcctggg ctgagaggta gtcctggttc tcgtggtctt 1200
   cctggagctg atggcagagc tggcgtcatg ggccctcctg gtagtcgtgg tgcaagtggc 1260
   cctgctggag tccgaggacc taatggagat gctggtcgcc ctggggagcc tggtctcatg 1320
   ggacccagag gtcttcctgg ttcccctgga aatatcggcc ccgctggaaa agaaggtcct 1380
   gtcggcctcc ctggcatcga cggcaggcct ggcccaattg gcccagctgg agcaagagga 1440
   gagcctggca acattggatt ccctggaccc aaaggcccca ctggtgatcc tggcaaaaac 1500
   ggtgataaag gtcatgctgg tcttgctggt gctcggggtg ctccaggtcc tgatggaaac 1560
   aatggtgctc agggacctcc tggaccacag ggtgttcaag gtggaaaagg tgaacagggt 1620
   ccccctggtc ctccaggctt ccagggtctg cctggcccct caggtcccgc tggtgaagtt 1680
   ggcaaaccag gagaaagggg tctccatggt gagtttggtc tccctggtcc tgctggtcca 1740
   agaggggaac gcggtccccc aggtgagagt ggtgctgccg gtcctactgg tcctattgga 1800
   agccgaggtc cttctggacc cccagggcct gatggaaaca agggtgaacc tggtgtggtt 1860
   ggtgctgtgg gcactgctgg tccatctggt cctagtggac tcccaggaga gaggggtgct 1920
   gctggcatac ctggaggcaa gggagaaaag ggtgaacctg gtctcagagg tgaaattggt 1980
   aaccctggca gagatggtgc tcgtggcgaa gctggggctg ctggtcctgc tggtcctgct 2040
   ggtcctcggg gaagccctgg tgaacgtggt gaggtcggtc ctgctggccc caatggattt 2100
   gctggtcctg ctggtgctgc tggtcaacct ggtgctaaag gagaaagagg agccaaaggg 2160
   cctaagggtg aaaacggtgt tgttggtccc acaggccccg ttggagctgc tggcccagct 2220
   ggtatttctg gccctcctgg tccccctggt cctgctggga aagaagggct tcgtggtcct 2280
   cgtggtgacc aaggtccagt tggccgaact ggagaagtag gtgcagttgg tccccctggc 2340
   ttcgctggtg agaagggtcc ctctggagag gctggtactg ctggacctcc tggcactcca 2400
   ggtcctcagg gtcttcttgg tgctcctggt attctgggtc tccctggctc gagaggtgaa 2460
   cgtggtctac caggtgttgc tggtgctgtg ggtgaacctg gtcctcttgg cattgccggc 2520
   cctcctgggg cccgtggtcc tcctggtgct gtgggtagtc ctggagtcaa cggtgctcct 2580
   ggtgaagctg gtcgtgatgg caaccctggg aacgatggtc ccccaggtcg cgatggtcaa 2640
   cccggacaca agggagagcg cggttaccct ggcaatattg gtcccgttgg tgctgcaggt 2700
   gcacctggtc ctcatggccc cgtgggtcct gctggcaaac atggaaaccg tggtgaaact 2760
   ggtccttctg gtcctgttgg tcctgctggt gctgttggcc caagaggtcc tagtggccca 2820
   caaggcattc gtggcgataa gggagagccc ggtgaaaagg ggcccagagg tcttcctggc 2880
   ttaaagggac acaatggatt gcaaggtctg cctggtatcg ctggtcacca tggtgatcaa 2940
   ggtgctcctg gctccgtggg tcctgctggt cctaggggcc ctgctggtcc ttctggccct 3000
   gctggaaaag atggtcgcac tggacatcct ggtacagttg gacctgctgg cattcgaggc 3060
   cctcagggtc accaaggccc tgctggcccc cctggtcccc ctggccctcc tggacctcca 3120
   ggtgtaagcg gtggtggtta tgactttggt tacgatggag acttctacag ggctgaccag 3180
   cctcgctcag caccttctct cagacccaag gactatgaag ttgatgctac tctgaagtct 3240
   ctcaacaacc agattgagac ccttcttact cctgaaggct ctagaaagaa cccagctcgc 3300
   acatgccgtg acttgagact cagccaccca gagtggagca gtggttacta ctggattgac 3360
   cctaaccaag gatgcactat ggatgctatc aaagtatact gtgatttctc tactggcgaa 3420
   acctgtatcc gggcccaacc tgaaaacatc ccagccaaga actggtatag gagctccaag 3480
   gacaagaaac acgtctggct aggagaaact atcaatgctg gcagccagtt tgaatataat 3540
   gtagaaggag tgacttccaa ggaaatggct acccaacttg ccttcatgcg cctgctggcc 3600
   aactatgcct ctcagaacat cacctaccac tgcaagaaca gcattgcata catggatgag 3660
   gagactggca acctgaaaaa ggctgtcatt ctacagggct ctaatgatgt tgaacttgtt 3720
   gctgagggca acagcaggtt cacttacact gttcttgtag atggctgctc taaaaagaca 3780
   aatgaatggg gaaagacaat cattgaatac aaaacaaata agccatcacg cctgcccttc 3840
   cttgatattg cacctttgga catcggtggt gctgaccagg aattctttgt ggacattggc 3900
   ccagtctgtt tcaaataaat gaactcaatc taaattaaaa aagaaagaaa tttgaaaaaa 3960
   ctttctcttt gccatttctt cttcttcttt tttaactgaa agctgaatcc ttccatttct 4020
   tctgcacatc tacttgctta aattgtgggc aaaagagaaa aagaaggatt gatcagagca 4080
   ttgtgcaata cagtttcatt aactccttcc cccgctcccc caaaaatttg aatttttttt 4140
   tcaacactct tacacctgtt atggaaaatg tcaacctttg taagaaaacc aaaataaaaa 4200
   ttgaaaaata aaaaccataa acatttgcac cacttgtggc ttttgaatat cttccacaga 4260
   gggaagttta aaacccaaac ttccaaaggt ttaaactacc tcaaaacact ttcccatgag 4320
   tgtgatccac attgttaggt gctgacctag acagagatga actgaggtcc ttgttttgtt 4380
   ttgttcataa tacaaaggtg ctaattaata gtatttcaga tacttgaaga atgttgatgg 4440
   tgctagaaga atttgagaag aaatactcct gtattgagtt gtatcgtgtg gtgtattttt 4500
   taaaaaattt gatttagcat tcatattttc catcttattc ccaattaaaa gtatgcagat 4560
   tatttgccca aatcttcttc agattcagca tttgttcttt gccagtctca ttttcatctt 4620
   cttccatggt tccacagaag ctttgtttct tgggcaagca gaaaaattaa attgtaccta 4680
   ttttgtatat gtgagatgtt taaataaatt gtgaaaaaaa tgaaataaag catgtttggt 4740
   tttccaaaag aacatat                                                4757
<210> 144
   <211> 1934
   <212> DNA
   <213> unknown
<220>
   <223> HDAC3 (HUGO ID) HISTONE DEACETYLASE 3 (HD3) (RPD3-2). [Source: SWISSPROT (015379)]
<210> 145
   <211> 1235
   <212> DNA
   <213> unknown
<220>
   <223> >HLA-DRA (HUGO ID) HLA CLASS II HISTOCOMPATIBILITY ANTIGEN, DR ALPHA CHAIN PRECURSOR. [Source: SWISSPROT (P01903)]
<400> 145
   attcttgtct gttctgcctc actcccgagc tctactgact cccaacagag cgcccaagaa 60
   gaaaatggcc ataagtggag tccctgtgct aggatttttc atcatagctg tgctgatgag 120
   cgctcaggaa tcatgggcta tcaaagaaga acatgtgatc atccaggccg agttctatct 180
   gaatcctgac caatcaggcg agtttatgtt tgactttgat ggtgatgaga ttttccatgt 240
   ggatatggca aagaaggaga cggtctggcg gcttgaagaa tttggacgat ttgccagctt 300
   tgaggctcaa ggtgcattgg ccaacatagc tgtggacaaa gccaacctgg aaatcatgac 360
   aaagcgctcc aactatactc cgatcaccaa tgtacctcca gaggtaactg tgctcacgaa 420
   cagccctgtg gaactgagag agcccaacgt cctcatctgt ttcatagaca agttcacccc 480
   accagtggtc aatgtcacgt ggcttcgaaa tggaaaacct gtcaccacag gagtgtcaga 540
   gacagtcttc ctgcccaggg aagaccacct tttccgcaag ttccactatc tccccttcct 600
   gccctcaact gaggacgttt acgactgcag ggtggagcac tggggcttgg atgagcctct 660
   tctcaagcac tgggagtttg atgctccaag ccctctccca gagactacag agaacgtggt 720
   gtgtgccctg ggcctgactg tgggtctggt gggcatcatt attgggacca tcttcatcat 780
   caagggattg cgcaaaagca atgcagcaga acgcaggggg cctctgtaag gcacatggag 840
   gtgatggtgt ttcttagaga gaagatcact gaagaaactt ctgctttaat ggctttacaa 900
   agctggcaat attacaatcc ttgacctcag tgaaagcagt catcttcagc attttccagc 960
   cctatagcca ccccaagagt ggttatgcct cctcgattgc tccatactct aacatctagc 1020
   tggcttccct gtctattgcc ttttcctgta tctattttcc tctatttcct atcattttat 1080
   tatcaccatg caatgcctct ggaataaaac atacaggagt ctgtctctgc tatggaatgc 1140
   cccatggggc atctcttgtg tacttattgt ttaaggtttc ctcaaactgt gatttttctg 1200
   aacacaataa actattttga agatcttggg tggaa                            1235
<210> 146
   <211> 5918
   <212> DNA
   <213> unknown
<220>
   <223> >OPA1 (HUGO ID) DYNAMIN-LIKE 120 KDA PROTEIN, MITOCHONDRIAL PRECURSOR (OPTIC ATROPHY 1 GENE PROTEIN). [Source: SWISSPROT (060313)]
<400> 146
   cacgggggct cccgcgtggc cgtctcggcg cctgcgtgac ctccccgccg gcgggatgtg 60
   gcgactacgt cgggccgctg tggcctgtga ggtctgccag tctttagtga aacacagctc 120
   tggaataaaa ggaagtttac cactacaaaa actacatctg gtttcacgaa gcatttatca 180
   ttcacatcat cctaccttaa agcttcaacg accccaatta aggacatcct ttcagcagtt 240
   ctcttctctg acaaaccttc ctttacgtaa actgaaattc tctccaatta aatatggcta 300
   ccagcctcgc aggaattttt ggccagcaag attagctacg agactcttaa aacttcgcta 360
   tctcatacta ggatcggctg ttgggggtgg ctacacagcc aaaaagactt ttgatcagtg 420
   gaaagatatg ataccggacc ttagtgaata taaatggatt gtgcctgaca ttgtgtggga 480
   aattgatgag tatatcgatt ttgagaaaat tagaaaagcc cttcctagtt cagaagacct 540
   tgtaaagtta gcaccagact ttgacaagat tgttgaaagc cttagcttat tgaaggactt 600
   ttttacctca ggtcacaaat tggttagtga agtcatagga gcttctgacc tacttctctt 660
   gttaggttct ccggaagaaa cggcgtttag agcaacagat cgtggatctg aaagtgacaa 720
   gcattttaga aaggtgtcag acaaagagaa aattgaccaa cttcaggaag aacttctgca 780
   cactcagttg aagtatcaga gaatcttgga acgattagaa aaggagaaca aagaattgag 840
   aaaattagta ttgcagaaag atgacaaagg cattcatcat agaaagctta agaaatcttt 900
   gattgacatg tattctgaag ttcttgatgt tctctctgat tatgatgcca gttataatac 960
   gcaagatcat ctgccacggg ttgttgtggt tggagatcag agtgctggaa agactagtgt 1020
   gttggaaatg attgcccaag ctcgaatatt cccaagagga tctggggaga tgatgacacg 1080
   ttctccagtt aaggtgactc tgagtgaagg tcctcaccat gtggccctat ttaaagatag 1140
   ttctcgggag tttgatctta ccaaagaaga agatcttgca gcattaagac atgaaataga 1200
   acttcgaatg aggaaaaatg tgaaagaagg ctgtaccgtt agccctgaga ccatatcctt 1260
   aaatgtaaaa ggccctggac tacagaggat ggtgcttgtt gacttaccag gtgtgattaa 1320
   tactgtgaca tcaggcatgg ctcctgacac aaaggaaact attttcagta tcagcaaagc 1380
   ttacatgcag aatcctaatg ccatcatact gtgtattcaa gatggatctg tggatgctga 1440
   acgcagtatt gttacagact tggtcagtca aatggaccct catggaagga gaaccatatt 1500
   cgttttgacc aaagtagacc tggcagagaa aaatgtagcc agtccaagca ggattcagca 1560
   gataattgaa ggaaagctct tcccaatgaa agctttaggt tattttgctg ttgtaacagg 1620
   aaaagggaac agctctgaaa gcattgaagc tataagagaa tatgaagaag agttttttca 1680
   gaattcaaag ctcctaaaga caagcatgct aaaggcacac caagtgacta caagaaattt 1740
   aagccttgca gtatcagact gcttttggaa aatggtacga gagtctgttg aacaacaggc 1800
   tgatagtttc aaagcaacac gttttaacct tgaaactgaa tggaagaata actatcctcg 1860
   cctgcgggaa cttgaccgga atgaactatt tgaaaaagct aaaaatgaaa tccttgatga 1920
   agttatcagt ctgagccagg ttacaccaaa acattgggag gaaatccttc aacaatcttt 1980
   gtgggaaaga gtatcaactc atgtgattga aaacatctac cttccagctg cgcagaccat 2040
   gaattcagga acttttaaca ccacagtgga tatcaagctt aaacagtgga ctgataaaca 2100
   acttcctaat aaagcagtag aggttgcttg ggagacccta caagaagaat tttcccgctt 2160
   tatgacagaa ccgaaaggga aagagcatga tgacatattt gataaactta aagaggctgt 2220
   taaggaagaa agtattaaac gacacaagtg gaatgacttt gcggaggaca gcttgagggt 2280
   tattcaacac aatgctttgg aagaccgatc catatctgat aaacagcaat gggatgcagc 2340
   tatttatttt atggaagagg ctctgcaggc tcgtctcaag gatactgaaa atgcaattga 2400
   aaacatggtg ggtccagact ggaaaaagag gtggttatac tggaagaatc ggacccaaga 2460
   acagtgtgtt cacaatgaaa ccaagaatga attggagaag atgttgaaat gtaatgagga 2520
   gcacccagct tatcttgcaa gtgatgaaat aaccacagtc cggaagaacc ttgaatcccg 2580
   aggagtagaa gtagatccaa gcttgattaa ggatacttgg catcaagttt atagaagaca 2640
   ttttttaaaa acagctctaa accattgtaa cctttgtcga agaggttttt attactacca 2700
   aaggcatttt gtagattctg agttggaatg caatgatgtg gtcttgtttt ggcgtataca 2760
   gcgcatgctt gctatcaccg caaatacttt aaggcaacaa cttacaaata ctgaagttag 2820
   gcgattagag aaaaatgtta aagaggtatt ggaagatttt gctgaagatg gtgagaagaa 2880
   gattaaattg cttactggta aacgcgttca actggcggaa gacctcaaga aagttagaga 2940
   aattcaagaa aaacttgatg ctttcattga agctcttcat caggagaaat aaattaaaat 3000
   cgtactcata atcagctctg catacatctg aagaacaaaa acatcaacgt cttttgtcca 3060
   gcctcttttt cttctgctgt tccacctttc taaacataca ataaagtcat gggataaaaa 3120
   taatcgatgt atgttacggg cgctttaacc atcagctgcc tctcgaatgg aagaacagtg 3180
   gtaatggatt aacatcctat tttgttgtac taaagtgaca aatcggaata atataattgg 3240
   tatggccatt aggttcagtc cttgaagata agaaacttgt tctctgtttg ttgtcttatt 3300
   tgtggtggca ctcgtttaat ggattaactg aggttgctca atgttcagtt tcttttccag 3360
   aaatacaatg ctaggtgttt tgaaataaaa cttatatagc aattgtttaa agttatcaat 3420
   tgtatataaa atcacagtag cctgctaaat cattgtatgt gtctgtagta ttctattccc 3480
   agaaactatt tgaccatgat aattcagttt atattcacca catgaaagaa aaatgggtaa 3540
   cagaagaacc cttaaaacag gttaatttgg attgtaacgt tcagtgaaag aaatttcaac 3600
   ccttcatagc cagcgaagaa atttgccttg gaagccaagt cagtaccagc ttacctattt 3660
   gattcagttg ctgttttctc actctctata tccatttgaa attgatttat tttagatgtt 3720
   gtatacttac gttaggcttt ctgttaatag tggtttttct cctgttgaca gagccaccgg 3780
   attatgacac aggatgagga agattaagga taatcaattg actaatttca tttagaatat 3840
   tatcaaacat ttcaactagg tatcagaaaa aggctttctt tcataagact attttaaata 3900
   gaaattattt caacaattaa agtaatgttg accatccccc tctcagctga ataaagaaaa 3960
   atttagttca atttattgca atttaattac aatactacct tcacaacatt ttcatgtgtt 4020
   ttaaataaat attttttaat tggctaaagg acattcaagc aaagaaatgc tttctttact 4080
   taaaatgtct atctcatttg ctgccttttc actaagcctt tactttgtta ataaaagtgt 4140
   ccattgtgtg atgtttttga ttttacagtt tgctaaatct tattttcttg gagttgcttt 4200
   ttggtaacag ccccattgct actccccatt ttattgtttt acatcaatgc atgcttcgtt 4260
   gtgatccctc aagatgtaac acttggtatg ctcggttgag gatatgaaaa aatacttccg 4320
   aaaccaggaa ttcaatgtat gtttgtttta tactgtttga taagaaaagt aggtccagcc 4380
   ttaagcagca cagatgcgct ggtagatgca tagtcaggaa ctttttttat ttcttttagg 4440
   tctagggaca ggagtgaata gaaagggagg agagctctat tatgttctat acacagatta 4500
   ggagatgacc ttactgggta cacccctcta accagtgctt acaggttaat gcatgttaat 4560
   gaatattttt gcagttgtaa agcataacaa ttacaactac acatctattt ctaaagaata 4620
   aaacaggacc atatttattt acttctgtca actatagaaa gaaagacctt cagctgtatt 4680
   tccacagatt tctcccaagg aaaaggctaa tattagtcac tactgttatc acatcccttt 4740
   gtataagttt taaaaagaga tggagggaga tcttcatttc tttgaggaga tcagtattgt 4800
   aacgtatgtg aatagatgat aacaattaat attactaaaa gtcccacatg agagtcctga 4860
   cgccctctcc atgccccaca gtaatgtggc ttctttcatg ggtttttttt tcttcttttt 4920
   agctgatctc atcctaagca tgctttattt ttccttgaaa gctaggtatt tatcaactgc 4980
   agatgttatt gaaagaaaat aaaattcagt ctcaagagta aaccctgtgt cttgtgtctg 5040
   tagttcaaaa gtcagaaatg attctaattt aaacaaaaag atactaaata tacagaagtt 5100
   aaattcgaac tagccacaga atcatttgtt tttatgtcag aatttgcaaa gagtggagtg 5160
   gacaaagctc tgtatggaag actgaacaac tgtaaataga tgatatccaa acttaatttg 5220
   gctaggactt caattttaaa aatcagtgta cctaggcagt gcacagcacg aaataagtgg 5280
   cccttgcagc ttccccgttt aacccactgt gctatagttg cgggtggaac agtcaacctt 5340
   tctagtagtt tatgatattg ccctctttgt attcccattt tctacagttt tttccgcaga 5400
   cttctttctg caaattattc agcctccaaa tgcaaatgaa tgatataaaa ataagtaggg 5460
   aacatggcag agagtggtgc ttcccagcct cacaatgtgg gaatttgaca taggatgaga 5520
   gtcagagtat aggtttaaaa gataaaatct ttagttaata attttgtatt tatttattct 5580
   agatgtatgt atctgaggaa agaaatctgg tatttttgct ttccaataaa ggggatcaaa 5640
   gtaatggttt ttctctcagt tctctaagct ggtctatgtt atagctctag cagtatggaa 5700
   atgtgcttta aaatatgctt accttttgaa tgatcatggc tatatgttgt tgagatattt 5760
   gaaacttacc ttgttttcac ttgtgcactg tgaatgaact ttgtattatt tttttaaaac 5820
   cttcacatta cgtgtagata ttattgcaac ttatattttg cctgagcttg atcaaaggtc 5880
   atttgtgtag atgagtaatt aaaaaatatt taaatcac                         5918
<210> 147
   <211> 16396
   <212> DNA
   <213> unknown
<220>
   <223> BPAG1 (HUGO ID) BULLOUS PEMPHIGOID ANTIGEN 1 ISOFORMS 1/2/3/4/5/8 (230 KDA BULLOUS PEMPHIGOID ANTIGEN) (BPA) (HEMIDESMOSOMAL PLAQUE PROTEIN) (DYSTONIA MUSCULORUM PROTEIN) (FRAGMENT). [Source: SWISSPROT (Q03001)]
<400> 147
   cagctgccac ttttcaccgt tagaagtaga gctttttcca gacctcctac cttttagtct 60
   actttgaaag gtgaaagaaa gaacatcgtt tcaggaataa aaatgcacag tagtagttat 120
   agttaccgta gcagtgattc tgtgtttagt aacactacca gcactcgaac cagtcttgat 180
   tcaaatgaaa atcttctctt ggttcattgt ggtccaacac tgatcaactc ttgcattagc 240
   ttcggcagtg aatcctttga tggacacagg ttagaaatgt tgcaacagat tgccaacaga 300
   gttcagaggg acagtgtcat ctgtgaagac aaactgattc ttgctggaaa tgctcttcag 360
   tctgattcta aaagattaga atcaggagtg cagtttcaga atgaagcaga aattgctggg 420
   tatatacttg aatgtgagaa ccttttacgc cagcatgtaa ttgatgtaca gattcttatt 480
   gatggaaaat actaccaggc agatcaattg gtacagaggg ttgcaaaact gcgtgacgaa 540
   attatggcct taaggaacga atgttcttct gtgtacagca aaggacgcat actgacaaca 600
   gaacagacaa agctcatgat atcaggaatc actcaaagtt taaactcagg atttgcacag 660
   accttacacc ctagtctgac ctcagggctg acccagagtt taacaccttc cctaacctct 720
   tctagtatga cttctggcct gtcatcaggg atgacttccc gcctgactcc atctgtcact 780
   ccagcttata cacctggttt cccatcagga ttagttccaa atttcagttc aggagtagag 840
   ccaaattcat tgcaaacttt gaagttgatg cagatccgaa aaccccttct aaagtcttct 900
   ttgctggatc aaaatttaac agaagaagaa atcaatatga aatttgttca ggatcttttg 960
   aattgggttg atgagatgca ggtacaactg gaccgcactg agtggggctc agatttgcca 1020
   agtgttgaaa gccatttaga aaatcataaa aatgttcata gagctattga agaatttgaa 1080
   tctagtctca aagaagctaa aatcagtgag attcaaatga cagcacctct taaactgact 1140
   tatgcagaaa agttgcacag attagagagt cagtatgcaa aactcttgaa tacatccagg 1200
   aatcaagaac ggcaccttga tacactccat aattttgtaa gtcgtgcgac taatgaactt 1260
   atttggttga atgaaaaaga agaggaggaa gttgcttatg actggagtga gagaaacacc 1320
   aacatagcta ggaaaaaaga ttatcatgct gaattaatga gagaacttga tcaaaaggaa 1380
   gaaaatatta aatcagttca ggagatagca gagcagctac ttctagaaaa tcatccagcc 1440
   cggttaacta ttgaggccta cagagcggca atgcagacgc agtggagctg gatcttacag 1500
   ctctgccagt gtgtggagca gcacataaag gagaacacag cgtatttcga gtttttcaat 1560
   gatgccaaag aagctactga ttacttaagg aatctaaaag atgccattca gcggaagtac 1620
   agctgtgata gatcaagcag cattcacaag ctagaagacc ttgttcagga atcaatggaa 1680
   gagaaagaag aacttctgca gtacaaaagc actatagcaa acctaatggg aaaagcaaaa 1740
   acaataattc aactgaagcc aaggaattct gactgtccac tcaaaacttc tattccgatc 1800
   aaagctatct gtgactacag acaaattgag ataaccattt acaaagacga tgaatgtgtt 1860
   ttggcgaata actctcatcg tgctaaatgg aaggtcatta gtcctactgg gaatgaggct 1920
   atggtcccat ctgtgtgctt caccgttcct ccaccaaaca aagaagcggt ggaccttgcc 1980
   aacagaattg agcaacagta tcagaatgtc ctgactcttt ggcatgagtc tcacataaac 2040
   atgaagagtg tagtatcctg gcattatctc atcaatgaaa ttgatagaat tcgagctagc 2100
   aatgtggctt caataaagac aatgctacct ggtgaacatc agcaagttct aagtaatcta 2160
   caatctcgtt ttgaagattt tctggaagat agccaggaat cccaagtctt ttcaggctca 2220
   gatataacac aactggaaaa ggaggttaat gtatgtaagc agtattatca agaacttctt 2280
   aaatctgcag aaagagagga gcaagaggaa tcagtttata atctctacat ctctgaagtt 2340
   cgaaacatta gacttcggtt agagaactgt gaagatcggc tgattagaca gattcgaact 2400
   cccctggaaa gagatgattt gcatgaaagt gtgttcagaa tcacagaaca ggagaaacta 2460
   aagaaagagc tggaacgact taaagatgat ttgggaacaa tcacaaataa gtgtgaggag 2520
   tttttcagtc aagcagcagc ctcttcatca gtccctaccc tacgatcaga gcttaatgtg 2580
   gtccttcaga acatgaacca agtctattct atgtcttcca cttacataga taagttgaaa 2640
   actgttaact tggtgttaaa aaacactcaa gctgcagaag ccctcgtaaa actctatgaa 2700
   actaaactgt gtgaagaaga agcagttata gctgacaaga ataatattga gaatctaata 2760
   agtactttaa agcaatggag atctgaagta gatgaaaaga gacaggtatt ccatgcctta 2820
   gaggatgagt tgcagaaagc taaagccatc agtgatgaaa tgtttaaaac gtataaagaa 2880
   cgggaccttg attttgactg gcacaaagaa aaagcagatc aattagttga aaggtggcaa 2940
   aatgttcatg tgcagattga caacaggtta cgggacttag agggcattgg caaatcactg 3000
   aagtactaca gagacactta ccatccttta gatgattgga tccagcaggt tgaaactact 3060
   cagagaaaga ttcaggaaaa tcagcctgaa aatagtaaaa ccctagccac acagttgaat 3120
   caacagaaga tgctggtgtc cgaaatagaa atgaaacaga gcaaaatgga cgagtgtcaa 3180
   aaatatgcag aacagtactc agctacagtg aaggactatg aattacaaac aatgacctac 3240
   cgggccatgg tagattcaca acaaaaatct ccagtgaaac gccgaagaat gcagagttca 3300
   gcagatctca ttattcaaga gttcatggac ctaaggactc gatatactgc cctggtcact 3360
   ctcatgacac aatatattaa atttgctggt gattcattga agaggctgga agaggaggag 3420
   aagtcactgg aagaagaaaa gaaagaacat gttgaaaaag ccaaagaatt gcaaaaatgg 3480
   gtatcaaata tcagcaagac attgaaagat gcagagaagg ctggaaaacc tcccttctct 3540
   aagcaaaaga tttccagtga ggaaatatca accaagaagg aacaattatc ggaagcactt 3600
   caaactatac agcttttttt ggcaaaacat ggagacaaaa tgacagatga agaaagaaat 3660
   gaattggaaa aacaagtgaa aactcttcag gaaagttata atttattatt cagtgaatct 3720
   ctgaaacagc tacaagaatc acaaacctca ggagatgtaa aggtagagga gaagattgta 3780
   gcagagcgtc agcaggagta taaagagaaa ctccaaggga tatgtgatct tcttacccaa 3840
   actgaaaacc gcctaattgg tcaccaagaa gccttcatga ttggagatgg cacagttgag 3900
   ttaaagaaat atcagtccaa gcaagaggaa ttacagaaag atatgcaagg aagtgcacag 3960
   gcattggctg aagtagtgaa aaacacagag aacttcttaa aagagaatgg tgaaaagctg 4020
   tcacaggaag ataaggcttt gattgaacag aaacttaatg aagctaagat aaagtgtgaa 4080
   cagcttaatt taaaagcaga acagtctaaa aaggagctgg ataaagttgt gacaacagca 4140
   atcaaggaag aaactgaaaa ggtagcagca gtgaagcagc tggaagagag caaaaccaaa 4200
   atagaaaacc ttttggactg gttgtcaaat gttgacaaag actcagaaag ggcagggaca 4260
   aaacacaaac aggtaatcga acagaacggg acccattttc aagaaggtga tggcaagtca 4320
   gcaattggag aagaggatga agttaatggt aacctgttgg agactgatgt tgatgggcaa 4380
   gttggaacca ctcaggagaa tctgaatcag caatatcaga aagttaaggc ccaacacgag 4440
   aagatcatct ctcagcacca agcagtgatc atagccactc agtctgcaca agtgttgctt 4500
   gagaaacagg gtcagtatct ctctcctgaa gaaaaagaga aactgcaaaa gaacatgaag 4560
   gaactgaaag tgcattatga aactgcactg gctgagtcag agaagaaaat gaagttaact 4620
   cactctctgc aggaagaatt agaaaagttt gatgctgact ataccgagtt tgagcactgg 4680
   ctacagcagt cagaacaaga acttgaaaac ctggaggcag gtgcagatga catcaatggt 4740
   ttaatgacca aattgaagag gcagaagagt ttctcagaag acgttatttc tcacaaaggt 4800
   gacttgagat acatcacaat ttctggaaac agagtgttgg aagctgccaa atcttgcagc 4860
   aagagagacg gtggcaaggt tgatacttct gcaacccaca gagaagtgca gcgcaagttg 4920
   gatcatgcta ctgatcggtt caggtctctc tactctaagt gcaatgttct tggaaataat 4980
   cttaaagatc tggtggataa atatcaacac tatgaagatg cttcatgtgg acttcttgct 5040
   ggactccagg cctgtgaggc cacagcgagc aaacacttat ctgaacctat tgcggtggac 5100
   cccaaaaatc ttcaaaggca attagaagag accaaggctt tgcaaggaca gatatcaagt 5160
   cagcaggttg ctgtagagaa actgaagaaa acggctgaag tgcttttaga tgccagggga 5220
   tctttacttc cagccaagaa tgatatccag aaaacacttg atgacattgt tgggagatat 5280
   gaggatctgt ccaagtctgt taatgaacga aatgaaaaac tccagataac cttgacccgt 5340
   tcactgagtg tgcaggatgg cttggatgaa atgctggact ggatgggaaa tgtggaaagt 5400
   tctctgaaag aacaaggtca agtgccttta aactctactg ctcttcagga tattatcagt 5460
   aaaaacatta tgttggaaca ggatattgca ggtcgtcaga gcagtataaa tgccatgaat 5520
   gaaaaagtga agaaatttat ggaaacaaca gatccatcca ctgcatcctc actgcaagcc 5580
   aaaatgaaag acttgtctgc tcggttttca gaagcaagtc ataaacacaa agaaactctt 5640
   gccaaaatgg aggagctaaa aaccaaagta gaactgtttg agaacctctc agaaaagctc 5700
   cagacatttt tagaaacaaa aactcaggct cttactgaag tagatgtgcc aggaaaagat 5760
   gttactgaat tgtctcagta tatgcaggaa tcaacttctg aatttttaga acacaagaaa 5820
   catcttgaag ttttgcatag tctcttgaag gagatatcca gccatggctt accaagtgat 5880
   aaggctttgg tccttgaaaa aacaaataat ttgtcaaaga aattcaagga aatggaggat 5940
   accatcaaag aaaaaaaaga agctgtaact tcttgtcaag aacagttgga tgctttccaa 6000
   gttcttgtta aatcattgaa gtcatggata aaagaaacaa caaaaaaagt tcccattgta 6060
   cagccttctt ttggtgcaga ggatttagga aaatctttgg aagacactaa gaaattacaa 6120
   gaaaagtgga gtttaaaaac accagagatt cagaaagtaa acaacagtgg gatctcacta 6180
   tgtaacttaa taagtgctgt gaccaccccc gcaaaggcaa tagcagcagt taaatcaggt 6240
   ggagcagtat taaatggtga aggaacagcc acaaatactg aggaattttg ggcaaataaa 6300
   ggtttaacat ccattaaaaa ggacatgact gacataagtc atggttatga agatcttggc 6360
   ctcttactca aggacaaaat agcggaactg aacactaaac tctccaaatt gcaaaaggct 6420
   caggaagaat caagtgcaat gatgcagtgg ttacagaaaa tgaacaaaac tgcaacaaaa 6480
   tggcagcaga cacctgcacc tacagatact gaagctgtga agactcaagt tgagcagaat 6540
   aagtcgtttg aggcagaact gaagcagaat gtaaacaaag tacaggagtt gaaagacaaa 6600
   ttgacagagc tgttggagga gaacccagat actcctgagg cccccagatg gaaacagatg 6660
   ttgacagaaa tagattctaa gtggcaagaa ctcaatcaat taacaattga tagacaacaa 6720
   aaactggaag aatcctccaa taatctaacc cagttccaga ctgtagaggc ccaattgaaa 6780
   cagtggcttg tggaaaaaga acttatggtc agtgttcttg ggcccttgtc aattgaccca 6840
   aatatgctaa acacacaaag gcagcaggtg cagattttgc tgcaagaatt cgccactcgg 6900
   aaacctcaat atgaacagct gacagcagct ggtcagggca ttctgagcag gcctggagaa 6960
   gacccttctt tacgtgggat tgtgaaagag caactggcag ctgtgaccca aaaatgggat 7020
   agcctaacag ggcaattgag tgacagatgt gactggattg accaagccat tgttaaaagc 7080
   acacagtatc aaagcctgct gagaagcctt tctgataaac tgagtgactt ggataataaa 7140
   ctcagcagca gtctggctgt gagcacgcac cctgatgcta tgaaccaaca gttggaaaca 7200
   gcccaaaaaa tgaagcagga gatacagcag gaaaagaagc agataaaagt ggcccaggca 7260
   ctctgtgagg atttgtcagc actggttaaa gaagagtact tgaaagcaga acttagtagg 7320
   caactagaag gcatcttaaa atcatttaag gatgttgaac agaaagcaga gaatcatgtc 7380
   cagcaccttc agtcggcctg tgcaagctct catcaatttc agcaaatgtc tagagatttt 7440
   caggcttggc tggatacaaa gaaagaagag caaaacaaat ctcatccaat atctgccaaa 7500
   ctcgatgtct tggagtcatt aattaaagat cataaagact ttagtaaaac tttgaccgct 7560
   cagtctcata tgtatgaaaa aaccattgca gaaggtgaaa atctgttatt aaaaacacaa 7620
   gggtctgaga aggcagcctt acagttacag cttaatacaa ttaaaaccaa ttgggataca 7680
   tttaataagc aggtgaaaga aagagaaaac aagttaaaag agtcattgga aaaagccctt 7740
   aagtataaag agcaagtaga gactctctgg ccatggatag acaaatgcca aaacaacctg 7800
   gaggaaataa aattttgctt ggatcctgct gaaggagaga attctattgc caagttaaag 7860
   tctctgcaga aggaaatgga ccaacacttt ggtatggtag aattactgaa caacacagcc 7920
   aatagcttgc tcagtgtctg tgagatagat aaagaagttg ttacagatga gaataagtca 7980
   ctgatccaga aggtggacat ggtcactgaa caacttcaca gtaagaaatt ctgtctggag 8040
   aacatgactc agaagtttaa agaatttcaa gaagtttcca aagaatctaa aaggcagctt 8100
   cagtgtgcaa aggagcagct agatatccat gattcgctgg gatcccaggc ttacagtaac 8160
   aaatacctga ccatgttgca aactcagcag aaatcacttc aggccttgaa gcatcaggta 8220
   gatttggcta aaagacttgc acaggacctt gtggtagagg cctcagactc aaagggaacc 8280
   tctgatgttt tattacaagt ggaaaccata gctcaagagc atagtacact aagtcagcag 8340
   gttgatgaaa agtgttcttt cttagaaacc aagcttcagg gcattgggca tttccagaat 8400
   accattcgag aaatgttttc tcagttcgca gagtttgatg atgaactgga tagcatggct 8460
   ccagtgggga gagatgcaga aacattgcaa aagcaaaagg aaactataaa agcctttcta 8520
   aagaaactag aagccctcat ggcaagcaat gacaatgcca ataaaacctg caagatgatg 8580
   ttagccacag aagaaacctc tcctgacctt gttggaatca aaagggactt ggaggcctta 8640
   agcaaacaat gcaacaagtt actggaccga gcccaagcca gagaagagca ggttgaaggg 8700
   acaattaagc gccttgaaga attttacagc aaattgaaag aattttctat tctgctccag 8760
   aaagccgaag aacatgaaga gtcacaaggt cctgttggta tggaaacgga gacaattaat 8820
   cagcagctta acatgttcaa ggtattccag aaagaagaga ttgaaccctt gcaaggtaaa 8880
   cagcaagatg taaactggtt aggtcaaggc cttattcaga gtgctgccaa aagcactagc 8940
   actcagggct tggagcatga cctggatgat gtcaatgcac ggtggaagac tctcaataag 9000
   aaggtggctc agcgagcagc ccagctgcag gaggccttgc tgcactgtgg gaggttccag 9060
   gatgccctgg agtccctgct cagctggatg gtggacactg aggagcttgt ggccaatcag 9120
   aagcccccgt cggctgagtt caaagtggta aaggcccaga tacaagaaca aaagcttctc 9180
   cagagattgt tggatgaccg aaaatctacg gtggaggtaa tcaaacgaga aggagaaaaa 9240
   attgctacaa cagcagagcc cgcagataaa gtgaagattt tgaaacagct cagtctcttg 9300
   gatagcagat gggaggcatt gcttaataaa gctgaaacaa ggaatcgtca gttggaaggt 9360
   atctcggtgg tagcacagca atttcatgaa accttagaac cactgaacga gtggcttaca 9420
   accatagaaa agaggctggt gaattgtgaa cccataggaa cccaagcatc taaacttgag 9480
   gaacaaattg cacagcacaa agccttagaa gatgacatca tcaatcacaa taaacattta 9540
   caccaggctg ttagcattgg ccagtcctta aaggttttga gctccaggga ggataaagat 9600
   atggtgcaga gtaaattaga cttctctcaa gtatggtaca ttgagattca agagaaaagt 9660
   cacagcaggt ctgagctcct ccagcaggcc ttatgtaatg ctaagatttt tggggaagat 9720
   gaagttgaac tgatgaactg gctgaatgaa gtgcatgaca aactgagcaa gctctcagtc 9780
   caggattaca gcactgaggg gctatggaag cagcagtctg aacttcgggt tctgcaagag 9840
   gacatcttac tcaggaaaca aaatgtagat caggctttac taaatggttt agaactactt 9900
   aaacaaacca caggtgatga agttttaata attcaagata aattggaagc cattaaagca 9960
   aggtacaaag acattactaa actgagcact gatgtggcca agactctgga acaggcgctg 10020
   cagcttgcaa ggcggctgca ctccacacac gaagagctgt gtacctggct ggacaaagtg 10080
   gaggtggaat tactttcata tgaaactcag gttctgaaag gagaagaagc aagtcaagca 10140
   caaatgagac caaaggaact gaaaaaggaa gctaagaaca acaaagcctt actggactcc 10200
   cttaatgaag tgagcagtgc tttgctggaa ctggtaccat ggagggcaag agaaggactt 10260
   gagaaaatgg tagctgagga caatgagcgc taccgattag tgagcgacac catcactcag 10320
   aaggtggagg agatcgatgc agccattctg cgatcacagc agtttgacca agcagctgat 10380
   gctgagttat cctggattac tgaaacagaa aaaaaattga tgtctctggg tgacatcagg 10440
   cttgagcaag accagacttc tgctcagctt caagttcaaa agacattcac catggagatt 10500
   ttgagacaca aggatattat tgatgacctt gttaaatctg ggcataaaat catgaccgca 10560
   tgcagtgaag aggaaaagca atcaatgaag aaaaaactgg acaaggtact gaagaactat 10620
   gataccatct gccagattaa ttcagaaagg tatctgcagc tggaacgggc acagtccctg 10680
   gttaaccaat tctgggaaac atatgaagaa ctttggccat ggctgacaga aacacaatca 10740
   atcatctctc agcttcccgc cccagccctt gaatatgaaa ctctaaggca gcagcaggaa 10800
   gaacatcggc aactgcgtga gttgatagct gaacacaagc ctcatataga taagatgaac 10860
   aaaactgggc cacagttact ggaattgagc cctggggaag gcttttctat ccaagagaag 10920
   tatgtggcag ccgacaccct ttacagtcaa attaaagaag atgtcaaaaa gcgtgctgtg 10980
   gcactggatg aagccatttc tcaatcaact cagttccatg acaagataga tcagatcctt 11040
   gagagcctgg aacgcatcgt ggaacgtctg aggcagccac cctctatctc tgcagaggtt 11100
   gagaagatca aggaacagat cagtgaaaat aagaatgtgt cagtagacat ggaaaagcta 11160
   cagccgttgt atgaaactct taaacagagg ggagaggaaa tgattgctag atctgggggg 11220
   actgataaag acatatctgc caaagctgtt caggataagc ttgaccaaat ggttttcatt 11280
   tgggagaaca tacacacact ggtggaagag agggaagcca aactactgga tgtgatggag 11340
   ctagcagaaa agttctggtg tgatcacatg tcattgatag ttaccattaa agatactcaa 11400
   gatttcatcc gggacctgga agatcctgga attgatcctt cagtagtaaa acaacagcaa 11460
   gaagcagcag agaccataag ggaagaaata gatggactac aggaggagct ggatatagtt 11520
   attaacctag gttctgaact cattgcggca tgtggggagc ctgataaacc cattgtcaag 11580
   aagagtatag atgagttaaa ttcagcatgg gattctctaa ataaagcttg gaaagaccgg 11640
   attgacaaac ttgaggaggc aatgcaggct gccgttcagt accaggatgg actgcaggcg 11700
   gtatttgact gggtagatat tgcaggtggt aaattagctt caatgtctcc aattggaaca 11760
   gatctcgaaa ctgtcaagca gcagattgaa gagctaaagc aatttaagtc tgaggcctat 11820
   caacagcaga tagaaatgga aagactgaat catcaagcag agcttttgct aaagaaagta 11880
   acagaagaga gtgacaaaca cactgttcaa gacccattaa tggaactgaa attgatatgg 11940
   gatagcctgg aggagagaat catcaacaga cagcataaac tggagggtgc tctattagcc 12000
   ttgggtcagt tccaacatgc cctggatgag ctcctggcat ggctgacaca caccgagggc 12060
   ttgctaagtg agcagaaacc tgttggagga gaccctaaag ccattgaaat tgaacttgcc 12120
   aagcatcatg tgctccaaaa tgatgtatta gcccatcagt ccacagtgga agccgttaat 12180
   aaagcaggaa atgatctaat tgaatcaagt gcaggagaag aagcaagcaa ccttcagaac 12240
   aagctagagg ttttaaatca acgctggcaa aatgttttgg aaaaaacaga acaaaggaag 12300
   cagcagctgg atggtgcctt gcgccaggcc aaagggttcc atggcgaaat tgaggatttg 12360
   cagcagtggc tgactgacac ggagcgtcat ctgttggcat ctaaaccgct gggaggttta 12420
   ccggaaacag ccaaggagca gcttaatgtc catatggaag tctgtgctgc ctttgaagct 12480
   aaagaagaaa catataagag tctgatgcag aaaggccagc agatgcttgc aagatgccca 12540
   aaatctgcag agacaaatat tgaccaagac ataaataact tgaaagaaaa atgggaatcg 12600
   gtggaaacca aactcaatga aaggaaaact aaactggaag aggctctcaa cttggcaatg 12660
   gagttccaca attctctcca agacttcatc aactggctta ctcaggctga acagacccta 12720
   aatgtagctt ctcggccaag tctcatcttg gacacagtct tatttcaaat tgacgaacac 12780
   aaggtttttg ccaatgaagt aaattctcat cgtgagcaga taatagagct ggacaaaact 12840
   ggaacccacc taaaatattt tagtcagaaa caagatgttg ttctaatcaa gaatctactt 12900
   atcagtgtac aaagtcgatg ggaaaaagtg gttcaacggt tggtagagag aggaagatct 12960
   ttggatgatg caaggaagag agccaagcag ttccatgaag cttggagtaa acttatggag 13020
   tggctagaag agtcagaaaa gtctttggat tctgaactgg aaatcgcaaa tgatccagac 13080
   aaaataaaaa cacaacttgc acaacataag gagtttcaga aatcactcgg agccaagcat 13140
   tctgtctacg acaccaccaa caggactgga cgttctctga aggagaaaac ctccctggct 13200
   gatgacaacc tgaaactgga tgacatgctg agtgaactca gagacaaatg ggataccata 13260
   tgtggaaaat ctgtggaaag acaaaacaaa ttggaggaag ccctgttatt ttctggacaa 13320
   ttcacagatg ccctacaggc tctcattgat tggttatata gagttgaacc ccagctggca 13380
   gaagaccagc ctgttcatgg agacattgat ttggtgatga atctgatcga taatcacaag 13440
   gccttccaaa aagagttggg gaagaggacc agcagtgtgc aggccctgaa gcgctcagcc 13500
   cgagaactca tagaaggcag tcgggatgac tcctcctggg tcaaggtcca gatgcaggaa 13560
   ttaagcacac gctgggagac cgtgtgtgca ctttctatat caaagcaaac acggttagaa 13620
   gcagccctgc gtcaggcaga ggaattccac tcggtggtac atgccctctt ggagtggctg 13680
   gctgaggcgg agcaaaccct gcgtttccat ggtgtcctcc cagatgatga ggatgctctc 13740
   cggactctca ttgatcagca taaagaattc atgaagaaac tggaagaaaa gagagctgaa 13800
   ctaaataaag ccaccactat gggcgacacc gttttggcta tctgccaccc cgactccatc 13860
   actaccatta agcactggat aacaatcatc cgggcgaggt ttgaggaggt gctggcctgg 13920
   gcaaagcaac atcagcagag attagcaagt gctctggctg ggcttattgc caaacaggaa 13980
   ttgttggaag ctttgctggc ttggttgcaa tgggctgaaa ctacacttac tgataaggat 14040
   aaagaagtca tcccccagga gatcgaagag gtgaaagcac tcattgcaga acaccagacc 14100
   ttcatggagg aaatgaccag aaaacagcct gatgttgata aagtaacgaa gacctataag 14160
   aggagagctg ctgatccttc ctcattacaa tcccatattc cagtcttgga taagggacga 14220
   gcaggaagaa aacgctttcc agcatcaagc ttgtatccct ctgggtcaca gacacaaatt 14280
   gaaaccaaaa atcctagggt aaacttactg gtgagcaaat ggcagcaagt ctggctcctg 14340
   gcgttggaaa gaaggaggaa actcaatgat gccttggaca gactagagga gctgagggaa 14400
   tttgctaact ttgattttga tatctggcgc aaaaaataca tgcgatggat gaatcacaag 14460
   aaatctcgag tgatggactt cttcaggaga attgataaag accaggatgg gaaaataacg 14520
   cggcaggaat ttattgatgg aattctttcc tcaaagtttc caaccagtcg cttggagatg 14580
   agcgcagttg cagacatctt tgacagagat ggcgatggat atattgacta ctatgaattt 14640
   gtagcagccc ttcacccaaa taaagatgca tataaaccta tcacagatgc cgacaaaatc 14700
   gaagatgagg tgacaaggca ggtagctaag tgtaaatgtg caaagcgatt tcaagttgag 14760
   cagattggtg ataataaata cagggtaaag gttctttctt tcttcactga acagtttgga 14820
   gactcccagc aactgcgact ggtccggatc ctgcggagta ctgtgatggt tcgtgttgga 14880
   ggtggatgga tggcacttga tgagttctta gtgaaaaatg atccttgcag ggccaaagga 14940
   aggacaaaca tggaactgcg tgagaagttc attttagcag atggtgccag ccagggtatg 15000
   gctgctttcc gaccccgagg ccgaagatcc cggccatcat cacgaggcgc ttcacccaac 15060
   agatccactt ctgtgtccag tcaggctgcg caggcggcct ccccacaggt ccctgccacc 15120
   accacaccca agattctcca tcctttaaca cgcaattatg gtaaaccatg gttgacaaac 15180
   agcaaaatgt caactccttg taaagcagca gagtgctcag actttcccgt gccatctgca 15240
   gagggaacgc caatacaagg aagcaagctt cgacttccag gatatttatc agggaaaggc 15300
   ttccactctg gggaggacag tggcttgata acaactgcag ctgccagagt ccgaacacag 15360
   tttgctgatt ccaagaagac tcccagccga ccaggaagtc gagctggaag caaagctggc 15420
   agcagggcca gcagccgccg aggcagtgat gcatcagact ttgacatttc agaaatccag 15480
   tccgtgtgct cagatgtgga aactgtcccc cagacacaca gacctacacc ccgagcaggt 15540
   tctcggccat ccacagcgaa gccttcaaaa atccccacgc cccagaggaa atcacctgcc 15600
   agcaaattgg acaagtcctc aaagagatag tgcaattggt tctaccaagg cccttccttg 15660
   agcatttatt atttaagttt gaacgatgta aaatatggtg tagaaattct tgtgaaatat 15720
   tgcaagaggc gagtttaaaa ttctgcagat ggccttattt gtgtatttgt ctttttattt 15780
   tatctgtata attttttttg tcagatattc tggggttaaa gtcacatcat atgtgaggag 15840
   gaaaagttta acatgaacta acatttctgc actgtaacgt gccgggcaca cactaaactc 15900
   agttactgta cctacaggta agtctacatc ctctctgaca gccacagcac tacatcaatc 15960
   cctgacgtta gggatacctc atgacatttt cctgttttta tggaaactct gagaagctga 16020
   atgatacatg caggggatat tttttgagat gatttaaatg taaaccaaaa gatggaagac 16080
   aaaaagacaa acacacccac acgcagtctt tgcagtatct gacagagaac tcacaggaag 16140
   ttacttcaag cacttgccag tactatgata ttcaagtacc ttgcagcatt tctctgccat 16200
   tgctttcaat gaggccagag gcatcctgga tattagacct attatactgt aagaatataa 16260
   gtataaagtg cgttcatata catgtgaggt tttcttttgc ttgagtggac agtagcacct 16320
   gtatcattga actcattttg tatcagagca attttgcttg cagaaagcta tgaaataaaa 16380
   cacgtccctt aactgc 16396
<210> 148
   <211> 1751
   <212> DNA
   <213> unknown
<220>
   <223> >DCN (HUGO ID) DECORIN PRECURSOR (BONE PROTEOGLYCAN II) (PG-S2) (PG40). [Source: SWISSPROT (P07585)]
<400> 148
   tttaactgtg ctatggagta gaagcaggag gttttcaacc tagtcacaga gcagcaccta 60
   ccccctcctc ctttccacac ctgcaaactc ttttacttgg gctgaatatt tagtgtaatt 120
   acatctcagc tttgagggct cctgtggcaa attcccggat taaaaggttc cctggttgtg 180
   aaaatacatg agataaatca tgaaggccac tatcatcctc cttctgcttg cacaagtttc 240
   ctgggctgga ccgtttcaac agagaggctt atttgacttt atgctagaag atgaggcttc 300
   tgggataggc ccagaagttc ctgatgaccg cgacttcgag ccctccctag gcccagtgtg 360
   ccccttccgc tgtcaatgcc atcttcgagt ggtccagtgt tctgatttgg gtctggacaa 420
   agtgccaaag gatcttcccc ctgacacaac tctgctagac ctgcaaaaca acaaaataac 480
   cgaaatcaaa gatggagact ttaagaacct gaagaacctt cacgcattga ttcttgtcaa 540
   caataaaatt agcaaagtta gtcctggagc atttacacct ttggtgaagt tggaacgact 600
   ttatctgtcc aagaatcagc tgaaggaatt gccagaaaaa atgcccaaaa ctcttcagga 660
   gctgcgtgcc catgagaatg agatcaccaa agtgcgaaaa gttactttca atggactgaa 720
   ccagatgatt gtcatagaac tgggcaccaa tccgctgaag agctcaggaa ttgaaaatgg 780
   ggctttccag ggaatgaaga agctctccta catccgcatt gctgatacca atatcaccag 840
   cattcctcaa ggtcttcctc cttcccttac ggaattacat cttgatggca acaaaatcag 900
   cagagttgat gcagctagcc tgaaaggact gaataatttg gctaagttgg gattgagttt 960
   caacagcatc tctgctgttg acaatggctc tctggccaac acgcctcatc tgagggagct 1020
   tcacttggac aacaacaagc ttaccagagt acctggtggg ctggcagagc ataagtacat 1080
   ccaggttgtc taccttcata acaacaatat ctctgtagtt ggatcaagtg acttctgccc 1140
   acctggacac aacaccaaaa aggcttctta ttcgggtgtg agtcttttca gcaacccggt 1200
   ccagtactgg gagatacagc catccacctt cagatgtgtc tacgtgcgct ctgccattca 1260
   actcggaaac tataagtaat tctcaagaaa gccctcattt ttataacctg gcaaaatctt 1320
   gttaatgtca ttgctaaaaa ataaataaaa gctagatact ggaaacctaa ctgcaatgtg 1380
   gatgttttac ccacatgact tattatgcat aaagccaaat ttccagttta agtaattgcc 1440
   tacaataaaa agaaattttg cctgccattt tcagaatcat cttttgaagc tttctgttga 1500
   tgttaactga gctactagag atattcttat ttcactaaat gtaaaatttg gagtaaatat 1560
   atatgtcaat atttagtaaa gcttttcttt tttaatttcc aggaaaaaat aaaaagagta 1620
   tgagtcttct gtaattcatt gagcagttag ctcatttgag ataaagtcaa atgccaaaca 1680
   ctagctctgt attaatcccc atcattactg gtaaagcctc atttgaatgt gtgaattcaa 1740
   tacaggctat g                                                      1751
<210> 149
   <211> 870
   <212> DNA
   <213> unknown
<220>
   <223> >CD47 (HUGO ID) LEUKOCYTE SURFACE ANTIGEN CD47 PRECURSOR (ANTIGENIC SURFACE DETERMINANT PROTEIN OA3) (INTEGRIN ASSOCIATED PROTEIN) (IAP) (MER6). [Source: SWISSPROT (Q08722)]
<400> 149
   ggatcagctc agctactatt taataaaaca aaatctgtag aattcacgtt ttgtaatgac 60
   actgtcgtca ttccatgctt tgttactaat atggaggcac aaaacactac tgaagtatac 120
   gtaaagtgga aatttaaagg aagagatatt tacacctttg atggagctct aaacaagtcc 180
   actgtcccca ctgactttag tagtgcaaaa attgaagtct cacaattact aaaaggagat 240
   gcctctttga agatggataa gagtgatgct gtctcacaca caggaaacta cacttgtgaa 300
   gtaacagaat taaccagaga aggtgaaacg atcatcgagc taaaatatcg tgttgtttca 360
   tggttttctc caaatgaaaa tattcttatt gttattttcc caatttttgc tatactcctg 420
   ttctggggac agtttggtat taaaacactt aaatatagat ccggtggtat ggatgagaaa 480
   acaattgctt tacttgttgc tggactagtg atcactgtca ttgtcattgt tggagccatt 540
   cttttcgtcc caggtgaata ttcattaaag aatgctactg gccttggttt aattgtgact 600
   tctacaggga tattaatatt acttcactac tatgtgttta gtacagcgat tggattaacc 660
   tccttcgtca ttgccatatt ggttattcag gtgatagcct atatcctcgc tgtggttgga 720
   ctgagtctct gtattgcggc gtgtatacca atgcatggcc ctcttctgat ttcaggtttg 780
   agtatcttag ctctagcaca attacttgga ctagtttata tgaaatttgt ggcttccaat 840
   cagaagacta tacaacctcc tagggtaagt                                  870
<210> 150
   <211> 941
   <212> DNA
   <213> unknown
<220>
   <223> beta2-microglobulin
<400> 150
   tcctgaagct gacagcattc gggccgagat gtctcgctcc gtggccttag ctgtgctcgc 60
   gctactctct ctttctggcc tggaggctat ccagcgtact ccaaagattc aggtttactc 120
   acgtcatcca gcagagaatg gaaagtcaaa tttcctgaat tgctatgtgt ctgggtttca 180
   tccatccgac attgaagttg acttactgaa gaatggagag agaattgaaa aagtggagca 240
   ttcagacttg tctttcagca aggactggtc tttctatctc ttgtactaca ctgaattcac 300
   ccccactgaa aaagatgagt atgcctgccg tgtgaaccat gtgactttgt cacagcccaa 360
   gatagttaag tgggatcgag acatgtaagc agcatcatgg agggtttgaa gatgccgcat 420
   ttggattgga tgaattccaa attctgcttg cttgcttttt aatattgata tgcttataca 480
   cttacacttt atgcacaaaa tgtagggtta taataatgtt aacatggaca tgatcttctt 540
   tataattcta ctttgagtgc tgtctccatg tttgatgtat ctgagcaggt tgctccacag 600
   gtagctctag gagggctggc aacttagagg tggggagcag agaattctct tatccaacat 660
   caacatcttg gtcagatttg aactcttcaa tctcttgcac tcaaagcttg ttaagatagt 720
   taagcgtgca taagttaact tccaatttac atactctgct tagaatttgg gggaaaattt 780
   agaaatataa ttgacaggat tattggaaat ttgttataat gaatgaaaca ttttgtcata 840
   taagattcat atttacttct tatacatttg ataaagtaag gcatggttgt ggttaatctg 900
   gtttattttt gttccacaag ttaaataaat cataaaactt g                     941

<210> 151
   <211> 484
   <212> DNA
   <213> unknown
<220>
   <223> >RPL27 (HUGO ID) 60S RIBOSOMAL PROTEIN L27. [Source: SWISSPROT (P08526)]
<400> 151
   tccttctttc ctttttgctg gtagggccgg gtggttgctg cagaaatggg caagttcatg 60
   aaacctggga aggtggtgct tgtcctggct ggacgctact ccggacgcaa agctgtcatc 120
   gtgaagaaca ttgatgatgg cacctcagat cgcccctaca gccatgctct ggtggctgga 180
   attgaccgct acccccgcaa agtgacagct gccatgggca agaagaagat cgccaagaga 240
   tcaaagataa aatcttttgt gaaagtgtat aactacaatc acctaatgcc cacaaggtac 300
   tctgtggata tccccttgga caaaactgtc gtcaataagg atgtcttcag agatcctgct 360
   cttaaacgca aggcccgacg ggaggccaag gtcaagtttg aagagagata caagacaggc 420
   aagaacaagt ggttcttcca gaaactgcgg ttttagatgc tttgttttga tcattaaaaa 480
   ttat                                                              484
<210> 152
   <211> 605
   <212> DNA
   <213> unknown
<220>
   <223> RPS16 (HUGO ID) 40S RIBOSOMAL PROTEIN S16. [Source: SWISSPROT (P17008)]
<400> 152
   aaggcgcctg cgcagaccct gaaaagcggc cagggtggcc cctagctttc cttttccggt 60
   tgcggcgccg cgcggtgagg ttgtctagtc cacgctcgga gccatgccgt ccaagggccc 120
   gctgcagtct gtgcaggtct tcggacgcaa gaagacagcg acagctgtgg cgcactgcaa 180
   acgcggcaat ggtctcatca aggtgaacgg gcggcccctg gagatgattg agccgcgcac 240
   gctacagtac aagctgctgg agccagttct gcttctcggc aaggagcgat ttgctggtgt 300
   agacatccgt gtccgtgtaa agggtggtgg tcacgtggcc cagatttatg ctatccgtca 360
   gtccatctcc aaagccctgg tggcctatta ccagaaatat gtggatgagg cttccaagaa 420
   ggagatcaaa gacatcctca tccagtatga ccggaccctg ctggtagctg accctcgtcg 480
   ctgcgagtcc aaaaagtttg gaggccctgg tgcccgcgct cgctaccaga aatcctaccg 540
   ataagcccat cgtgactcaa aactcacttg tataataaac agtttttgag ggattttaaa 600
   gtttc                                                             605
<210> 153
   <211> 2054
   <212> DNA
   <213> unknown
<220>
   <223> NM_018479 (RefSeq ID) UNCHARACTERIZED HYPOTHALAMUS PROTEIN HCDASE. [Source: RefSeq (NM_018479)]
<400> 153
   agtcaccacc gtggcctgcg acgaaatggc gaaaagtctt ttgaagacag cctctctgtc 60
   tggaaggaca aaattgctac atcaaacagg attgtcactt tatagtacat cccatggatt 120
   ttatgaggaa gaagtgaaaa aaacacttca gcagtttcct ggtggatcca ttgaccttca 180
   gaaggaagac aatggcattg gcattcttac tctgaacaat ccaagtagaa tgaatgcctt 240
   ttcaggtgtt atgatgctac aacttctgga aaaagtaatt gaattggaaa attggacaga 300
   ggggaaaggc ctcattgtcc gtggggcaaa aaatactttc tcttcaggat ctgatctgaa 360
   tgctgtgaaa tcactaggaa ctccagaggc aattttttct tcaatataca gacttccttt 420
   aataagtgtt gcgctggttc aaggttgggc attgggtgga ggagcagaat ttactacagc 480
   atgtgatttc aggttaatga ctccagagag taagatcaga ttcgtccaca aagagatggg 540
   cataatacca agctggggtg gcaccacccg gctagttgaa ataatcggaa gtagacaagc 600
   tctcaaagtg ttgagtgggg cccttaaact ggattcaaaa aatgctctaa acataggaat 660
   ggttgaagag gtcttgcagt cttcagatga aactaaatct ctagaagagg cacaagaatg 720
   gctaaagcaa ttcatccaag ggccaccgga agtaattaga gctttgaaaa aatctgtttg 780
   ttcaggcaga gagctatatt tggaggaagc attacagaac gaaagagatc ttttaggaac 840
   agtttggggt gggcctgcaa atttagaggc tattgctaag aaaggaaaat ttaataaata 900
   attggttttt cgtgtggatg tactccaagt aaagctccag tgactaatat gtataaatgt 960
   taaatgatat taaatatgaa catcagaatt actttgaagg ctactattaa tatgcagact 1020
   tacttttaat catttgaata tctgaactca tttacctcat ttcttgccaa ttactcactt 1080
   gggtatttac tgcgtaatct ggaacattta gctaaaatat acacttttgg cttaaaaatt 1140
   attgctgtca attccaataa taattcttag cttataacca aagagcagtg tttaaaagga 1200
   gagcttctat acaaaaccta ttcctggcgt tacttttcat acaatttttg ttctgtttta 1260
   cctggaaata atttaccaaa ataactgagt gttgctgcta aagaacaaaa gtggggaggt 1320
   atcagggaac aagaaaacaa gaaagggtat gatcaatcat tttcttctgc tccaaacagc 1380
   tggagtaaaa ttcatgggaa atggcccttc atttaaaaaa agatgtacct cactacccac 1440
   tacaaatttg gaactttgtt cttttcaata attagttttc tattgtaaat tacctactaa 1500
   acagtggtag ccatgacatg gaaagtcaac tgattctaca attggacatt catttgtgtg 1560
   ccctggaatt tccaactagt aataaacaac tactgttgat gtagttttaa accacttgaa 1620
   gggactcatg aagcatcctg caacataaat ttgcattttt acatcagatt tctttttttt 1680
   cctgaaaaac aactaacctt ctaacaacta tctttcaaaa gtaaatgtaa taaaaatgca 1740
   caacataaaa tgtttatgat cccagcaata cactttttaa aaaatgtgaa agtcaaagaa 1800
   ttaagttcta gttctgactc atcacaagag gtcaaaagta tttgctactg taacattcaa 1860
   ttcacatttg agaatcatgg taaaaataac ttgcatttgc cttaccatca tgatcctact 1920
   gttgagttag gaaaatatgg ttagacagac tcacattact ttttttcaga ggtaaactct 1980
   agattactgt gtcaacccaa tactatttgg ccatagatgt aaaaactacc aaataaaagt 2040
   ggattttgtg gtct                                                   2054
<210> 154
   <211> 170
   <212> DNA
   <213> unknown
<220>
   <223> >OIC1 AJ617288
<400> 154
   gtaaagcgta tttggttatt cctttgtgtt ctcaattttc tggacagtaa gatgtgcaag 60
   aagtgtttgt attctcatgg ggcatgtgga acaggtaact atacccccaa caactcccac 120
   cccatctctt catcttgcac acacaacacc aatcaccatc atgtctttcc 170
<210> 155
   <211> 2037
   <212> DNA
   <213> unknown
<220>
   <223> >Chr 5 clone (EST) ENSESTT00000023592
<400> 155
   cactaggagg gaggatgcta cgatttcaga cctcagctat taaatagtaa atctggaaaa 60
   ctagtggata aatttgccat ctccattcta tgttattaag accaagatag tgtactggtt 120
   ttcttttaaa tcacattcca ttatgtcatt taaaatgtca caaaacccac acgaagcatc 180
   tcatttacac taagatgctt atttagctaa caccactaat gataaatgct gttacaaata 240
   tgcatagtca gatgatagta accacataca gaaaaaaaat ttgaacaagt atttatttct 300
   taaaatttac ttaagggatt agagctaata tataatagaa catttaatat aacatttgga 360
   gttatgtcaa cataaaaata gctgtggtta caattagcac atgcaattca ctgcaaaggt 420
   aaaaatacat gctatactct agacaagcct tccaaatgaa gttagagtag atggggtaaa 480
   acagcaagtg aacatgaaag gattgcactt agaagaaagt gggacatagc taggatataa 540
   aagaaacata cctaatgcta gtcagtcact gcattgtcct actagcaaat tgcacattta 600
   tttttagagt atattcaata cacatacata ttgagactag agaattttca aatatctacc 660
   tttgaaatat ccctttgttt ctaacacatc acattatggt attaatgtaa cagcacttaa 720
   aacctgtagt tatcattcaa agcttgtgtt caagagataa aagaacatca ttcagacaaa 780
   gcctgtgttc aagaaataaa aaaaacatct atcactatcg gccttaaatg catctcatca 840
   cacgacccat caactttaat gaaatgggta atgaagttaa actaaccagt ttgtacaaac 900
   tattgactga gaaaaagccc ttaaaaattt aggaacaaat gagcaacaga agagctcacc 960
   ctgagctgcc accttttaat ttttaatgca atgtatatga gaatatcatg ctaatgcaca 1020
   tactagtagc atgtatgatg gaaaaaaata ggttaatgca aaagataata cgcttggctt 1080
   tgtaaagttt tgttttgact tacatcatct gaattctgtt tatcgagtct gaaaaggaac 1140
   tgctgccaag gaccagtcca aagaagaatt aggctgctaa tcctaaaaca cttgccattt 1200
   tatgactcca tattctttta atttacagaa aagtacaaat tttttagaaa gtaatcttat 1260
   cagcacaata taatctaaca atacctgtaa tagaaagcta tcccagtaaa ttttttttga 1320
   acaattgaac ttttgtctta tgcagcttat aactttgtta ttttattcat ctcaacaaaa 1380
   taaaacttta ttgaaacaaa agtgtatggt ttaggaatat gctcttaata acaactgagc 1440
   aaaattagaa tgatgtggac tataacagaa tgaaggaaga tctctcctgc ttgatgtact 1500
   tggtcaatat atgttagtaa acagaaatat tttagggaat tctatgatta ttctacttct 1560
   acatttacat gtcatggttt taactagtta aatgggtaga aaaaagacat ctttaataat 1620
   tgatttctat ttctcaaact agatgctcta ctttaaatat gagctttaat gaatgttttg 1680
   taacatttta aagttgtaac atttctaagt atactccact aacttcacaa taagagctag 1740
   ggtcactgga tgacttcatg tggtaaaatc ccttatcaag gaataaggct gaaatttaaa 1800
   tgaacaatta ggtagagaag aggtatgaac acagaacatg tgaatgaaat ttgctttttt 1860
   tccttgctgc tgttcccgag tgccgactga tccatagtaa aaaaggacaa gattaaaatt 1920
   ttaggtttta atggtacaaa gtgtgaagtt ttatagtttt ctaataatta ttcagtatta 1980
   tccctttcag agatgagggt aggataccac agacatcagt aactgacaag ttataat 2037
<210> 156
   <211> 228
   <212> DNA
   <213> unknown
<220>
   <223> >Chromosome 16 clone (EST) No ESTs or genes
<400> 156
   cattctgtta gtgcagagct acaaatctta tttgttacat agctcaaaga catcttccac 60
   aggaagaaaa atacctgcca ttagtaaatt cttaaccggt atgaacctat gcttctattt 120
   taaagaggtt agtgcacata tgccttctaa atatctcata aaaaaccacc gtagcacatt 180
   taaaatcctg gtattattac ttaataccac cctacaacac aaaaatca              228
<210> 157
   <211> 166
   <212> DNA
   <213> unknown
<220>
   <223> >Chromosome-1 clone hypothetical protein XP_211586
<400> 157
   gacatgtctc actgtttgta tttctctctc ctgtcaaagt gaatgatata ttcttgaaaa 60
   cccccaattt cttgaaatgg gtgttctgtt cattcatggg caggggtcag gattgaagtt 120
   catatatgaa acaactgggg atgtagatag gaaagaatgt ctgctg                166
<210> 158
   <211> 536
   <212> DNA
   <213> unknown
<220>
   <223> >gi|5446595|gb|A1825924|sr|IMAGp998F065401.1|cl|IM AGp998F0165401|AI825924 to92d03.×1 NCI_CGAP_Gas4 Homo sapiens cDNA clone IMAGE:2185733 3', mRNA sequence.
<221> misc_feature
   <222> 495
   <223> n=a, t, c, or q
<400> 158
   cacttttttc attttttttt attaatcaaa tgatacaaaa caaccatcat tctgtcaatg 60
   cccaagcacc cagctggtcc tctccccaca tgtcacactc tcctcagcct ctcccccaac 120
   cctgctctcc ctcctcccct gccctagccc agggacagag tctaggagga gcctggggca 180
   gagctggagg caggaagaga gcactggaca gacagctatg gtttggattg gggaagagat 240
   taggaagtag gttcttaaag accctttttt agtaccagat atccagccat attcccagct 300
   ccattattca aatcatttcc catagcccag ctcctctctg ttctccccct actaccaatt 360
   ctttggctct tacacaattt ttatccctca aatattcatc cctggcccaa ccagtcccct 420
   gagcctccct ctggtggaga ctcctccacc catgagctcc ccagagcatc caagacagag 480
   tgcacagaga cctgnggaag gaagctgaac tttgcagaga tgaggacagg tgcagg     536
<210> 159
   <211> 699
   <212> DNA
   <213> unknown
<220>
   <223> >gi|4534249|gb|AI570875|sr|IMAGp998J065499.1|cl|IM AGp998J065499|AI570875 tr68d03.×1 NCI_CGAP_Panl Homo sapiens cDNA clone IMAGE:2223461 3' similar to gb:M35252 TUMOR-ASSOCIATED ANTIGEN CO-029 (HUMAN);, mRNA sequence
<221> misc_feature
   <222> 526, 538, 549, 631, 632, 637, 652, 654, 661
   <223> n=a, t, c, or g
<400> 159
   ttcaccaacg taaacagtta cttttatttt gagtaaaaat acacattcat atcattttcc 60
   cttatatccc tcaaatctta aatgtgttca atatgtctag aagatatctg tggtctagct 120
   agccgagaca ttttaaaaag acagctgctc ctgacttata tagcacttac atatttaaat 180
   ttacaaagcc aaagcaacat tttaaagggg tttgactgac gatagcttga tgcatccaca 240
   gattcatttg ttcccgatct ggcaatacag gaccatagaa aacaccaaac ccagtatctc 300
   aataactgcc agtccaaatg ctattccaat aactataatc aaattttttg ccaagaagtc 360
   ttttatgaaa gaaatacagg tctctttgta aacttgtttt ccattatagc tttggcatgg 420
   tctctgctta tctagacagg cacataattc aggataggtt gaaaattatt tcccaatcag 480
   cagctccatt gaccaaaccg cagcatttaa actcttcttg aaacancatt atggctcncc 540
   tggaatgtnt tcacttttcc ctgtggcgct caaaaaagcc ttgtgttcat atgagagtca 600
   tttccacaat cgatcagact tagattgaaa nncagtncta tggtatcctg cncncccttc 660
   ncggggagca gatagaggca agcctttgaa aacacaaca 699
<210> 160
   <211> 717
   <212> DNA
   <213> unknown
<220>
   <223> >sr|IMAGp998A05535.2|cl|IMAGp998A05535|gb|BX114906 |oid|234564199 BX114906 Soares fetal liver spleen 1NFLS Homo sapiens cDNA clone IMAGp998A05535 ; IMAGE:247564, mRNA sequence
<221> misc_feature
   <222> 696, 702
   <223> n=a, t, c, or g
<400> 160
   ctttatcttg agtttcatgt cttagggttg gagaagggac caagcctccc ctgaaagctg 60
   aggggtttat ggtcaagaag aaagagtaac tatggccacc tttaggtcac cagccaataa 120
   tactgcttct ctatcctgtt ctgctgtgac atttgtccca gcaggtgttc cagtctgtcc 180
   tacagtagtg agaccacctg atatggtgag gaaggcaggt aattgatctt actccatcat 240
   atcatcacat ctatttccac cccccgccct tggagacaag gtctcactcc atcatttagg 300
   ccggaatgca gtggtgcaat catagctcat tgtagtctca aacacctggg cccaagtgat 360
   cctcactgta gtctcccaag tagctaggac tacaggcaca tgccaccaca cctggctaat 420
   taaaaaaaat tttttttgca cagagatggg ggtcttgata tgttgcccag gctggtctgg 480
   aactcctggc cacaagtgat cctcccacct cagcctccca aagcactggg attactgttg 540
   tgagccacca tgaccagccc catcacttct tttgtaggtt tgtatttgtt tttatttttg 600
   acaaagactt ttatttcacg gtgacttctt cacaagtttc ctcagatgct agtctgaaca 660
   aaggagagca gggaactaga aatgtagact ttctgntttt tncagctatg gcatcac 717
<210> 161
   <211> 600
   <212> DNA
   <213> unknown
<220>
   <223> >gi|1382358|sr|IMAGp998819790|cl|IMAGp998B19790|gb |W72422|W72422 zd65c10.sl Soares fetal heart NbHH19W Homo sapiens cDNA clone 345522 3' similar to SW:SC13_YEAST Q04491 PROTEIN TRANSPORT PROTEIN SEC13. [1]
<221> misc_feature
   <222> 530
   <223> n=a, t, c, or g
<400> 161
   tataagcaat atgactttat ttagttcctt tggaaacaaa accccccaaa taatgcctga 60
   acccaaaggt acataaaaat gacccaaaat agatttgaac atcacttgta gtttcttcct 120
   cgtaacatga gtgctttcag ctggacagta gattacaaag catctccgat cacgttaagg 180
   cagatgatca atctgtggct gcatctgtaa ctcctcctgg gaaaataatc ctgttggagt 240
   tgggggctct tcccagttgt ctggttagtt ggcccaggaa ggggaagtcc tggagctggc 300
   gggtggggag ccaggcccca cctgtcttgt cactgctccg ttctgctggc cctctgtcac 360
   ttgatgctga tacggagccc tggcccttgt tgacatcact gatgcacacc cactgcccat 420
   caactgactc cttccacagg gtcaccttat tgtctccacc agagacagcc aggatgttgg 480
   ctgtgatgga caagctcaca tgccacacca catcgttgaa cttgtgcaan atttagggga 540
   ccacgtattg cttgaggcat catcacaggt ccaaatgaac acacgaacat cctgggagca 600
<210> 162
   <211> 202
   <212> DNA
   <213> unknown
<220>
   <223> >gi|4648553|gb|AI623622|sr|IMAGp998M105514.1|cl|IM AGp998M105514|AI623622 ts22b05.×1 NCI_CGAP_Panl Homo sapiens cDNA clone IMAGE:2229297 3', mRNA sequence.
<400> 162
   tttttttttt tttttttttt tttttttttt tttttttttt tttttttttt tttttttttt 60
   tttttggaaa aaaagggctt tgtttggtcc ctttttaacc ccccgaattt ggttttcccc 120
   cattttggag gttttttttt ttcccccccc cccccccccg gttttaaacc gggggcgccc 180
   cccctttttt tttttttttt gg                                          202
<210> 163
   <211> 407
   <212> DNA
   <213> unknown
<220>
   <223> >gi|4984008|gb|AI696108|sr|IMAGp9980095541.1|cl|IM AGp9980095541|AI696108 tt03e05.×1 NCI_CGAP_GC6 Homo sapiens cDNA clone IMAGE:2239712 3' similar to contains L1.t3 L1 repetitive element **;,** mRNA sequence
<400> 163
   gagacataac aatatttaat gtgtatgtgc ctgacaacag agtataaaaa tatgtgaggc 60
   aaaacccata gaaatatgag gagaaataaa tgcatacagt atcataattg acttcaacac 120
   tccaacagaa atggacagat ccagtaggca gaaaatctgt atgtgcatag ctaaaattaa 180
   ccataccatc aatcaaatag atataatgga catctactga ctacttcatc caacaacagc 240
   agaatacata tttttctcaa gctcacatga aacattcaac aaaatagact gtattttggg 300
   ccttcagaat caccttaaac acattttaaa cataaaaatt acataatgtc tgggtgtctg 360
   accacaatgg acttgaacta gaaatcaata acagaaaaat agctgag               407
<210> 164
   <211> 380
   <212> DNA
   <213> unknown
<220>
   <223> >gi|2139170|gb|AA434256|sr|IMAGp998D121896.1|cl|IM AGp998D121896|AA434256 zw24h06.sl Soares ovary tumor NbHOT Homo sapiens cDNA clone IMAGE:770267 3', mRNA sequence
<400> 164
   cgaatgaatt gatcagtctt tattgatgat tattacattt ttcatttgcc tggaacttac 60
   cacaccctaa agtattctgg ctctccatat tttctgttca taagaatatg cagagcagtt 120
   gattatcttc tgggtccttt agccctttag ctctcctgag ggtagcagag gactccctgg 180
   ttttcctttg gatggaagtt tcctgacttt ctacttctgg aaccagggcc agtgtccttg 240
   acatgggctt cctccaggat gaaatcggac aactgcccaa tgacagcaag taaagaaagt 300
   atactcgcct ataatcccag cactttggga ggccaaggcg ggtggatcac gaggtcagct 360
   ggataacata ctggctaacg                                             380
<210> 165
   <211> 479
   <212> DNA
   <213> unknown
<220>
   <223> >sr|IMAGp998H235725.2|cl|IMAGp998H235725|gb|BX1120 57|oid|234558491 BX112057 NCI_CGAP_GC6 Homo sapiens cDNA clone IMAGp998H235725 ; IMAGE:2310214, mRNA sequence
<400> 165
   ccgatcgcct cggaagcccc gtagaccatc acggacgccg agcttccagt aactcacagt 60
   gaaagcttgt ttaatgaagc tagcttcaat gctgtgagct gctctataga cgcccatgta 120
   ggaggaaact caaggggccc ccagccacta gccattgagg aactgaggcc ctcaatccaa 180
   caacccacaa ggaactgaat cctgccaaca accactgagt gagcctggaa gcggatcctg 240
   cccagctgaa actcgagaag attgtagctc tgctgccctg accactattc taactgcacc 300
   cttgagagag accctcagcc agctaatctg tacctggatt cctaacctgg agaaactgaa 360
   atagcaacgc atgttgatgt aagtgctaag tttgggagta atttgttatg taacaataaa 420
   taactaataa tacttattat ttaggcaata aaaccataga aaataaaaaa aaaaaaaaa  479
<210> 166
   <211> 716
   <212> DNA
   <213> unknown
<220>
   <223> >sr|IMAGp998P03372.1|cl|IMAGp998P03372|gb|BX118749 |oid|234571874 BX118749 Soares fetal liver spleen 1NFLS Homo sapiens cDNA clone IMAGp998P03372 ; IMAGE:195698, mRNA sequence
<400> 166
   ttttttttta gagttttaga ggaatataat gtatgaaaga aaattccaat aacataatgt 60
   acaaaaatgt tgtcacatac agaagagcaa aaatctacgt attggggact attgctgtgg 120
   gaagaagggc ctgatcttca tactcatctt cctcattgag taccatgacc ccttccaatt 180
   catccatact acaccatcat ctgtgccatg ctttgccatg tcccaggtgt actgtccacc 240
   ccagtagtat ctgccgtttg gattggctgc atgacatcta ttataccacc atccaccacc 300
   gtcttcttta gaacactgtt ttctgggatc tgatgttaac ctgaaagaaa atgtgtttgg 360
   cgttattaga aacagaacta cggtggtcaa gttacgtttt gcagattacc cataaaagac 420
   tgctacactc gtgtgcacag tagtctttcc tttgggtgct atacatagag tcacactaat 480
   acaccaaaga cctcctgtgc cctatagcaa gaaagagaga taactgggcc tttgtggcca 540
   atccatctag ggactgtgtt tgttacttta tactggaata atagctttta caacccttag 600
   tatcagagaa ccctggaaat gaaaatgaaa gttttcacac acactttaga atatagagat 660
   ataggtattt atctaaatgc tcattattca gaaagtaact gtgctcaaat tctagt 716
<210> 167
   <211> 747
   <212> DNA
   <213> unknown
<220>
   <223> >sr|IMAGp998B071870.3|cl|IMAGp998B0718|gb|BX0956 83|oid|234525737 BX095683 Soares_total_fetus_Nb2HF8_9w Homo sapiens cDNA clone ÏMAGp998B071870 ; IMAGE:760230, mRNA sequence
<221> misc_feature
   <222> 737
   <223> n=a, t, c, or g
<400> 167
   ccgcatccta gccgccgact cacacaaggc aggtgggtga ggaaatccag agttgccatg 60
   gagaaaattc cagtgtcagc attcttgctc cttgtggccc tctcctacac tctggccaga 120
   gataccacag tcaaacctgg agccaaaaag gacacaaagg actctcgacc caaactgccc 180
   cagaccctct ccagaggttg gggtgaccaa ctcatctgga ctcagacata tgaagaagct 240
   ctatataaat ccaagacaag caacaaaccc ttgatgatta ttcatcactt ggatgagtgc 300
   ccacacagtc aagctttaaa gaaagtgttt gctgaaaata aagaaatcca gaaattggca 360
   gagcagtttg tcctcctcaa tctggtttat gaaacaactg acaaacacct ttctcctgat 420
   ggccagtatg tccccaggat tatgtttgtt gacccatctc tgacagttag agccgatatc 480
   actggaagat attcaaatcg tctctatgct tacgaacctg cagatacagc tctgttgctt 540
   gacaacatga agaaagctct caagttgctg aagactgaat tgtaaagaaa aaaaatctcc 600
   aagcccttct gtctgtcagg ccttgagact tgaaaccaga agaagtgtga gaagactggc 660
   tagtgtggaa gcatagtgaa cacactgatt aggttatggt ttaatgttac aacaactatt 720
   ttttaagaaa aacaagnttt agaaatt 747
<210> 168
   <211> 435
   <212> DNA
   <213> unknown
<220>
   <223> >gi|1425401|sr|IMAGp998D08822|cl|IMAGp998D08822|gb |W95494|W95494 ze02h04.s1 Soares fetal heart NbHH19W Homo sapiens cDNA clone 357847 3'
<221> misc_feature
   <222> 387, 432
   <223> n=a, t, c, or g
<400> 168
   taatatgcaa accatttatt gaactattta ctattttaaa atatcattct taaaagaggc 60
   cacaaaaatt ataacattca gaaattttag gtaaactcca aaagtaagct tttttttttt 120
   ttggtagtca acttgtacca agtttagcag caagaggata cttccttaga gactttcagt 180
   ggacttaaac tcagtttccg ctggtgctat gtaaagcatc cacgatggtt ttattgtact 240
   ctgcaatctg cttggtcaca tttttcttaa ttggctggta atcactctct tgactcttgg 300
   ttgctatgaa ttctttcatc gcaaaattat tttggctcaa ggtgttgcca ctttctctcc 360
   aaatttgtaa gctgagaatg gtggtcncat tttcttggca attgtgtttt tagtggcctc 420
   atactctatg tnttt 435
<210> 169
   <211> 410
   <212> DNA
   <213> unknown
<220>
   <223> >gi|1423675|sr|IMAGp998I08822|cl|IMAGp998I08822|gb |W94545|W94545 ze04b04.s1 Soares fetal heart NbHH19W Homo sapiens cDNA clone 357967 3'
<400> 169
   cccaacgttt tcaagtttta tttttaaaat atattacaac atcacattaa aaacagtaga 60
   ggaaaacaag caaacatgat agaattactt ttttccaact caatagtact tagctttacc 120
   tagaattttc atacagttga tgctaacttc ttctgaaccc tttctgacta gtattttacc 180
   ttatgtattc tttatagtat ttccatataa tctttaccga gacctattca gagcataaca 240
   tagacagggc agcacaagca actgacacct ttgttggtac tacagtgttt tttcaaccag 300
   tttatgagag aatgaaaaga atgaaagtga aaattttact taacttaggg ttatgtagct 360
   ctaggtaatc tcttcagaac tcccctggct tcaccttctc aagttaatca 410
<210> 170
   <211> 132
   <212> DNA
   <213> unknown
<220>
   <223> >ImageID=357848 IMAGP971CO935_S1 CC FromEXP of IMAGp971C0935_s1.exp April 26, 2002 12:43
<221> misc feature
   <222> 15, 33, 73, 107, 123, 127
   <223> n=a, t, c, or g
<400> 170
   caagcatcac cctgngaggt tcctgaggga ttnctcataa atgagggctg cacattgcct 60
   gttctgcttc gangtattca ataccgctca gtattttaaa tgaagcngat tctaagattt 120
   ggntggngat ca 132
<210> 171
   <211> 390
   <212> DNA
   <213> unknown
<220>
   <223> >sr|IMAGp998K045001.2|cl|IMAGp998K045001|gb|BX1091 64|oid|234552707 BX109164 Barstead prostate BPH HPLRB4 1 Homo sapiens cDNA clone IMAGp998K045001 ; IMAGE:2032251, mRNA sequence.
<400> 171
   atccacccag tctctcaaga ccaggaacga gaaaaaagaa gtatcagtga cagcgatgaa 60
   ttagcttcag ggttttttgt gttcccttac ccatatccat ttcgcccact tccaccaatt 120
   ccatttccaa gatttccatg gtttagacgt aattttccta ttccaatacc tgaatctgcc 180
   cctacaactc cccttcctag cgaaaagtaa acaagaagga aaagtcacga taaacctggt 240
   cacctgaaat tgaaattgag ccacttcctt gaagaatcaa aattcctgtt aataaaagaa 300
   aaacaaatgt aattgaaata gcacacagca ttctctagtc aatatcttta gtgatcttct 360
   ttaataaaca tgaaagcaaa aaaaaaaaaa 390
<210> 172
   <211> 322
   <212> DNA
   <213> unknown
<220>
   <223> >gi|5366932|gb|AI801460|sr|IMAGp998K125400.1|cl|IM AGp998K125400|AI801460 to89f06.xl NCI CGAP Gas4 Homo sapiens cDNA clone IMAGE:2185475 3' similar to contains element MER22 repetitive element ;, mRNA sequence
<221> misc_feature
   <222> 278, 279
   <223> n=a, t, c, or g
<400> 172
   tttttttttt tttttttttt tttttttttt tttttttttt tttttttttt tttttttttt 60
   tttttttttt tttttttttt tttttttttt ttttaaaaaa aaaaggggcc ccctgggggg 120
   gaaacaaatt tggttccccc cccggccccc tgaaacccaa tttttaaaac tgggtttttt 180
   taaaaaaaag ggggggggcc caaaaaccca accccaaaag ggggggtgcc ttttgccccc 240
   cccccccccg gtttttcccc ctttttttgg gttaaaannc aagggccccc aaaaaaaagg 300
   ggggggggga tttttttttt tt 322
<210> 173
   <211> 460
   <212> DNA
   <213> unknown
<220>
   <223> >gi|2069924|gb|AA410759|sr|IMAGp998K151776.2|cl|IM AGp998K151776|AA410759 zt35e08.r1 Soares ovary tumor NbHOT Homo sapiens cDNA clone IMAGE:724358 5' similar to gb:Y00503 KERATIN, TYPE I CYTOSKELETAL 19 (HUMAN);, mRNA sequence
<400> 173
   cccttggacc ataaattttt attggcaggt caggagaaga gccgggggta agggtccctt 60
   ccttcccatc cctctaccca gaagacaccc tccaaaggac agcagaagcc ccagagcctg 120
   ctgcctcaga ggaccttgga ggcagacaaa ttgttgtagt gatcttcctg tccctcgagc 180
   aggctgcggt aggtggcaat ctcctgctcc agccgcgact tgatgtccat gagccgctgg 240
   tactcctgat tctgccgctc actatcagct cgcacatcgc ccagctgggc ttcaataccg 300
   ctgatcagcg cctggatatg cgccacgtgg gctccaaagc gcgcctccgt ttctgccagt 360
   gtgtcttcca aggcagcttt catgctcagc tgtgactgca gctcaatctc aagaccctga 420
   agggtgcgcc gcaggtcagt aacctcggac ctgctcatct 460
<210> 174
   <211> 797
   <212> DNA
   <213> unknown
<220>
   <223> >gi|5054911|gb|AI733798|sr|IMAGp998I164581.2|cl|IM AGp998I164581|AI733798 qk35b08.x5 NCI_CGAP_Co8 Homo sapiens cDNA clone Image:1870935 3' similar to gb:X02761_cdsl FIBRONECTIN PRECURSOR (HUMAN);, mRNA sequence
<221> misc_feature
   <222> 621, 665
   <223> n=a, t, c, or g
<400> 174
   tttttttttt ggggaatgga aatcttttat taaaacagtt gtctttccac agtagtaaag 60
   ctttggcaca tacagtataa aaaataatca cccaccataa ttataccaaa ttcctcttat 120
   caactgcata ctaagtgttt tcaatacaat tttttccgta taaaaatact gggaaaaatt 180
   gataaataac aggtaagaga aagatatttc taggcaatta ctaggatcat ttggaaaaag 240
   tgagtactgt ggatatttaa aatatcacag taacaagatc atgcttgttc ctacagtatt 300
   gcgggccaga cacttaagtg aaagcagaag tgtttgggtg actttcctac ttaaattttt 360
   ggtcatatca tttcaaaaca tttgcatctt ggttggctgc atatgctttc ctattgatcc 420
   caaaccaaat cttagaatca cttcatttaa aatactgagc ggtattgaat acttcgaagc 480
   agaacaggca atgtgcagcc ctcatttatg agaaaaccct caggaaactc ccagggtgat 540
   gcttggagaa gctgtgagtg agctgaagct ggagaacttc ctccagagca aagggcttaa 600
   gaaagaaaga agaactccta ngctggtctg ctaccattca ctcagtttaa aatgatcctg 660
   caganagaca atcttgtcct tttggaattg gaaagagatt acctctcgga atcctctctg 720
   tcagcctgac atctaaaggc atgaagcact caatttggca ataaaacaaa tagggttggt 780
   ctctgatgga tctctga                                                797
<210> 175
   <211> 408
   <212> DNA
   <213> unknown
<220>
   <223> >sr|IMAGp998F044825.2|cl|IMAGp998F044825|gb|BX1062 84|oid|234546949 BX106284 NCI_CGAP_Ov23 Homo sapiens cDNA clone IMAGp998F044825 : IMAGE:1964547, mRNA sequence

<221> misc_feature
   <222> 81, 254, 338, 378, 403
   <223> n=a, t, c, or g
<400> 175
   tttttttttt ttttaaacat cagattttct atttttatta aaaactcaca aatttattca 60
   acatgttttc tttcatacag ngaatggtct aatatgcact ggaggtcaca caagcttagg 120
   tttattagaa caataaaaga catatgagaa atttaatata taaagaaaaa gtagcagctg 180
   ttgactgcat atttgaccat aaaatttaaa tattttggac ttttatttta aagacacaaa 240
   aataaaacct gtgngggtct atataagtca tattaacaat tccatgaatg ttcaacagga 300
   caaaaaaatt agcaaagatg ttttttttta aatcttgnaa cacttttttt tttttttttt 360
   tttaacactt tctcaggntg ctggggccag gcccctttac agnatttg          408
<210> 176
   <211> 293
   <212> DNA
   <213> unknown
<220>
   <223> >gi|5593910|gb|AI888746|sr|IMAGp998L206082.1|cl|IM AGp998L206082|AI888746 wn22h10.x1 NCI_CGAP_Gas4 Homo sapiens cDNA clone IMAGE:2446243 3' similar to contains element MSR1 repetitive element ;, mRNA sequence.
<400> 176
   tttttttttt tttttttttt tttttttttt tttttttttt tttttttttt tgggtttttt 60
   ttttttttaa aaaagggctt aaatttaaaa ttctcaaaaa aaacaaaaaa aaaaaaaccc 120
   cttgggtcca agccaaaacc ccgggaaaac ccccctaaaa cagggcgggg ggccctacaa 180
   aaaaaacaaa aaagggccaa gggaaattta aaacatttaa atccttaaag gtccaccagg 240
   aaaaaggggg cccccccccc gggaaatcat tttaaaaaaa aacaccgggc att 293
<210> 177
   <211> 404
   <212> DNA
   <213> unknown
<220>
   <223> >sr|IMAGp998D085368.2|cl|IMAGp998D085368|gb|BX1070 55|oid|234548491 BX107055 Barstead colon HPLRB7 Homo sapiens cDNA clone IMAGp998D085368 ; IMAGE:2173015, mRNA sequence.
<221> misc_feature
   <222> 9
   <223> n=a, t, c, or g
<400> 177
   ctagtaatna ctagtaatat tactagtgaa ttcactagta atgccagagc caacctggac 60
   ttcctgtcat tttcacaatc ttggggctga tgaagaaggg ggtgggggga gatctacagg 120
   agctcagcat ggcatctgtg atattcctgc ccaacataca tgacagaatc caatcatgga 180
   aaagcaccaa aaaacccaag ttgaaggaca agtctaccaa gtagctggcc aggtaatctt 240
   cacaaaggtc cgtgtcatga aagacaaagc agcagaaact ttcaaattag aggggacagc 300
   taaatgcaac acatggtctt ggattgtttt gttataaagg acattatcat gaagtctgaa 360
   taaagtctgt agatgagatg agagaattcc ctgtttttag gaaa 404
<210> 178
   <211> 270
   <212> DNA
   <213> unknown
<220>
   <223> >sr|IMAGp998D20611.1|cl|IMAGp998D20611|gb|BX115533 |oid|234565441 BX115533 Soares_multiple_sclerosis_2NbHMSP Homo sapiens cDNA clone IMAGp998D20611 ; IMAGE:276835, mRNA sequence
<221> misc_feature
   <222> 267, 270
   <223> n=a, t, c, or g
<400> 178
   atattaacat aattatgttg gagagaagaa aaacccacac ttagaagtca ggtttctttt 60
   aaaactcctt gttacaaata gaatcaatga ttatatctca gacataataa tgggtcaaac 120
   tcacaaaatc agtgctacaa aatacagtat ttttccatca cttttagttg taatgtacac 180
   tatgtacaga aagataatac tacctatttt aaactaaata tagatgaaca aacagcttgt 240
   taaaaaccag aatcagggct tataaanagn 270
<210> 179
   <211> 669
   <212> DNA
   <213> unknown
<220>
   <223> >sr|IMAGp998B14742.3|cl|IMAGp998B14742|gb|BX100503 |oid|234535382 BX100503 Soares_fetal_heart_NbHH19W Homo sapiens cDNA clone IMAGp998B14742 ; IMAGE:327085, mRNA sequence
<221> misc_feature
   <222> 558
   <223> n=a, t, c, or g
<400> 179
   tagtgttttc ttaaaataag actgatttaa agcttatcta gcagatagtt ttaaacacag 60
   attaaagaaa actagattgg gtacaaatgt ttggcttccc aagtgagagg ccacatgtac 120
   atcaacagta ctttccaact taacactacg acccccattt tgatggtgac agaattggaa 180
   acgggggaca gtgatgagag catcaccctc ctgtcactta cagatggtcc cttgggactg 240
   gatttttcct gggagaccat tgaccccaag aaccctgaaa acatgatatg cggtagcaga 300
   atggattaga ttgctgttag ctttctggta ggtgagggga cctgaaaaga acagggccac 360
   cagcatttca agggaacagc cattccaatt ttcagaggtt gattctcttg gaattatttg 420
   acctgaggcc ataggaaagc aaagggttaa aaaagtcacg tttgacatgc cagcctgggg 480
   agctggacat tggatacccg tgtgagcctc gtggaacacc ataatcaaat gtgaaaatta 540
   gagttgatta attaaagnaa aagaatttaa gtctttattt ctaaactgat gcatgaggac 600
   ccccagtgag caatattccc ccaaggcaaa tcaaccaaaa gcaaaactgt tttgaaaaaa 660
   aaaaaaaaa 669
<210> 180
   <211> 711
   <212> DNA
   <213> unknown
<220>
   <223> >sr|IMAGp998B07407.1|cl|IMAGp998B07407|gb|BX111875 |oid|234558127 BX111875 Soares fetal liver spleen 1NFLS Homo sapiens cDNA clone IMAGp998B07407 ; IMAGE:208806, mRNA sequence
<221> misc_feature
   <222> 676
   <223> n=a, t, c, or g
<400> 180
   ctcattcttg gcaggatggc ttctcatcgt ctgctcctcc tctgccttgc tggactggta 60
   tttgtgtctg aggctggccc tacgggcacc ggtgaatcca agtgtcctct gatggtcaaa 120
   gttctagatg ctgtccgagg cagtcctgcc atcaatgtgg ccgtgcatgt gttcagaaag 180
   gctgctgatg acacctggga gccatttgcc tctgggaaaa ccagtgagtc tggagagctg 240
   catgggctca caactgagga ggaatttgta gaagggatat acaaagtgga aatagacacc 300
   aaatcttact ggaaggcact tggcatctcc ccattccatg agcatgcaga ggtggtattc 360
   acagccaacg actccggccc ccgccgctac accattgccg ccctgctgag cccctactcc 420
   tattccacca cggctgtcgt caccaatccc aaggaatgag ggacttctcc tccagtggaa 480
   ctgaaggacg agggatggga tttcatgtaa ccaagagtat tccattttta ctaaagcagt 540
   gttttcacct catatgctat gttagaagtc caggcagaga caataaaaca ttcctgtgaa 600
   aggcactttt cattccactt taacttgatt ttttaaattc ccttattgtc ccttccaaaa 660
   aaaagagaat caaaanttta caaagaatca aaggaattct agaaagtatc t          711
<210> 181
   <211> 474
   <212> DNA
   <213> unknown
<220>
   <223> >sr|IMAGp998C16522.3|cl|IMAGp998C16522|gb|BX097461 |oid|234529295 BX097461 Soares fetal liver spleen 1NFLS Homo sapiens cDNA clone IMAGp998C16522 ; IMAGE:242631, mRNA sequence
<400> 181
   aggacagaga cgctggctag gccgccctcc ccactgttac caacatgaag ctgctcgcag 60
   caactgtgct actcctcacc atctgcagcc ttgaaggagc tttggttcgg agacaggcaa 120
   aggagccatg tgtggagagc ctggtttctc agtacttcca gaccgtgact gactatggca 180
   aggacctgat ggagaaggtc aagagcccag agcttcaggc cgaggccaag tcttactttg 240
   aaaagtcaaa ggagcagctg acacccctga tcaagaaggc tggaacggaa ctggttaact 300
   tcttgagcta tttcgtggaa cttggaacac agcctgccac ccagtgaagt gtccagacca 360
   ttgtcttcca accccagctg gcctctagaa cacccactgg ccagtcctag agctcctgtc 420
   cctacccact ctttgctaca ataaatgctg aatgaatcca aaaaaaaaaa aaaa       474
<210> 182
   <211> 462
   <212> DNA
   <213> unknown
<220>
   <223> >gi|4739393|gb|AI655414|sr|IMAGp998O055540.1|cl|IM AGp998O055540|AI655414 ts98e03.x1 NCI_CGAP_GC6 Homo sapiens cDNA clone IMAGE:2239324 3' similar to gb:S70057 GASTRIN/CHOLECYSTOKININ TYPE B RECEPTOR (HUMAN);, mRNA sequence.
<400> 182
   ttcaggagga agccaaacaa tttttatttc ttgaacagtt tgggtaagga aggagagggc 60
   agggccacgg tccttaactg gaaagggatg aagaaccttt tcagtgcaga acagcctgtt 120
   ggtcagaggt atgagattag gcctgacggt attttcagtg ctgattggga agggtcactg 180
   tgaggtcaaa ctaggagcat cccagagtca gtcctgtgcc aggctccata gtctctaaaa 240
   caatcagtgc cattaatcta tgtgtgtgag aggcaggtca gcccttgtca gagttcccac 300
   tgtgtagaaa tagttcactg ctagggacgc tgtgtctctc caaggtcagt gtcatgtttc 360
   tatgggccag gctcagtccc atatcagagg catgcctcct ttcccatgta ctgctccatt 420
   cttattcctc ttgtgtcagg taagctacct tttcctgtgc gt                    462
<210> 183
   <211> 466
   <212> DNA
   <213> unknown
<220>
   <223> >sr|IMAGp998A244944.2|cl|IMAGp998A244944|gb|BX0909 80|oid|234516327 BX090980 NCI_CGAP_GC6 Homo sapiens cDNA clone IMAGp998A244944 ; IMAGE:2010143, mRNA sequence
<400> 183
   atggcttaca tattcaaaag gttggatttg ggaagcaatg ctaagcagtg gaatggacat 60
   cgacatagag agatcagctc cacacttata ctctgccact caacttcccc atgtgacttg 120
   aggatcactc taactccaaa acatagcaag ctcgcggaac atcagggttc atgcaaagta 180
   ttccaaggag ccccttgaag caacagaatg gattgctctt ctatggtgga atggcaccct 240
   ggatgattaa aaccgtagca gcaaacaaaa cctccatcaa gtaagaattc agagtgtgag 300
   atatcacgca cagccacgcg tggatcttta tatacgtgtc aatgtgtttg attgtatttt 360
   tgctttcaaa gtatgtattg agcatttctt ctaggtcctc agtaacatct ttttttaaaa 420
   aaaataaatg cttagggaat tgttttatat taaaaaaaaa aaaaaa 466
<210> 184
   <211> 16966
   <212> DNA
   <213> unknown
<220>
   <223> >Homosapiens chromosome:NCBI34:4:74736257:74753222:1
<400> 184
   agcttttctc ttctgtcaac cccacacgcc tttggcacaa tgaagtgggt aacctttatt 60
   tcccttcttt ttctctttag ctcggcttat tccaggggtg tgtttcgtcg agatgcacgt 120
   aagaaatcca tttttctatt gttcaacttt tattctattt tcccagtaaa ataaagtttt 180
   agtaaactct gcatctttaa agaattattt tggcatttat ttctaaaatg gcatagtatt 240
   ttgtatttgt gaagtcttac aaggttatct tattaataaa attcaaacat cctaggtaaa 300
   aaaaaaaaaa ggtcagaatt gtttagtgac tgtaattttc ttttgcgcac taaggaaagt 360
   gcaaagtaac ttagagtgac tgaaacttca cagaataggg ttgaagattg aattcataac 420
   tatcccaaag acctatccat tgcactatgc tttatttaaa aaccacaaaa cctgtgctgt 480
   tgatctcata aatagaactt gtatttatat ttattttcat tttagtctgt cttcttggtt 540
   gctgttgata gacactaaaa gagtattaga tattatctaa gtttgaatat aaggctataa 600
   atatttaata atttttaaaa tagtattctt ggtaattgaa ttattcttct gtttaaaggc 660
   agaagaaata attgaacatc atcctgagtt tttctgtagg aatcagagcc caatattttg 720
   aaacaaatgc ataatctaag tcaaatggaa agaaatataa aaagtaacat tattacttct 780
   tgttttcttc agtatttaac aatccttttt tttcttccct tgcccagaca agagtgaggt 840
   tgctcatcgg tttaaagatt tgggagaaga aaatttcaaa gccttgtaag ttaaaatatt 900
   gatgaatcaa atttaatgtt tctaatagtg ttgtttatta ttctaaagtg cttatatttc 960
   cttgtcatca gggttcagat tctaaaacag tgctgcctcg tagagttttc tgcgttgagg 1020
   aagatattct gtatctgggc tatccaataa ggtagtcact ggtcacatgg ctattgagta 1080
   cttcaaatat gacaagtgca actgagaaac aaaaacttaa attgtattta attgtagtta 1140
   atttgaatgt atatagtcac atgtggctaa tggctactgt attggacagt acagctctgg 1200
   aacttgcttg gtggaaagga ctttaatata ggtttccttt ggtggcttac ccactaaatc 1260
   ttctttacat agcaagcatt cctgtgctta gttgggaata tttaattttt tttttttttt 1320
   aagacagggt ctcgctctgt cgcccaggct ggagtgcagt ggcgcaatct cggctcactg 1380
   caaactccgc ctcccgggtt cacgccattc tcctgcctca gcctcccgag tagctgggac 1440
   tacaggcgcc cgccatcacg cccggctaat cttttgtatt tttagtagag atggggtttc 1500
   accgtgtgcc aggatggtct caatctcctg acatcgtgat ctgcccacct cggcctccca 1560
   aagtgctggg attacaggag tgagccaccg cgcccggcct atttaaatgt tttttaatct 1620
   agtaaaaaat gagaaaattg tttttttaaa agtctaccta atcctacagg ctaattaaag 1680
   acgtgtgtgg ggatcaggtg cggtggttca cacctgtaat cccagcactt tggaaggctg 1740
   atgcaggagg attgcttgag cccaggagtt caagaccagc ctgggcaagt ctctttaaaa 1800
   aaaacaaaac aaacaaacaa aaaaattagg catggtggca catgcctgta gtcctagcta 1860
   cttaggaggc tgacgtagga ggatcgtttg gacctgagag gtcaaggcta cagtgagcca 1920
   tgattgtgcc actgcactcc agcctgggtg acagagtgag actctgtctc aaaaaagaaa 1980
   aaggaaatct gtggggtttg ttttagtttt aagtaattct aaggacttta aaaatgccta 2040
   gtcttgacaa ttagatctat ttggcataca atttgcttgc ttaatctatg tgtgtgcata 2100
   gatctactga cacacgcata catataaaca ttagggaact accattctct ttgcgtagga 2160
   agccacatat gcctatctag gcctcagatc atacctgata tgaataggct ttctggataa 2220
   tggtgaagaa gatgtataaa agatagaacc tatacccata catgatttgt tctctagcgt 2280
   agcaacctgt tacatattaa agttttatta tactacattt ttctacatcc tttgtttcag 2340
   ggtgttgatt gcctttgctc agtatcttca gcagtgtcca tttgaagatc atgtaaaatt 2400
   agtgaatgaa gtaactgaat ttgcaaaaac atgtgttgct gatgagtcag ctgaaaattg 2460
   tgacaaatca cttgtaagta cattctaatt gtggagattc tttcttctgt ttgaagtaat 2520
   cccaagcatt tcaaaggaat tttttttaag ttttctcaat tattattaag tgtcctgatt 2580
   tgtaagaaac actaaaaagt tgctcataga ctgataagcc attgtttctt ttgtgataga 2640
   gatgctttag ctatgtccac agttttaaaa tcatttcttt attgagacca aacacaacag 2700
   tcatggtgta tttaaatggc aatttgtcat ttataaacac ctctttttaa aatttgaggt 2760
   ttggtttctt tttgtagagg ctaataggga tatgatagca tgtatttatt tatttattta 2820
   tcttatttta ttatagtaag aacccttaac atgagatcta ccctgttata tttttaagtg 2880
   tacaatccat tattgttaac tacgggtaca ctgttgtata gcttactcat cttgctgtat 2940
   taaaactttg tgcccattga ttagtaaccc ctcgtttcgt cctcccccag ccactggcaa 3000
   ccagcattat actctttgat tctatgagtt tgactacttt agctacctta tataagtggt 3060
   attatgtact gtttatcttt ttatgactga cttatttccc ttagcatagt gcattcaaag 3120
   tccaaccatg ttgttgccta ttgcagaatt tccttctttt caaggctgaa taatattcca 3180
   gtgcatgtgt gtaccacatt ttctttatcc attaatttgt tgattgatag acatttaggt 3240
   tggttttcta catcttgact atcatgaata gtgttgcaat gaacacagga gagctactat 3300
   ctcttagaga tgatatcatg gtttttatca tcagaaaaca cccactgatt tctatgctaa 3360
   ttttgttacc tgggtggaat aatagtacag ctatatattc ctcattttag atatctttgt 3420
   atttctacat acaataaaaa agcagagtac ttagtcatgt tgaagaactt taaactttta 3480
   gtatttccag atcaatcttc aaaacaagga caggtttatc tttctctcac cactcaatct 3540
   atatatacct cttgtgggca aggccagttt ttatcactgg agcctttccc ctttttatta 3600
   tgtacctctc cctcacagca gagtcaggac tttaacttta cacaatacta tggctctaca 3660
   tatgaaatct taaaaataca taaaaattaa taaattctgt ctagagtagt atattttccc 3720
   tggggttaca gttactttca taataaaaat tagagataag gaaaggactc atttattgga 3780
   aagtgatttt aggtaacatt tctggaagaa aaatgtctat atcttaatag tcacttaata 3840
   tatgatggat tgtgttactc ctcagttttc aatggcatat actaaaacat ggccctctaa 3900
   aaagggggca aatgaaatga gaaactctct gaatgttttt ctcccctagg tgaattcacc 3960
   tgctgcttag aagcttattt tctcttgatt tctgttataa tgattgctct taccctttag 4020
   ttttaagttt caaaatagga gtcatataac tttccttaaa gctattgact gtctttttgt 4080
   cctgttttat tcaccatgag ttatagtgtg acagttaatt cttatgaaaa ttatatagag 4140
   atggttaaat catcagaaac tgtaaacctc gattgggagg ggaagcggat ttttaaatga 4200
   tttcctgacc aagcttaacc agtatattaa atcctttgta ctgttctttg gctataaaga 4260
   aaaaaggtac tgtccagcaa ctgaaacctg ctttcttcca tttagcatac cctttttgga 4320
   gacaaattat gcacagttgc aactcttcgt gaaacctatg gtgaaatggc tgactgctgt 4380
   gcaaaacaag aacctgagag aaatgaatgc ttcttgcaac acaaagatga caacccaaac 4440
   ctcccccgat tggtgagacc agaggttgat gtgatgtgca ctgcttttca tgacaatgaa 4500
   gagacatttt tgaaaaagta agtaatcaga tgtttatagt tcaaaattaa aaagcatgga 4560
   gtaactccat aggccaacac tctataaaaa ttaccataac aaaaatattt tcaacattaa 4620
   gacttggaag ttttgttatg atgatttttt aaagaagtag tatttgatac cacaaaattc 4680
   tacacagcaa aaaatatgat caaagatatt ttgaagttta ttgaaacagg atacaatctt 4740
   tctgaaaaat ttaagataga caaattattt aatgtattac gaagatatgt atatatggtt 4800
   gttataattg atttcgtttt agtcagcaac attatattgc caaaatttaa ccatttatgc 4860
   acacacacac acacacacac acacttaacc cttttttcca catacttaaa gaatgacaga 4920
   gacaagacca tcatgtgcaa attgagctta attggttaat tagatatctt tggaatttgg 4980
   aggttctggg gagaatgtcg attacaatta tttctgtaat attgtctgct atagaaaagt 5040
   gactgttttt ctttttcaaa atttagatac ttatatgaaa ttgccagaag acatccttac 5100
   ttttatgccc cggaactcct tttctttgct aaaaggtata aagctgcttt tacagaatgt 5160
   tgccaagctg ctgataaagc tgcctgcctg ttgccaaagg tattatgcaa aagaatagaa 5220
   aaaaagagtt cattatccaa cctgattttg tccattttgt ggctagattt agggaacctg 5280
   agtgtctgat acaaactttc cgacatggtc aaaaaagcct tccttttatc tgtcttgaaa 5340
   atctttcatc tttgaaggcc tacactctcg tttcttcttt taagatttgc caatgatgat 5400
   ctgtcagagg taatcactgt gcatgtgttt aaagatttca ccacttttta tggtggtgat 5460
   cactatagtg aaatactgaa acttgtttgt caaattgcac agcaaggggc cacagttctt 5520
   gtttatcttt tcatgataat ttttagtagg gagggaattc aaagtagaga attttactgc 5580
   atctagatgc ctgagttcat gcattcattc cataaatata tattatggaa tgctttattt 5640
   tcttttctga ggagtttact gatgttggtg gaggagagac tgaaatgaat tatacacaaa 5700
   atttaaaaat tagcaaaatt gcagcccctg ggatattagc gtactctttc tctgactttt 5760
   ctcccacttt taaggctctt tttcctggca atgtttccag ttggtttcta actacatagg 5820
   gaattccgct gtgaccagaa tgatcgaatg atctttcctt ttcttagaga gcaaaatcat 5880
   tattcgctaa agggagtact tgggaattta ggcataaatt atgccttcaa aatttaattt 5940
   ggcacagtct catctgagct tatggagggg tgtttcatgt agaatttttc ttctaatttt 6000
   catcaaatta ttcctttttg tagctcgatg aacttcggga tgaagggaag gcttcgtctg 6060
   ccaaacagag actcaagtgt gccagtctcc aaaaatttgg agaaagagct ttcaaagcat 6120
   ggtaaatact tttaaacata gttggcatct ttataacgat gtaaatgata atgcttcagt 6180
   gacaaattgt acatttttat gtattttgca aagtgctgtc aaatacattt ctttggttgt 6240
   ctaacaggta gaactctaat agaggtaaaa atcagaatat caatgacaat ttgacattat 6300
   ttttaatctt ttcttttcta aatagttgaa taatttagag gacgctgtcc tttttgtcct 6360
   aaaaaaaggg acagatattt aagttctatt tatttataaa atcttggact cttattctaa 6420
   tggttcatta tttttataga gctgtaggca tggttcttta tttaattttt taaagttatt 6480
   tttaattttt gtggatacag agtaggtata catatttacg ggtatatgag gatattttga 6540
   tataagtata caacatatat aatcccttta tttaatttta tcttcccccc aatgatctaa 6600
   aactatttgc ttgtcctttt atgtcttata gttaaattca gtcaccaact aagttgaagt 6660
   tacttcttat ttttgcatag ctccagctct gatcttcatc tcatgttttt gcctgagcct 6720
   ctgttttcat attacttagt tggttctggg agcatacttt aatagccgag tcaagaaaaa 6780
   tactagctgc cccgtcaccc acactcctca cctgctagtc aacagcaaat caacacaaca 6840
   ggaaataaaa tgaaaataat agacattatg catgctctct agaaactgtc aattgaactg 6900
   tatttgctca tcattcctac catctacacc accaaaatca accaaattta tgaaaaaaaa 6960
   cagccccaac ataaaattat acacagataa acaggctatg attggttttg ggaaagaagt 7020
   cacctttacc tgatttaggc aactgtgaaa tgactagaga atgaagaaaa ttagacgttt 7080
   acatcttgtc atagagtttg aagatagtgc tggatctttc tttttataag taagatcaat 7140
   aaaaactccc tcattctgta gaagttatga tttcttttct aagagacctt tagaagtcag 7200
   aaaaaatgtg tttcaattga gaaaaaagat aactggagtt tgtgtagtac ttcccagatt 7260
   ataaaatgct tttgtatgta ttatctaatt taatcctcaa aacttcttca atttagcatg 7320
   ttgtcatgac actgcagagg ctgaagctca gagaggctga gccctctgct aacaagtcct 7380
   actgctaaca agtgataaag ccagagctgg aagtcacatc tggactccaa acctgatgct 7440
   tctcagcctg ttgccccttt tagagttcct ttttaatttc tgcttttatg acttgctaga 7500
   tttctaccta ccacacacac tcttaaatgg ataattctgc cctaaggata agtgattacc 7560
   atttggttca gaactagaac taatgaattt taaaaattat ttctgtatgt ccattttgaa 7620
   ttttcttatg agaaatagta tttgcctagt gttttcatat aaaatatcgc atgataatac 7680
   cattttgatt ggcgattttc tttttagggc agtagctcgc ctgagccaga gatttcccaa 7740
   agctgagttt gcagaagttt ccaagttagt gacagatctt accaaagtcc acacggaatg 7800
   ctgccatgga gatctgcttg aatgtgctga tgacagggta aagagtcgtc gatatgcttt 7860
   ttggtagctt gcatgctcaa gttggtagaa tggatgcgtt tggtatcatt ggtgatagct 7920
   gacagtgggt tgagattgtc ttctgtgctt tcgtctgtcc tatcttcaat ctttccctgc 7980
   ctatggtggt ggtacctttc tgtttttaac ctggctataa attaccagat aaacccattc 8040
   actgatttgt aactcctttc agtcatgctc taactgtaaa tgaaggctta aactgaagta 8100
   gaacagttac aaggttttac ttggcagaac atcttgcaag gtagatgtct aagaagattt 8160
   ttttttcttt ttttaagaca gagtttcgct cttgtttccc aggctggggt gcaatggtgt 8220
   gatcttggct cagcgcaacc tctgcctcct gggttcaagt gattctcatg cctcagcctc 8280
   ccaagtagct gggattacag gcatgcgcca ccacacctgg ctaattttgt atttttagta 8340
   gaggcggggt ttcaccatat tgtccagact ggtctcgaac tcctgacctc aggtgatcca 8400
   cccgccttgg cctcccaaag tgctgggatt acaggcatga gccaccttgc ccagcctaag 8460
   aagatttttt gagggaggta ggtggacttg gagaaggtca ctacttgaag agatttttgg 8520
   aaatgatgta tttttcttct ctatattcct tcccttaatt aactctgttt gttagatgtg 8580
   caaatatttg gaatgatatc tcttttctca aaacttataa tattttcttt ctccctttct 8640
   tcaagattaa acttatgggc aaatactaga atcctaatct ctcatggcac tttctggaaa 8700
   atttaaggcg gttattttat atatgtaagc agggcctatg actatgatct tgactcattt 8760
   ttcaaaaatc ttctatattt tatttagtta tttggtttca aaaggcctgc acttaatttt 8820
   gggggattat ttggaaaaac agcattgagt tttaatgaaa aaaacttaaa tgccctaaca 8880
   gtagaaacat aaaattaata aataactgag ctgagcacct gctactgatt agtctatttt 8940
   aattaagtgg gaatgttttt gtagtcctat ctacatctcc aggtttagga gcaaacagag 9000
   tatgttcata gaaggaatat gtgtatggtc ttagaataca atgaatatgt tctgccaact 9060
   taataaaggt ctgaggagaa agtgtagcaa tgtcaattcg tgttgaacaa tttccaccaa 9120
   cttacttata ggcggacctt gccaagtata tctgtgaaaa tcaagattcg atctccagta 9180
   aactgaagga atgctgtgaa aaacctctgt tggaaaaatc ccactgcatt gccgaagtgg 9240
   aaaatgatga gatgcctgct gacttgcctt cattagctgc tgattttgtt gaaagtaagg 9300
   atgtttgcaa aaactatgct gaggcaaagg atgtcttcct gggcatgtaa gtagataaga 9360
   aattattctt ttatagcttt ggcatgacct cacaacttag gaggatagcc taggcttttc 9420
   tgtggagttg ctacaatttc cctgctgccc agaatgtttc ttcatccttc cctttcccag 9480
   gctttaacaa tttttgaaat agttaattag ttgaatacat tgtcataaaa taatacatgt 9540
   tcatggcaaa gctcaacatt ccttactcct taggggtatt tctgaaaata cgtctagaaa 9600
   cattttgtgt atatataaat tatgtatact tcagtcattc attccaagtg tatttcttga 9660
   acatctataa tatatgtgtg tgactatgta ttgcctgtct atctaactaa tctaatctaa 9720
   tctagtctat ctatctaatc tatgcaatga tagcaaagaa gtataaaaag aaatatagag 9780
   tctgacacca ggtgctttat atttggtgaa aagaccagaa gttcagtata atggcaatat 9840
   ggtaggcaac tcaattacaa aataaatgtt tacatattgt cagaagttgt ggtgataaac 9900
   tgcatttttg ttgttggatt atgataatgc actaaataat atttcctaaa attatgtacc 9960
   ctacaagatt tcactcatac agagaagaaa gagaatattt taagaacata tctctgccca 10020
   tctatttatc agaatccttt tgagatgtag tttaaatcaa acaaaatgtt aataaaaata 10080
   acaagtatca ttcatcaaag acttcatatg tgccaagcag tgtgtgcttt gtgtagatta 10140
   tgtcatatag ttctcataat ccaccttccg agacagatac tatttatttt ttgagacaga 10200
   gttttactct tgttgcccag gctggagtgc aatggtgcca tctcggctca ccacaacctc 10260
   cgcctcccag gttcaagcga ttctcctgcc tcagcctcct gggattacag gcatgcacca 10320
   ccatgcctgg ctaattttgt atttttagta gagatggggt ttcaccatgt tggtcagact 10380
   ggtctcaaac tcctgacctc tggtgatatg cctgcctcag cctcctaaag tgctgggatt 10440
   acaggcatga gccactgtgc ccagccgaca gatactatta ttatttccat tctaccgaga 10500
   aggagactaa ggctctgatc atttaaataa gttgcctaag gtgatgcagt gatataagta 10560
   gcagagctag gaattgagcc ttggtaactt taactctgga ccccaagtcc ttagctacta 10620
   agctttactg catggggttt agtcaaatta agacttttgg aatatgagtt acttttgaga 10680
   ttagctttgt gatatttttt gtgctcattt gtccaacaaa gtctatttta ttttcatctt 10740
   aattaggttt ttgtatgaat atgcaagaag gcatcctgat tactctgtcg tgctgctgct 10800
   gagacttgcc aagacatatg aaaccactct agagaagtgc tgtgccgctg cagatcctca 10860
   tgaatgctat gccaaagtgg taggtttatt gttggaaaaa aatgtagttc tttgactgat 10920
   gattccaata atgagaaaga aaaataatgc aagaatgtaa aatgatatac agtgcaattt 10980
   agatcttttc ttgagatggt ttcaattctg gaatcttaaa catgaaagaa aaagtagcct 11040
   tagaatgatt aacaaaattt agactagtta gaatagaaag atctgaatag agcaatctct 11100
   aaaaaatttt gatctttttt tctctttttc acaatcctga gaacaaaaaa aaattaaatt 11160
   taaatgttaa ttagaagata tttaacttag atgtaaagtg agttaacctg attccaggat 11220
   taatcaagta ctagaattag tatcttatgg caaattatag aacctatccc tttagaatat 11280
   tttcaaatct ttttgaggat gtttaggaat agttttacaa gaaattaagt taggagagga 11340
   aatctgttct ggaggatttt tagggttccc actagcatat gtaatggttt ctgaactatt 11400
   cagaatcaga gaaaactcat ttttcctgct ttcaagaagc tactgtatgc caggcaccat 11460
   gcacaaacaa tgaccaacgt aaaatctctc attttggaga gcctggaatc taactggaaa 11520
   ggtgaactaa taataataat atgtacaatc atagccatca tttattaaac ttttattata 11580
   tgcaaggcac tgtttaattt cattagctta cctggtttac agagcagctc tatgagatga 11640
   gtgccatctt tgcccctatt ttagggataa ggattctgaa atgtggagat ggtaagtaaa 11700
   attgcacaac tgaagaatga gttacatgac ttggctcaaa tactggtcat tgaactccag 11760
   agcctgaata ttcttaacca cttacatgat gcaagctcac caaataaata gttcgaatgt 11820
   attgtgacag agcggcattg atattcatct attcatgtgg ctttgagtag gaagaagaaa 11880
   ggatatcatt ctgaccagag gggtgaaaaa caacctgcat ctgatcctga ggcataatac 11940
   tattaacaca attcttttat gtttcagttc gatgaattta aacctcttgt ggaagagcct 12000
   cagaatttaa tcaaacaaaa ttgtgagctt tttgagcagc ttggagagta caaattccag 12060
   aatgcgtaag taatttttat tgactgattt tttttatcaa tttgtaatta tttaagactt 12120
   aatatatgag ccacctagca tagaactttt aagaatgaaa atacattgca tatttctaat 12180
   cactctttgt caagaaagat aggagaggag agataaaata gttgatgggg tggagaggtc 12240
   tatatttgaa tgtagtctaa aaattgttct cttaagattg gaagtatgta ggctgggagg 12300
   gtaaatacca aatcttggta tatcagaact gagcatgtcc cttgaaggtt aagaaatagt 12360
   taatgggcaa atagagcatg gcaatatttt gtagagcagc aagtagtagg ccttgaatag 12420
   atgtcgctca aaaagtaata tgtaagctga acacaaaaat gtaacaaatg aatttagata 12480
   catatttgaa tattaaattc aggttgtttg ggagatgcac ctagtctttg atggttaaac 12540
   ctttccctcc atagaagaga cagagacaga atggcttgct ggactaatgt cccaattcaa 12600
   tagagtctta tctatgaagg ttaaaaacaa gaagagacat attatacagt agatatttat 12660
   tgtgtggctc atacacatgg tgctcttctg attatggatt ttagagataa taacagtgaa 12720
   caagacatag tttctttcct cgagtagatt aaagtcatac attgactttt aatggtgact 12780
   ggcattctta atacatgatt attatatatt aggtaccatg tcagattaat tataatactt 12840
   tactactttt aatttaaccc ttgaactatc cctattgagt cagatatatt tccttccatt 12900
   ttctacttgt atctttcaag tttagcatat gctgatacat atgaagctct ctccaggttt 12960
   tattgaaaga agaaattaat aaatttatta atgtcactga attaggcaac tcactttccc 13020
   aagattatgc aagtggtaca ggtggaactc aaagccaagt ttaactagtt gttcaggaga 13080
   atgttttcta ccctccacta acccactact ctgcagatgg agataatatg atgaatggaa 13140
   catagcaaca tcttagttga ttccggccaa gtgttctctg ttttatctac tatgttagac 13200
   agtttcttgc cttgctgaaa acacatgact tctttttttc aggctattag ttcgttacac 13260
   caagaaagta ccccaagtgt caactccaac tcttgtagag gtctcaagaa acctaggaaa 13320
   agtgggcagc aaatgttgta aacatcctga agcaaaaaga atgccctgtg cagaagacta 13380
   tgtgagtctt taaaaaaata taataaatta ataatgaaaa aattttacct ttagatattg 13440
   ataatgctag ctttcataag cagaaggaag taatgtgtgt gtgtgcatgt ttgtgtgcat 13500
   gtgtgtgtgc atgcacgtgt gtgtatgtgt gatattggca gtcaaggccc cgaggatgat 13560
   aatttttttt ttttttttga gacggagtct cgctttgttg tccaggctgg agtgcagtgg 13620
   tgccatctcg gctcactgca acctccgcct cccaggttca agccattctc ctgcctcagc 13680
   ctcccaagta gctgggacta caggtgcatg ccaccatgcc tggctaattt tttgtatttt 13740
   tagtagaaaa ttttcagctt cacctctttt gaatttctgc tctcctgcct gttctttagc 13800
   tatccgtggt cctgaaccag ttatgtgtgt tgcatgagaa aacgccagta agtgacagag 13860
   tcaccaaatg ctgcacagaa tccttggtga acaggcgacc atgcttttca gctctggaag 13920
   tcgatgaaac atacgttccc aaagagttta atgctgaaac attcaccttc catgcagata 13980
   tatgcacact ttctgagaag gagagacaaa tcaagaaaca aacgtgagga gtatttcatt 14040
   actgcatgtg tttgtagtct tgatagcaag aactgtcaat tcaagctagc aactttttcc 14100
   tgaagtagtg attatatttc ttagaggaaa gtattggagt gttgccctta ttatgctgat 14160
   aagagtaccc agaataaaat gaataacttt ttaaagacaa aatcctctgt tataatattg 14220
   ctaaaattat tcagagtaat attgtggatt aaagccacaa tagaataaca tgttaggcca 14280
   tattcagtag aaaaagatga acaattaact gataaatttg tgcacatggc aaattagtta 14340
   atgggaacca taggagaatt tatttctaga tgtaaataat tattttaagt ttgccctatg 14400
   gtggccccac acatgagaca aacccccaag atgtgacttt tgagaatgag acttggataa 14460
   aaaacatgta gaaatgcaag ccctgaagct caactcccta ttgctatcac aggggttata 14520
   attgcataaa atttagctat agaaagttgc tgtcatctct tgtgggctgt aatcatcgtc 14580
   taggcttaag agtaatattg caaaacctgt catgcccaca caaatctctc cctggcattg 14640
   ttgtctttgc agatgtcagt gaaagagaac cagcagctcc catgagtttg gatagcctta 14700
   ttttctatag cctccccact attagctttg aagggagcaa agtttaagaa ccaaatataa 14760
   agtttctcat ctttatagat gagaaaaatt ttaaataaag tccaagataa ttaaattttt 14820
   aaggatcatt tttagctctt taatagcaat aaaactcaat atgacataat atggcacttc 14880
   caaaatctga ataatatata attgcaatga catacttctt ttcagagatt tactgaaaag 14940
   aaatttgttg acactacata acgtgatgag tggtttatac tgattgtttc agttggtctt 15000
   cccaccaact ccatgaaagt ggattttatt atcctcatca tgcagatgag aatattgaga 15060
   cttatagcgg tatgcctgag ccccaaagta ctcagagttg cctggctcca agatttataa 15120
   tcttaaatga tgggactacc atccttactc tctccatttt tctatacgtg agtaatgttt 15180
   tttctgtttt ttttttttct ttttccattc aaactcagtg cacttgttga gctcgtgaaa 15240
   cacaagccca aggcaacaaa agagcaactg aaagctgtta tggatgattt cgcagctttt 15300
   gtagagaagt gctgcaaggc tgacgataag gagacctgct ttgccgagga ggtactacag 15360
   ttctcttcat tttaatatgt ccagtattca tttttgcatg tttggttagg ctagggctta 15420
   gggatttata tatcaaagga ggctttgtac atgtgggaca gggatcttat tttacaaaca 15480
   attgtcttac aaaatgaata aaacagcact ttgtttttat ctcctgctct attgtgccat 15540
   actgttaaat gtttataatg cctgttctgt ttccaaattt gtgatgctta tgaatattaa 15600
   taggaatatt tgtaaggcct gaaatatttt gatcatgaaa tcaaaacatt aatttattta 15660
   aacatttact tgaaatgtgg tggtttgtga tttagttgat tttataggct agtgggagaa 15720
   tttacattca aatgtctaaa tcacttaaaa ttgcccttta tggcctgaca gtaacttttt 15780
   tttattcatt tggggacaac tatgtccgtg agcttccgtc cagagattat agtagtaaat 15840
   tgtaattaaa ggatatgatg cacgtgaaat cactttgcaa tcatcaatag cttcataaat 15900
   gttaattttg tatcctaata gtaatgctaa tattttccta acatctgtca tgtctttgtg 15960
   ttcagggtaa aaaacttgtt gctgcaagtc aagctgcctt aggcttataa catcacattt 16020
   aaaagcatct caggtaacta tattttgaat tttttaaaaa agtaactata atagttatta 16080
   ttaaaatagc aaagattgac catttccaag agccatatag accagcaccg accactattc 16140
   taaactattt atgtatgtaa atattagctt ttaaaattct caaaatagtt gctgagttgg 16200
   gaaccactat tatttctatt ttgtagatga gaaaatgaag ataaacatca aagcatagat 16260
   taagtaattt tccaaagggt caaaattcaa aattgaaacc aaagtttcag tgttgcccat 16320
   tgtcctgttc tgacttatat gatgcggtac acagagccat ccaagtaagt gatggctcag 16380
   cagtggaata ctctgggaat taggctgaac cacatgaaag agtgctttat agggcaaaaa 16440
   cagttgaata tcagtgattt cacatggttc aacctaatag ttcaactcat cctttccatt 16500
   ggagaatatg atggatctac cttctgtgaa ctttatagtg aagaatctgc tattacattt 16560
   ccaatttgtc aacatgctga gctttaatag gacttatctt cttatgacaa catttattgg 16620
   tgtgtcccct tgcctagccc aacagaagaa ttcagcagcc gtaagtctag gacaggctta 16680
   aattgttttc actggtgtaa attgcagaaa gatgatctaa gtaatttggc atttatttta 16740
   ataggtttga aaaacacatg ccattttaca aataagactt atatttgtcc ttttgttttt 16800
   cagcctacca tgagaataag agaaagaaaa tgaagatcaa aagcttattc atctgttttt 16860
   ctttttcgtt ggtgtaaagc caacaccctg tctaaaaaac ataaatttct ttaatcattt 16920
   tgcctctttt ctctgtgctt caattaataa aaaatggaaa gaatct 16966
<210> 185
   <211> 364
   <212> DNA
   <213> unknown
<220>
   <223> >gi|4292568|gb|AI433922|sr|IMAGp998K105256.1|cl|IM AGp998K105256|AI433922 ti11f05.x1 NCI CGAP_Pan1 Homo sapiens cDNA clone IMAGE:2130177 3' similar to TR:Q08805 Q08805 SALIVARY PROLINE-RICH PROTEIN L ;, mRNA sequence
<400> 185
   tttttttttt tttttttttt tttttttttt tttttttttt tttttttttt tttttttttt 60
   tttttttttt tttttttttt tttttttttt ttttggggcg gtcccaaaac caaccctttt 120
   aattggaaaa aaaggggaaa cccccccccc ccgggggggg gggggggaaa aaaaaaaaaa 180
   accccctttt caaaaaaagg cgggggggga accaattccc cccccgggga aacccccccc 240
   ccccaaaacc ctggaagttc cctgggaaaa aaaaaaaaaa aagggggggg gggaaaaaag 300
   gggggacccc cccaaacccc tttgcccccc cccccaaggg gggggaaaaa ccccccggtc 360
   cccc 364
<210> 186
   <211> 474
   <212> DNA
   <213> unknown
<220>
   <223> >ImageID-1752104 IMAGP971B2475_S1_M13-RP CC FromEXP of IMAGp971B2475_s1_M13-RP.exp April 26, 2002 12:41
<400> 186
   catcatcatc cccactgtgg acaggtcgca gtaagaacag gagagtcaca tcattctaat 60
   aatggtctca gagatacatc acaatgactc ccctgggcag aaagaaggga taagagtcac 120
   atcacctgtg ggctaggccc agagatgtca ctcttacttc tgtgggcatg tctcaggctg 180
   gagaggagaa tcacattacc taaacactgg accaagaaat acgtcacagt ctttctcatg 240
   ggcaaagtcc aggacctcgg caaaacctga gtcctgtcct ctcgctttcc tccccgtgca 300
   cagcgagctt caccacttgc tccgcacctt ctccatcaac tactacctgt ccctgtgatc 360
   tgtccagtcg cccaactaaa gtgctcagca ggtcagcagc gcggcgagct tctatgcagg 420
   cgtggggtct ggggctcctg gatctctgtg tcccatttca cagagattgc cacc 474
<210> 187
   <211> 667
   <212> DNA
   <213> unknown
<220>
   <223> >sr|IMAGp998G193982.2|cl|IMAGp998G193982|gb|BX0901 35|oid|234514632 BX090135 NCI_CGAP_GC4 Homo sapiens cDNA clone IMAGp998G193982 ; IMAGE:1571370, mRNA sequence
<400> 187
   ctcagcccac ctgcacccag gtgaaataaa cagccatgta gctcacacaa cgcctgtttg 60
   gtggtctctt cacacggacg cacatgaaat ttggtgccct gactcggatc gggggacctc 120
   ccttgggaga tcaatcccct gtcctcctgt tctttgctcc ctgagaaaga tccacctatg 180
   acttcaggtc ctcagactga ccagcccaag gaacatctca ccaattttaa atcagcggcc 240
   aggcgttcct ccagaacctc ctcccacagg aacttgctac acgtgctgga aatctggcaa 300
   ctggaccaag gaatgcccgc agcccgggat tcctaagccg catcccatct gtgtgggacc 360
   ccacggaaaa tcggactgtt caactcacct ggcagccact cccagagccc ctggaactct 420
   ggcccaaggc tctctgactc cttcccagat cttctcggcc tagcggttga agactgacac 480
   tgcccgatca cctcggaagc cccctagacc atcacggacg ccgagcttca gatacgcgca 540
   tgaaaggaag attaagaaaa acaaaaaaat gtaaatttac tggacactga atatatacta 600
   agtactgctt aagtgattta catgtcaaga ttaaaatatt ttgctttact ttaaaaaaaa 660
   aaaaaaa 667
<210> 188
   <211> 444
   <212> DNA
   <213> unknown
<220>
   <223> >gi|5340943|gb|AI793227|sr|IMAGp998P194992.3|cl|IM AGp998P194992|AI793227 gz35g10.x5 NCI_CGAP_Kid11 Homo sapiens cDNA clone IMAGE:2028930 3', mRNA sequence
<400> 188
   tgtgcttcag tgatttattg agctttaact tcgggccagg gcagcagtag gcgccgctga 60
   gaaaagatga atgaacaagt ccccaaacca aaaataagca gcccactcct ccaaaataaa 120
   cacccactcc cagcattaag tttaaattac aatcactcag ggctcaggac tttcttgttg 180
   gcagcagctg ttggcttcat agctttgacc attctgccac ctctctggga tcccacagcc 240
   catctttgag tgtctgtttt cagttcaaga cagctagctc atgccagcac tggcagaggt 300
   gagtccccaa ctgcacacag gcacctactg gtataggtct ggcagatttg agttcggata 360
   gagttgccat cacttcctcg ctgtctggag ctgcacgcgt taatatgcca ttagagcccc 420
   agtttcttca tatgccagta gcac 444
<210> 189
   <211> 476
   <212> DNA
   <213> unknown
<220>
   <223> >gi|5110011|gb|AI741723|sr|IMAGp998F015873.1|cl|IM AGp998F015873|AI741723 wg22c01.x1 Soares_NSF_F8 9W_OT_PA_P S1 Homo sapiens cDNA clone IMAGE:2365824 3', mRNA sequence
<400> 189
   tttttttttt tttttttttg catttaaata tgggctcaag tagtaggact tggctgagtt 60
   tattactctc aagagtcaaa cagaaagaga aggagtgagg aaaccaagaa acagtggggg 120
   tcaggctggg agtagaggaa gtactagaac ttgggttggc agagttcatc tttgtagttc 180
   aagaagtctg aagttttaca gttatttgca tcacctggat agaagacttt cttggcttgc 240
   aaagctttct gccacatcca taaagatctt gtttttattt acttgctggt tttctttatt 300
   tctttatttc atgaggctga atagaaatga aggaaacttg gttggtgatt ctagaagctg 360
   ctctctggga tgatctcctt gttaatgatc ccctatggat tcatctttga tgggcaataa 420
   agaattgctt tcttgaggga cgcacagctt cctcagacct cttcatgcta ctgcag     476
<210> 190
   <211> 417
   <212> DNA
   <213> unknown
<220>
   <223> >gi|3930893|gb|AI288116|sr|IMAGp998A054888.1|cl|IM AGp998A054888|AI288116 qv88a03.x1 NCI_CGAP_Ut2 Homo sapiens cDNA clone IMAGE:1988620 3' similar to gb:J03075 PROTEIN KINASE C SUBSTRATE, 80 KD PROTEIN, HEAVY CHAIN (HUMAN);, mRNA sequence
<400> 190
   tttttttttt tttttttttt tttttttttt tttttttttt tttttttttt tttttttttt 60
   tttttttttt tttttttttt tggggggggg gaaaaaaata aatggatggg gggccaaggg 120
   aaaaacatcc acaaaccccc caaaaaaggg ggggcagaaa cagggctggg gggggggggg 180
   caaaacacca aggggggggg cccaaggggg gaaacccccc taacccattt ttaaaggggg 240
   ggggggcccg gaaaagggaa ccaccccccc cggggcccca aaaaagggga aaaaacccca 300
   aaaagggcct gccccccaaa aagagggcaa aaaaaccaca ggcagcccca aagggggggg 360
   gggacacgac tgaaggggcg ggtttaaacc ctttttaagg ggttttgcgc ccaacca    417
<210> 191
   <211> 488
   <212> DNA
   <213> unknown
<220>
   <223> >sr|IMAGp998A04528.1|cl|IMAGp998A04528|gb|BX101034 |oid|234536444 BX101034 Soares fetal liver spleen 1NFLS Homo sapiens cDNA clone IMAGp998A04528 ; IMAGE:244875, mRNA sequence.
<400> 191
   aaatatgtga ttttctctaa tgtaacttcc tcttaggatg tttcaccaag gaaatattga 60
   gagagaagtc ggccaggtag gatgaacaca ggcaatgact gcgcagagtg gattaaaggc 120
   aaaagagaga agagtccagg aaggggcggg gagaagcctg ggtggctcag catcctccac 180
   gggctgcgcc gtctgctcgg ggctgagctg gcgggagcag tttgcgtgtt tgggtttttt 240
   aattgagatg aaattcaaat aacctaaaaa tcaatcactt gaaagtgaac aatcagcggc 300
   atttagtaca tccagaaagt tgtgtaggca ccacctctgt cacgttctgg aacattctgt 360
   catcaccccg tgaagcaatc atttcccctc ccgtcttcct cctcccctgg caactgctga 420
   tcgactttgt gtctctgttg tctaaaatag gttttccctg ttctggacat ttcatataaa 480
   tggaatca                                                          488
<210> 192
   <211> 526
   <212> DNA
   <213> unknown
<220>
   <223> >sr|IMAGp998H22207.1|cl|IMAGp998H22207|gb|BX096159 |oid|234526691 BX096159 Soares placenta Nb2HP Homo sapiens cDNA clone IMAGp998H22207 ; IMAGE:140997, mRNA sequence
<400> 192
   ctggaagacc tgggctgggc cgattgggtg ctgtcgccac gggaggtgca agtgaccatg 60
   tgcatcggcg cgtgcccgag ccagttccgg gcggcaaaca tgcacgcgca gatcaagacg 120
   agcctgcacc gcctgaagcc cgacacggtg ccagcgccct gctgcgtgcc cgccagctac 180
   aatcccatgg tgctcattca aaagaccgac accggggtgt cgctccagac ctatgatgac 240
   ttgttagcca aagactgcca ctgcatatga gcagtcctgg tccttccact gtgcacctgc 300
   gcggaggacg cgacctcagt tgtcctgccc tgtggaatgg gctcaaggtt cctgagacac 360
   ccgattcctg cccaaacagc tgtatttata taagtctgtt atttattatt aatttattgg 420
   ggtgaccttc ttggggactc gggggctggt ctgatggaac tgtgtattta tttaaaactc 480
   tggtgataaa aataaagctg tctgaactgt taaaaaaaaa aaaaaa                526
<210> 193
   <211> 755
   <212> DNA
   <213> unknown
<220>
   <223> >ImageID=451838 IMAGP971M0340_S1 CC FromEXP of IMAGp971M0340_s1.exp April 26, 2002 13:37
<221> misc_feature
   <222> 580, 694, 704
   <223> n=a, t, c, or g
<400> 193
   agaaacatgt atttcagcaa acccatccag tgtaccacgt aaaacctggt gggccagtaa 60
   atctcctgac aataaacctg tttggtatgg tcttgatatg aacagagggt ctcagttcgc 120
   ttatggagac caccaatcac ctaatacagc cattactcag atgacttttt tgcgcctttt 180
   atcaaaagaa gcctcccaga acatcactta catctgtaaa aacagtgtag gatacatgga 240
   cgatcaagct aagaacctca aaaaagctgt ggttctcaaa ggggcaaatg acttagatat 300
   caaagcagag ggaaatatta gattccggta tatcgttctt caagacactt gctctaagcg 360
   gaatggaaat gtgggcaaga ctgtctttga atatagaaca cagaatgtgg cacgcttgcc 420
   catcatagat cttgctcctg tggatgttgg cggcacagac caggaattcg gcgttgaaat 480
   tgggccagtt tgttttgtgt aaagtaagcc aagacacatc gacaatgagc accaccatca 540
   atgaccaccg ccattcacaa gaactttgac tgtttgaagn tgatcctgag actcttgaag 600
   taatggctga tcctgcatca gcattgtata tatggtctta agtgcctggc ctccttatcc 660
   ttcagaatat ttattttact tacaatcctc aagntttaat tgantttaaa tatttttcaa 720
   tacaacaggt taggttttaa gatgaccaat gacaa                            755
<210> 194
   <211> 467
   <212> DNA
   <213> unknown
<220>
   <223> >gi|4740154|gb|AI656175|sr|IMAGp998K055550.1|cl|IM AGp998K055550|AI656175 tt38e03.x1 NCI_CGAP_GC6 Homo sapiens cDNA clone IMAGE:2243068 3', mRNA sequence
<400> 194
   actttgtgtc attcagttta gagcaattta atcttttttt ctccaaatcc atttttgaag 60
   ctgagtttaa cttttgcaac ccatggcaaa tcttaaatgc cctcatttac caatctttac 120
   caaactccta tttaagcctc taaaagtcaa tactggccat cagacccaaa tttcagaaga 180
   caatagtgaa aaattactta cgtttaatct ccagtcgtgt cccttggccg aaggtgatcc 240
   acagtgttaa cttaattact ttcccctaaa caaaaatctc ttttcgctgt taatatcact 300
   aacctgaccg atgcagagaa aatctacaat tgagatgcct cacttaactg gctagcgctt 360
   ggctgttcct taagatgaac tacttttcta tcccttactc atctgacttt ttgaaagaat 420
   ctggtactct tttgaattga cctgagctca tatctcaaac acaaaaa 467
<210> 195
   <211> 474
   <212> DNA
   <213> unknown
<220>
   <223> >sr|IMAGp998A065260.2|cl|IMAGp998A065260|gb|BX1195 12|oid|234573224 BX119512 NCI_CGAP_Kid11 Homo sapiens cDNA clone IMAGp998A065260 ; IMAGE:2131469, mRNA sequence
<400> 195
   tcttaatttt ttttttttaa ttggaaaact cgatctctag gaggaaagaa aaaaaaggcc 60
   tgggtcccag aaaactggtt gtagccctag cttcttaatt tattggccat gtgactatag 120
   actatttgct tacttgctct ggtcctcagt tttctggtct gcagaatggg tatcttgatg 180
   tgtgttgttc ttctcctcca cgatatgaaa ccaaaggcct ttatagcttg ttttctgagg 240
   ctccttccag gtgaaaatcc tataactccc aataaaacag ctggagaggc tgtctctact 300
   gccttttctg tctacccagg agtttccttg gttgatggga agctgtttaa gaactgaaat 360
   gagagagaag aggcagacag acccaaccgc tacagctcct atcagaaagg gcagcgcccg 420
   tggaattgta ggtagcctgt tactggctca gagaacaagc aggcctactt atgt 474
<210> 196
   <211> 498
   <212> DNA
   <213> unknown
<220>
   <223> >sr|IMAGp998J076121.2|cl|IMAGp998J076121|gb|BX0968 30|oid|234528032 BX096830 NCI_CGAP_Pr22 Homo sapiens cDNA clone IMAGp998J076121 ; IMAGE:2461158, mRNA sequence
<400> 196
   gcccccacct tcccatccag acagccgagc tcgctccgga acttgtcatc tccaacgaca 60
   aaaggagctt ttgccactga ctcggccgtg tcctgacacc tccagaacgc aggtgctggc 120
   gcccgttctg cctgggaccc cgggaacctc tcctgccgga agccggacgg cagggatggg 180
   ccccaacttc gccctgccca cttgacttca ccaaatccct tcctggagac tgaacctggt 240
   gctcaggagc gaaggactgt gaacttgtgg cctgaagagc cagagctagc tctggccacc 300
   agctgggcga cgtcaccctg ctcccacccc acccccaagt tctaaggtct tttcagagcg 360
   tggaggtgtg gaaggagtgg ctgctctcca aactatgcca aggcggcggc agagctggtc 420
   ttctggtctc cttggagaaa ggttctgttg ccctgattta tgaactctat aatagagtat 480
   ataggttttg tacctttt 498
<210> 197
   <211> 584
   <212> DNA
   <213> unknown
<220>
   <223> >sr|IMAGp998L10662.1|cl|IMAGp998L10662|gb|BX102297 |oid|234538974 BX102297 Soares fetal liver spleen 1NFLS Homo sapiens cDNA clone IMAGp998L10662 ; IMAGE:296601, mRNA sequence
<221> misc_feature
   <222> 512, 538, 552, 570, 579, 584
   <223> n=a, t, c, or g
<400> 197
   tccgatatgg cctcaatgaa gtgaagtgca aagggaatga gtcttccttg tgggattgtc 60
   ctgccagacg ctggggccat agtgagtgtg ggcacaagga agacgctgca gtgaattgca 120
   cagatatttc agtgcagaaa accccacaaa aagccacaac aggtcgctca tcccgtcagt 180
   catcctttat tgcagtcggg atccttgggg ttgttctgtt ggccattttc gtcgcattat 240
   tcttcttgac taaaaagcga agacagagac agcggcttgc agtttcctca agaggagaga 300
   acttagtcca ccaaattcaa taccgggaga tgaattcttg cctgaatgca gatgatctgg 360
   acctaatgaa ttcctcagga ggccattctg agccacactg aaaaggaaaa tgggaattta 420
   taacccagtg agttcagcct ttaagatacc ttgatgaaga cctggactat tgaatggagc 480
   agaaattcac ctctctcact gactattaca gntgcatttt tatggagttc ttcttctnct 540
   atgattccta anactgctgc tgaatttatn aaaattaanc ttgn 584
<210> 198
   <211> 732
   <212> DNA
   <213> unknown
<220>
   <223> >ImageID=114388 IMAGP971C043_S1 CC FromEXP of IMAGp971C043_sl.exp April 26, 2002 12:42
<400> 198
   aaattccctc tggggatcta tgagctaatg agtttcttca tgcttatccc atgtgatgtt 60
   gggaatccag actacagggc aaagtagata ctttaactcc attttacaga taagagtgag 120
   gttcagagag atgattgagt tagtgaggac tgagcctggc ctcaaactcg agttgactac 180
   aagcacctct tctctgcaca gtgttactca tgaaagagac ttccctgcaa gacctgggcc 240
   ttttacttca aagggcaaag agacttattt ttcctttcat gcctttccat ggcccactct 300
   ttcattctgg tttcaggttg ccagtctggc tgcagtcctc tgtcattggt cggtaacacc 360
   ctgggtcata ttccacactt aaagatggag gtaattgctt caagatgtgg ccctttaagg 420
   agagaatagg ttgaaaggca aattcttttt ttcagcttta gtcagagcct gagttttgga 480
   aaaaggaaat gatgagttgc tccgagatat aaactttctt tcatctctta gaagaagtat 540
   ttgtccttct gaaaaggtta gtggcagttt tgtccattga tttttttctt aaacctttat 600
   tacccagttt catggtccat cacagtaagt aaaacaaacc accctccccc agaccacctc 660
   cctccttatt tgcacaggga accttcaaac aattgggagc agaagcagaa tgatgcctgt 720
   gaagcctttg cc 732
<210> 199
   <211> 528
   <212> DNA
   <213> unknown
<220>
   <223> >sr|IMAGp998G06783.2|cl|IMAGp998G06783|gb|BX118770 |oid|234571916 BX118770 Soares_fetal_heart_NbHH19W Homo sapiens cDNA clone IMAGp998G06783 ; IMAGE:342941, mRNA sequence
<221> misc_feature
   <222> 438, 463, 498, 517
   <223> n=a, t, c, or g
<400> 199
   tcttatacta atttatacaa agatattaag gccctgttca ttaagaaatt gttcccttcc 60
   cctgtgttca atgtttgtaa agattgttct gtgtaaatat gtctttataa taaacagtta 120
   aaagctgaca gttcgccctt actcttggag gtcatgttca ggaggggcat tcctttcccc 180
   tgggggtcat gggtgtcccc atgcccacat attgcacgtg cagggaggta agtgcctgca 240
   tcccaaatcg gttctaggtc aactggcctc aaactgattt gccatgagct cacaaaatga 300
   atccctatgc ttaatgacca ggtcacataa aatccagccc acttacaggt tttctggcat 360
   ctgtttgggt gtcctaattt ttttggcagt gtcatttgaa gaattttttt aaagcagttt 420
   atttaagaac atactganta aatgcaggat cgctactaaa aantgctttg tatccttggt 480
   gggtgtcttc tgctattnta tctacttttg aacactntca ggactttt 528
<210> 200
   <211> 489
   <212> DNA
   <213> unknown
<220>
   <223> >sr|IMAGp998K0B276.1|cl|IMAGp998K08276|gb|BX090262 |oid|234514887 BX090262 Soares infant brain 1NIB Homo sapiens cDNA clone IMAGp998K08276 ; IMAGE:47615, mRNA sequence
<400> 200
   tttttttttt ttttctggaa ggttctcagg tctttatttg ctctctcaaa ttccaggaat 60
   tgacttattt aattaatcca tcaacctctc atagcaaata tttgagaaaa caaatttata 120
   ttcagattct tattttcagt agggaagtaa gaagttgcag ctcagtgcac gtaaagttga 180
   gacagagatg gagacatcca gccccacctc tctggaacaa gaaagatgac tggggaggaa 240
   acacaggtca gcatgggaac aggggtcaca gtggacacaa gggtgggctg tctctccacc 300
   tcctcacatt atgctaacag ggacgcagac acattcaggt gcctttgcag aaagagatgc 360
   cagaggctct tgaagtcaca aaggggaggc gtgaagaaat cctgcatctc agtccctcac 420
   aagacagctg tctcaggctt ttcaagctgt gagagacaca tcagagccct gggcactgtc 480
   gctggacgc 489
<210> 201
   <211> 364
   <212> DNA
   <213> unknown
<220>
   <223> >gi|2224139|gb|AA494352|sr|IMAGp998F152230.1|cl|IM AGp998F152230|AA494352 ne27c08.sl NCI_CGAP_Co3 Homo sapiens cDNA clone Image : 898574 3' similar to contains element LTR5 LTR5 repetitive element ; mRNA sequence.
<400>201
   ctttcactca ttggtttatt cccgcactcc ttccctcact cattcattca ttgactcttg 60
   caggtagtgc acaccaaggg tgtgaagggg tgaggtgtct ggttctctgc tcagacctct 120
   gcaagtgccc gcagaggtgt tctctgctca gacctctgca agtgcccgca gaggtgactg 180
   ggcggcctcc acttctggcc tggccttgca cccctgtgct cttgggggcc atggctgggc 240
   agggcaggtg tccccctctg cacaggccca gtgtgtctca gcctcagagc tggcagggca 300
   gggcaggcat cccctctgca ctggcccagt gtctctcagc ctcagaacca gatgtgagaa 360
   gacg 364
<210> 202
   <211> 3159
   <212> DNA
   <213> unknown
<220>
   <223> >ENST00000322244 Coding sequence
<400> 202
   atggaaggat ccgagcctgt ggccgcccat cagggggaag aggcgtcctg ttcttcctgg 60
   gggactggca gcacaaataa aaatttgccc attatgtcaa cagcatctgt ggaaatcgat 120
   gatgcattgt atagtcgaca gaggtacgtt cttggagaca cagcaatgca gaagatggcc 180
   aagtcccatg ttttcttaag tgggatgggt ggtcttggtt tggaaattgc aaagaatctt 240
   gttcttgcag ggattaaggc agttacaatt catgatacag aaaaatgcca agcatgggat 300
   ctaggaacca acttctttct cagtgaagat gatgttgtta ataagagaaa cagggctgaa 360
   gctgtactta aacatattgc agaactaaat ccatacgttc atgtcacatc atcttctgtt 420
   cctttcaatg agaccacaga tctctccttt ttagataaat accagtgtgt agtattgact 480
   gagatgaaac ttccattgca gaagaagatc aatgactttt gccgttctca gtgccctcca 540
   attaagttta tcagtgcaga tgtacatgga atttggtcaa ggttattttg tgatttcggt 600
   gatgaatttg aagttttaga tacaacagga gaagaaccaa aagaaatttt catttcaaac 660
   ataacgcaag caaatcctgg cattgttact tgccttgaaa atcatcctca caaactggag 720
   acaggacaat tcctaacatt tcgagaaatt aatggaatga caggtttaaa tggatctata 780
   caacaaataa cggtgatatc gccattttct tttagtattg gtgacaccac agaactggaa 840
   ccatatttac atggaggcat agctgtccaa gttaagactc ctaaaacagt tttttttgaa 900
   tcactggaga ggcagttaaa acatccaaag tgccttattg tggattttag caaccctgag 960
   gcacctttag agattcacac agctatgctt gccttggacc agtttcagga gaaatacagt 1020
   cgcaagccaa atgttggatg ccaacaagat tcagaagaac tgttgaaact agcaacatct 1080
   ataagtgaaa ccttggaaga gaagcctgat gtaaatgctg acattgtgca ttggctctct 1140
   tggactgccc aaggcttttt atctccactt gctgcagcag taggaggtgt tgccagccaa 1200
   gaagtattga aagctgtaac aggaaaattt tctcctttgt gccagtggtt atatcttgaa 1260
   gcagcagata ttgttgaatc actaggcaaa cctgaatgtg aagaatttct cccacgagga 1320
   gatagatatg atgccttaag agcttgcatt ggagacactt tgtgtcagaa actgcaaaat 1380
   ttaaacatct tcttagtagg gtgtggagcc ataggctgtg aaatgttgaa aaattttgct 1440
   ttacttggtg ttggcacaag caaagagaaa ggaatgatta cagttacaga tcctgacttg 1500
   atagagaaat ccaacttaaa tagacagttc ctatttcgtc ctcatcacat acagaaacct 1560
   aaaagctaca ctgctgctga tgctactctg aaaataaatt ctcaaataaa gatagatgca 1620
   cacctgaaca aagtatgtcc aaccactgag accatttaca atgatgagtt ctatactaaa 1680
   caagatgtaa ttattacagc attagataat gtggaagcca ggagatacgt agacagtcgt 1740
   tgcttagcaa atctaaggcc tcttttagat tctggaacaa tgggcactaa gggacacact 1800
   gaagttattg taccgcattt gactgagtct tacaatagtc atcgggatcc cccagaagag 1860
   gaaataccat tttgtactct aaaatccttt ccagctgcta ttgaacatac catacagtgg 1920
   gcaagagata agtttgaaag ttccttttcc cacaaacctt cattgtttaa caaattttgg 1980
   caaacctatt catctgcaga agaagtctta cagaagatac agagtggaca cagtttagaa 2040
   ggctgttttc aagttataaa gttacttagc agaagaccta gaaattggtc ccagtgtgta 2100
   gaattagcaa gattaaagtt tgaaaaatat tttaaccata aggctcttca gcttcttcac 2160
   tgtttccctc tggacatacg attaaaagat ggcagtttat tttggcagtc accaaagagg 2220
   ccaccctctc caataaaatt tgatttaaat gagcctttgc acctcagttt ccttcagaat 2280
   gctgcaaaac tatatgctac agtatattgt attccatttg cagaagagga cttatcagca 2340
   gatgccctct tgaatattct ttcagaagta aagattcagg aattcaagcc ttccaataag 2400
   gttgttcaaa cagatgaaac tgcaaggaaa ccagaccatg ttcctattag cagtgaagat 2460
   gagaggaatg caattttcca actagaaaag gctattttat ctaatgaagc caccaaaagt 2520
   gaccttcaga tggcagtgct ttcatttgaa aaagatgatg atcataatgg acacatagat 2580
   ttcatcacag ctgcatcaaa tcttcgtgcc aaaatgtaca gcattgaacc agctgaccgt 2640
   ttcaaaacaa agcgcatagc tggtaaaatt atacctgcta tagcaacaac cactgctaca 2700
   gtttctggct tggttgcctt ggagatgatc aaagtaactg gtggctatcc atttgaagct 2760
   tacaaaaatt gttttcttaa cttagccatt ccaattgtag tatttacaga gacaactgaa 2820
   gtaaggaaaa ctaaaatcag aaatggaata tcatttacaa tttgggatcg atggaccgta 2880
   catggaaaag aagatttcac cctcttggat ttcataaatg cagtcaaaga gaagtatgga 2940
   attgagccaa caatggtggt acagggagtc aaaatgcttt atgttcctgt aatgcctggt 3000
   catgcaaaaa gattgaagtt aacaatgcat aaacttgtaa aacctactac tgaaaagaaa 3060
   tatgtggatc ttactgtgtc atttgctcca gacattgatg gagatgaaga tttgccggga 3120
   cctccagtaa gatactactt cagtcatgac actgattaa 3159
<210> 203
   <211> 166
   <212> DNA
   <213> unknown
<220>
   <223> >gi|2524282|gb|AA620J43|sr|IMAGp998A022575.1|cl|IM AGp998A022575|AA620343 af07b01.s1 Soares_testis_NHT Homo sapiens cDNA clone IMAGE:1030921.3', mRNA sequence.
<400> 203
   ttttggtagc tattgaatca gggccacaca tttaattgat attatgatca agatgttcaa 60
   ggcaaaaaat actattactt atttaatgtg gaacaagtct agtctttctc ttgagctccc 120
   acctgctggt taggaggcaa caatgttatt tggatcctgt ttagag                166
<210> 204
   <211> 408
   <212> DNA
   <213> unknown
<220>
   <223> >sr|IMAGp998E122300.2|c1|IMAGp998E122300|gb|BX0954 46|oid|234525261 BX095446 NCI_CGAP_Co3 Homo sapiens cDNA clone IMAGp998E122300; IMAGE:925427, mRNA sequence
<400> 204
   aaatatgtat aaatgtgggc accgtcattt gtctagaaaa atctatagta aattgttttg 60
   ttttcctttc attgcaaagt ctcatacttg ttcttccaaa atcagttcta tgagttgctt 120
   taaaattagc ctgggacgtc caagaagtgt taagacatct gcagtaactg gggcagccag 180
   tccctggagg ccaactcagg gaacaacaaa ctccttcgcc aagccgtcca cacatgccag 240
   ctgatggtct aggacccaag tatgtgccaa gagtgaggtg tcttacttct aaggagaggc 300
   aagcagtttg ctatgcaagc gggtcgctgg ccctggacct aaagatcttc tctctaactc 360
   catattcttt aaataaactt gtcttttatg aggaaaaaaa aaaaaaaa             408
<210> 205
   <211> 398
   <212> DNA
   <213> unknown
<220>
   <223> >gi|879189|sr|IMAGp998C12278|cl|IMAGp998C12278|gb| H14369|H14369 ym18f06.s1 Homo sapiens cDNA clone 48417 3'.
<221> misc_feature
<222> 105, 342, 363
<223> n=a, t, c, or g
<400> 205
   tttttttttt ttgccttttc atttgtcttt ttattttaca tttaaaaagt ggttattgct 60
   ctaagtggat cagaggtaag gacctctccc ctagggagcc ttggncttgc agccccattc 120
   agcagggatg gaagtcacaa gacaatgagt ggagcctcat gccctcccat gaggaagccc 180
   ttagtattgc tgacatctgc cctttatcct gtctctcctc cccagtgctg tcacacttgg 240
   gcaaagcaga gtggtggcag acccagcctt gagagctctt gtaggaccgg aaggaagggg 300
   cggtcattag gtgatgggct tctgggctct ctgggcttgg gngaccaatg ctcttattca 360
   ttncattttc aacaaatatt taactggagg taccgggc                         398
<210> 206
   <211> 654
   <212> DNA
   <213> unknown
<220>
   <223> >sr|IMAGp99BE191782.3|cl|IMAGp99SE191782|gb|BX1105 07|oid|234555393 BX110507 Soares_testis_NHT Homo sapiens cDNA clone IMAGp998E191782 ; IMAGE:726522, mRNA sequence
<400> 206
   caaattcaac ccaccacata ggccctcccg aggccacctc tgcgtcatct ttgtctacag 60
   attctgtgtc cgtcttcctc tcctcattga ctctgcatga aggggatttc tggtggagta 120
   agggtagcag ctcttgccgc acgtgcagag agagaggaac ttccagtgcc cgctatggag 180
   ccacagccca cagcatgggg gaagtcccca tccagaggca gtgctgcagc tagaggtagc 240
   tccagagtcc tccgggccct gcaccgatac agccaaagac cagcaaagcg acaagctgcc 300
   agacctcatg ccacctgccg tagccactgg gctcagccct ggagctgaga gcatcgctgg 360
   agatagacgt ggcagagaag aggttgcgag catggcccca gccagcagct cccacgctgc 420
   ccctagtcct gggcatggag cgagccttgg tgtcagagac cagggtgtgc agtctgagct 480
   cctctacctt actaaagaga ggcctctttt atttaccaga gccacagccc tgctgcctca 540
   ggaccttttc attctgccgg tgctggggct gtctatctgc aagctggagg tgttgagagc 600
   aggaaaaggg aggctgtgag gaaggggttc gggcagctcc tcctgctctc agag       654
<210> 207
   <211> 417
   <212> DNA
   <213> unknown
<220>
   <223> >sr|IMAGp9980124335.2|cl|IMAGp9980124335|gb|BX0992 72|oid|234532920 BX099272 Soares_fetal_heart_NbHH19W Homo sapiens cDNA clone IMAGp9980124335 ; IMAGE:1707107, mRNA sequence
<221> misc_feature
   <222> 20, 26
   <223> n=a, t, c, or g
<400> 207
   agcatggccc cagccctggn acaagnacac gttctgccag ggcaaagctg atgggctcta 60
   tcccaatcct cgggaacggt ccagcttcta cagctgtgca gcggggcggc tgttccagca 120
   aagctgcccg acaggcctgg tgttcagcaa ctcctgcaaa tgctgcacct ggaattgagt 180
   cgctaaagcc cctccagtcc cagctttgag gctgggccca ggatcactct acagcctgcc 240
   tcctgggttt tccctggggg ccgcaatctg gctcctgcag gcctttctgt ggtcttcctt 300
   tatccaggct ttctgctctc agccttgcct tccttttttc tgggtctcct gggctgcccc 360
   tttcacttgc aaaataaatc tttggtttgt gcccctcttc ccaaaaaaaa aaaaaaa    417
<210> 208
   <211> 767
   <212> DNA
   <213> unknown
<220>
   <223> >sr|IMAGp998K102640.2|cl|IMAGp99SK102640|gb|BX0989 17|oid|234532208 BX098917 Soares_testis_NHT Homo sapiens cDNA clone IMAGp998K102640 ; IMAGE:1056129, mRNA sequence
<221> misc_feature
   <222> 7, 759
   <223> n=a, t, c, or g
<400> 208
   cgcaagnata atggattgtc aaaaaggagg caatatggca aacggaagtt gtttggacct 60
   gaagtcagaa gaatcaagtt cagctcactt tgattctgat gggatctacc aaagcatgtt 120
   tgttgatatg atcaatccaa aagagaaggt gagactgatg aacaggagga ctggagtctt 180
   gtagtaggag gagtaaatgg gattcatatc tcacatcagc accttattta tttgacactg 240
   ctgatactgt taatttaact actaaagtct ggatgtcctg gcatggcaga caaaagtttc 300
   attcattatc cagtgaaaca ctgaaatgaa attaggatga gcaagtcact gagttctttg 360
   gaacgggaat cctcacggtt ctcctacagt cctaagaatg cttacaaaac aacgggctta 420
   tatcaatgcc agatctggaa gccatgggct aacttgacaa gcccactgga tttgacttta 480
   gcttcttgct ctagccagag gttaccaaca cctaaacaca aaatagacta tctgctcctg 540
   actgctgcag aactaaagag ctgactttat tcttgagttc tttattcaga gtcccagaaa 600
   attatttcta aaatgcatgc tgggataact ttcaaaatca ggatttggtt tactttgcaa 660
   cagatattac cacacaaaaa atattaaatt ttagaaaaca agacaaatca tctttaactt 720
   aatgccctag tcttccttaa caaaagcaaa gacacaaant taagaag 767
<210> 209
   <211> 468
   <212> DNA
   <213> unknown
<220>
   <223> >gi|4735332|gb|AI651353|sr|IMAGp998F035711.1|cl|IM AGp998F035711|AX651353 wb05c02.x1 Ncr_CGAP_GC6 Homo sapiens cDNA clone IMAGE:2304770 3' similar to contains Alu repetitive element;contains element TAR1 repetitive element ;, mRNA sequence
<400> 209
   cttctttttg cttctctttt ctttctttct ttctttcttt ctttctttct ttctttcttt 60
   ttctttttct ctcttcccct ctttctttcc tgccttcctg cctttcttct tttcttcttt 120
   cctcccttcc tcccttcctt ctttcctccc gcctcagcct cccaaagtgc tgggatgact 180
   ggcgggaggc accatgcctg cttggcccaa agagaccctc ttggaaagtg agacgcagag 240
   agcgccttcc agtgatctca ttgactgatt tagagacggc atctcgctcc gtcaccccgg 300
   cagtggtgcc gtcgtaactc actccttgca gcgtggacgc tcctggactc gagcgatcct 360
   tccacctcag cctccagagt acagagcctg ggaccgcggg cacgcgccac tgtgcccaca 420
   ccgtttttaa ttgttttttt ttcccccgag acagagtttc actctcgt 468
<210> 210
   <211> 726
   <212> DNA
   <213> unknown
<220>
   <223> >sr|IMAGp9980184348.2|cl|lMAGp9980184348|gb|BX1054 24|oid|234545229 BX105424 Soares_pregnant_uterus_NBHPU Homo sapiens cDNA clone IMAGp9980184398 ;IMAGE:1712105, mRNA sequence.
<221> misc_feature
   <222> 637, 721
   <223> n=a, t, c, or g
<400> 210
   acggatttta ttaaaaccta tgttattttt tgttgctaat catttgacac ttaatcgaaa 60
   cagaataagt tactgtggaa aaaaggatga tccaactcct gaggattgcc gcctgtgtgt 120
   ctttaattga ttatctttga tagttagagt tatttttcag atttttcccc ctcatactgg 180
   attgtgatgc tctgatatga gcatgcaatg actgatacag aaataggctg gagcttcggt 240
   gtgtgctttt gctatacagc ttacagttct aatgcctgat gtagagatga tgaatttctc 300
   ctttttaaac ctatcttctg tttactcctt ttgcattatg aaggccctct tcaatgatgt 360
   atgtttctga tggaatcaga aaatttcaca ctagaaaagt gtacctaact tgattcagtg 420
   tgagctcttg ggacagggag gggagccagg ggtagagctt aaagaagctg gctctgtaag 480
   agttgtatat caaagtacct tttgctacaa ggtgcatata gaagtctaag ataagaaatg 540
   taccctctat actttatttt tcatgcccct atcatgtttg gtgtgcctta aatcttacct 600
   ttaaatgaag actctatgga ttgtttacaa atgaagnttt acactaggac cagatcgtcc 660
   atacatgatt ggtcttctac aataataatg ttcaattctt tcattttaaa tctttcttta 720
   nggtca                                                            726
<210> 211
   <211> 391
   <212> DNA
   <213> unknown
<220>
   <223> >gi|1512724|sr|IMAGp99BB201153|cl|IMAGp998B201153| gb|AA037568|AA037588 zk36d10.r1 Soares pregnant uterus NbHPU Homo sapiens cDNA clone 484915 5'
<221> misc feature
   <222> 85, 96, 167, 174, 218, 242,297, 312, 332, 366
   <223> n=a, t, c, or g
<400> 211
   ccaaattgct caacagtatg tatgtttaga ggggttagac tccttttgaa aatctggata 60
   tctaaccacc tacttaaatc tgttngatag tgtcanacca cccccaccct tgatcctccc 120
   acccccaaga aaaagaaaag atgctcctgt taaatgaagg ctctttnggg gtangtgcta 180
   ggtatactct tggctagatg gattcagctc agaatttntg actttttaaa gctaagtcct 240
   tngtagtcac atggttgact tgaaaggatg aggtggtgtg tgcatttatg acgcttntcc 300
   tacccccctg anagaaactt cattggccgc cntggttact caccttgaag agagtaccat 360
   ccattntacg tgaaaaacac atctgtcacc c                                391
<210> 212
   <211> 658
   <212> DNA
   <213> unknown
<220>
   <223> >sr|IMAGp998P08S67.1|cl|IMAGp998P08567|gb|BX099307 |oid|234532990 BX099307 Soares_placenta_8to9weeks_2NbHP8to9W Homo sapiens cDNA clone IMAGp998P08567; IMAGE:260215, mRNA sequence
<221> misc_feature
   <222> 564, 584, 601, 612, 616, 619, 627, 630, 644, 656
   <223> n=a, t, c, or g
<400> 212
   tctcgtctca catttgcact ctctccttcc cacattctgt gctcagctca ctcagtggtc 60
   agcgtttcct gcacacttta cctctcatgt gcgtttcccg gcctgatgtt gtggtggtgt 120
   gcggcgtgct cactctctcc ctcatgaaca cccacccacc tcgtttccgc agcccctgcg 180
   tgctgctcca gaggtgggtg ggaggtgagc tgggggctcc ttgggccctc atcggtcatg 240
   gtctcgtccc attccacacc atttgtttct ctgtctcccc atcctactcc aaggatgccg 300
   gcatcaccct gagggctccc ccttgggaat ggggtagtga ggccccagac ttcaccccca 360
   gcccactgct aaaatctgtt ttctgacaga tgggttttgg ggagtcgcct gctgcactac 420
   atgagaaagg gactcccatt tgcccttccc tttctcctac agtccctttt gtcttgtctg 480
   tcctggctgt ctgtgtgtgt gccattctct ggacttcaga gccccctgag ccagtcctcc 540
   cttcccagcc tccctttggg cctncctaac tccacctagg ctgncaggga ccggagtcag 600
   ntggttcaag gncatnggna gctctgnctn caagtctacc cttnccttcc ggactnct 658
<210> 213
   <211> 548
   <212> DNA
   <213> unknown
<220>
   <223> >sr|IMAGp99BA191006.1|cl|IMAGp99BA191006|gb|BX1180 17|oid|234570410 BX118017 Soares_fetal_liver_spleen_1NFLS_S1 Homo sapiens cDNA clone IMAGp998A191006 : IMAGE:428442, mRNA sequence
<400> 213
   cagatctcta ccagctgctc cagagactct tcaaaagcca ctcatctctg gagggattgc 60
   tcaaagccct gagccaggct agcacagatc ctaaggaatc aacatctccc gagaaacgtg 120
   acatgcatga cttctttgtg ggacttatgg gcaagaggag cgtccagcca gactctccta 180
   cggatgtgaa tcaagagaac gtccccagct ttggcatcct caagtatccc ccgagagcag 240
   aataggtact ccacttccgg actcctggac tgcattagga agacctcttt ccctgtccca 300
   atccccaggt gcgcacgctc ctgttaccct ttctcttccc tgttcttgta acattcttgt 360
   gctttgactc cttctccatc ttttctacct gaccctggtg tggaaactgc atagtgaata 420
   tccccaaccc caatgggcat tgactgtaga ataccctaga gttcctgtag tgtcctacat 480
   taaaaatata atgtctctct ctattcctca acaataaagg atttttgcat atgaaaaaaa 540
   aaaaaaaa                                                         548
<210> 214
   <211> 528
   <212> DNA
   <213> unknown
<220>
   <223> >sr|IMAGp998B02152.1|cl|IMAGp998B02152|gb|BX110160 |oid|234554699 BX110160 Soares infant brain 1NIB Homo sapiens cDNA clone IMAGp998B02152 ; IMAGE:31971, mRNA sequence
<400> 214
   tttttttttt ttttgaaggg acaccgattt attgagtagc agagctgagg gaaggcgtag 60
   gatggctcca gcttccggtc agtggctaca tggtcagttc catgatggcg ttgacgatgt 120
   cactgtggtt gtctctcaga gcccgcacgg ccttggccct ggacacattg gcctgcgcca 180
   tcaccagctc aatgtcacgc agttccagcc ccgcctcgtc cacctcttcc tcctcctcct 240
   cttcctcttc cttgcactcc agcctcaccc ggggcctggg tgctgactca gggaccaagg 300
   ctgagggctc tgagggcacc ttaaacttct cagctgcggc tttgtgcact tgctgggaca 360
   ggtcctcaat cttggcctcg ccaaagacca cataagtgtc tgaggctggg ctcttgaaga 420
   catcaggctt ggcgatgaca aagaggatgt tcttggactt ctggatggtg atcctggtga 480
   ctccctgaat ctgccgcaag cccagctttg acattgcctt tcgggcct             528
<210> 215
   <211> 2341
   <212> DNA
   <213> unknown
<220>
   <223> >Homo sapiens|chromosome:NCBI34:7:64716658:64718998:1
<400> 215
   gcagattctc caggcccagc atctgcctca ccgtggcccc ccacaagcca agcgcctgcc 60
   tttcagcagc ctctacacac ccagctcctg ccacccaatg gctctttagg ccaagctcat 120
   acctcacgat gatttttcca ggcccaactt ttgtctcatg gcaaccttcc ctggccaagt 180
   ttccacctat ttcctggcag cctggacagg cccaggtcct gccacacact ggcctctcta 240
   cgcccagctc atgcctcaca gtggcctctc caggcccagc tcctgtcccg ggacatcatc 300
   tccaggccca aaacttcctc aagtcggcct ctccaggccc agttgctgcc tcccggcatt 360
   ctctccaggc ctagctcttc ctcctggctg tatctacaag accaactcct gcctcacaac 420
   aaccttttat ggctcagctc ctgcccaact actgccggcc tttgtaggcc caaaacttcc 480
   tcaagtcaag ctctttaggc ccaccttctg ccttgcagtg gcctgtacag acccagctct 540
   ggcttgagaa cagcctctgc aggccctgct cttgcctctt agctccctct ccaggcccat 600
   ctcttgcctc acagtggctt ccgtgggcca agttcccgcc tgcctcccag cagcctcaac 660
   aggcctagct cctccctcac aatggcttgt ttaggtccag ttgatgcctc tggcaacctg 720
   tccaggccca gctcctgcct cacactggcc tctctaggcc gaggtccttt ctcatactgg 780
   cctgtttagg cccagctcat tcctcttgtc atctctccag gcccagcttt tgcctgttgt 840
   tggcctctac ctcacagtgc accttccagt cccacctctt gcctcaccat ggcctcctct 900
   gaccaggttc ctgcctttcg gcagcctcta caggcctagc tgctgcctcc caatggcctt 960
   tgtaggccac gctcatgcct cactgtggcc tttccaggcc tagctttcgc tttttggcca 1020
   ctccaggccc agaacttccc ccagtcagcc tctccaggcc cagctcttcc tcccagcaac 1080
   ctctgcaggc ccaaatcatc ctcaaattgg cctcttcttt cccagctcct gcctcctggt 1140
   ggcctctgaa gacccaaatc gtcctccagt tggtttttcc aggcccagct cctgcctttt 1200
   ggtggcctct ccaggtgcaa aacttcctcc catcagcctg tccaggccca gctcatgcct 1260
   cttggtggcc ttctcaggcc ctgcttttga cttggtggcc tcttcaggcc cagaacttga 1320
   actcaagtca gcctctccag gcccagctcc tgccttctta aggtctgtac aggcccagcc 1380
   tctacctcac agcggactct ccacacccag ctcttgcctc actgtagcct ccccagtcca 1440
   aaactcctgc cttttggcag cttcgacaag cccagctcct gcctttcaat gacctcttta 1500
   ggccccgctc attccttaca acggcctttc caggcccagt ttttcccttt tggcggcctc 1560
   tccaggccca gaacttcctc aagtcggcct ctttaggccc agttgctgcc tcctggcatc 1620
   ctctgcaggc cgagctcttc ctccctgctg tgtctacagg cccaactcct gcctcacaac 1680
   aacctccttg gactcagctt ctgcccagct cctggtggcc tttgtaggct caaaattttc 1740
   tcaaatcaag ctctccaggc ctactgtcag cctcgtggca gcctaaacag gcccagctcc 1800
   tgcctgacaa tggcctctcc aggcttttct cctgcctcgc agcaggcttt ccaggcccag 1860
   ctcttgcctc atggtggcct tccccggcca tgttcctatc tgacttctgg cagcctcaac 1920
   cggcccagct tctgcctcac actggcctct ctaggcccag ctcctttttc acagtggcct 1980
   cactaggccc atctcctacc tcagatctgc ctcccaagac ccagctcctg tctcatggtg 2040
   gtctctctta caccagctcc tgcctcacaa tggcctcgtc tggcccatct tctgcctcac 2100
   agtggccact caaggcccat cttttgcctc atggtagcct cttctggttt tgctcttgcc 2160
   tcacagttgc ctcttccaga tccagcttta agcctttgat ggtcaacagc atcaaggagc 2220
   ctaaagcttc cctggactct catttgttca ctttacagca gagtgcctta gcaaaaactg 2280
   tctcttaacc ttgagagtgg atttctgaca aatcgatagt aaattctgcc tgtgtggttt 2340
   c                                                                 2341
<210> 216
   <211>407
   <212> DNA
   <213> unknown
<220>
   <223> >gi|944178|gb|R85772|sr|IMAGp998I12605.2|cl|IMAGp9 98I12605/R85772 yq23e06.s1 Soares retina N2b4HR Homo sapiens cDNA clone IMAGE:274643 3', mRNA sequence.
<400> 216
   gacacaattc caatgtgtga acatttcagt tgtcatgttt cattcaacaa caccagtctc 60
   ccaacaaact ggctcaaatt tcagttacct tggtattcag atgagtaact gcatctcagt 120
   ttcttcagtc cacatattac aatgtgaata acagatgtac attgtgatca gtgaccggtc 180
   acgtcacttc cttcaaagtc tgtcaatgat tggtcattgc ccttctgtta tttggctcac 240
   acacagacag caaaacatgt gttgccttcc tgtctctcag cagtagacag atactataga 300
   ccaaataatt taaccattaa agcactacaa ctattgggat aatgctcttg ggtcacccta 360
   attctcttgg gggattgaaa ttaaaatgtg aaaatttgct ttatacc              407
<210> 217
   <211> 585
   <212> DNA
   <213> unknown
<220>
   <223> >gi|4373025|gb|AI479857|sr|IMAGp990F015343.1|cl|IM AGp998F015343|AI479857 tm70c01.x1 NCI_CGAP_Brn25 Homo sapiens cDNA clone IMAGE:2163456 3' similar to TR:Q13231 Q13231 CHITOTRIOSIDASE PRECURSOR. ;, mRNA sequence.
<221> misc_feature
   <222> 319
   <223> n=a, t, c, or g
<400> 217
   cattcagtaa ctatttattg gatgtctaat acatgcttag tatggtctct ggtcatgcaa 60
   cgaatgttaa ttcccctgtc tctttgaagg aaggactaat ggctggacac taatattaaa 120
   agagtcagtg gtattcaata aaagggcttg ggagagtagt gctcttgtca tgaaatagca 180
   cttcagtctg gaaatcctct aagtgatttc tgcactcagt tcctgccgta gcgtctggat 240
   gagggggtat cggccctggt tgcaggagaa gccggcaaag tcatttaagt ccagtgccca 300
   gaccatggcc ccgcccagnc ccttctgctt cagatagctg accttggttt tgaagctctc 360
   cacatcatca aagcccaccc actggttgtc ccggaagatg tagggcacct tctgatcctg 420
   gattctctgt ttggtggccc ccttccaaga gcagactata taataggcca acatcccttc 480
   tttcttggtg aaggggcctg gagggccaca ccctgtgtgt ggggccccct actcttggtt 540
   cttatgaggc ggcagggaga aggagccccc cgaggtggcc tcccc                585
<210> 218
   <211> 534
   <212> DNA
   <213> unknown
<220>
   <223> >sr|IMAGp998H171824.3|cl|IMAGp998B171824|gb|BX1158 55|oid|234566085 BX115855 Soares_testis_NHT Homo sapiens cDNA clone IMAGp998B171824 ; IMAGE:742576, mRNA sequence.
<400> 218
   ttggcagaca tgaggcaacg attgctcccg tccgtcacca gccttctcct tgtggccctg 60
   ctgtttccag gatcgtctca agccagacat gtgaaccact cagccactga ggctctcgga 120
   gaactcaggg aaagagcccc tgggcaaggc acaaacgggt ttcagctgct acgccacgca 180
   gtgaaacggg acctcttacc accgcgcacc ccaccttacc aagggacagg ccagcagcac 240
   agacaacgct gtggataagg agagtagaga cttcctcttc ctgcctcctg tcttctggag 300
   ttgtgaccgc agggtggccc aggtggatcg gcttcagagg ccaggaggca gctctctgca 360
   gccgaagagc aggagcctca cccagggtct gtggctctga ctaagcctgg actgcctctc 420
   ggctgtgctc cgtggactgg ctcctcaggg atccatgtga gagaccggag tatgatcctc 480
   agtgcgagga caaataaaag tagtgattat gtccacccca aaaaaaaaaa aaaa 534
<210> 219
   <211> 741
   <212> DNA
   <213> unknown
<220>
   <223> >sr|IMAGp998L29923.2|cl|IMAGp998L24923|gb|BX096399 |oid|234527172 BX096399 Soares pineal gland 3NbHPG Homo sapiens cDNA clone IMAGp998L24923 ; Image:396839, mRNA sequence.
<221> misc_feature
   <222> 687, 713
   <223> n=a, t, c, or g
<400> 219
   ttagtcccta attctagacc ttacccattt tacacacact attagctaaa tcgtgttcct 60
   gaggcatggc tctgattact ttactctggc tttagccttg gcctcatccg ttaaattgaa 120
   actgttcacc agacctgtat gcattaaaaa ttttttattt agaaaaatca gtgtcttgct 180
   agccatgtat gatagctatc aaattgatca ttctgtaaat ggtagcaata agtgtatcaa 240
   cactttgacc tcctctgtgt ctagtaacat ttgcatatag tacaactgag cacatagcac 300
   cagaatattg gaacagatgc ttctccaaag ccaagggttg ccttacattt agagtgggaa 360
   aagaggaaaa tgagttagca gaggagcagc ggggaggggg gtaaattaca ggacataatt 420
   tgcttctaaa aatttgtgtt tcgtgttgta gttatataat taacttttct attgtactga 480
   ttgacagatt tggcacagac atcctctcat tgtgaatata tatgaatata tcagagtttt 540
   ggactctggg gtatatttac ataaacacaa gtattctgaa agcattgata accacactgg 600
   tgtctcttaa ttttgtctat aagatgacct tataaaatcc ttcaagaatt atttaaatca 660
   agtgtacgaa gctctgcagt tttttgntat gtctgaaata ttcattaaaa agnttattaa 720
   aacctgaaaa aaaaaaaaaa a 741
<210>220
   <211> 393
   <212> DNA
   <213> unknown
<220>
   <223>sr|IMAGp998E135802.2|cl|IMAGp998E135802|gb|BX0968 12|oid|234527996 BX096812 NCI_CGAP_Lu24 Homo sapiens cDNA clone IMAGp998E135802 ; IMAGE:2338548, mRNA sequence.
<400> 220
   cccctcttcc caaagatgtg gtgacttaag aggctctcta agcacactgt tgactccaaa 60
   acatccgcag gtcagagcca ggtgggaagg tggtccgtgc aggatgtgcc aggccctgtg 120
   gcaggtcttg ccccatgagt ccatatgcca gtgggcagtt tccagtacac aggtgatggc 180
   agccagagcg cggccccaag tgcaacacgt tctttggtta ttggctcaga agcctggaaa 240
   caggagcttc tgcggacctt cacgcacgtg ttattcccaa aatgccacta gatggcagca 300
   gagaagaact tttccctgat ttaagcgctc tgcgagcagc cactcagcat tacactaaga 360
   attcagaccc ctggtactca ggggtcttcg aga 393
<210> 221
   <211> 620
   <212> DNA
   <213> unknown
<220>
   <223> >sr|IMAGp9980171153.1|cl|IMAGp998D1711531|gb|BX0985 06|oid|234531385 BX098506 Soares_pregnant_uterus-NbHPU Homo sapiens cDNA clone IMAGp998D171153 ; IMAGE:484960, mRNA sequence.
<400> 221
   cgaagggacc ctgggcgtct gaggccctga tccgggcggg ccttcaggat gttagcagtc 60
   cctgtccggt tgaaggtagg aagccgaaaa cccgagtggg ggacgaacag acttacctcc 120
   tgtcccgcaa aagatccact agatcgtcgt ctccaaaacc tgagagaccg cgaacgggtt 180
   ccagagccgc agcgttctct cagacctggc gttcaggaag attctagaga gcacgggcag 240
   gtcccggagg tctcagaccc gcaggcctgc ggggcggggg cggggtggcg cccaggacga 300
   ctccccgggc tcagcttggc tgcctgcctc cttctcaaac aatgagtacc tactgtgtgt 360
   agaagcagca ggacagcctg tcatcactgg gaaagaggcc agaagagccg tttggccagg 420
   gtctccaatt taggctttca acattatctc taaagaaggt tatacattat gtcggctcca 480
   ctggctgatg ggatcaaaga ccctccttgt atatccttaa acctgtactc tattaccagg 540
   ccactgtgcc tgtggagcac tgtaagttcc taccttttaa taaattttat aattatcttg 600
   tgagcaaaaa aaaaaaaaaa                                             620
<210> 222
   <211> 696
   <212> DNA
   <213> unknown
<220>
   <223> >sr|IMAGp998F17593.1|cl|IMAGp998F17593|gb|BX117945 |oid|234570266 BX117945 Soares melanocyte 2NbHM Homo sapiens cDNA clone IMAGp998F17593 ; IMAGE:269968, mRNA sequence.
<400> 222
   ctactgtacc ctggcctggg aacacattgc cctccccaca caaaactgga gggcttggct 60
   tctgcgtgtg agaaatcaac atttttaaag cacttgcctt ctaccaaccc cagcttgcaa 120
   tcactgggcc ttcccctcct atccaagggg ttggaggggc cccttggctc tccttttggc 180
   aggaggagcc tgcttcatta caccaatgac tctgccatcc ccctccctgg ccctagaccc 240
   caaacacatc tccctctacc caatttactc ttctcgcccc acctagggac agattccccc 300
   tgctcttttt gtcctagaaa ccccgctagt ttgggatggt agcgtctggg gtggggaggg 360
   cttccccttc cccactcgag ggtgcgggtg gggaaggggg ggtggggtgg agacagccct 420
   ggggcaggga ggatggtctc tccactgtag aaagtagagt aggattgtgg tcagacttaa 480
   tttgaggcat ctagtgaaga cacgtacaaa tccaccaagg aaaaagattt caaaagcaaa 540
   ataaaagcgg gaaataaaac agacccaaga ataatcaagt caaagtgatg ttgcacaaaa 600
   tgcagagaaa ccaagaaggg ggagggttaa tgtattaaat gtgctattaa gaacttaatt 660
   ttattaaaag tactattact taaaaaaaaa aaaaaa                           696
<210> 223
   <211> 215
   <212> DNA
   <213> unknown
<220>
   <223> >gi|917103|sr|IMAGp998N11360|el|IMAGp998N11360|gb| H410511H41051 yp52c06.s1 Homo sapiens cDNA clone 191050 3'.
<221> misc feature
   <222> 16, 36, 38, 44, 54, 123, 170, 211
   <223> n=a, t, c, or g
<400> 223
   tccaacccgc ggggancagg tgtccccgga ctgggntnct gttnggaaga tggncgaccc 60
   cgggcatgag ctggcagcag cactattacg gcggctcgcg gccaaattcg cgccctcgcc 120
   ggnccaccgc acagctggct gggcacagca ttgactacag ccaggagatn cacctgaaaa 180
   taagcaagaa aatcgcccag ctcaaccaag nttaa 215
<210> 224
   <211> 473
   <212> DNA
   <213> unknown
<220>
   <223> >sr|IMAGp998I152328.2|cl|IMAGp998I152328|gb|BX0952 10|oid|234524789 BX095210 NCI_CGAP_Co3 Homo sapiens cDNA clone IMAGp998I152328 ; IMAGE:936278, mRNA sequence.
<400> 224
   atgacctctt tagactcagc tcattttcac tgctacatct tcaagccatt ctcctgcctc 60
   ttggcaacct ctagtggccc agcttctgcc tcacagcagc ctctccatgc atgcctagct 120
   cctgcctctt tagggaactt acaggcctaa aactttctta atttgggctt ctcaggccca 180
   gctcctgcct tctgttggcc tctacaggcc tggcatcatc ctttcaacag cctctttagg 240
   cccggcctct ccaggaccaa aacatcctta agtcaacctc accaggcccg gctcctgtct 300
   ccttgcggcc tccagaggcc gagcttttgc ctgccaatgg cctctctagc cccagctttt 360
   gcctgccaat ggcctctcta gccccagctc ctgcctctca gcaccctctt taggcccagc 420
   tcctgcctta ataaatttga ataaattatt gttatgtgaa aaaaaaaaaa aaa 473
<210> 225
   <211> 279
   <212> DNA
   <213> unknown
<220>
   <223> >sr|IMAGp998I112449.2|cl|IMAGp998I112449|gb|BX1058 75|oid|234546131 BX105875 NCI_CGAP_Pr12 Homo sapiens cDNA clone IMAGp998I112449 ; IMAGE:982738, mRNA sequence
<400> 225
   aattcgcggc cgcgtcgact tttttttttt ttatcgtttt gttttaaata taaagagcta 60
   ttttagctct cttgaatcta aaaaagcaac ttaagtttgt cggttactgc tattgggatt 120
   accttatgaa atcataacat aatgttgttt ttaaatatga aatcattttc ctacgaattt 180
   cccaatttgt gtttcataaa cattccagta agagaaaatg attgattaca aagcagaata 240
   tcaaaggttt ttaataaatt gtcgacgcgg ccgcgaatt 279
<210> 226
   <211> 547
   <212> DNA
   <213> unknown
<220>
<223> >sr|IMAGp998AI7282.1|cl|IMAGp998AI7282|gb|BX091082 |oid|1234516531 BX091082 Soares infant brain 1NIB Homo sapiens cDNA clone IMAGp998A17282 ; IMAGE:50008, mRNA sequence.
<400> 226
   tttttttttt ttttaacact tcttaaatat aaagttttgc aacaaacctt tgatttatat 60
   caaatataat taaaaattaa caaatgaata cattgtatca aaaagtacga agatatacat 120
   tatcatgcac atacttgtat atatgtatat atgtatatat gttacatgca cacataactt 180
   gttcaaacta cagccactgc ttgactggtt ccagatgcag acaactgggt gatgcacttc 240
   aaatttggca gctcactatg agattcaggg gacatatatg atagccggag aagacacagt 300
   gagaaacaaa acccaaagtc aggaaaacaa gacataatgt ctcctttccc cgctccctgt 360
   agttttcatc aattccaatg cctagaagag tctagacaag ccagctaggc tgtgtgggtt 420
   tagaaaagtg gattctcgac ccaacactgc ctggggaaat ggctgtggtt ctgcatggaa 480
   tggacacatt cgacacccaa gataacatgg gctgggggct ggggaacaag agacaaaacg 540
   tcagtgt                                                           547
<210> 227
   <211> 328
   <212> DNA
   <213> unknown
<220>
   <223> >gi|32463731|gb|AI026885|sr|IMAGp998J014173.1|cl|IM AGp996J014173|AI026885 ov92c01.x1 Soares_testis_NHT Homo sapiens cDNA clone IMAGE:1644768 3', mRNA sequence.
<400> 227
   ttttgtattc actagacttt tatttcgttc cagggcttat gacagagccc aagagagtca 60
   gagaagcttt ctacaaattc agctgtcgca ttcattttct tacacactag agttgccact 120
   taaaaatact aaaataaaat aaaaaattaa tgttgaccat ctaacaggaa tacttgactc 180
   agctactaag ttacaaattt gcttcatttt tggaagacat ctagcatctg agaattggac 240
   ttttcccaga agttcacttg gtaacctcag gtaagtcctg gttcatcctg gagaggatcc 300
   gaggggaaag cctgatggga cccacggc                                    328
<210> 228
   <211> 262
   <212> DNA
   <213> unknown
<220>
   <223> >gi|4892899|gb|AI682667|sr|IMAGp9980235762.1|cl|IM AGp9980235762|AI682667 wc64e12.x1 NCI_CGAP_Pan1 Homo sapiens cDNA clone IMAGE:2323438 3', mRNA sequence.
<400> 228
   tttttttttt tttttttttt tttttttttt tttttttttt ttttttttta aacaattaaa 60
   aaatttatgg gcttccgggg gccccccatc agcgggcaaa aaggcccctt taagcaaaag 120
   gttttcagtt ttcccgggcc ccatttggtg aacaggaacc catccctggg gaaaattctt 180
   tttaaaaaaa ctgggcaaaa accccttggg ccgggaaggg ggattttttt ttttactttt 240
   ccttcccccc attttttcct tt                                          262
<210> 229
   <211> 477
   <212> DNA
   <213> unknown
<220>
   <223> >sr|IMAGp998C01743.1|cl|IMAGp998C01743|gb|BX099107 |oid|234532590 BX099107 Soares_fetal_heart_NbHH19W Homo sapiens cDNA clone IMAGp998C01743 ; IMAGE:327480, mRNA sequence.
<221> misc_feature
   <222> 470
   <223> n=a, t, c, or g
<400> 229
   gcaaggctag aaagatgcta cgttgggcct tcagtcacct gatcagcaga gagactctca 60
   gctgtggtac tgccctgtaa gaacctgccc ccgcaaaact ctggagtccc tgggacacac 120
   cctatccaag aagacccagg ggtggaacag cggctgctgt tgctcctggc ctcatcagcc 180
   tccaaactca accacaacca gctgcctctg cagttggaca agacttggcc cccggacaag 240
   actcgcccag cacttgcggc tgggcccggg gagcagtgag tggaaatccc ccacgagggt 300
   ctagctctac cacattcagg aggcctcagg aggccagcct gccatgagag cacatgtcct 360
   ctggccagga gtagtggctg agctctgtga tcgctgtgat gtggacccag cctccaggga 420
   gcagagtgtc ggggatggag gggcccagcc tggactgact gctacttccn gtctctg    477
<210> 230
   <211> 468
   <212> DNA
   <213> unknown
<220>
   <223> >gi|4081545|gb|AI394339|sr|IMAGp998D175081.1|cl|IM AGp998D175081|AI344339 tc03g09.x1 NCI_CGAP_Co16 Homo sapiens cDNA clone IMAGE:2062816 3' similar to contains TAR1-t3 TAR1 repetitive element ;, mRNA sequence.
<400> 230
   gcggccgcca aactccgtaa ggatggcgtc ggccgcccgg cctgcccctc cctgggcccc 60
   gctgccctgc acctccgcct tcagccatag gcgctggagg ttgtgctgac gctcgaaggt 120
   ccagaccagg gcctcctcgc gactgcgggc aaaggtgcac cggtttcggt ggcgccggca 180
   gccgagccca gggctgtagt agtggcagac ttggtagcac aggggccttg ggaaggtggg 240
   ccggcagccc accacgcgcc agaccttgct cttggtggcc tgcttaaagc gggccagcag 300
   gatctcgcgg gagcagtcat gctccaccct acggaggacg taggtgcttt cattgagccg 360
   ctgggtgcaa cgggaggcag cccagaccca ggtccaccag ggcacacagc cgggccaggg 420
   acgggcctct ggtggccgcg gggctgttgg gcagcagggt ggaccctg               468
<210> 231
   <211> 367
   <212> DNA
   <213> unknown
<220>
   <223> >sr|IMAGp998B2974.1|cl|IMAGp998B2474|gb|BX089396|o id|234513140 BX089396 Soares infant brain 1NIB Homo sapiens cDNA clone IMAGp998B2474 ; IMAGE:23877, mRNA sequence.
<221> misc_feature
   <222> 55
   <223> n=a, t, c, or g
<400> 231
   tttttttttt ttttaagtaa tggtaaagtt tatttcattt ttaataacaa ttagnaggac 60
   aaaatgttta aaatttgcag tttaaaacat gcacattcac aaaaggccaa tgacacataa 120
   cactgcatag aaataatatt actcaatttt aataactata aaacacagtg catcaaacat 180
   caagataaac aaacctgaga aaactactat aaccacagat tcaatactct ccactcatgc 240
   agcttcacaa tttctacagc agtttcagca ggaatggttt ttcagggagc tgaaaatact 300
   actttatctt taacgcaaaa ctgcagtttt ctgtagtagc tgccttccaa ggctgccctg 360
   tcttttc                                                           367
<210> 232
   <211> 498
   <212> DNA
   <213> unknown
<220>
   <223> >sr|IMAGp998I094494.2|cl|IMAGp998I094494|gb|BX0959 09|oid|234526189 BX095909 Scares_testis_NHT Homo sapiens cDNA clone IMAGp9981094494 IMAGE:1837520, RNA sequence
<400> 232
   gcaacctcta gtggcccagc ttctgcctca cagcagcctc tccatgcatg cctagctcct 60
   gcctctttag ggaacttaca ggcctaaaac tttcttaatt tgggcttctc aggcccagct 120
   cctgccttct gttggcctct acaggcctgg catcatcctt tcaacagcct ctttaggccc 180
   ggcctctcca ggaccaaaac atccttaagt caacctcacc aggcccggct cctgtctcct 240
   tgcggcctcc agaggccgag cttttgcctg ccaatggcct ctctagcccc agcttttgcc 300
   tgccaatggc ctctctagcc ccagcttctg cctttcatcg gtctctccag gcttagctcc 360
   tttctcttca cggcctctgc aggcctaaaa cttcctcaat ttggcatctc caggcccagc 420
   tcctgcctcc aggccgcctc tgcaggccta tctcaagcct tacaacagcc tctttacccc 480
   cagctccttt ctccgact                                              498
<210> 233
   <211> 591
   <212> DNA
   <213> unknown
<220>
   <223> >sr|IMAGp998B08733.1|cl|IMAGp998B08733|gb|BX102114 |oid|234538608 BX102114 Soares_senescent_fibroblasts NbHSF Homo sapiens cDNA clone IMAGp998B08733 ; IMAGE:323623, mRNA sequence.
<221> misc_feature
   <222> 462, 482, 513, 558, 564
   <223> n=a, t, c, or g
<400> 233
   ataactgata cttagctaac aattgtccat aacttgttta agtgtaagaa ataccatttc 60
   aggaatgaaa aagaaggaat actactttct aagaaagaag atcttataat agacatattt 120
   agtaggttaa actactcctt tgaaagaata agttttggtt caaagtcaat aatgaagacg 180
   agatttgttt ttctcttctc tggctgattt ttaaggatat tatgtagttc atttagttaa 240
   cagaattgaa atgtttgata tggcaagata ggtatggtaa tttcaaagtg aattgggaat 300
   tcctctggct catagaaccc tttttttttt cctttaagta ttcttgagat acaaaaaaaa 360
   aaagtaaata aaatttcaaa aaaaaagttc cggatctgtt tttaagctcc atctggtcct 420
   cataacctgc aagatttttc ttaaaacctt tcagctgaaa gngggggtaa aggtggagta 480
   anctgtggat tcgcttctgt tgtcttttaa aangtcaaat atataacatg taattttttt 540
   aaaaaccacc agatacanaa atgngcttta acatcagcct gaaacctaaa t         591
<210> 234
   <211> 8483
   <212> DNA
   <213> unknown
<220>
   <223> >Homo sapiens|chromosome:NCBI34:4:155279369:155287851:1
<400> 234
   ataggtaaaa caggatgtaa agtttatata caagaatata atgtttatct gaaatattta 60
   cagtgttggt taaagcaata tttttacaac ttttaaaggt aaactactat gtatattaca 120
   ggtaagctac aatgggttta atttgcaaaa gttaagtaag aaatgtttta aacaaggctt 180
   aaagtactca agtcaattat aaaatttata tcttttgcct tttacttgaa gaaatcatgc 240
   tatagaaatg gttaatgtgc ttctaataaa tggaagtatt gtagctggaa tgtgatacat 300
   gtaacagttt aagttcccat tgaaggtata aaatgatgaa ttgttgtaag acttagacac 360
   tgagtctcag tctggagctg atgaagatgt tgagataaca gccagcttta tctcaacagg 420
   gtttgtgacc cacaagtttg ggccacagag aaaattgaag caatttgcat gttatgacaa 480
   cctcagtggg aagtgaaaat cagctgactc aaaacaacaa acaacaacca accagaccca 540
   agtcacagtt gcacctattc aaaactagct ttaaagtgag ctatttttaa acttcataaa 600
   aatattcatg attttattag tttgaatatt tctacaagat tcgggtgggc ttttccttta 660
   ggtgaaaaca gctatccact cctgtggcct tataactcag gaaatgctgg ggatgcaaac 720
   gtgcaaaagg cagggggaag ctgcccaggc tgagactgga gcagctagga gtgtgcttgg 780
   ggaacgggag ctgagatccc ggagcagaaa tggtcagccg tgctctggag caggcctgtc 840
   ggagccatca ggatgccggg actagcactg cagcttgcgg atgctgcggg agatgcgctt 900
   gaactctctc tgccccttct gccaccgctt caccgaggtg atcaccagct ccccaccctg 960
   tttctgtccc atgaccagat agggcgcgtt gatgtcgttc atctcctcac aggtgcactg 1020
   caagctgtct ttgagccaca gcaccgattt cttcaggtcc ctttcggaca caccgttcag 1080
   cttgtaaatg gtcttgctct tggtctccag gatgattttg gtatctcggt tgatgtaggt 1140
   tatctccttc acttttattt tcagtgctat gagggagggc agaaagagtc atgccagtta 1200
   taatgaggga atacagtata cctcccccca ttctgccaac tcgtgtgact tggcctggat 1260
   gaggaaagag aggcttccta gagctaatgg gaccctcaga ggctgcagat cgggggttgg 1320
   gagctagggg cagcaggtgg ctccactgct aaagcctcaa agcatttgcc aggcacttgg 1380
   tttccaatca gccatttttt caaaactctc tctttgcggg actcaattaa cttttcttct 1440
   cctttcctca ctcacccttg tgtttgtaag aaagttgcat gtggtgtgtg ctctctccaa 1500
   caaatgactt cctctgttgg aaacttaagc atgaaaagaa atgcctgact tttaggtgaa 1560
   atatgatccc tctgtggccg taaagtttaa actcaattta ttatgcaaga ggtttttgga 1620
   caaacctgta aggaaactgt gtcacgcaat cattgttttc taaagatcag attacaggga 1680
   aggagaattg atcttacagc tgcatttgat ttggtaaagc aggagaaaca atatgcaagt 1740
   ctctttttaa cagcacaacg tggttagtca taaaagtgtt tttctgaaga cctcatcctt 1800
   atatgctctc atttgccagt ccaaggccaa ggcgtttacg gatcatatat gtactgtttc 1860
   caaaatgtat atgtagactc gttagaatgc gaagaccaaa agtaagtggt ttactataaa 1920
   ggacacgttt tatagtcatg aacagtatgc tctgggaaag aaagccctac aaacgtgtat 1980
   gctggaatta aatttcaaga ctccatgaaa gcaggaaaag aatagaattt taaggttaga 2040
   agggatcatc ccattaaaat tgcttgttaa tacacaaaga atcttaaatc cagagaagtt 2100
   tagtgacttg catgaggtca cccagtttag gttgtttgct agaactccag ttgccaaacc 2160
   tcctcctgta ataccactgt ggtgtcttga ggcaatgtcc atgtttttgt agtggttgca 2220
   gcataaactt gcccagctgc aatgtgtcct aagaccctgt gtgcccctga cagctattct 2280
   gtatagatga tgtgtaagta tgacttcctg actttgatgg agtttaatct atgtttaaat 2340
   tagtaataga ggaagatttt tctgtgttgc gtacaactgt ttttgagatt ttcttttttt 2400
   tttttttaaa tatcagaatg ggaatttgac tgagtatggc tttctagtca accaggaaat 2460
   tatagaatct ctagcaggta aatcaaaaat gctatgactt ggccaaccat ggttttgtct 2520
   ggtatgatat gcatctgaac tgtggtactg aattctcagt ttttgagact caagtcgtgg 2580
   aataagccat acttgtggaa atgtttcatg cttttgtgtc ctctgcattg ggagtagggt 2640
   gttggataga cctcaccatg gtgggagaaa tggactgatt acactttacc tgggagcctt 2700
   gtctgtagta caggtagggc atatataaga tttcacctgg ccaaatgaaa cattctcaag 2760
   ctccaaacat cagaaaaaac catcactttt aaccaggagg aagatttttt agtcatcttt 2820
   gtctagctct aaaataacta attttgcaaa tgtttatcat gagaaaacat tagggatttt 2880
   ctccctggat tttcagctag gcccaaacag agagaattga atcccccaca taacctttcc 2940
   tgtcctggag gatgtgactg aggatgcagg atgtgatcgc aggcagaggt gatgatactt 3000
   tctggtccta caataggttg tatatatagg aaggtagttc tgaaccattt tttattcacc 3060
   agtacttgca ccactgaatt tcccttgtac ttcaggagca cagtagggaa gaggtgatgc 3120
   cttcccagag tagtcaagtc aaataaatca gatttctatg cttgttcctc ctacttttcc 3180
   attattcaca aagaacagag gcaagatgga atagtgctta taaaccaata atcttatgat 3240
   gcagttctta aaattctgct tcattatccc tctatgggtc aaaaagagtg aagaaataga 3300
   aaacaaagct tgtgaggtat ctatgcatgt gataccttaa gtgtgataac ttctgtgttg 3360
   ttttgaaagg ctttctctct gtaaggaggg agcacactaa ggcacacagg catccatgtt 3420
   ttggctcatg tcttgatagg gcatccttag aaagggggtc ttctgtaagg tcagccacaa 3480
   agcaagcaag taataggtgt gtgtgcatgt ttcccaaagc caattgttat ggagctccac 3540
   agtgtaacaa tcaacttttc atggaagtca ggaaggatct gttttatgtg cagtgcaaag 3600
   gatataaact gccagaagaa catgatattt tataggcttt ttaatagaaa ttttataaaa 3660
   tgatattttt ctatcttgcc cttaatgaag tgatgtagca tgggggctga gaactgtgtt 3720
   ttggtaaggc tctggtgatt tggaattatc tgctgctaac atgcctggct cttagccagt 3780
   ttgagggttg tatacatttg aaaacaggca tatgaaaccc cgtcagcccc atttgtggct 3840
   cctcctgagg ggaaaagttg ctaaggactt tgagctacct cccttctttc tctttcctca 3900
   gcagtgtgaa gcctttccca acaccgggac tatggtagtt ctgggaccag tcagagttaa 3960
   acccaggaca ctttctggaa gagacctgga gttgtcaaag agcagggatt ggatggttcc 4020
   agagttttag ggacctgacc aggcctgaga tttctgggat ggcaaggtaa ggagagtgag 4080
   agctggagtg atttccctca gtctcgagga ctatgttgaa tatgtagaag tgttaatatg 4140
   ggtctattag actccattat tgattgacag agcagggaac atatctgctt atgagtaagt 4200
   tcagccttgt gcctgaataa atactttttt aaaaggtacc aataattcca acacaccaaa 4260
   aggtacctgc ccttggaaaa ggataaaagc aagaatgcct ctgggtgaag ttcctggtca 4320
   ttcccaactc tgaatttagc attgatcctg tgtctagctg ggctcctctt ctgacctgag 4380
   agccctgaag tcagcagatg aggtgctggt agagttccat gtagacaaag ccccagctct 4440
   ctttcccctt ataatcctgt cttcagggga gtgacttgca acttatggaa catatcacat 4500
   gtgtttttta ttgtccccaa acaccatgtg atctatcatg tatcttgcta aagtaaattt 4560
   ctaagctttt tttttccttt ttaaaactat tttcgaaact gtggcaaatg tgtctagact 4620
   tagtcataca aaatttacaa aaacctttta aaagggattt taagatggtg ttttaaggtt 4680
   gttaggtgtt ccatgttagg tgtcttctca gtaagccact cttctttttc aacttaagga 4740
   acccctcctt gacaaattat taatcagtgc cttggatata ttcactaatg gtagaacaca 4800
   gaggtttaaa atcattatgg gttataaaga aagtaaaaat atttttattt gtggccaatt 4860
   taaagccttg aatctgtttg gctggggcta atatttacac ttccatccct ggtgcacatg 4920
   ttaagtaatc aaaagaaaat agaactcggg attatgcctg tcggcaaaaa aaaaaaaaaa 4980
   aaaaaaggga tttaaaatgt gccactggaa attgataatc tgaataattt tcattatttg 5040
   attattggct taacagaaca atgagtctca cctaacaaga tacaactcaa tgctagacag 5100
   ttacataagg aggaaactag gatagatcaa gttagatgat tttgggatac atgagctata 5160
   atggtgtttt acccaacctt tatctagcaa catcataatg atttgcacct tgaagtttta 5220
   ctggtaaata aaacttctga atgtgaaatc tgttgttgtt gttgtattac ttaccaaaat 5280
   catttttaca aagcgtttcc attatgtcgt tgtcatcatc atttttattt ttgcaggctt 5340
   cacatacctt tggagctaga aatgtgaaaa acagtcttta gtaagtttgc ttaaaagaga 5400
   acagtaggtg gcgtttgtaa acaactcaaa gggacttatc cagacaaaag tagcaattgt 5460
   cctatttaac ctcagcttct gcctcctcta taaaagcagg aacactcaat cctaagaaag 5520
   ttttcccatg cttcccaatt tttttggggg ggaggggtgg tgggagcaaa atgagaatgc 5580
   ctatatgttg ttgtgtttaa taaattatga ctctcaaaag ggagttgtgg gcagagtata 5640
   caagtgtatg tagttatagt taactgagac agggtgcagc ttttcagctc atcctcccaa 5700
   cctctgggaa attcagtctg aaaaagttat tcccaaggcc ttttgaagga gaaagcaccc 5760
   aaagaaccaa agatacttga ggaatttggg aaaactggta actagaaaat atagacaaaa 5820
   gattgtcttt ctcagccaca ggacaccagt gtagttggct ctactcggca gagctctgta 5880
   tctcaggggg gccaagtctg gactgggagg ggtgactggc tagcgttcca catgtgggct 5940
   gtttccacct tgaaaacaca tctcactaat tacaacagcc tgctctaagt ggttctgtgt 6000
   caccttgtgc ctctgccaag attgtgcttg agttaggcag cattcatttc attgttatct 6060
   gcaagctccc tggtacaggt tcttcttgcc cttctgtgcc tgtttcaaac tctcaaaaca 6120
   aacaaataaa taaacctgtt tctgaagcta atcaaagacg aagcaaaaat ggtttagtgg 6180
   tctgatcaaa cttagggatt gtatactagc acaattatca gaagacctgc taaaagctct 6240
   tctaagagtt tgatttcttg ggagaagtaa aaaaaaaaaa aaaagaacaa ttatattgcc 6300
   cttctctcat ttataaatgt gttaattatg tctttaaaat ttaggtgaaa tgagattttc 6360
   actaatgtga aatttaatgt tcatagaaaa ctatgtgact taatagaagc gttatttatc 6420
   aaattagaaa aatcacaaag ctggaatttt gaagaacata aacttttatt ttgaaggaga 6480
   aaagtggctt tttggagaaa tgtaagatgc gtaatgttgc tgctgatagt gggaagaaat 6540
   aaaagcaaca tacataaaac tgcccttaag tcccctcacc caaacaaaat caaacatttc 6600
   caattaggag ctctcaggat gtatataaaa tattactaac ctgtattggt tggtctatgg 6660
   tacatgttgg acaaatgttt gaaaaatgcc aaacatttgc aataaattag aaatctaacc 6720
   aataagatga ttgcaaactc atggtccctg ccaggaaaga ttgctaaaga actttgcatt 6780
   tgctttaact aatgatgtct gtaggaaaac tactagagat tgaaagggag aggctgctga 6840
   agccctgaaa gggattagga agctcccttt tccaacccag gggacccaaa tcagctcaga 6900
   ggcattgtat atgttttcct ttcagaaaaa atattgagag cccatgaggt tttcattgct 6960
   ttgatggttt ttgcacagga actctccctc cgttttctaa aaacttggga atctcttttg 7020
   catgattttc tccagctaat caaaaccact cctaagttaa aaactgatag caatagaaaa 7080
   gtgatttcca gagattccct ttatgccctc ttcctcaagt atctgagcag ttacaaccaa 7140
   tgttcaattt tccacacact ccgtgccagt cccggtttta tttccaaaat ctaaattgac 7200
   aaaggacaaa aagaccctta aaaggttatc tacttgtgca tcctggtttt cctttctgta 7260
   caggaaatgt tcctgtgact agaatcctgc ccagtggtag gaaggtgcag aatgaggctg 7320
   agcgaggggc ctctggccag aatgctcacc acaaactcct ccaccaaaca ccacttccaa 7380
   cgctctgcgc agctccagtg ctagcatctt taacaataat cgtgtatcta ttttttttta 7440
   aaccgccgtt cagttcttcg tttttttagt tttgttgttt ttgtttgttt gttttatttt 7500
   taaactgccc ttaagtatag ctggtacaac tcgggaggag aatgcgcgag acttgggcgg 7560
   cgtgggaggg gtgcctcaaa tttgatacag gcttgttgtg atgacagacc aggtcaggca 7620
   gaacttctgc ccttcccgct actggcaccc caagcaggga tgcactggga tgcgtggcag 7680
   gggcgggatc tcctgggagc gtctcagccc agcagggagt ggggaagcaa gagggaaggc 7740
   ttaccttcct cggtggctgg caggaggtgg tcgctgctag cgagggggat gcaaaggtcg 7800
   ttgtcctggg ggaaacggtc gcactcaagc atgtcgggcc aggggaagcc gaaggcggac 7860
   atgaccgggg cgcagcggtc cttcacctgc acgcagagcg agtggcatgg ctggatggtc 7920
   tcgtctaggt catcgaggca gacgggggcg aagagcgagc acaggaactt cttggtgtcc 7980
   gggtggcact gcttcatgac cagcgggatc caagcgccgg cctgctccag cacctccttc 8040
   atggtctcgt ggcccagcag gttgggcagc cgcatgttct ggtattcgat gccgtggcac 8100
   agctgcaggt tggcagggat gggcttgcaa ttgctgcgct tgtaggagaa gtcgggctgg 8160
   ccaaagagga agagcccgcg cgccgagccc aggcagcagt gcgaggcgag gaagagcagc 8220
   agcagcgagc cagggccctg cagcatcgtg ggcgcgcgac cccgaggggg cagagggagc 8280
   ggagccgggg aagggcgagg cggccggagt tcgagcttgt cccgggcccg ctctcttcgc 8340
   tgggtgcgac tcggggcccc gaaaagctgg cagccggcgg ctggggcgcg gagaagcggg 8400
   acaccgggag gacagcgcgg gcgaggcgct gcaagcccgc gcgcagctcc ggggggctcc 8460
   gacccggggg agcagaatga gcc                                         8483
<210> 235
   <211> 153
   <212> DNA
   <213> unknown
<220>
   <223> >gi|2714120|gb|AA704202|sr|IMAGp998K211090,1|cl|IM AGp998K211090|AA704202 zj77e11.s1 Soares_fetal_liver_spleen_1NFLS_S1 Homo sapiens cDNA clone IMAGE:460940 3', mRNA sequence.
<400> 235
   aaatactttt actgggatac tcagtggtag aaaatggtac ttggacagtt gaactgattt 60
   ttagcagtaa ccaagatctg tcagctctga gcttggggaa catcattctg tttggtaccc 120
   aaagcaacaa gtgactattg agcaggggtt acc                              153
<210> 236
   <211> 382
   <212> DNA
   <213> unknown
<220>
   <223> >gi|21864311|gb|AA461311|sr|IMAGp998A181964.1|cl|IM AGp99BA181964|AA461311 2x65b09.s1 Soares_total_fetus_Nb2HF8_9w Homo sapiens cDNA clone IMAGE:796313 3', mRNA sequence.
<400> 236
   ttacaatttt actctttctt agcaccaact ccatcatgca agactgacag ggaagaatca 60
   caacatgtaa aagctaaaag aaacccctta tacagtccag tgcttacaac ctttaaaaca 120
   cattaaaagc ttgaggaatt tgttaacatg caaattccca ggcctaacca cagagacact 180
   gatttagatg atatggggta gatcccagta aagcagccca ggtgtttttg aaatagatgg 240
   atcatgaacc aaactttagg aaataacaat atactgcctc acttttagat taagaaattt 300
   aaactcagag agcttagtga ttttcctgag gccatgtaat tagctagggg caaagaagac 360
   attagactag tagcctccta tc 382
<210> 237
   <211> 672
   <212> DNA
   <213> unknown
<220>
   <223> >sr|IMAGp998H04617.1|cl|IMAGp998H04617|gb|BX1001500 |oid|234534676 BX100150 Soares_multiple_sclerosis_2NbHMSP Homo sapiens cDNA clone IMAGp998H04617 ; IMAGE:279219, mRNA sequence.
<221> misc_feature
   <222> 588, 592, 604, 618, 621, 623, 630, 644, 645, 661, 663, 664, 669, 672
   <223> n=a, t, c, or g
<400> 237
   atacctgata cgcgcttctg ggctttgtct gtggctgcca ggtggtcagc gtgtttacgg 60
   aggaagagga cagctttgat ttcattggtg gatttgatcc atttcctctc taccatgtca 120
   atgaaaagcc atccagtctc ttgtccaagc aggtgtactt gcctgcgtaa gtgaggaaac 180
   agctgatcct gctcctgtgg cctccagcct cagcgaccga ccagtgacaa tgacaggagc 240
   tcccaggcct tgggacgcgc ccccacccag caccccccag gcggccggca gcacctgccc 300
   tgggttctaa gtactggaca ccagccaggg cggcagggca gtgccacggc tggctgcagc 360
   gtcaagagag tttgtaattt cctttctctt aaaaaaaaaa aagaaaagaa aacatacaaa 420
   agaaaaggca aaaccccaca tgcccacctc ctctggcaac atgggggtca cagctctgcc 480
   cccaggctgt cgtctcgtcg aggagcccct ccctcaggtg cccacctggg gctgctggac 540
   cctcgggctg caagcactgc tgctgggatg cagccctccc caggaagnca angcgaggcc 600
   cganacccct caagcggnga ngncccctgn tgaacatgga gggnntccta acccccaaac 660
   ncnngccana an 672
<210> 238
   <211> 551
   <212> DNA
   <213> unknown
<220>
   <223> >sr|IMAGp998I12670.3|cl|IMAGp998I12670|gb|BX089595 |oid|234513538 BX089595 Soares_fetal_lung_NbHL19W Homo sapiens cDNA clone IMAGp998I12670 ; IMAGE:299603, mRNA sequence
<400> 238
   gaggagaaga aatagaagaa agttctgcaa caaccatgtc tgagaacttc cagtattaat 60
   gtcagacacc aaaccaaaga tccaggaagc tccgagaaca ccaggcagaa taaatgccaa 120
   caacctacac ttggacatat aattttcaaa ctatatgaaa taaaagataa aggaaaactc 180
   tgaaagaaac cagaggtggg gcagaaaaca ccttacctac agagacacaa agataagaac 240
   tgcattcaac attgcagaaa ctgtgaaagc aagaagacag tgaaatgaaa aattcaaaat 300
   gttgacagaa aaaaacccac caacctaagt ttctgtaccc actgaaaaca cccttcaaaa 360
   gtgaaggaga attaaggcct tcctcagaaa aataaaaatt caagaaactt gttgccagga 420
   gacctgtctt gcaagaaatt ttaaatgaaa ttctttaggg aaacaaaaga tatgtaactg 480
   aaacctggat caacattttt ttaaaaagag cattaaagaa agaattgtgg tacaataaaa 540
   aaaaaaaaaa a 551
<210> 239
   <211> 239
   <212> DNA
   <213> unknown
<220>
   <223> >gi|5445722|gb|AI825133|sr|IMAGp998D145711.1|cl|IM AGp998D145711|AI825133 wb04h07.x1 NCI_CGAP_GC6 Homo sapiens cDNA clone IMAGE:230733 3', mRNA sequence.
<400> 239
   tttttttttt tttttttttt tttttttttt ttcaaaaatg gaaaaatttt atgtttttta 60
   tataaccatt ccctagggtt aacagatttt atttttatga tacatatctt taagtggggg 120
   cccactgggg acatagcaaa tttgcaataa atccttatta agctgaaggg aactaacaaa 180
   aaccattaat tacccctttt ttaacaccca aaccagtttt ggtttttaag ggtttgggg  239
<210> 240
   <211> 525
   <212> DNA
   <213> unknown
<220>
   <223> >sr|IMAGp998L06178.1|cl|IMAGp998L06178|gb|BX113044 |oid|234560465 BX113044 Soares infant brain 1NIB Homo sapiens cDNA clone IMAGp998L06178 ; IMAGE:38015, mRNA sequence.
<221> misc_feature
   <222> 1, 13, 35
   <223> n=a, t, c, or g
<400> 240
   nacaacactg agnactcacg ataaaacaga cggcncatgt ctacaactat atccccccac 60
   ctgtgggtca gatgtgccaa aaccccatct acatgcagaa ggaaggagac ccagtagcct 120
   attaccgaaa cctgcaagag ttcagctata gcaacctgga ggagaaaaaa gaagagccag 180
   ccacacctgc ttacacaata agtgccactg agctgctaga aaagcaggcc acaccaagag 240
   agcctgagct gctgtattaa aatattgctg agcgagtcaa ggaacttccc agcgcaggcc 300
   tagtccacta taacttttgt accttaccta aaaggcagtt tgccccttcc tatgaatctc 360
   gacgccaaaa ccaagacaga atcaataaaa ccgttttata tggaactccc aggaaatgct 420
   ttgtggggca gtcaaaaccc aaccaccctt tactgcaagc taagccgcaa tcagaaccgg 480
   actacctcga agttctggaa aaacaaactg caatcagtca gctgt                525
<210> 241
   <211> 364
   <212> DNA
   <213> unknown
<220>
   <223> >sr|IMKGp998I135799.2|cl|IMAGp998I135799|gb|BX1007 17|oid|234535810 BX100717 NCI_CGAP_Lu24 Homo sapiens cDNA clone IMAGp998I135799 ; IMAGE:2337492, mRNA sequence.
<400> 241
   ttttttttta catcgacagt tgtattagag aaaagcttaa agacaaggaa aaattaagga 60
   aatgaggcac aaaacgtaga gtagatacaa ccaacaaaac caaattggat tctttgagaa 120
   gattagtaca cttgagaaag ccctggtaca ctagtcagag aaaagagagc agggacaaat 180
   tacaatgtca gaatctacga acccttcaga cataaaaact tgaggctgtc tcaaaagtca 240
   gtgggaaacg ttaaatgcaa ttgacaaatt actaggaaaa aactgctatc agaagaaata 300
   gaacatgtta ctagctcctg tctttcagaa atcttaccta tatcttgtca aaaaaaaaaa 360
   aaaa 364
<210> 242
   <211> 493
   <212> DNA
   <213> unknown
<220>
   <223> >sr|IMAGp998D154762.2|cl|IMAGp998D154762|gb|BX1032 41|oid|234540862 BX103241 Soares_total_fetus_Nb2HF8_9w Homo sapiens cDNA clone IMAGp998D154762 ; IMAGE:1940318, mRNA sequence.
<400> 242
   aggtttttcc agaattagtc tggatcagtg cctaacaaag ctgttattaa ttctccacac 60
   atgagtctgg ctaactgggc cagctctcat gttttactcc tgtctggcta caaatagcac 120
   tgtgcatagt agccctgttt ccccatggtg ttggactgtg gtaaagtatg ttgtgacata 180
   ctggaattct aataatcctg ttgtaagtat gtacagaatc tatgtaactt attgttgaca 240
   tacagaggtt tgggaagtag tctaatggac attccttaca atagaatggc tgggaaggga 300
   aagaaaaaaa aaaaaaaact acctaactcc aatcttaatg ttgtagtttt atagcaaaca 360
   aaaattgtca gctttttgta ttgaaaggtc agtggtggta agacaaggtg tcttgtaaat 420
   taagatttta agaagtgcat taaaatgttt tagaattact ctgggaattc tgtatatcac 480
   actaaaattt tta 493
<210> 243
   <211> 751
   <212> DNA
   <213> unknown
<220>
   <223> >sr|IMAGp998P20366.1|cl|IMAGp998P20366|gb|BX097677 |oid|234529727 BX097677 Soares fetal liver spleen 1NFLS Homo sapiens cDNA clone IMACp998P20366 ; IMAGE:193411, mRNA sequence.
<400> 243
   caccctgcag ccccagcact gcctggctcc acgtgcctcc tggtctcagt atggcgctgt 60
   cctgggttct tacagtcctg agcctcctac ctctgctgga agcccagatc ccattgtgtg 120
   ccaacctagt accggtgccc atcaccaacg ccaccctgga ccggatcact ggcaagtggt 180
   tttatatcgc atcggccttt cgaaacgagg agtacaataa gtcggttcag gagatccaag 240
   caaccttctt ttactttacc cccaacaaga cagaggacac gatctttctc agagagtacc 300
   agacccgcca gaaccagtgc ttctataact ccagttacct gaatgtccag cgggagaatg 360
   ggaccgtctc cagatacgag ggaggccgag aacatgttgc tcacctgctg ttccttaggg 420
   acaccaagac cttgatgttt ggttcctacc tggacgatga gaagaactgg gggctgtctt 480
   tctatgctga caagccagag acgaccaagg agcaactggg agagttctac gaagctctcg 540
   actgcttgtg cattcccagg tcagatgtca tgtacaccga ctggaaaaag gataagtgtg 600
   agccactgga gaagcagcac gagaaggaga ggaaacagga ggagggggaa tcctagcagg 660
   acacagcctt ggatcaggac agagacttgg gggccatcct gcccctccaa cccgacatgt 720
   gtacctcagc tttttccctc acttgcatca a 751
<210> 244
   <211> 276
   <212> DNA
   <213> unknown
<220>
   <223> >gi|3785069|gb|AI216028|sr|IMAGp998C034512.1|cl|IM AGp998C034512|AI216028 qh10e02.×1 Soares_NFL_T_CBC_S1 Homo sapiens cDNA clone IMAGE:1844282 3', mRNA sequence.
<400> 244
   tttgtgtgat cgcacttatt taacatttat tttgagttag ggtgtgagtg ctggggcagg 60
   tgactaacca tcccctgccc tctgtggtgg cctgagagag aagcaggaat gccccgtgca 120
   ggttggggat cagcaaacag tctagaccag cttctctcct gtatcccgtt tggagagtcc 180
   ctgggtcagg ggccagtcca tcctgcagca ttgaagggcg tgaggaatga gcagggctgt 240
   ggggctcaca gagcaggcag ctctggggag aagcgg 276
<210> 245
   <211> 468
   <212> DNA
   <213> unknown
<220>
   <223> >sr|IMAGp998G01115.1|cl|IMAGp998G01115|gb|BX111632 |oid|234557641 BX111632 Soares fetal liver spleen 1NFLS Homo sapiens cDNA clone IMAGp998G01115 ; IMAGE:122136, mRNA sequence.
<400> 245
   acagttctcc ctgcccctag tatgcagcag catttgtttc cccttggtaa tacacccgag 60
   tcagtaaacc ccttatttgc ttgttggttc agtgcccaga agggcccaaa acagcaggtg 120
   agcctcaatg tacaattcag tgagctgttg tcttgtgttc cccggaagca ttcctcctgt 180
   aaaaaccaag ccctccaaac cagtagaact ccagttgagg ggacaggtga gaggaaccac 240
   tcctacttct atcccttgat tcctggaccc gtgaatcctg gttataggag agacaacaca 300
   tgcaaagcag aaccttgggg gcagcaccca aaagttgtct cccaagtggt gctgtaacgg 360
   agtcttcacg aggccgtttc actctaatgt ttgtccagct gcttcctcct gctgagatac 420
   gtggtatgac tgtgattacc tggtcacaag cccattgcct cactttct 468
<210> 246
   <211> 752
   <212> DNA
   <213> unknown
<220>
   <223> >sr|IMAGp998B22787.1|cl|IMAGp998B22787|gb|BX109149 |oid|234552677 BX109149 Soares_fetal_heart_NbHH19W Homo sapiens cDNA clone IMAGp998B22787 ; IMAGE:344373, mRNA sequence.
<400> 246
   aggctagtgg ctggacctgc actcagatgg caattcaggt ccagttcatg agatcttgga 60
   ctgttttgac aaatgatttg gggaaaggga tagggagaga gttgtcagta ccgatgctga 120
   gactgtgaga gccatttctg agtggaggtg aggaaaaacg gagcgatgac cgtgagggtc 180
   tttgcgtggc gcctcgagac cacacctggg aacggtgact ttggaactgg ctgacgtgtt 240
   tatttggtgt gaggtcaact acagctctga gggcaataac atgatcctca taaaacagac 300
   cccccaggct gggcctcagc ccgcttgctg ccaagcaggg gtggtggcct aataagtgag 360
   acattgctat caactctctg taaatggtgg gttcattcac tctgtggatg acatagcctg 420
   ttctttgccc ttctgtgtca gccccattga ttgcttttta accaccttct tgctttgtgc 480
   tgcttctttc agatgaaaag gaagggaaaa gagacttcat ttccataccc actaaccatg 540
   ccaacagaag gctttgtgaa gaaggaagta aaagctatcg gtaggagtta gaaagaaaca 600
   tttctagtta caagaacagc agaaaaagac ctgaacttca gagtggacca caaatgaata 660
   tgaattaata acacagatct cttttcaaaa gccaacacaa aatgaggatg tgttttttgc 720
   aaaagcagtt gttcttgcca ctgggcttat gc 752
<210> 247
   <211> 505
   <212> DNA
   <213> unknowns
<220>
   <223> >sr|IMAGp998J134174.2|cl|IMAGp998J134174|gb|BX1068 51|oid|234548083 BX106651 Soares_testis_NHT Homo sapiens cDNA clone IMAGp998J134174 ; IMAGE:1645164, mRNA sequence.
<221> misc_feature
   <222> 9
   <223> n=a, t, c, or g
<400> 247
   accaccagna atgtgccttt tccttatcaa aaagaattgc tgccccttac aggacaggag 60
   gactccaatt gtgaccaagt tggagcagcc ttgagccaca actcagactt attttcattc 120
   tctgctcatt ggccattagg aacccgatcc ttatgacctg cagtgacctg ctgctaccag 180
   aactgactcg cttcaaagtg acggatgaaa cccatatgga gatattcctg gggtgggtag 240
   gccagttgga agacagagtg tttacacctg agagaggact tgagaatttt actcagtcac 300
   gttgctcact gctgcgcact cctgcagacg catcccagtg gactctgcgc tgcatcccca 360
   gagtacagcg agtttcccgc ctcccgttag agatccaaca catatttttt ttgaaagaag 420
   aaactgatgg aagacaggaa gcaaaaagga gaggaaggaa agggatagga aggaggggaa 480
   ggaaagggaa aggaaggaaa agaaa                                        505
<210> 248
   <211> 327
   <212> DNA
   <213> unknown
<220>
   <223> >gi|711665|sr|IMAGp998D04101|cl|IMAGp998D04101|gb| T83377|T83377 ye03h02.s1 Homo sapiens cDNA clone 116691 3'.
<221> misc_feature
   <222> 72, 238, 278, 294, 320
   <223> n=a, t, c, or g
<400> 248
   ttttaacata aaacagggct atttattttt attattaaac atgagtactt ctgaaatatg 60
   acatttcatg gnacataaaa tgatgtgtat gacaaaatct gttgctaact ccaaatatta 120
   acttaatata gcttagaaaa atgacataat atactctgca tttttctgac cattctattt 180
   ttacaagtac tccagttata tttagcattc aatttaaaag acaagtgcac tgaaggantt 240
   tgaggttata atataattaa cccaaatatt atataacnat acacacttaa atgnctggtt 300
   gtggtttatt ggatttggcn gggtatc                                     327
<210> 249
   <211> 327
   <212> DNA
   <213> unknown
<220>
   <223> >gi|711665|sr|IMAGp998D04101|cl|IMAGp998D04101|gb| T83377|T83377 ye03h02.s1 Homo sapiens cDNA clone 116691 3'.
<221> misc_feature
   <222> 72, 238, 278, 294, 320
   <223> n=a, t, c, or g
<400> 249
   ttttaacata aaacagggct atttattttt attattaaac atgagtactt ctgaaatatg 60
   acatttcatg gnacataaaa tgatgtgtat gacaaaatct gttgctaact ccaaatatta 120
   acttaatata gcttagaaaa atgacataat atactctgca tttttctgac cattctattt 180
   ttacaagtac tccagttata tttagcattc aatttaaaag acaagtgcac tgaaggantt 240
   tgaggttata atataattaa cccaaatatt atataacnat acacacttaa atgnctggtt 300
   gtggtttatt ggatttggcn gggtatc                                     327
<210> 250
   <211> 334
   <212> DNA
   <213> unknown
<220>
   <223> >sr|IMAGp99800470.2|cl|IMAGp99800970|gb|BX10121410 id|234536804 BX101214 Soares infant brain 1NIB Homo sapiens cDNA clone IMAGp99800470 ; Image:22040, mRNA sequence.
<221> misc_feature
   <222> 168
   <223> n=a, t, c, or g
<400> 250
   tttttttttt ttttaaaggt tagagaatcc aagtttatta gaaacactcc aacaaaaaac 60
   aaaggtgaga agagagggcc cagcccacct ccactcctcc ctttcctcca gaacagaata 120
   ccagtttgta tccggcaaac tggctccttc cccagggttg gtcccagngg ggatttacat 180
   ggcactgcca aagcaggacg ggagccctag tcctcagggc actgcaggat gtcataggtc 240
   acgtagccca cttggtccac ctggttcaac tcactccggg aactcacgcg ccagtagaag 300
   cggtcctggc agaaataggc tttctctcgg tact                              334
<210> 251
   <211> 474
   <212> DNA
   <213> unknown
<220>
   <223> >sr|IMAGp998A125385.2|cl|IMAGp998A125385|gb|BX1135 74|oid|234561525 BX113574 NCI_CGAP_Ut2 Homo sapiens cDNA clone IMAGp998A125385 ; IMAGE:2179475, mRNA sequence.
<400> 251
   tttttttttt ttttcttgtg caaatgcttt actgagggag tactttctgg agaaacctgt 60
   gagggaacca ggaaaactgg ataggccaag gggtcagacc aagcagagag gtggttccag 120
   gagaggcctg gcctcagccc gatcccagga acaatttgag agtgtcattc aacccagaca 180
   atcctgcaca gacacggggg agctggcaat tgtaccctcc atggtgtcac tctcctggcc 240
   atgagtgaca ggtggcctct tggtgggaga ggtggggcag ggggaaggca gctcccacta 300
   gaccagggca gtatttaggg gaaggaggca gattggagca gttggctgcc aacacctggc 360
   ctcactcagg gagaggggcc ttgggcctgg atgatatctg ggtgaggtac ccacaccatc 420
   tgctgcaacc tctgtgtatc cattatgggc tcacgccatt cacaggcatg aatt        474
<210> 252
   <211> 406
   <212> DNA
   <213> unknown
<220>
   <223> >sr|IMAGp998D194160.2|cl|IMAGp998D194160|gb|BX0988 70|oid|234532114 BX098870 Soares_testis_NHT Homo sapiens cDNA clone IMAGp998D194160 ; IMAGE:1639650, mRNA sequence.
<221> misc_feature
   <222> (0)...(0)
   <223> n=a, t, c, or g
<400> 252
   caggttgtag tgacctagcc tgcaaaaatg agctccagna cacccctgag caaggttaaa 60
   agaaaaaaaa caaattcctt tactgtctct cctgtactga actattagtt atgactatgt 120
   ttgccaatgc ttatatttag taaaatttat aaattcctgt ttttctttca acgtagctgg 180
   aaggtcacta gctatacaag gccacaagtt atgccaagtc aacagttatg ccataaatta 240
   catgacttgt aatttatgta attaccgcct ttgttttgct cttgtatact agcctatata 300
   agctaaactc tgtctttgtt tggggctcag gtttttaaat gtgaatccac tgagccagtg 360
   cgtaccttaa aataaatatc ctcctatatt caaaaaaaaa aaaaaa 406
<210> 253
   <211> 486
   <212> DNA
   <213> unknown
<220>
   <223> >gi|2191401|gb|AA465239|sr|IMAGp998L132010.1|cl|IM AGp998L132010|AA465234 aa24g07.s1 NCI_CGAP_GCB1 Homo sapiens cDNA clone IMAGE:814236 3', mRNA sequence.
<400> 253
   acattttcaa tcattttaat gaaaagggcg acacagccat ggtgtgttca gatactcagc 60
   atgacaccgc ctgcaagctc tattgtcttt cgctgattaa actgggctgg ttgagaatta 120
   catttctgca cccatggttc cgctgtggtg aacaccggga gtaagcaagc tcttattcag 180
   gtcccaagca agccacagcc agcatgtcag aaagcaagat tcaagtcagg gtccccttgg 240
   cttcgactgg ctctactgga tcaaacagct ccagcgggga cagaggcagc aggaaagagc 300
   ttcctcaata aggaaattga agagcaggag gaaggaaaaa ataacctgaa actcagatgg 360
   gcaagattag ggtggggagg tgagagccag agatcatgtg tcagcaccca gagcggagga 420
   gaaagaattc tgattccagt ccacaccacc ctagactgca gcattgagca ccccagggct 480
   ggcgtt 486
<210> 254
   <211> 591
   <212> DNA
   <213> unknown
<220>
   <223> >gi|2186490|gb|AA461370|sr|IMAGp998G081965.1|cl|IM AGp998G081965|AA461370 zx70f04.r1 Soares_total_Fetus_Nb2HF8_9w Homo sapiens cDNA clone IMAGE:796831 5' similar to SW:ECE1_BOVIN P42891 ENDOTHELIN-CONVERTING ENZYME 1 ;, mRNA sequence.
<400> 254
   aaatattgct gataatggag gcctgcggga agcttttagg gcttacagga aatggataaa 60
   tgacagaagg cagggacttg aggagcctct tctaccaggc atcacattca ccaacaacca 120
   gctcttcttc ctgagttatg ctcatgtgag gtgcaattcc tacagaccag aagctgcccg 180
   agaacaagtc caaattggtg ctcacagtcc ccctcagttt agggtcaatg gtgcaattag 240
   taactttgaa gaattccaga aagcttttaa ctgtccaccc aattccacga tgaacagagg 300
   catggactcc tgccgactct ggtagctggg acgctggttt atggcatcct gagacagttg 360
   cacagtgcca gcggaggctt gcactgagcc ttcatcgccc attgctttag gcctggagac 420
   tttcattttt agtgcatttt cattatttgg gtaggtgacc tgcttggatc tagacagcat 480
   ctgttcaaag ttgtagggct tataaagtgg aatataagaa tgaactaagt atgtttcttt 540
   agaaaatcaa accaacaaaa ataaatccct aggctacttt tgttaaaatg c          591
<210> 255
   <211> 693
   <212> DNA
   <213> unknown
<220>
   <223> >sr|IMAGp998B072455.2|cl|IMAGp9988072955|gb|BX1183 94|oid|234571164 BX118394 NCI_CGAP_Pr21 Homo sapiens cDNA clone IMAGp990B07245 ; IMAGE:984870, mRNA sequence.
<400> 255
   tgaaattccc ggcgctcacg cactactggc ccctgatccg gttcttggtg cccctgggca 60
   tcaccaacat agccctcgac ttcggggagc agccatctcc tttttatatt tacaagaggt 120
   cacaagagga cagttaaaac tgatgacgcc taaggacaga tcacacgtaa tagaagagct 180
   gccgcagccg gttcccatta gaagggcctc caagaacaac acacacagga actcgcctgc 240
   tcttctcctg atggtccaca gtcacccctg ccctttgagc aggaggagcg tgacctcctg 300
   ccacagctac agccacaatt attgaaattg tggtcaacat tccatttaaa gcattaaagt 360
   ctgttgtttt agtcctgact aagcagtagg cttctatgga ttgggtattt tttttttttt 420
   acccacccaa atgtgtttta tgttgagatg tttcgagttt ttttgggggg ttgagggtgg 480
   tgcaggtcgt gaggggcaag agctgctgca taggagataa ttctctttaa ctgattaatg 540
   tttttgcttt attcaacagc tattgaatta ggcagtctgt acctttagtg ttttccagaa 600
   caaatgtgtt gaagtggcag aagggtggaa gaattgaaaa ttattcttgc acagttgcac 660
   ctgagttgta ttattgctcc atgtctcctt atg                              693